# FASCICULE DE BREVET EUROPEEN

(11) **EP 1 003 888 B1**
(45) Date de publication et mention de la délivrance du brevet: **30.04.2008**
(21) Numéro de dépôt: 98942748.9
(22) Date de dépôt: 14.08.1998
(51) Int. Cl.: C12N 15/74, C12N 15/31, C07K 14/35, A61K 48/00, A61K 39/04, C07K 19/00, C12Q 1/68, C07K 16/12, G01N 33/50, G01N 33/53, C12N 15/52, C12N 15/65

(54) **SEQUENCES NUCLEIQUES DE POLYPEPTIDES EXPORTES DE MYCOBACTERIES, VECTEURS LES COMPRENANT ET APPLICATIONS AU DIAGNOSTIC ET A LA PREVENTION DE LA TUBERCULOSE**
NUKLEOTIDSEQUENZEN, DIE FÜR SEKRETIERTE POLYPEPTIDE AUS MYCOBACTERIUM KODIEREN, VEKTOREN, DIE DIESE ENTHALTEN, UND ANWENDUNGEN ZUR DIAGNOSTIK UND VERHÜTUNG VON TUBERKULOSE
POLYPEPTIDE NUCLEIC SEQUENCES EXPORTED FROM MYCOBACTERIA, VECTORS COMPRISING SAME AND USES FOR DIAGNOSING AND PREVENTING TUBERCULOSIS

(30) Priorité: 14.08.1997 FR 9710404; 11.09.1997 FR 9711325
(43) Date de publication de la demande: 31.05.2000
(73) Titulaire: INSTITUT PASTEUR, 75015 Paris (FR)
(72) Inventeur: GICQUEL, Brigitte, F-75014 Paris (FR); PORTNOI , Denis, F-75004 Paris (FR); LIM, Eng-Mong, F-75015 Paris (FR); PELICIC, Vladimir, F-75009 Paris (FR); GUIGUENO, Agnès, F-62026 Arras (FR); GOGUET DE LA SALMONIERE, Yves, F-75015 Paris (FR)
(74) Mandataire: Warcoin, Jacques
(86) Numéro de dépôt international: PCT/FR1998/001813
(87) Numéro de publication internationale: WO 1999/009186

(56) Documents cités:
- WO-A-95/06726
- WO-A-96/07745
- WO-A-96/26275
- DATABASE EMBL [en ligne] 15 mai 1997 (1997-05-15) HAMLIN N., CHURCHER C.M: "Mycobacterium leprae cosmid L622" Database accession no. MLCL622 XP002282088
- ENG MONG LIM ET AL.: "Identification of Mycobacterium tuberculosis DNA sequences encoding exported proteins by using phoA gene fusions" JOURNAL OF BACTERIOLOGY, vol. 177, no. 1, 1 janvier 1995 (1995-01-01), pages 59-65, XP000560419
- RONALD ET AL: "Construction of broad-host-range vectors for the selection of divergent promoters" GENE, vol. 90, 1990, pages 145-148,

## Description

L'invention a pour objet de nouveaux vecteurs recombinants de criblage, de clonage et/ou d'expression se répliquant chez les mycobactéries. Elle a également pour objet un procédé de criblage de séquences codant pour des polypeptides exportés et/ou sécrétés induits ou réprimés lors de l'ingestion des mycobactéries par les macrophages mettant en oeuvre un secteur de l'invention. L'invention concerne enfin des mycobactéries recombinantes transformées par un secteur selon l'invention.

Le genre Mycobacterium, qui comprend au moins 56 espèces différentes, inclut des pathogènes humains majeurs tels *que M. leprae* et *M. tuberculosis,* les agents responsables de la lèpre et de la tuberculose, qui restent des problèmes graves de santé publique dans le monde entier.

La tuberculose continue d'être un problème de santé publique dans le monde. Aujourd'hui, cette maladie est la cause de 2 à 3 millions de morts dans le monde et environ 8 millions de nouveaux cas sont observés chaque année (Bouvet, 1994). Dans les pays développés *M. tuberculosis* est la cause la plus commune des infections mycobactériennes. En France il apparaît environ 10 000 nouveaux cas par an et parmi les maladies à déclaration obligatoire c'est la tuberculose qui comprend le plus grand nombre de cas. La vaccination par le BCG (Bacille de Calmette et Guérin), une souche avirulente dérivée de *M*. *bovis* et qui est très utilisée comme vaccin contre la tuberculose, est loin d'être efficace au sein de toutes les populations. Cette efficacité varie environ de 80 % dans les pays occidentaux comme l'Angleterre, à 0 % en Inde (résultats du dernier essai de vaccination à Chingleput., publiés en 1972 dans Indian J. Med. Res.). De plus, l'apparition de souches de *M. tuberculosis* résistantes aux antituberculeux et le risque accru chez les patients immunodéprimés, patients atteints du SIDA, de développer une tuberculose, rendent nécessaire la mise au point de méthodes rapides, spécifiques et fiables pour le diagnostic de la tuberculose et la mise au point de nouveaux vaccins. Par exemple, une étude épidémiologique réalisée en Floride, et dont les résultats ont été publiés en 1993 dans AIDS thérapies, a montré que 10 % des malades atteints de SIDA sont atteints de tuberculose au moment du diagnostic du SIDA ou 18 mois avant celui-ci. Chez ces malades, la tuberculose apparaît dans 60 % des cas sous une forme disséminée donc non repérable par les critères de diagnostic classiques comme la radiographie pulmonaire ou l'analyse de crachats.

Actuellement, une certitude sur le diagnostic apporté par la mise en évidence de bacilles cultivables dans un prélèvement provenant du malade n'est obtenue que pour moins de la moitié des cas de tuberculose, même dans les cas de tuberculose pulmonaire. Le diagnostic de la tuberculose et des autres mycobactéries apparentées est donc difficile à réaliser, et cela pour différentes raisons : les mycobactéries sont souvent présentes en faible quantité, leur temps de génération est très long (24h pour *M. tuberculosis*) et leur culture est difficile. (Bates et al., 1986).

D'autres techniques sont utilisables en clinique, pour identifier une infection mycobactérienne :
a) L'identification directe des microorganismes au microscope ; cette technique est rapide, mais ne permet pas l'identification de l'espèce mycobactérienne observée et manque de sensibilité (Bates, 1979).
   Les cultures, lorsqu'elles sont positives, ont une spécificité approchant 100 % et permettent l'identification de l'espèce mycobactérienne isolée ; néanmoins, comme précisé ci-dessus, la croissance des mycobactéries in vitro est longue (ne peut être réalisée qu'en 3 à 6 semaines de cultures répétées (Bates, 1979 ; Bates et al., 1986)) et coûteuse.
b) Les techniques sérologiques peuvent s'avérer utiles dans certaines conditions, mais leur utilisation est parfois limitée par leur sensibilité et/ou leur spécificité faibles (Daniel et al., 1987).
c) La présence de mycobactéries au sein d'un échantillon biologique peut aussi être déterminée par hybridation moléculaire avec de l'ADN ou de l'ARN en utilisant des sondes d'oligonucléotides spécifiques des séquences recherchées (Kiehn et al., 1987 ; Roberts et al., 1987 ; Drake et al., 1987). Plusieurs études ont montré l'intérêt de cette technique pour le diagnostic des infections à mycobactéries. Les sondes utilisées sont constituées d'ADN, d'ARN ribosomique ou de fragments d'ADN mycobactériens provenant de banque de gènes. Le principe de ces techniques repose sur le polymorphisme des séquences nucléotidiques des fragments utilisés ou sur le polymorphisme des régions avoisinantes. Dans tous les cas, elles nécessitent l'utilisation de cultures et ne sont pas applicables directement sur les échantillons biologiques.

La faible quantité de mycobactéries présentes au sein d'un échantillon biologique et en conséquence la quantité faible d'ADN cible à détecter dans cet échantillon peut nécessiter le recours à une amplification spécifique in vitro de l'ADN cible avant sa détection à l'aide de la sonde nucléotidique et en utilisant des techniques d'amplification *in vitro* telles que la PCR (amplification en chaîne à la polymérase. L'amplification spécifique de l'ADN par la technique PCR peut constituer la première étape d'un procédé de détection de la présence d'un ADN mycobactérien dans un échantillon biologique, la détection proprement dite de l'ADN amplifié étant effectuée dans un second temps à l'aide d'une sonde oligonucléotidique capable de s'hybrider spécifiquement à l'ADN amplifié.
Un test de détection de mycobactéries appartenant au complexe de *Mycobacterium tuberculosis,* par hybridation sandwich (test utilisant une sonde de capture et une sonde de détection) a été décrit par Chevrier et al. en 1993. le complexe de *Mycobacterium tuberculosis* est un groupe de mycobactéries qui comprend *M. bovis-BCG, M. bovis, M. tuberculosis, M. africanum* et *M. microti*.

Un procédé de détection de faibles quantités de mycobactéries, appartenant au complexe tuberculosis, par amplification génique et hybridation directement sur des échantillons biologiques a été mis au point. Ledit procédé utilise la séquence d'insertion IS*6110* (Brevet européen EP 0 490 951 B1). Thierry et al. ont décrit en 1990 une séquence spécifique du complexe *Mycobacterium* tuberculosis et nommée IS 6110. Certains auteurs ont proposé d 'amplifier spécifiquement l'ADN provenant de *Mycobacterium* en utilisant des amorces nucléiques dans une méthode d'amplification, telle que la réaction de polymérase en chaîne (PCR). Patel et al. ont décrit en 1990 l'utilisation de plusieurs amorces nucléiques choisies à partir d'une séquence connue en tant que sonde dans l'identification de *M. tuberculosis.* Cependant, la longueur des fragments obtenus en utilisant ces amorces était différente de la longueur théorique attendue et plusieurs fragments de taille variable étaient obtenus. De plus, les auteurs ont observé l'absence d'hybridation des produits amplifiés avec le plasmide ayant servi à déterminer les amorces. Ces résultats indiquent que ces amorces ne seraient pas appropriées dans la détection de la présence de *M. tuberculosis* dans un échantillon biologique et confirment la nature critique du choix des amorces. La même année, J.L. Guesdon et D. Thierry ont décrit une méthode de détection de *M. tuberculosis,* de grande sensibilité, par amplification d'un fragment d'ADN de *M. tuberculosis* localisé au sein de la séquence IS6110 (Brevet européen EP 461 045) à l'aide d'amorces générant des fragments d'ADN amplifié de longueur constante, même lorsque le choix des amorces conduisait à l'amplification de fragments longs (de l'ordre de 1000 à 1500 bases) où le risque d'interruption de la polymérisation est élevée en raison des effets de la structure secondaire de la séquence. D'autres amorces spécifiques de la séquence IS6110 sont décrites dans le brevet européen N° EP-0490 951.

Les inventeurs ont montré (résultats non publiés) que certains isolats cliniques de *Mycobacterium tuberculosis* étaient exempts de la séquence d'insertion IS6110 et ne pouvaient donc être détectés à l'aide des oligonucléotides spécifiques de cette séquence pouvant conduire ainsi à des résultats de diagnostic faussement négatifs. Ces résultats confirment une observation similaire faite par Yuen et al. en 1993. L'impossibilité de détecter ces souches pathogènes potentiellement présentes dans un échantillon biologique prélevé sur un patient est ainsi susceptible de conduire à des difficultés voire des erreurs de diagnostic. La disponibilité de plusieurs séquences spécifiques du Bacille de la tuberculose, à l'intérieur desquelles des amorces appropriées pour l'amplification seront choisis, est importante. La séquence DP428 décrite ici pourra être utilisée.

*M. bovis et M. tuberculosis*, les agents causals de la tuberculose, sont des bactéries facultatives intracellulaires.

Ces agents ont développé des mécanismes pour assurer leur survie et leur réplication à l'intérieur du macrophage, un des types cellulaires qui est supposé éradiquer l'invasion par des microorganismes. Ces agents sont capables de moduler l'évolution normale de leur phagosome et de les empêcher de se différencier en un compartiment acide riche en hydrolase (Clemens, 1979 ; Clemens et al., 1996; Sturgill-Koszycki et al., 1994 et Xu et al., 1994). Cependant, cette modulation n'est possible que si la bactérie est vivante au sein du phagosome, suggérant que des composés synthétisés de manière active et/ou sécrétés à l'intérieur de la cellule font partie de ce mécanisme. Des protéines exportées sont probablement impliquées dans ce mécanisme. En dépit des problèmes majeurs de santé liés à ces organismes pathogènes, on sait peu de choses sur leurs protéines exportées et/ou sécrétées. Des analyses en SDS-PAGE de filtrat de culture de *M. tuberculosis* montrent au moins 30 protéines sécrétées (Altschul et al., 1990 ; Nagal et al., 1991 et Young et al., 1992). Certaines d'entre elles ont été caractérisées, leurs gènes clonès et séquencés (Borremans et al., 1989 ; Wiker et al., 1992 et Yamaguchi et al., 1989). D'autres, bien qu'il s'agisse d'antigènes immunodominants d'importance majeure pour induire une immunité protectrice (Anderson et al.,1991 et Orme et al., 1993), ne sont pas totalement identifiés. En outre, il est probable que de nombreuses protéines exportées restent fixées sur la membrane cellulaire et par conséquent ne soient pas présentes dans les surnageants de culture. Il a été montré que les protéines localisées à la surface externe de diverses bactéries pathogènes, telles que l'invasine de 103 kDa de *Yersina* Pseudotuberculosis (Isberg et al., 1987) ou l'internaline de 80 kDa de *Listeria monocytoqenes* (Gaillard et al., 1991 et Dramsi et al., 1997) jouent un rôle important dans les interactions avec les cellules hôtes et par conséquent, dans la pathogénicité comme dans l'induction de réponses protectrices. Ainsi, une protéine liée à la membrane pourrait être importante pour l'infection à *M. tuberculosis* comme pour l'induction de réponse protectrice contre cette infection. Ces protéines pourraient revêtir un intérêt certain pour la préparation de vaccins.

Récemment, il a été décrit l'adaptation aux mycobactéries d'une méthodologie génétique pour l'identification et la sélection phénotypique de protéines exportées (Lim et al., 1995). Cette méthode utilise la phosphatase alkaline (PhoA) périplasmique d'*E. coli*. Un vecteur plasmidique a été construit permettant la fusion de gènes entre un gène PhoA tronqué et des gènes codant pour des protéines exportées (Manoil et al., 1990).

Par cette méthode, il a pu être identifié un gène de *M. tuberculosis* (erp (Berthet et al., 1995)) présentant des homologies avec une protéine exportée de 28 kDa de *M. leprae,* qui est une cible fréquente des réponses humorales de la forme lépromateuse de la lèpre. Une protéine présentant des motifs aminoacides caractéristiques de la désaturase de plante (des) a aussi été caractérisée par la technique de fusion avec PhoA.

Cependant, cette méthode génétique d'identification de protéines exportées ne permet pas d'évaluer facilement l'expression intracellulaire des gènes correspondants. Une telle évaluation est d'une importance primordiale à la fois pour la sélection de bons candidats vaccins et pour la compréhension des interactions entre les bactéries et leurs cellules hôtes. L'induction de l'expression de facteur de virulence par contact de cellule cible pathogène a été décrite. C'est le cas par exemple pour les facteurs de virulence Yops (Petersson et al., 1996) de *Yersinia pseudotuberculosis.* Shigella par contact avec les cellules cibles relargue les protéines Ipa dans le milieu de culture, et Salmonella synthétise de nouvelles structures de surface.

On peut également, citer le document Ronal et al. (Gene, 90 (1990) 145-148) qui décrit la construction de secteurs plasmidiques comprenant la combinaison de deux séquences de gènes sans promoteur codant pour des enzymes et leur utilisation pour la détection d'activité de promoteur.

Compte tenu de ce qui précède, il existe aujourd'hui un grand besoin de développer de nouveaux vaccins contre les mycobactéries pathogènes ainsi que de nouveaux tests de diagnostic spécifiques, fiables et rapides. Ces développements nécessitent la mise au point d'outils spécifiques encore plus performants permettant, d'une part, d'isoler ou d'obtenir des séquences de nouveaux polypeptides spécifiques, notamment immunogènes, et, d'autre part, de mieux comprendre le mécanisme des interactions entre les bactéries et leurs cellules hôtes comme notamment l'induction de l'expression de facteur de virulence . Ceci est précisément l'objet de la présente invention.

Les inventeurs ont défini et réalisé dans ce but de nouveaux vecteurs permettant le criblage, le clonage et/ou l'expression de séquences d'ADN de mycobactéries afin d'identifier parmi ces séquences, des acides nucléiques codant pour des protéines d'intérêt, de préférence des protéines exportées, pouvant être localisées sur la membrane bactérienne et/ou sécrétées, et d'identifier parmi ces séquences celles qui sont induites ou réprimées lors de l'infection (croissance intracellulaire).

### Description

La présente invention décrit l'utilisation du gène rapporteur *phoA* chez les mycobactéries. Il permet d'identifier des systèmes d'expression et d'exportation dans un contexte mycobactérien. Beaucoup de gènes ne sont exprimés que dans un tel contexte, ce qui montre l'avantage de la présente invention. Au cours du clonage de segments d'ADN de souches du complexe M. *Tuberculosis* en fusion avec phoA dans une autre mycobactérie comme *M. smegmatis,* le début du gène, ses régions régulatrices et son régulateur seront clonés ce qui permettra d'observer une régulation. Si cette régulation est positive, le clonage du régulateur constituera un avantage pour observer l'expression et l'exportation.

Dans le contexte de l'invention, on entend par mycobactérie toutes les mycobactéries appartenant aux diverses espèces énumérées par Wayne L. G. and Kubica G. P. (1980). Family Mycobacteriaceae in Bergey's manual of systematic bacteriology, J. P. Butler Ed. (Baltimore USA : Williams et Wilkins P. 1436-1457).

Dans certains cas les gènes clonés sont soumis dans leur hôte d'origine à une régulation négative rendant l'observation de l'expression et de l'exportation difficile chez l'hôte d'origine. Dans ce cas, le clonage du gène en absence de son régulateur négatif, dans un hôte ne le contenant pas, constituera un avantage.

L'invention décrit aussi de nouveaux polypeptides et de nouveaux polynucléotides de mycobactéries ayant pu être isolés au moyen des vecteurs précédents et susceptibles d'entrer dans la réalisation de compositions pour la détection d'une infection par des mycobactéries, ou pour la protection contre une infection due à des mycobatéries ou pour la recherche d'inhibiteurs comme cela est décrit précédemment pour DP428.

L'invention a donc pour objet un vecteur recombinant de criblage, de clonage et/ou d'expression, caractérisé en ce qu'il se réplique chez des mycobactéries et en ce qu'il contient :
1) un réplicon fonctionnel chez les mycobactéries ;
2) un marqueur de sélection ;
3) une cassette rapporteur comprenant :
   a) un site de clonage multiple (polylinker),
   b) éventuellement un terminateur de transcription actif chez les mycobactéries, en amont du polylinker,
   c) une première séquence nucléotidique codant pour un marqueur d'exportation et/ou de sécrétion de protéine, ladite séquence nucléotidique étant dépourvue de son codon d'initiation et de ses séquences de régulation, et
   d) en aval, une seconde séquence nucléotidique codant pour un marqueur d'activité de promoteurs contenus dans le fragment cloné, ladite séquence nucléotidique étant pourvue de son codon d'initiation. Eventuellement, le vecteur recombinant contient également un réplicon fonctionnel chez E. coli.

De manière préférée, le marqueur d'exportation et/ou de sécrétion est placé dans la même orientation que le marqueur d'activité de promoteurs.

Préférentiellement, le vecteur recombinant de criblage selon l'invention comprendra, en outre, un terminateur de transcription placé en aval du marqueur d'activité de promoteurs, ce qui est de nature à permettre l'obtention de transcrits courts qui se révèlent plus stables et qui, par conséquent, permettent un plus haut niveau d'expression des produits de traduction.

Le marqueur d'exportation et/ou de sécrétion est une séquence de nucléotides dont l'expression suivie de l'exportation et/ou de la secrétion dépend des éléments de régulation qui contrôlent son expression.

Par "séquences ou éléments de régulation de l'expression de la production de polypeptides et de sa localisation", on entend une séquence promotrice de la transcription, une séquence comprenant le site de liaison au ribosome (RBS), les séquences responsables de l'exportation et/ou la sécrétion telles que la séquence dite séquence signal.

Un premier marqueur intéressant d'exportation et/ou de sécrétion est une séquence codante issue du géne *phoA.* Le cas échéant, elle est tronquée de telle façon que l'activité phosphatase alcaline est cependant susceptible d'être restaurée lorsque la séquence codante tronquée est placée sous le contrôle d'un promoteur et d'éléments de régulation appropriés.

D'autres marqueurs d'exportation et/ou de sécrétion peuvent être utilisés. On citera à titre d'exemples une séquence du gène β-agarase, de la nucléase d'un staphylocoque ou d'une β-lactamase.

Parmi les marqueurs intéressants d'activité de promoteurs contenus dans le fragment cloné, on préfère une séquence codante issue du géne *luc* de luciférase de luciole pourvue de son codon d'initiation.

D'autres marqueurs d'activité de promoteurs contenus dans le fragment cloné peuvent être utilisés. On citera à titre d'exemples une séquence du gène de la GFP (Green Fluorescent Protein).

Le terminateur de transcription doit être fonctionnel chez les mycobactéries. Un terminateur avantageux est à cet égard le terminateur du coliphage T4 (tT4). D'autres terminateurs appropriés pour la réalisation de l'invention peuvent être isolés en utilisant la technique présentée dans les exemples, par exemple au moyen d'une cassette "omega" (Prentki et al., 1984).

Un vecteur particulièrement préféré pour la réalisation de l'invention est un plasmide choisi parmi les plasmides suivants déposés à la CNCM (Collection Nationale de Cultures de Microorganismes, 25 rue de Docteur Roux, 75724 Paris cedex 15, France) :
a) pJVEDa déposé à la CNCM sous le N° I-1797, le 12/12 1996,
b) pJVEDb déposé à la CNCM sous le N° I-1906, le 25 juillet 1997,
c) pJVEDc déposé à la CNCM sous le N° 1-1799 , le 12/12 1996.

Pour la sélection, ou l'identification de séquences d'acides nucléiques de mycobactéries codant pour des polypeptides susceptibles d'être incorporés dans des compositions immunogènes, ou antigéniques pour la détection d'une infection, ou susceptibles d'induire ou de réprimer un facteur de virulence de mycobactéries, le vecteur de l'invention comprendra, en l'un des sites de clonage multiple du polylinker, une séquence de nucléotides d'une mycobactérie chez laquelle on détecte la présence de séquences correspondant à des polypeptides exportés et/ou sécrétés pouvant être induits ou réprimés lors de l'infection, ou encore exprimés ou produits de façon constitutive, leurs séquences promotrices et/ou régulatrices associées susceptibles de permettre ou de favoriser l'exportation et/ou la sécrétion desdits polypeptides d'intérêt, ou tout ou partie de gènes d'intérêt codant pour lesdits polypeptides.

De préférence, cette séquence est obtenue par fragmentation physique ou par digestion enzymatique de l'ADN génomique ou de l'ADN complémentaire d'un ARN d'une mycobactérie, de préférence *M. tuberculosis* ou choisie parmi *M. africanum, M. bovis*, *M. avium* ou *M. leprae.*

Les vecteurs de l'invention peuvent en effet également être utilisés pour déterminer la présence de séquences d'intérêt, de préférence correspondant à des protéines exportées et/ou sécrétées, et/ou capables d'être induites ou réprimées ou produites de façon constitutive lors de l'infection, notamment lors de la phagocytose par les macrophages, et selon ce qui a été exposé précédemment, chez des mycobactéries telles que *M. africanum, M. bovis, M. avium* ou *M. leprae* dont on aura traité l'ADN ou l'ADNc par fragmentation physique ou avec des enzymes déterminées.

Selon un premier mode de réalisation de l'invention la digestion enzymatique de l'ADN génomique ou de l'ADN complémentaire est effectuée à partir de *M. tuberculosis.*
De préférence cet ADN est digéré avec une enzyme telle que sau3A, BclI,BglII.

D'autres enzymes de digestion telles que ScaI, ApaI, SacII, KpnI ou encore des nucléases ou des polymérases, peuvent naturellement être mises en oeuvre, dès lors qu'elles permettent l'obtention de fragments dont les extrémités peuvent être insérées dans l'un des sites de clonage du polylinker du vecteur de l'invention.
Le cas échéant, des digestions avec différentes enzymes seront effectuées simultanément.

Des vecteurs recombinants préférés pour la réalisation de l'invention sont choisis parmi les vecteurs recombinants suivants déposés à la CNCM :
a) p6D7 déposé le 28 janvier 1997 à la CNCM sous le N°I-1814,
b) p5A3 déposé le 28 janvier 1997 à la CNCM sous le N°I-1815,
c) p5F6 déposé le 28 janvier 1997 à la CNCM sous le N°I-1816,
d) p2A29 déposé le 28 janvier 1997 à la CNCM sous le N°I-1817,
e) pDP428 déposé le 28 janvier 1997 à la CNCM sous le N°I-1818,
f) p5B5 déposé le 28 janvier 1997 à la CNCM sous le N°I-1819.
g) p1C7 déposé le 28 janvier 1997 à la CNCM sous le N°I-1820,
h) p2D7 déposé le 28 janvier 1997 à la CNCM sous le N°I-1821,
i) plB7 déposé le 31 janvier 1997 à la CNCM sous le N°I-1843,
j) pJVED/M. tuberculosis déposé le 25 juillet 1997 à la CNCM sous le N° I-1907,
k) pMlC25 déposé le 4 août 1998 à la CNCM sous le n°I-2062.

L'invention à également pour objet un procédé de criblage de séquences de nucléotides issues de mycobactéries pour déterminer la présence de séquences correspondant à des polypeptides exportés et/ou sécrétés pouvant être induits ou réprimés lors de l'infection, leurs séquences promotrices et/ou régulatrices associées susceptibles notamment de permettre ou de favoriser l'exportation et/ou la sécrétion desdits polypeptides d'intérêt, ou tout ou partie de gènes d'intérêt codant pour lesdits polypeptides, caractérisé en ce qu'il met en oeuvre un vecteur recombinant selon l'invention.
L'invention concerne aussi un procédé de criblage, selon l'invention, caractérisé en ce qu'il comprend les étapes suivantes :
a) la fragmentation physique des séquences d'ADN de mycobactéries ou leur digestion par au moins une enzyme déterminée et la récupération des fragmentsobtenus ;
b) l'insertion des fragments obtenus à l'étape a) dans un site de clonage, compatible le cas échéant avec l'enzyme de l'étape a), du polylinker d'un vecteur selon l'invention ;
c) si besoin, l'amplification desdits fragments contenus dans le vecteur, par exemple par réplication de ce dernier après insertion du vecteur ainsi modifié dans une cellule déterminée, de préférence *E coli ;*
d) la transformation des cellules hôtes par le vecteur amplifié à l'étape c), ou en l'absence d'amplification, par le vecteur de l'étape b) ;
e) la culture des cellules hôtes transformées dans un milieu permettant la mise en évidence du marqueur d'exportation et/ou de sécrétion, et /ou du marqueur d'activité de promoteurs contenu dans le vecteur ;
f) la détection des cellules hôtes positives (colonies positives) pour l'expression du marqueur d'exportation et/ou de sécrétion, et /ou du marqueur d'activité de promoteurs ;
g) l'isolement de l'ADN des colonies positives et l'insertion de cet ADN dans une cellule identique à celle de l'étape c) ;
h) la sélection des insertions contenues dans le vecteur, permettant l'obtention de clones positifs pour le marqueur d'exportation et/ou de sécrétion, et /ou pour le marqueur d'activité de promoteurs ;
i) l'isolement et la caractérisation des fragments d'ADN de mycobactéries contenues dans ces insérats.

Dans l'un des modes de réalisation préférés du procédé de criblage selon l'invention, les cellules hôtes positives, détectées à l'étape f), pour le marqueur d'exportation et/ou de sécrétion sont, éventuellement dans un second temps, testées pour la capacité de l'insert nucléotidique sélectionné à stimuler l'expression du marqueur d'activité de promoteurs lorsque lesdites cellules hôtes sont phagocytées par des cellules du type macrophagique.

De manière plus spécifique, on compare la stimulation de l'expression du marqueur d'activité de promoteurs chez des cellules hôtes placées en culture axénique (cellules hôtes seules en culture) à la stimulation de l'expression du marqueur d'activité de promoteurs chez des cellules hôtes cultivées en présence de macrophages et ainsi phagocytées par ces derniers.

La sélection de cellules hôtes positives pour le marqueur d'activité de promoteurs peut être réalisée dès l'étape e) du procédé de criblage décrit ci-dessus, ou encore après l'une quelconque des étapes f), g), h) ou i), c'est-à-dire une fois que les cellules hôtes ont été sélectionnées positivement pour le marqueur d'exportation et/ou de sélection.

La mise en oeuvre de ce procédé permet la construction de banques d'ADN comportant des séquences correspondant à des polypeptides susceptibles d'être exportés et/ou sécrétés, et/ou susceptibles d'être induits ou réprimés lors de l'infection lorsqu'ils sont produits au sein de mycobactéries recombinantes. L'étape i) du procédé peut comprendre une étape de séquençage des insertions sélectionnées.

De préférence, dans le procédé selon l'invention, le vecteur utilisé est choisi parmi les plasmides pJVEDa (CNCM, N° I-1797) ,pJVEDb (CNCM, N° I-1906), pJVEDc (CNCM, N° I-1799) ou pJVED/*M*. *tuberculosis* (CNCM, N°I-1907), et la digestion des séquences d'ADN de mycobactéries est effectuée au moyen de l'enzyme Sau3A.

Selon un mode de réalisation préféré de l'invention, le procédé de criblage est caractérisé en ce que les séquences de mycobactéries sont issues d'une mycobactérie pathogène, par exemple de *M. tuberculosis, M. bovis, M. avium, M. africanum* ou *M. leprae.*

Dans la présente invention, on entend désigner par "séquences nucléiques" ou "séquences d'acides aminés" SEQ ID N° X à SEQ ID N° Y, où X et Y peuvent représenter indépendamment un nombre ou un caractère alphanumérique, respectivement l'ensemble des séquences nucléiques ou l'ensemble des séquences d'acides aminés représentées par les figures X à Y, extrémités comprises.

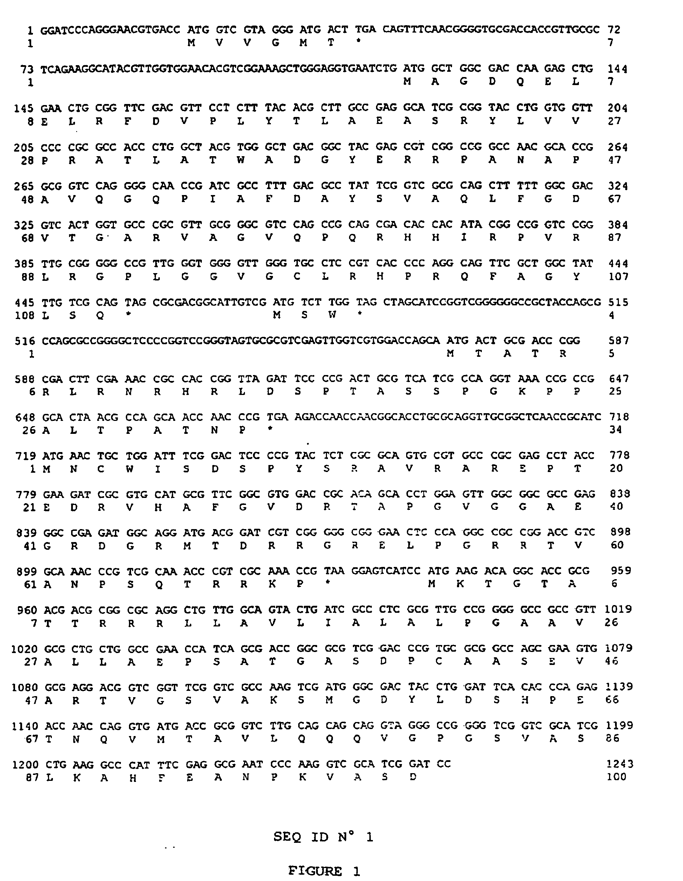

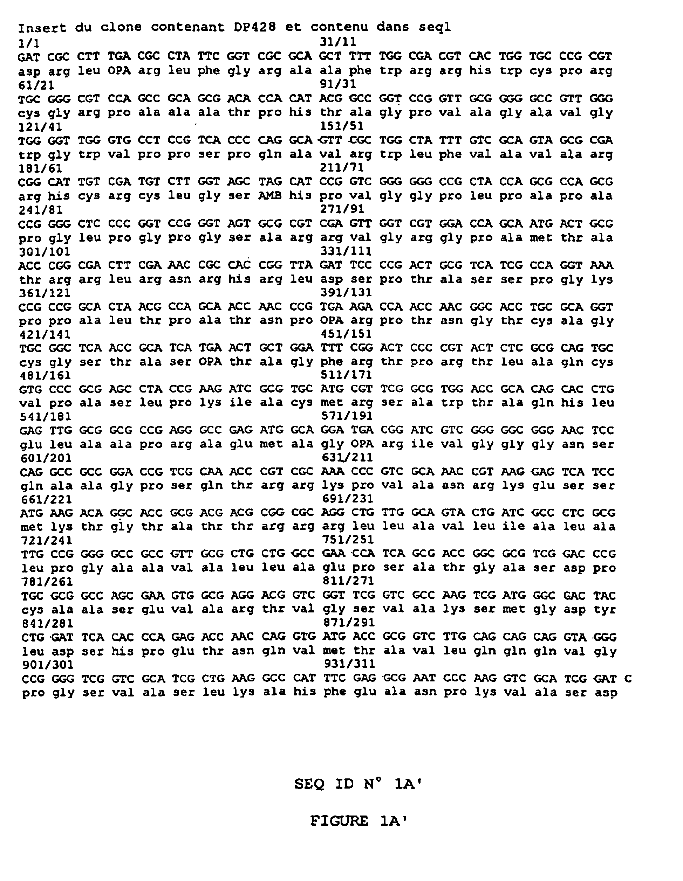

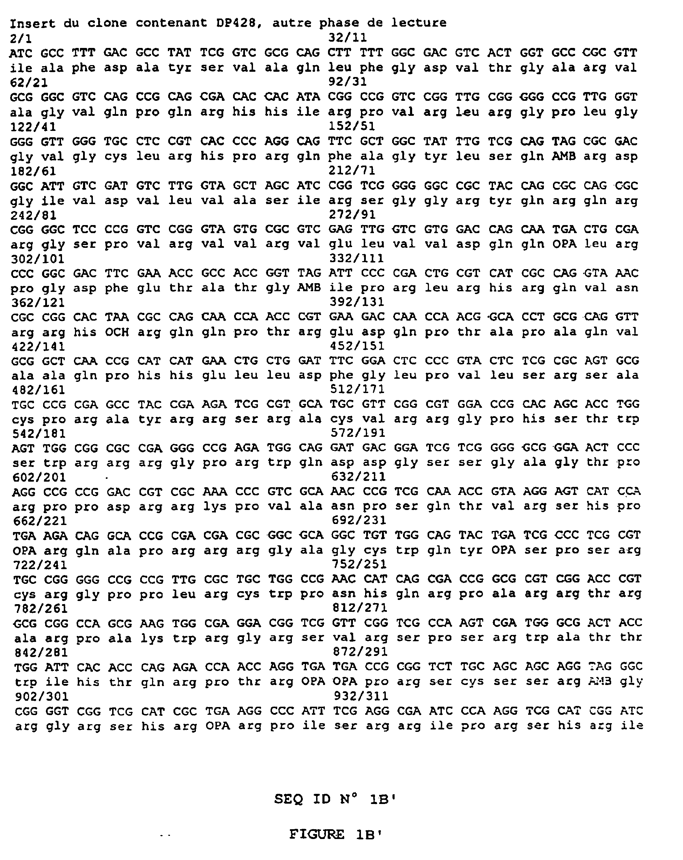

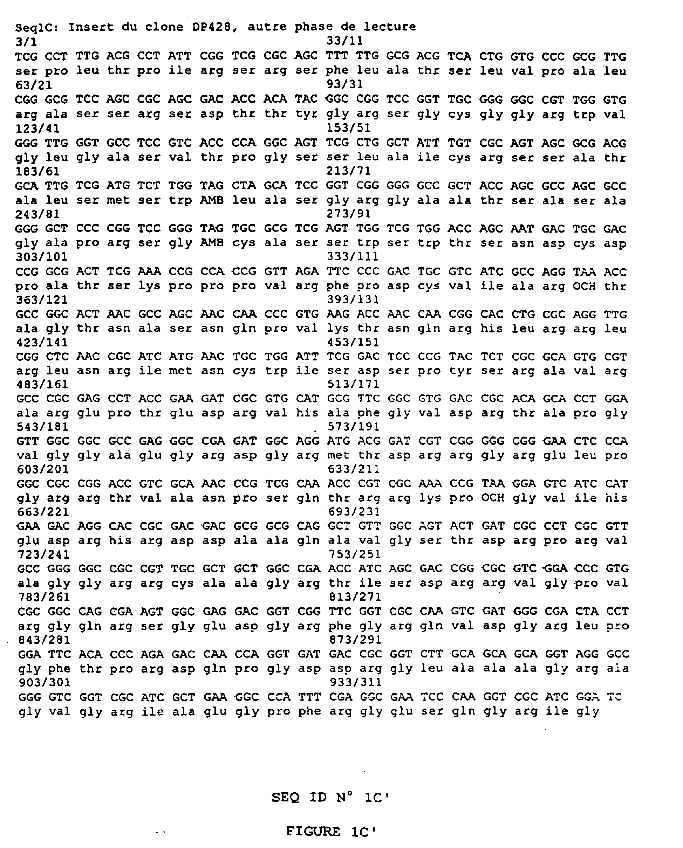

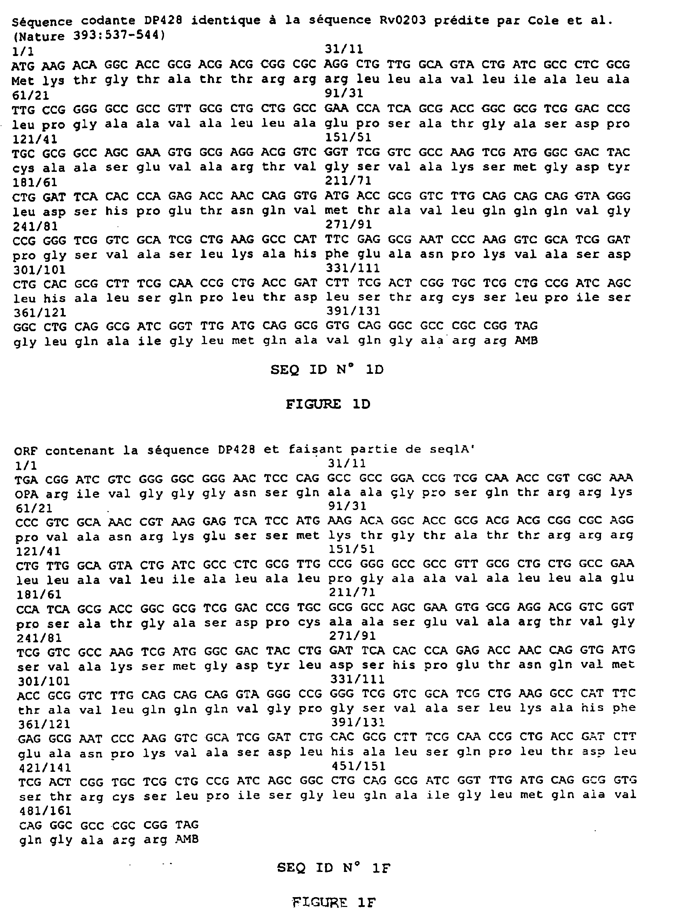

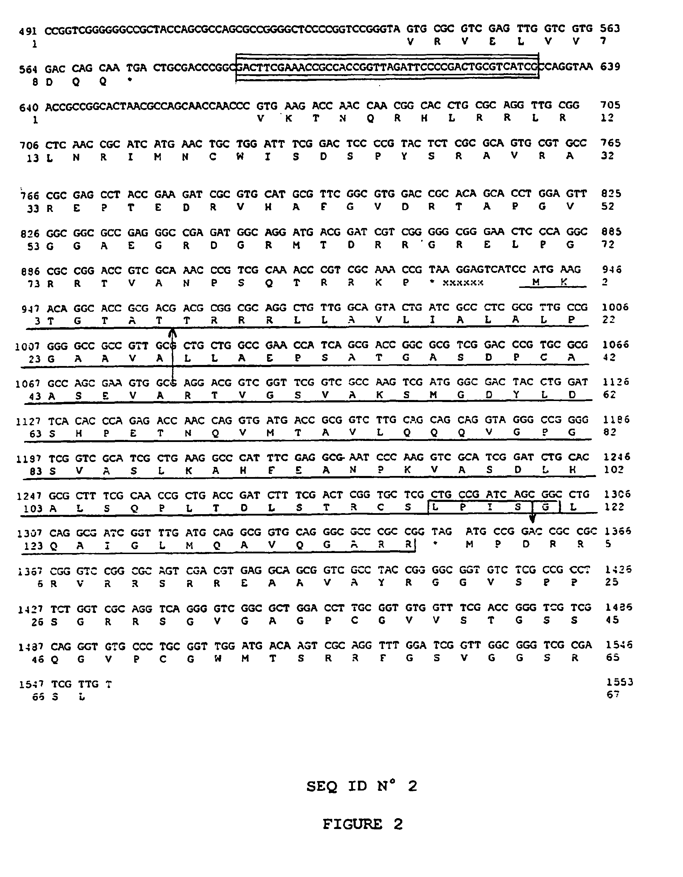

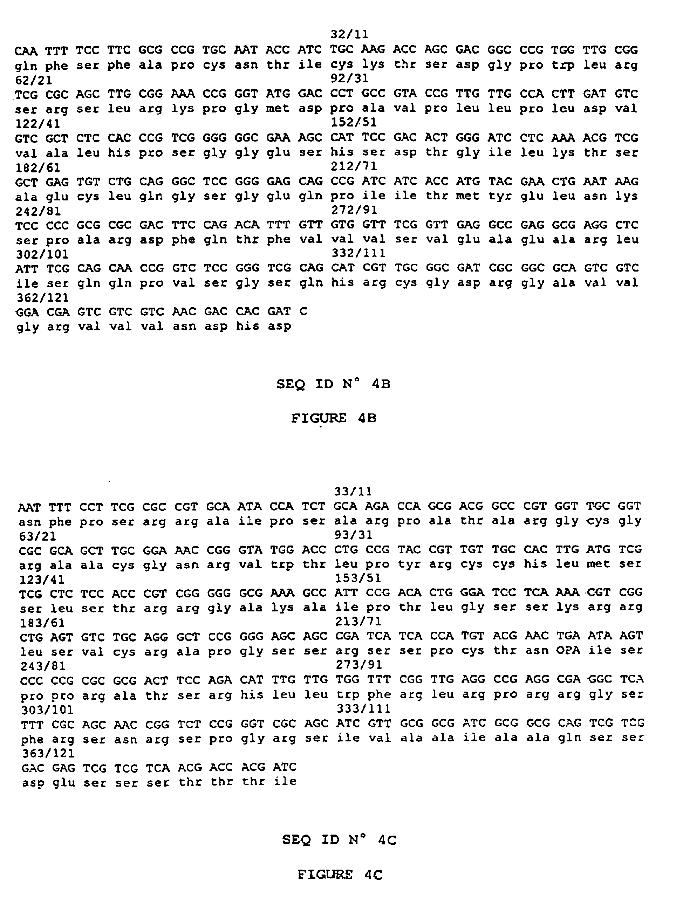

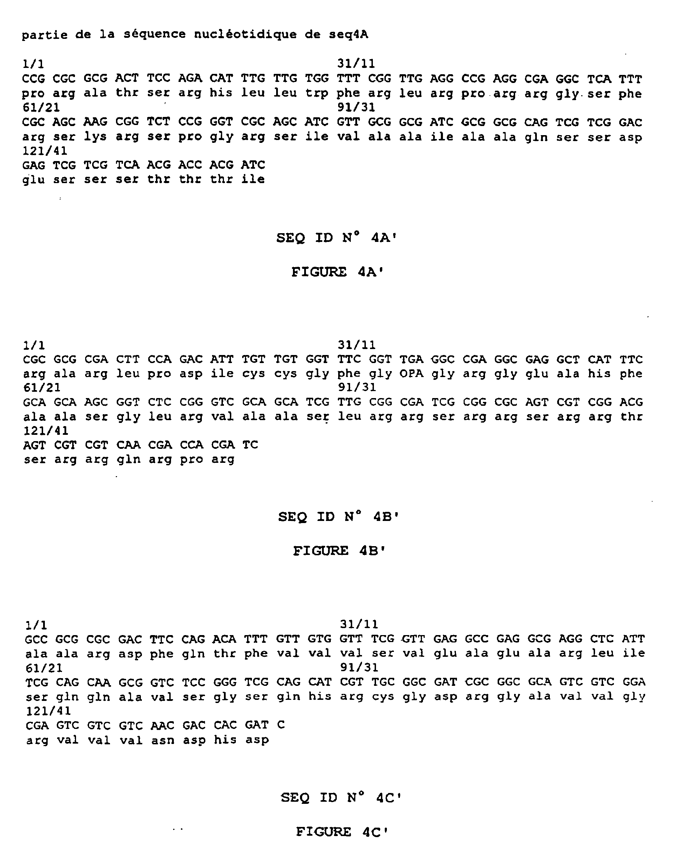

Par exemple, les séquences nucléiques ou les séquences d'acides aminés SEQ ID N° 1 à SEQ ID N° 4N sont respectivement les séquences nucléiques ou les séquences d'acides aminés représentées par les figures 1 à 4N, c'est-à-dire respectivement les séquences nucléiques ou les séquences d'acides aminés SEQ ID N° 1, SEQ ID N° 1A', SEQ ID N° 1B' , SEQ ID N° 1C' , SEQ ID N° 1D, SEQ ID N° 1F, SEQ ID N° 2, SEQ ID N° 3A, SEQ ID N° 3B, SEQ ID N° 3C, SEQ ID N° 4A, SEQ ID N° 4B, SEQ ID N° 4C, SEQ ID N° 4A', SEQ ID N° 4B', SEQ ID N° 4C', SEQ ID N° 4F, SEQ ID N° 4J, SEQ ID N° 4K, SEQ ID N° 4L, SEQ ID N° 4M et SEQ ID N° 4N.

La présente invention permet de déterminer un fragment de gène codant pour un polypeptide exporté. La comparaison avec la séquence du génome publiée par Cole et al. (Cole et al., 1998, Nature, 393, 537-544) permet de déterminer le gène en entier portant la séquence identifiée selon la présente invention.

Lorsque la séquence codant pour le marqueur d'exportation et/ou de sécrétion est une séquence issue du gène *phoA,* l'exportation et/ou la sécrétion du produit du gène *phoA,* le cas échéant tronqué, n'est obtenue que lorsque cette séquence est insérée en phase avec la séquence ou élément de régulation de l'expression de la production de polynucléotides et sa localisation placée en amont, qui contient les éléments contrôlant l'expression, l'exportation et/ou la sécrétion issus de séquence de mycobactéries.

Les vecteurs recombinants de l'invention peuvent bien entendu comprendre des sites de clonage multiples décalés de un ou deux nucléotides par rapport à un vecteur selon l'invention, permettant ainsi d'exprimer le polypeptide correspondant au fragment d'ADN de mycobactérie inséré et susceptible d'être traduit selon l'un des trois cadres de lecture possibles.

Par exemple les vecteurs préférés pJVEDb et pJVEDc de l'invention se distinguent du vecteur préféré pJVEDa par un décalage respectif de un et de deux nucléotides au niveau du site de clonage multiple.

Ainsi, les vecteurs de l'invention sont capables d'exprimer chacun des polypeptides susceptibles d'être codés par un fragment d'ADN de mycobactérie inséré.

L'invention a aussi pour objet des mycobactéries recombinantes contenant un vecteur recombinant selon l'invention décrit précédemment. Une mycobactérie préférée est une mycobactérie du type *M. smegmatis*.
*M. smegmatis* permet avantageusement de tester l'efficacité de séquences de mycobactéries, pour le contrôle de l'expression, de l'exportation- et/ou de la sécrétion, et/ou de l'activité de promoteurs d'une séquence donnée, par exemple d'une séquence codant pour un marqueur tel que la phosphatase alcaline et/ou la luciférase.

Une autre mycobactérie préférée est une mycobactérie du type *M. bovis,* par exemple la souche BCG utilisée actuellement pour la vaccination contre la tuberculose.
Une autre mycobactérie préférée est une souche de *M. tuberculosis, M. bovis* ou *M. africanum* possédant potentiellement tous les systèmes de régulation appropriés.

Les inventeurs ont ainsi caractérisé en particulier un polynucléotide constitué par une séquence de nucléotides présente chez toutes les souches testées de mycobactéries appartenant au complexe de *Mycobacterium tuberculosis.* Ce polynucléotide, dénommé *DP428* contient un cadre ouvert de lecture (ORF) codant pour un polypeptide d'environ 12 kD. Le cadre de lecture ouvert (ORF) codant pour le polypeptide DP428 s'étend du nucléotide en position nt 941 au nucléotide en position nt 1351 de la séquence SEQ ID N° 2, le polypeptide DP428 ayant la séquence en acides aminés SEQ ID N° 28 suivante :

MKTGTATTRRRLLAVLIALALPGAAVALLAEPSATGASDPCAASEVARTVGSVAKSMGD
YLDSHPETNQVMTAVLQQQVGPGSVASLKAHFEANPKVASDLHALSQPLTDLSTRCSLP
ISGLQAIGLMQAVQGARR.

Ce poids moléculaire(PM) correspond au PM théorique de la protéine mature obtenue après clivage de la séquence signale, le PM de la protéine ou polypeptide DP428 étant d'environ 10 kD après ancrage potentiel au peptidoglycane et coupure potentielle entre S et G du motif LPISG.

Ce polynucléotide inclut, d'une part, un cadre ouvert de lecture correspondant à un gène de structure et, d'autre part, les signaux de régulation de l'expression de la séquence codante en amont et en aval de cette dernière.Le polypeptide DP428 est composé d'un peptide signal, d'une région centrale hydrophile et d'une région C-terminale hydrophobe. Cette dernière se termine par deux résidus arginines (R),signal de rétention, et est précédé par un motif LPISG qui rappelle le motif LPXTG d'ancrage au peptidoglycane (Schneewind et al., 1995).

Par gène de structure aux fins de la présente invention, on entend un polynucléotide codant pour une protéine, un polypeptide ou encore un fragment de ces derniers, ledit polynucléotide ne comprenant que la séquence correspondant au cadre ouvert de lecture (ORF), ce qui exclut les séquences du côté 5' du cadre ouvert de lecture (ORF) qui dirigent l'initiation de la transcription.

On entend par séquence nucléotidique, polynucléotide ou acide nucléique, selon la présente invention, aussi bien un ADN double brin, un ADN simple brin que des produits de transcription desdits ADN.

Par pourcentage d'identité au sens de la présente invention, on entend un pourcentage d'identité entre les bases de deux polynucléotides, ce pourcentage étant purement statistique et les différences entre les deux polynucléotides étant réparties au hasard et sur toute leur longueur.

Une hybridation dans des conditions de forte stringence signifie que les conditions de température et de force ionique sont choisies de telle manière qu'elles permettent le maintien de l'hybridation entre deux fragments d'ADN complémentaires.

A titre illustratif, des conditions de forte stringence de l'étape d'hybridation aux fins de définir les fragments polynucléotidiques décrits ci-dessus, sont avantageusement les suivantes :
l'hybridation est réalisée à une température préférentielle de 65°C, en présence de tampon commercialisé sous le nom de rapid-hyb buffer par Amersham (RPN 1636) et 100 µg/ml d'ADN de E.coli.
   Les étapes de lavage peuvent, par exemple, être les suivantes :
   - deux lavages de 10 min, préférentiellement à 65°C, dans un tampon 2 x SSC et 0,1% SDS;
   - deux lavages de 10 min, préférentiellement à 65°C, dans un tampon 1 x SSC et 0,1% SDS;
   - un lavage de 10 min, préférentiellement à 65°C, dans un tampon de 0,1 x SSC et 0,1% SDS.
   1 x SSC correspond à 0,15 M NaCl et 0,05M citrate de Na et une solution de 1 x Denhardt correspond à 0,02% Ficoll, 0,02% de polyvinylpyrrolidone et 0,02% de sérum albumine bovine.

Avantageusement, un fragment nucléotidique répondant à la définition précédente aura au moins 8 nucléotides, de préférence au moins 12 nucléotides, et encore plus préférentiellement au moins 20 nucléotides consécutifs de laséquence dont il est issu. Les conditions d'hybridation de forte stringence décrites ci-avant pour un polynucléotide d'une taille d'environ 200 bases, seront adaptées par l'homme du métier pour des oligonucléotides de taille plus grande ou plus petite, selon l'enseignement de Sambrook et al., 1989.

Pour les conditions de mise en oeuvre des enzymes de restriction dans le but d'obtenir des fragments nucléotidiques des polynucléotides selon l'invention, on se référera avantageusement à l'ouvrage de Sambrook et al., 1989.

Par polypeptide homologue, on entendra désigner les polypeptides présentant, par rapport au polypeptide naturel tel que le polypeptide DP428, certaines modifications comme en particulier une délétion, addition ou substitution d'au moins un acide aminé, une troncation, un allongement, une fusion chimérique, et/ou une mutation. Parmi les polypeptides homologues, on préfère ceux dont la séquence d'acides aminés présente au moins 30%, de préférence 50%, d'homologie avec les séquences d'acides aminés des polypeptides naturels. Dans le cas d'une substitution, un ou plusieurs acides aminés consécutifs ou non consécutifs, sont remplacés par des acides aminés « équivalents ». L'expression acide aminé « équivalent » vise ici à désigner tout acide aminé susceptible d'être substitué à l'un des acides aminés de la structure de base sans cependant modifier essentiellement les propriétés immunogènes des peptides correspondants. En d'autres termes, les acides aminés équivalents seront ceux qui permettent l'obtention d'un polypeptide de séquence modifiée qui permet l'induction *in vivo* d'anticorps ou de cellules capables de reconnaître le polypeptide dont la séquence d'acides aminés est comprise dans la séquence d'acides aminés de polypeptide naturel telle que les séquences d'acides aminés SEQ ID N°1 à SEQ ID N° 2, ou un polypeptide de séquence d'acides aminés SEQ ID N°28 (polypeptide DP428) ou l'un de ses fragments ci-dessus définis.
Ces aminoacyles équivalents peuvent être déterminés soit en s'appuyant sur leur homologie de structure avec les aminoacyles auxquels ils se substituent, soit sur les résultats des essais d'immunogénicité croisée auxquels les différents peptides sont susceptibles de donner lieu.

A titre d'exemple, on mentionnera les possibilités de substitutions susceptibles d'être effectuées sans qu'il en résulte une modification approfondie de l'immunogénicité des peptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Par fragment biologiquement actif, on entendra désigner en particulier un fragment de séquence d'acides aminés de polypeptide présentant au moins une des caractéristiques des polypeptides décrits dans la présente invention, notamment en ce qu'il est :
- capable d'être exporté et/ou sécrété par une mycobactérie, et/ou d'être induit ou réprimé lors de l'infection par la mycobactérie ; et/ou
- capable d'induire, de réprimer ou de moduler, directement ou indirectement, un facteur de virulence de mycobactérie ; et/ou
- capable d'induire une réaction d'immunogénicité dirigée contre les mycobactéries ; et/ou
- capable d'être reconnu par un anticorps spécifique de mycobactérie .

Par fragment de polypeptide, on entend désigner un polypeptide comportant au minimun 5 acides aminés, de préférence 10 acides aminés et 15 acides aminés.

Un polypeptide, ou un de ses fragments, tels que définis précédemment, est susceptible d'être reconnu spécifiquement par les anticorps présents dans le sérum de patients infectés par des mycobactéries et préférentiellement des bactéries appartenant au complexe de *Mycobacterium tuberculosis* ou par des cellules de l'hôte infecté.

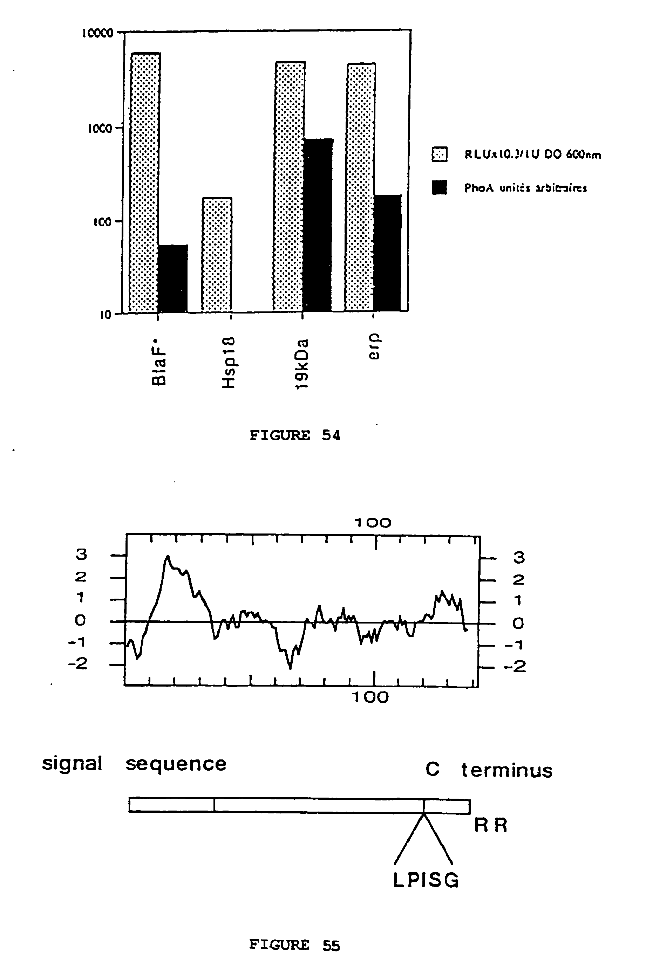

Une analyse de l'hydrophilicité du polypeptide DP428 a été réalisée à l'aide du logiciel DNA Strider^{™} (commercialisé par le CEA Saclay), sur la base d'un calcul du caractère hydrophile de la région codante pour le DP428 de la SEQ ID N°28. Les résultats de cette analyse sont présentés à la figure 54, où sont détaillés, pour chacun des acides aminés (AA) de position définie dans la SEQ ID N°28, l'indice d'hydrophilicité. Plus l'indice d'hydrophilicité est élevé, plus l'acide aminé considéré est susceptible d'être exposé au solvant dans la molécule native, et est en conséquence susceptible de présenter un degré d'antigénicité élevé. Ainsi, un enchaînement d'au moins sept acides aminés possédant un indice élevé d'hydrophilicité (>0,3) peut constituer la base de la structure d'un peptide candidat immunogène.

Les réponses immunitaires cellulaires de l'hôte à un polypeptide, peuvent être mises en évidence selon les techniques décrites par Colignon et al., 1996.

D'après les données de la carte d'hydrophilicité présentée à la Figure 54, les inventeurs ont pu définir des régions du polypeptide DP428 préférentiellement exposées au solvant, plus particulièrement la région localisée entre les acides aminés 55 et 72 de la séquence SEQ ID N° 28 et la région localisée entre les acides aminés 99 et 107 de la SEQ ID N° 28.

Les régions peptidiques du polypeptide DP428 définies ci-dessus peuvent être avantageusement mises en oeuvre pour la réalisation de composition immunogène ou de composition vaccinale.

L'invention décrit en outre l'utilisation d'une séquence d'acide nucléique de polynucléotides comme sonde ou amorce, pour la détection et/ou l'amplification de séquence d'acide nucléique. Parmi ces séquences d'acide nucléique utilisables comme sonde ou amorce, on décrit particulièrement les séquences d'acide nucléique ou leur séquence complémentaire, comprises entre le nucléotide en position nt 964 et le nucléotide en position nt 1234, extrémités inclues, de la séquence SEQ ID N°1.

Parmi les polynucléotides utilisables comme amorces nucléotidiques, on décrit particulièrement les polynucléotides de séquence SEQ ID N°25 et SEQ ID N°26.

Les polynucléotides décrit dans l'invention peuvent ainsi être utilisés pour sélectionner des amorces nucléotidiques, notamment pour la technique PCR (Erlich, 1989 ; Innis et al., 1990, et, Rolfs et al., 1991).

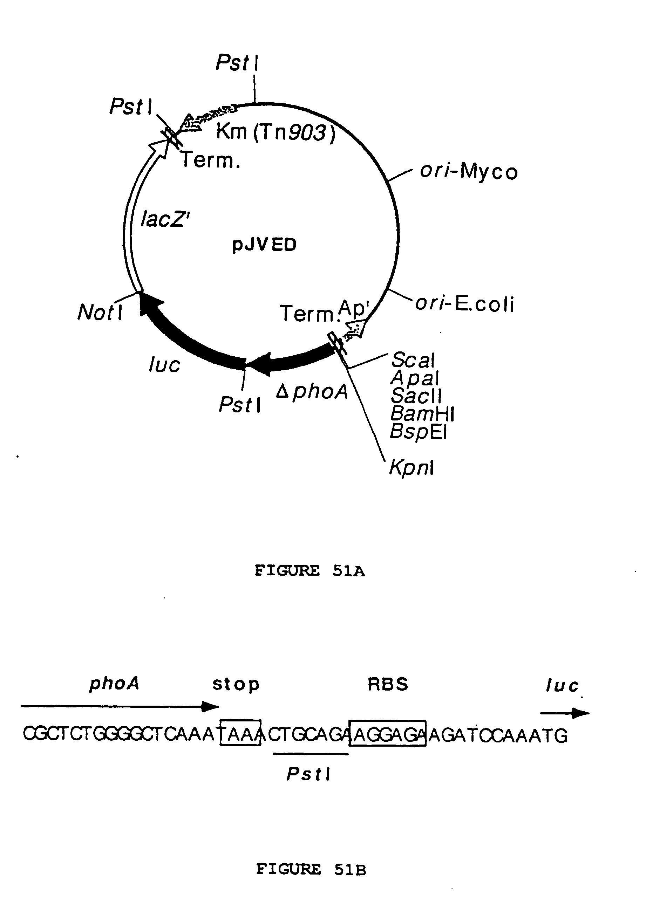

Les résultats présentés à la figure 51, montrent que la séquence codant pour le polypeptide DP428 (SEQ ID N° 28) n'est pas retrouvée dans les ADNs de *M*. *fortuitum*, *M. simiae*, *M. avium, M. chelonae, M. flavescens, M. gordonae*, *M. marinum* et *M. kansasii*

L'invention décrit également les cellules hôtes transformées par un vecteur dans des conditions permettant notamment l'expression d'un polypeptide recombinant.

Il est décrit en particulier une souche *E. coli* transformée par le plasmide pDP428 déposé le 28 janvier 1997 à la CNCM sous le N°I-1818 ou transformée par le plasmide pMlC25 déposé le 4 août 1998 à la CNCM sous le n° 1-2062 ou une mycobactérie appartenant à une souche de *M. tuberculosis*, *M.bovis* ou *M.africanum* possédant potentiellement tous les systèmes de régulation appropriés.

Les polypeptides décrits identifiés par les vecteurs ou procédés selon l'invention peuvent avantageusement être mis en oeuvre dans un procédé pour la détection *in vitro* d'anticorps dirigés contre lesdits polypeptides, notamment le polypeptide DP428, et ainsi d'anticorps dirigés contre une bactérie du complexe Mycobacterium *tuberculosis,* dans un échantillon biologique (tissu ou fluide biologique) susceptible de les contenir, ce procédé comprenant la mise en contact de cet échantillon biologique avec un tel polypeptide dans des conditions permettant une réaction immunologique in vitro entre ledit polypeptide et les anticorps éventuellement présents dans l'échantillon biologique, et la mise en évidence *in vitro* des complexes antigène-anticorps éventuellement formés.

Ces polypeptides peuvent également et avantageusement être mis en oeuvre dans un procédé pour la détection d'une infection par une bactérie du complexe *Mycobacterium tuberculosis* dans un mammifère basé sur la détection in vitro d'une réaction cellulaire indiquant une sensibilisation préalable du mammifère audit polypeptide comme par exemple la prolifération cellulaire, la synthèse de protéines telles que l'interféron gamma.

L'invention décrit également un nécessaire ou kit pour le diagnostic in vitro d'une infection par une mycobactérie appartenant au complexe *Mycobacterium tuberculosis,* comprenant en particulier un tel polypeptide.

L'invention décrit également des anticorps mono ou polyclonaux ou leurs fragments, ou anticorps chimériques capables de reconnaître spécifiquement un tel polypeptide.

Ces anticorps pourront également être marqués tel qu'un marquage de type enzymatique, fluorescent ou radioactif.

L'invention décrit en outre un procédé pour la détection spécifique de la présence d'un antigène d'une mycobactérie, comme une bactérie du complexe *Mycobacterium tuberculosis,* dans un échantillon biologique, mettant en oeuvre en particulier un tel anticorps mono ou polyclonal.

Est décrit également dans le cadre de l'invention, un nécessaire ou kit pour le diagnostic *in vitro* sur un échantillon biologique, de la présence de souches de mycobactéries, telles que des bactéries appartenant au complexe de *Mycobacterium tuberculosis,* comme *M. tuberculosis,* caractérisé en ce qu'il comprend en particulier
un tel anticorps polyclonal ou monoclonal le cas échéant marqué.

La présente invention décrit également un procédé de détection et d'identification rapide des mycobactéries et préférentiellement des bactéries de *M. tuberculosis* dans un échantillon biologique, caractérisé en ce qu'il met en oeuvre des amorces, sondes ou polypeptides.

L'invention décrit aussi un nécessaire ou kit pour la mise en oeuvre du procédé décrit ci-dessus, destiné à la détection de la présence des mycobactéries et préférentiellement des bactéries du complexe *Mycobacterium tuberculosis* dans un échantillon biologique, caractérisé en ce qu'il comprend en particulier de telles amorces et/ou sondes.

La présente invention décrit aussi une composition immunogène comprenant un tel polypeptide ou polynucléotide identifié au moyen de la présente invention.

Il est décrit que la composition immunogène ci-dessus définie peut être constitutive d'un vaccin, lorsqu'elle est présentée en association avec un véhicule pharmaceutiquement acceptable et éventuellement un ou plusieurs adjuvants de l'immunité tels que l'alun ou un représentant de la famille des muramyl peptides ou encore l'adjuvant incomplet de Freund.

Aujourd'hui, divers types de vaccins sont disponibles pour protéger l'homme contre des maladies infectieuses : micro-organismes vivants atténués (*M. bovis -* BCG pour la tuberculose), micro-organismes inactivés (virus de la grippe), des extraits acellulaires (*Bordetella pertussis* pour la coqueluche), protéines recombinées (antigène de surface du virus de l'hépatite B), des polyosides (pneumocoques). Des vaccins préparés à partir de peptides de synthèse ou de micro-organismes génétiquement modifiés exprimant des antigènes hétérologues sont en cours d'expérimentation. Plus récemment encore, des ADN plasmidiques recombinés portant des gènes codant pour des antigènes protecteurs ont été proposés comme stratégie vaccinale alternative. Ce type de vaccination est réalisé avec un plasmide particulier dérivant d'un plasmide de *E. coli* qui ne se réplique pas *in vivo* et qui code uniquement pour la protéine vaccinante. Les principaux composants fonctionnels de ce plasmide sont : un promoteur fort permettant l'expression dans les cellules eucaryotes (par exemple celui du CMV), un site de clonage approprié pour insérer le gène d'intérêt, une séquence de terminaison-polyadénylation, une origine de réplication procaryote pour produire le plasmide recombiné *in vitro* et un marqueur de sélection (par exemple le gène de résistance à l'ampicilline) pour faciliter la sélection des bactéries qui contiennent le plasmide. Des animaux ont été immunisés en injectant simplement l'ADN plasmidique nu dans le muscle. Cette technique conduit à l'expression de la protéine vaccinale *in situ* et à une réponse immunitaire en particulier de type cellulaire (CTL) et de type humoral (anticorps). Cette double induction de la réponse immunitaire est l'un des principaux avantages de la technique de vaccination avec de l'ADN nu. Huygen et al. (1996) et Tascon et al. (1996) ont réussi a obtenir une certaine protection contre *M. tuberculosis* en injectant des plasmides recombinés contenant des gènes de *M. leprae* (*hsp65, 36kDa pra*) comme inserts. *M. leprae* est l'agent responsable de la lèpre. L'utilisation d'un insert spécifique de *M*. *tuberculosis* comme par exemple tout ou partie du gène *DP428,* conduirait probablement à une meilleure protection contre la tuberculose Tout ou partie du gène *DP428,* ou tout polynucléotide identifié au moyen de la présente invention, peut être facilement inséré dans les plasmides vecteurs V1J (Montgomery et al, 1993), pcDNA3 (Invitrogen, R & D Systems) ou pcDNAl/Neo (Invitrogen) qui possèdent les caractéristiques nécessaires pour une utilisation vaccinale.

Il est décrit aussi une méthode de criblage de molécules capables d'inhiber la croissance de mycobactéries ou le maintien de mycobactéries dans un hôte, caractérisée en ce que lesdites molécules bloquent la synthèse ou la fonction des polypeptides codés par une séquence nucléotidique identique au moyen de la présente invention ou par un polynucléotide tel que décrit supra.

Dans ladite méthode de criblage, les molécules peuvent être des anti-messagers ou peuvent induire la synthèse d'anti-messagers.
D'autres caractéristiques et avantages de l'invention apparaissent dans les exemples et les figures suivants :

### FIGURES

### La série de Figures 1 :

La série de Figures 1 illustre la série de séquences nucléotidiques SEQ ID N°1 correspondant à l'insert du vecteur pDP428 (déposé à la CNCM sous le N° I-1818) et la série de séquences d'acides aminés SEQ ID N°1 des polypeptides codés par la série des séquences nucléotidiques SEQ ID N°1.

### Figure 2 :

Illustre la séquence nucléotidique SEQ ID N°2 correspondant à la région incluant le gène codant pour le polypeptide DP428 (région soulignée). Sur cette figure ont été pris en compte à la fois les codons ATG et GTG d'initiation de la traduction. La figure fait apparaître que le polypeptide DP428 fait probablement partie d'un opéron comprenant au moins trois gènes. La région doublement encadrée inclut probablement les régions promotrices.
La région simplement encadrée correspond au motif LPISG rapellant le motif LPXTG décrit chez les bactéries à Gram positifs comme permettant l'ancrage aux peptidoglycannes.

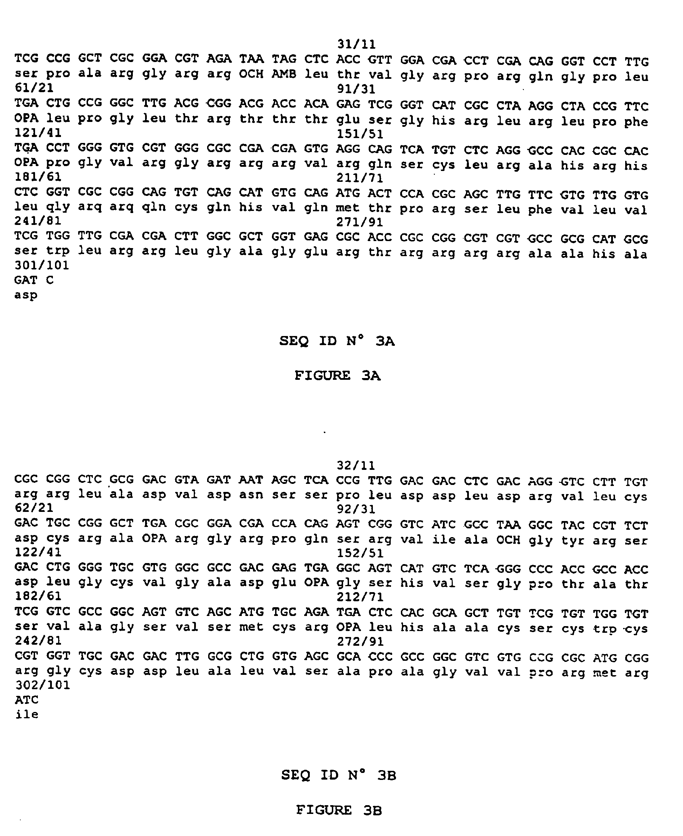

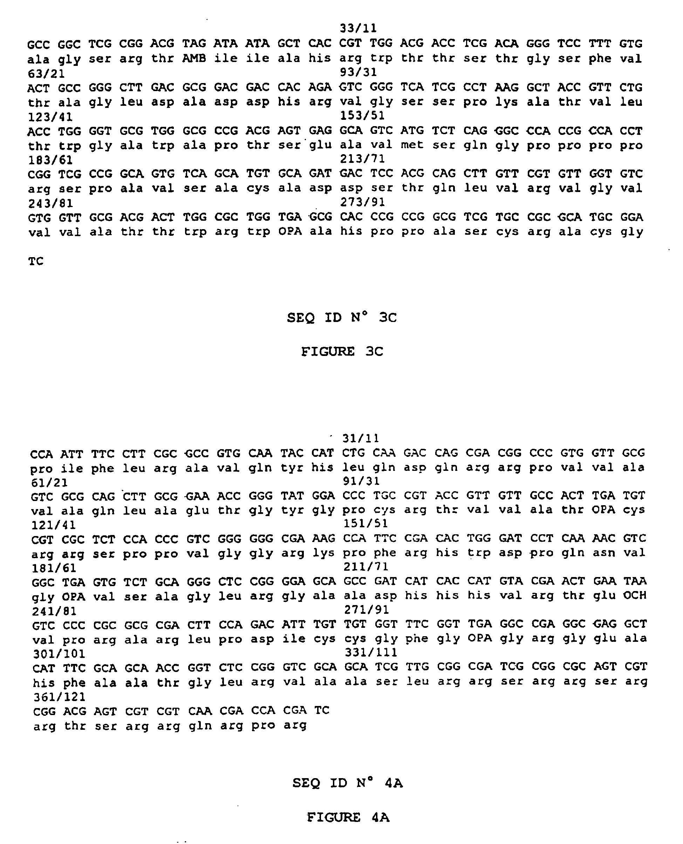

### La série de Figures 3 :

La série de Figures 3 représente la série de séquences nucléotidiques SEQ ID N°3 correspondant à l'insert du vecteur p6D7 (déposé à la CNCM sous le N° I-1814).

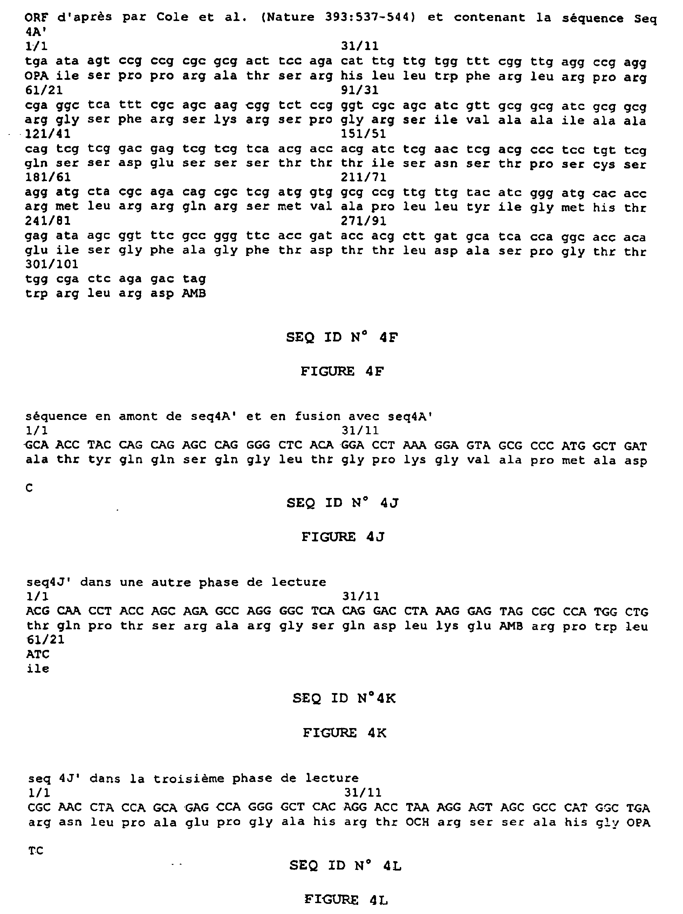

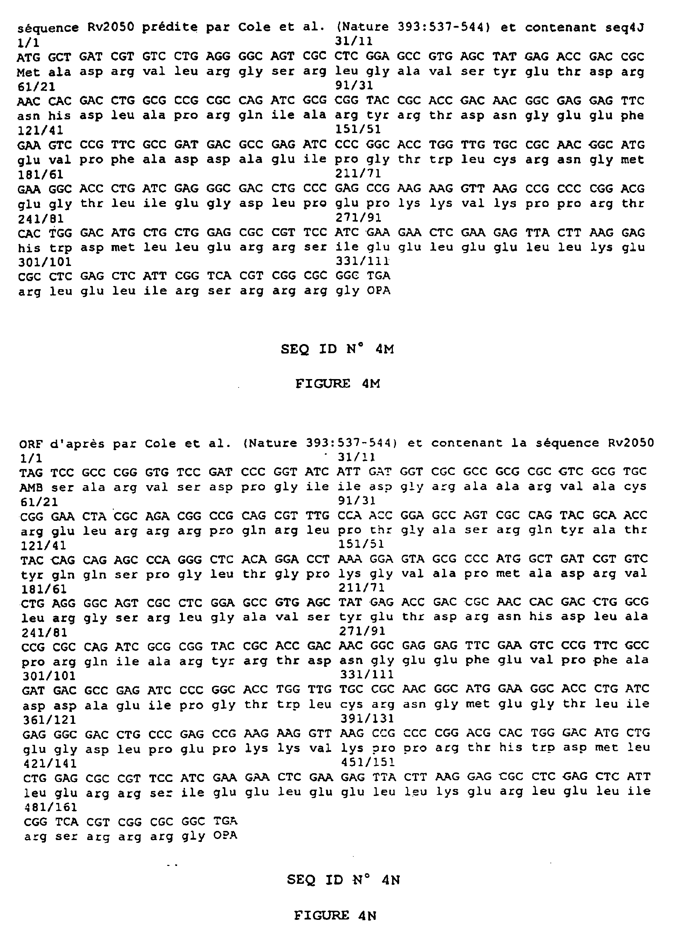

### La série de Figures 4 :

La série de Figures 4 représente la série de séquences nucléotidiques SEQ ID N°4 correspondant à l'insert du vecteur p5A3 (déposé à la CNCM sous le N° I-1815.

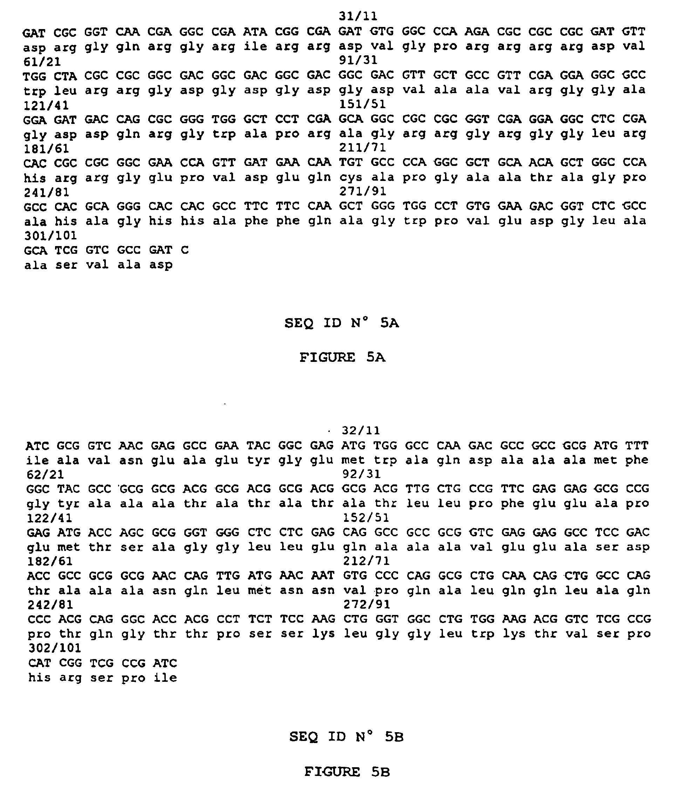

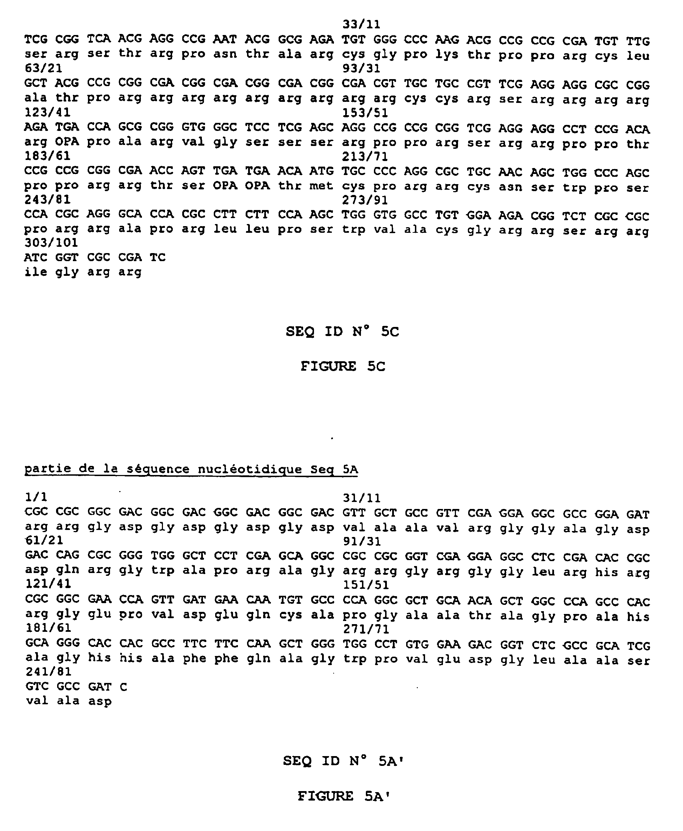

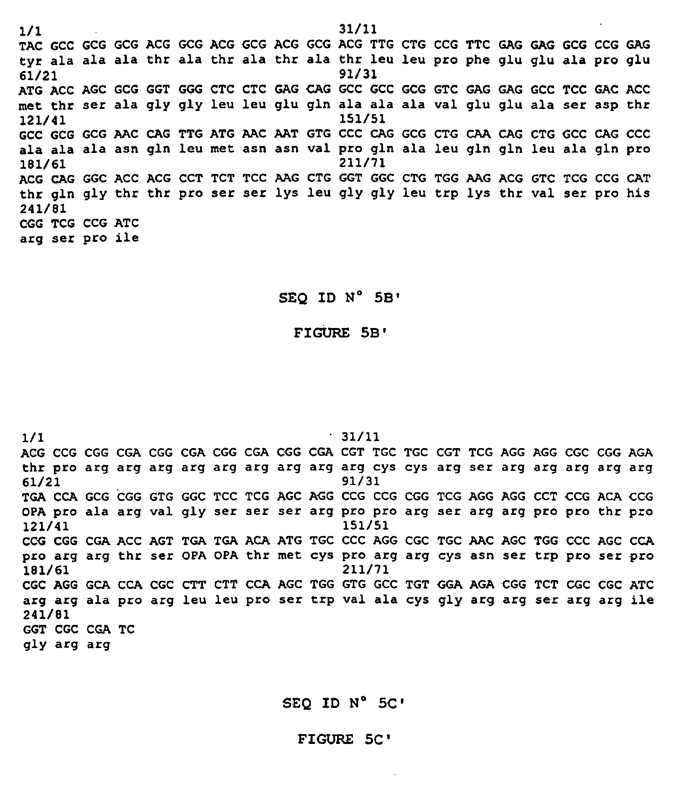

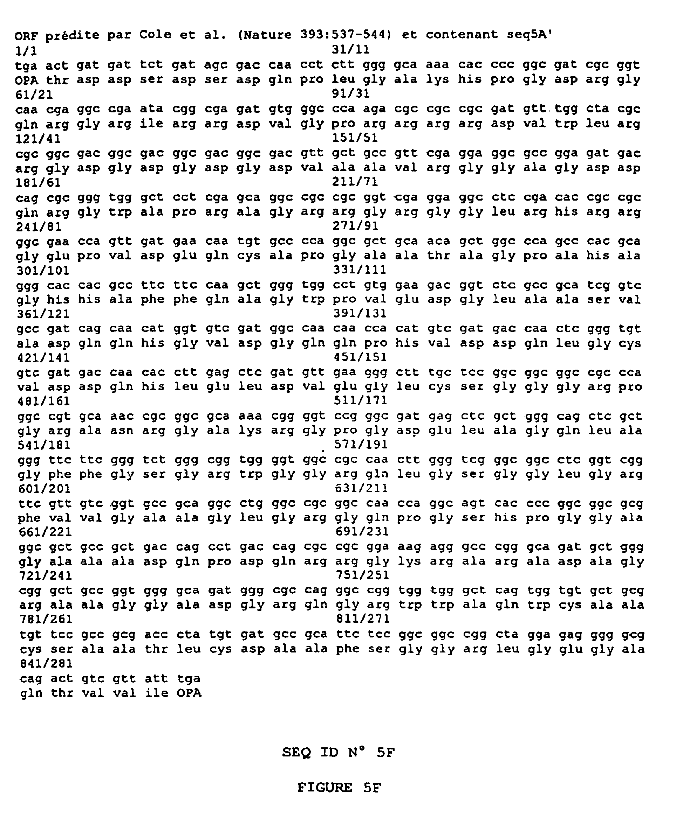

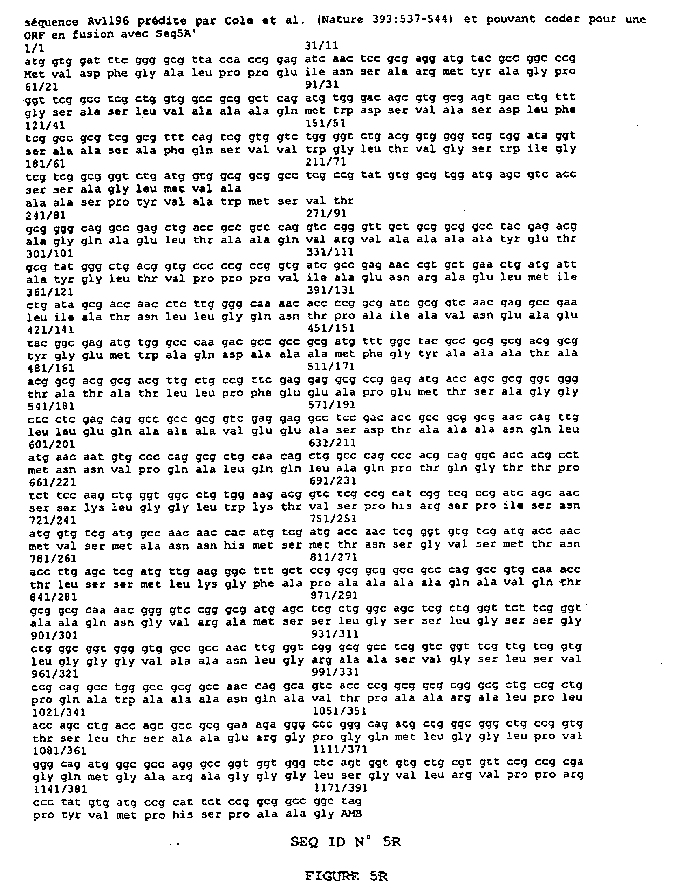

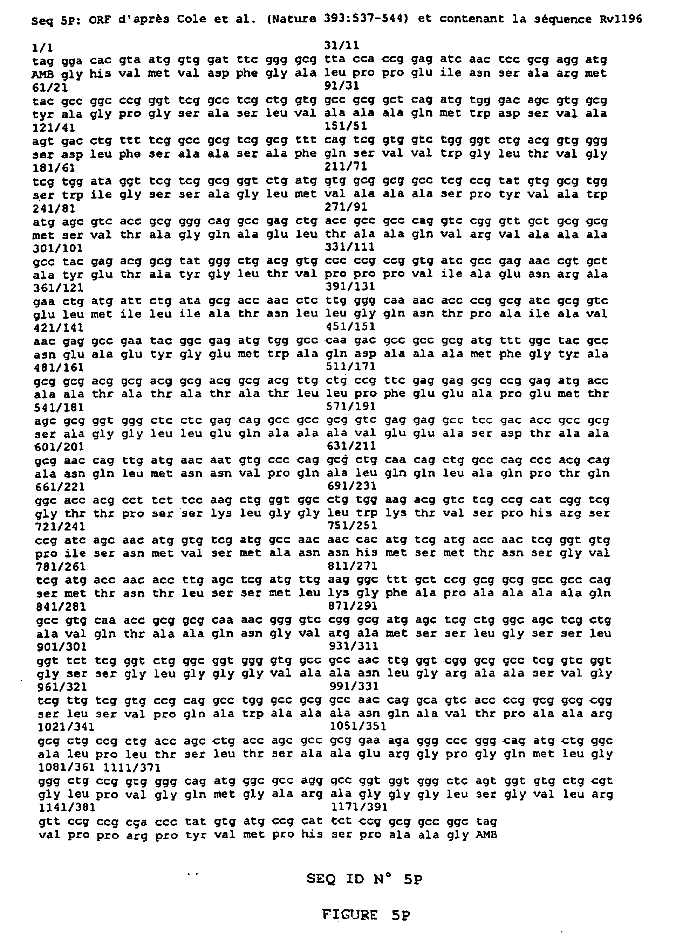

### La série de Figures 5 :

La série de Figures 5 représente la série de séquences nucléotidiques SEQ ID N°5 correspondant à l'insert du vecteur p5F6 (déposé à la CNCM sous le N° I-1816).

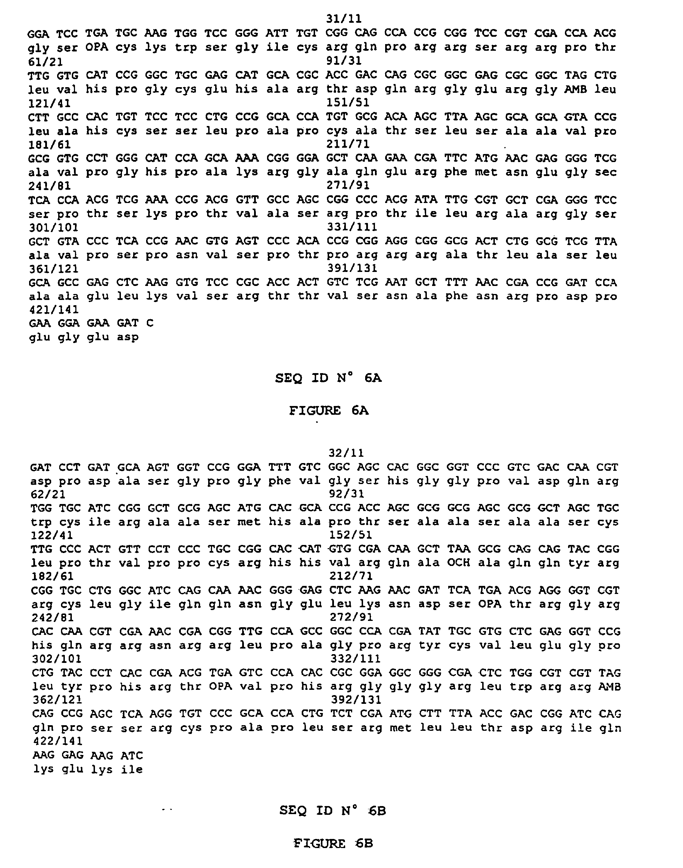

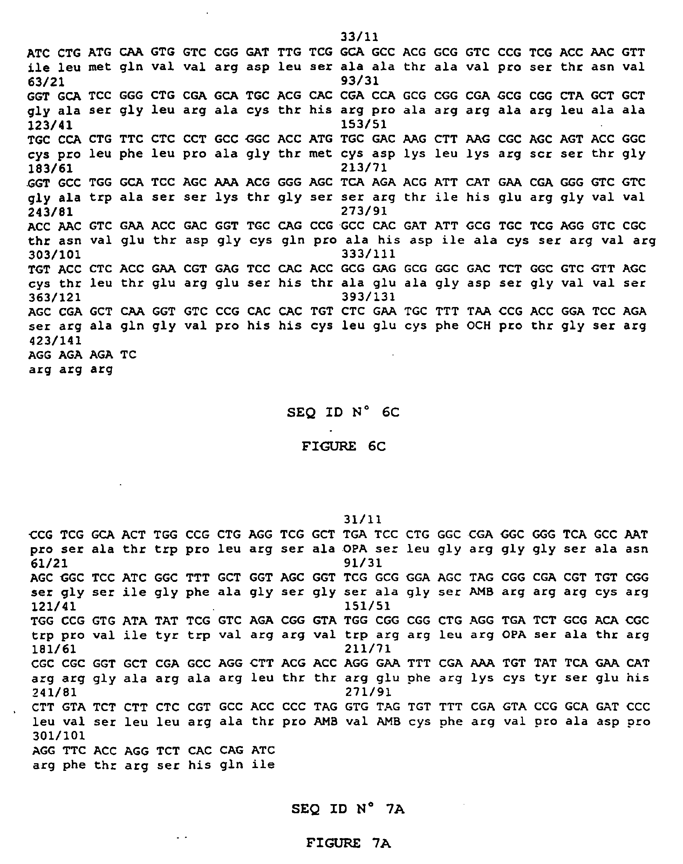

### La série de Figures 6 :

La série de Figures 6 représente la série de séquences nucléotidiques SEQ ID N°6 correspondant à l'insert du vecteur p2A29 (déposé à la CNCM sous le N° I-1817).

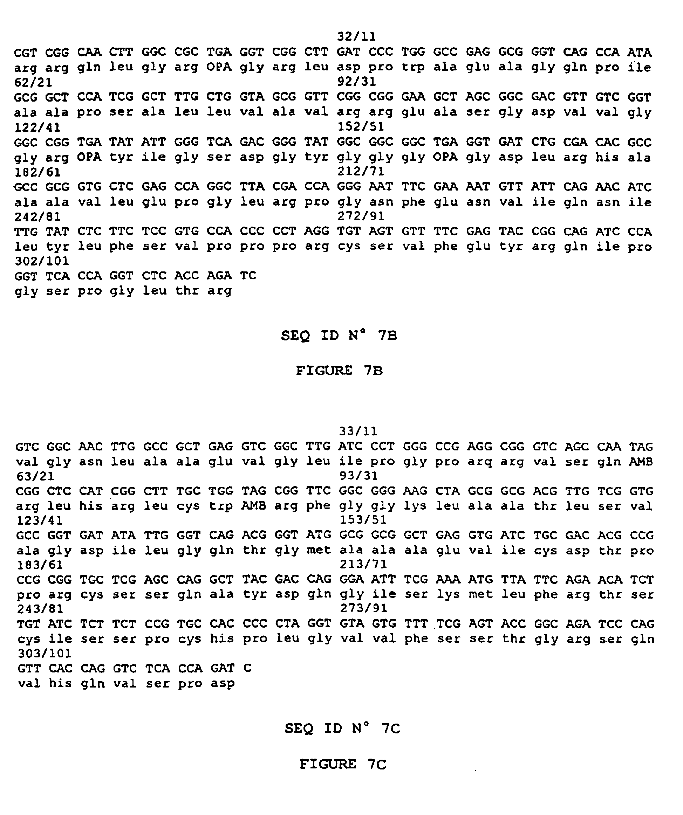

### La série de Figures 7 :

La série de Figures 7 représente la série de séquences nucléotidiques SEQ ID N°7 correspondant à l'insert du vecteur p5B5 (déposé à la CNCM sous le N° I-1819).

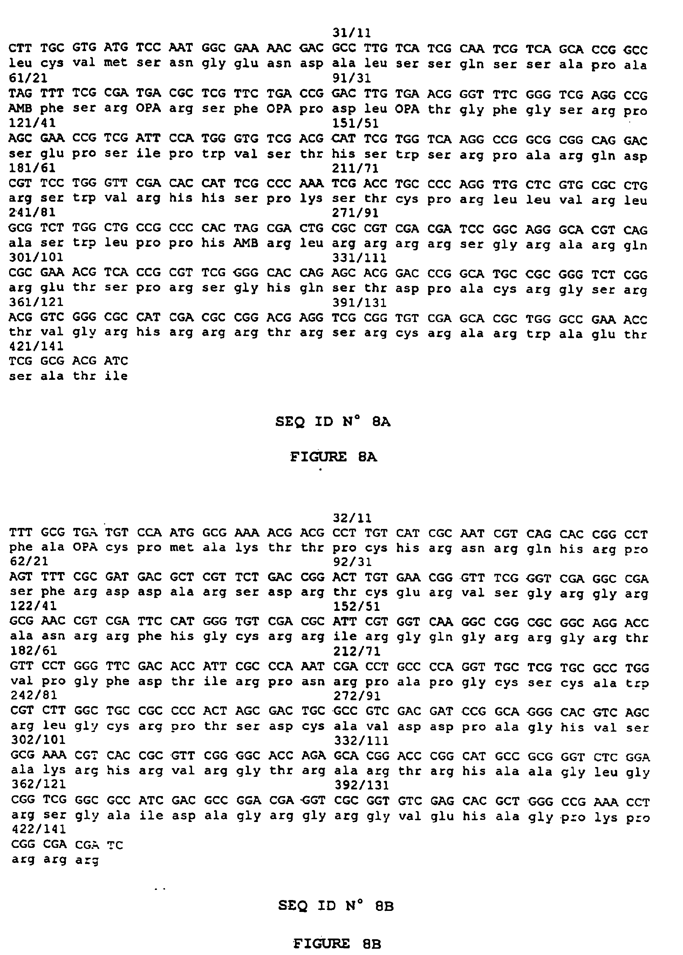

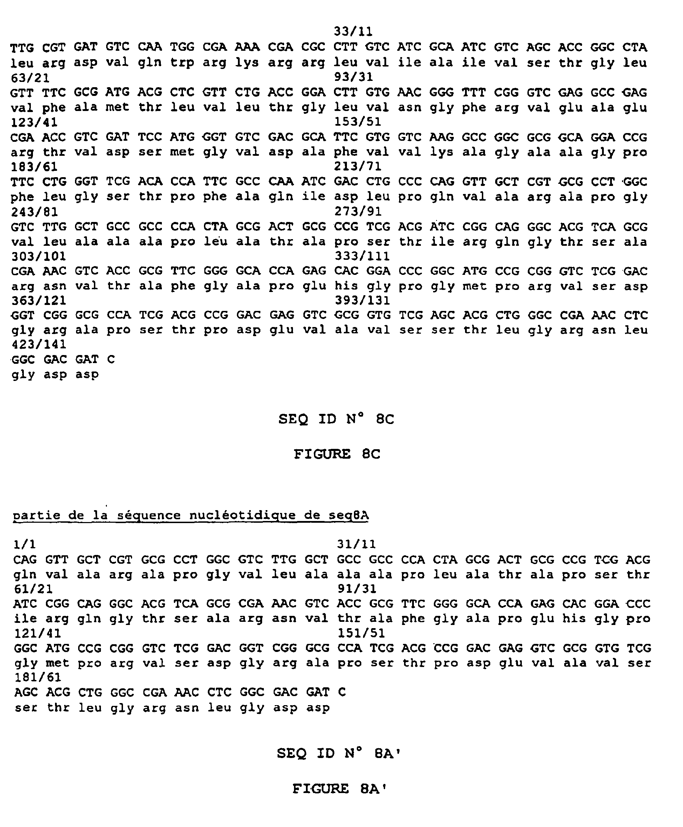

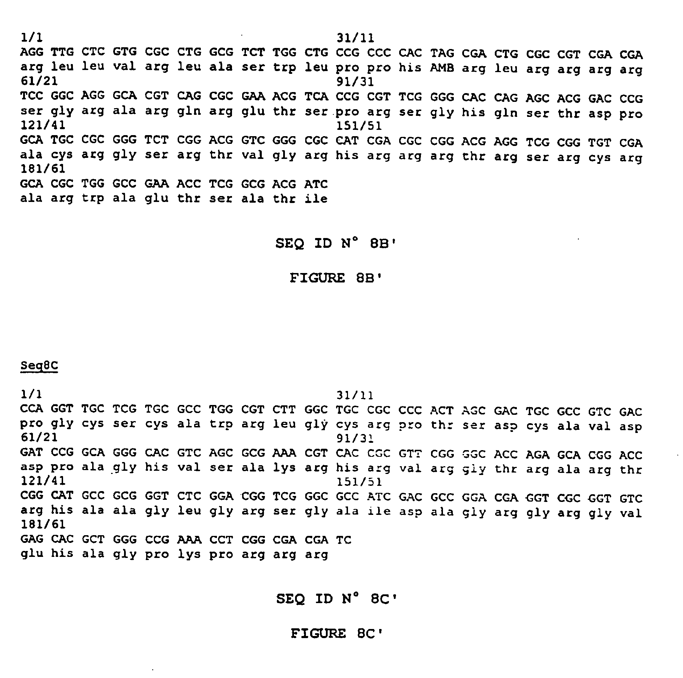

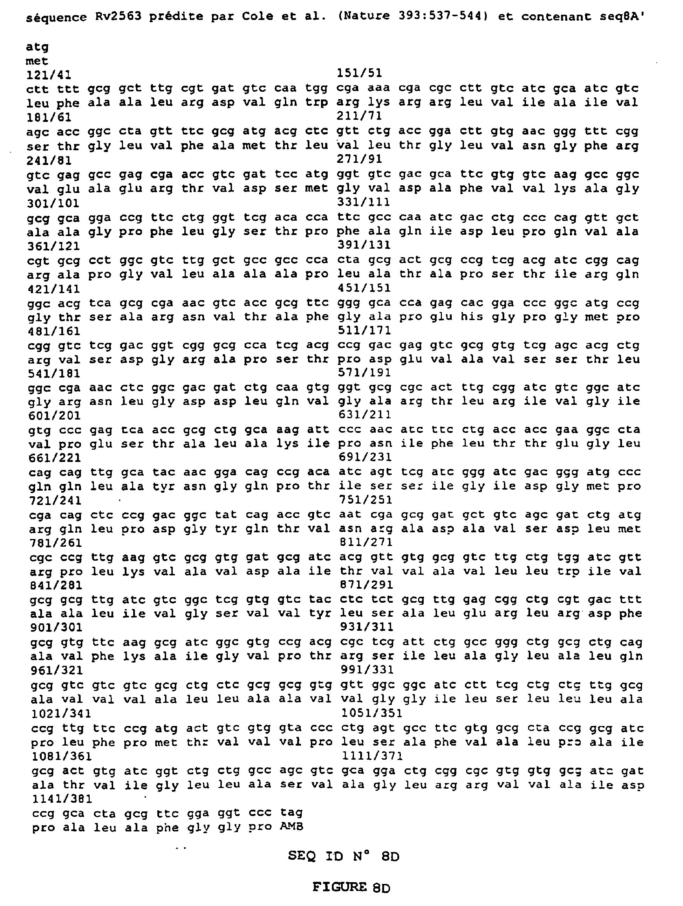

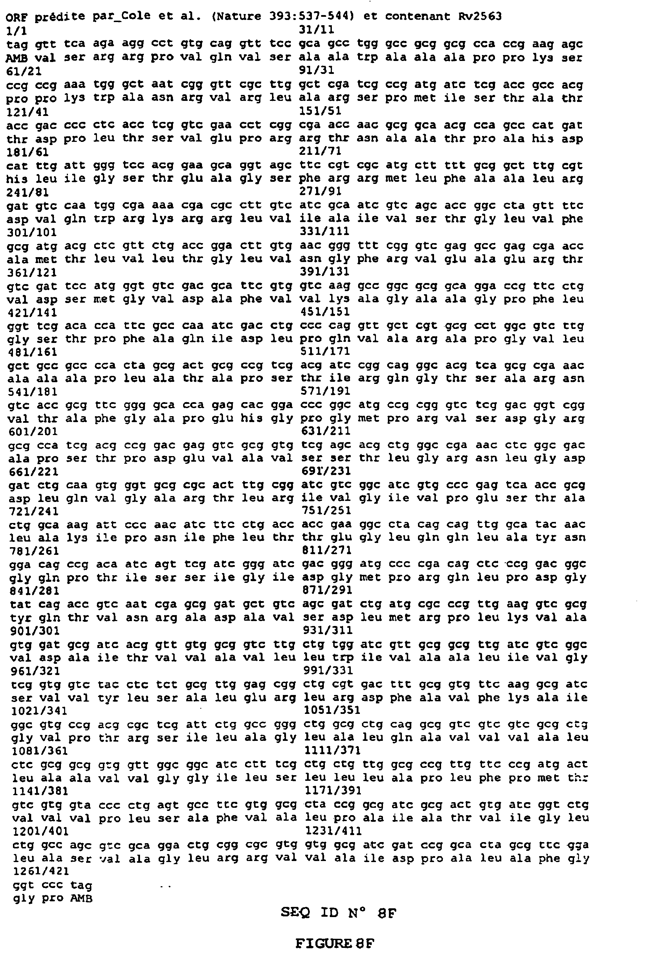

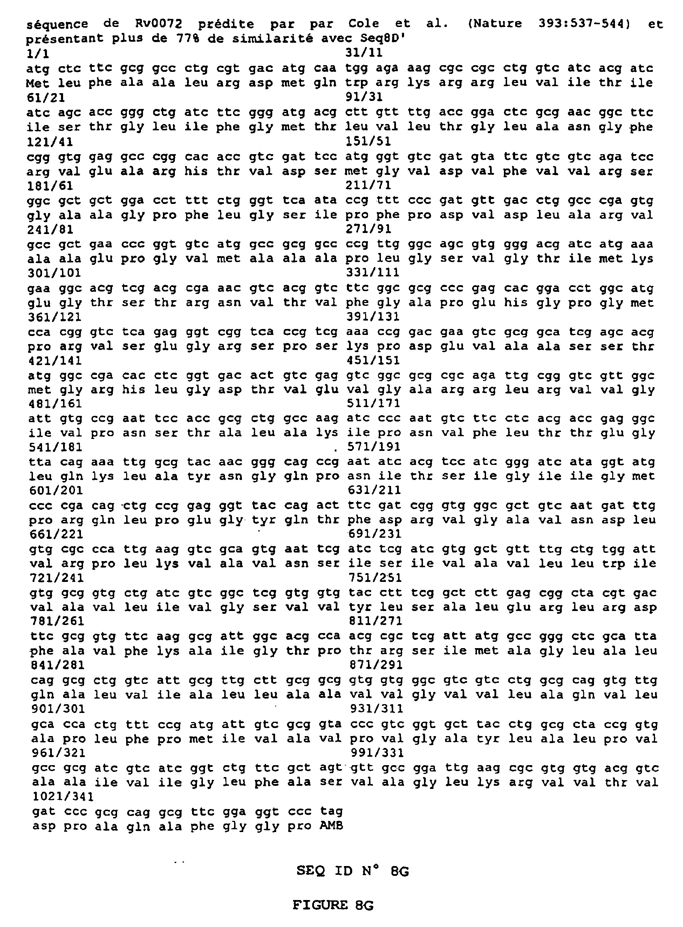

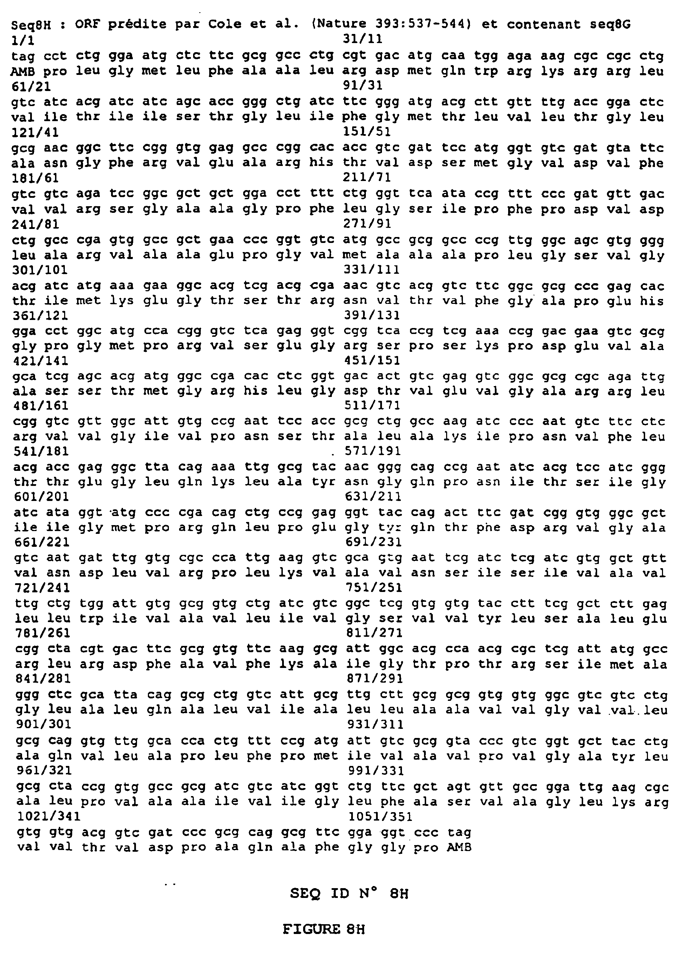

### La série de Figures 8 :

La série de Figures 8 représente série de séquences nucléotidiques SEQ ID N°8 correspondant à l'insert du vecteur plC7 (déposé à la CNCM sous le N° I-1820).

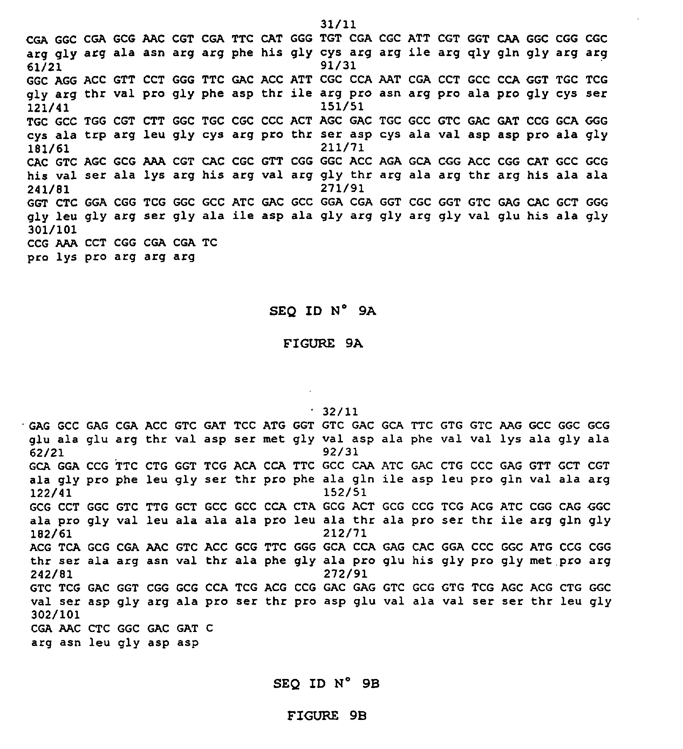

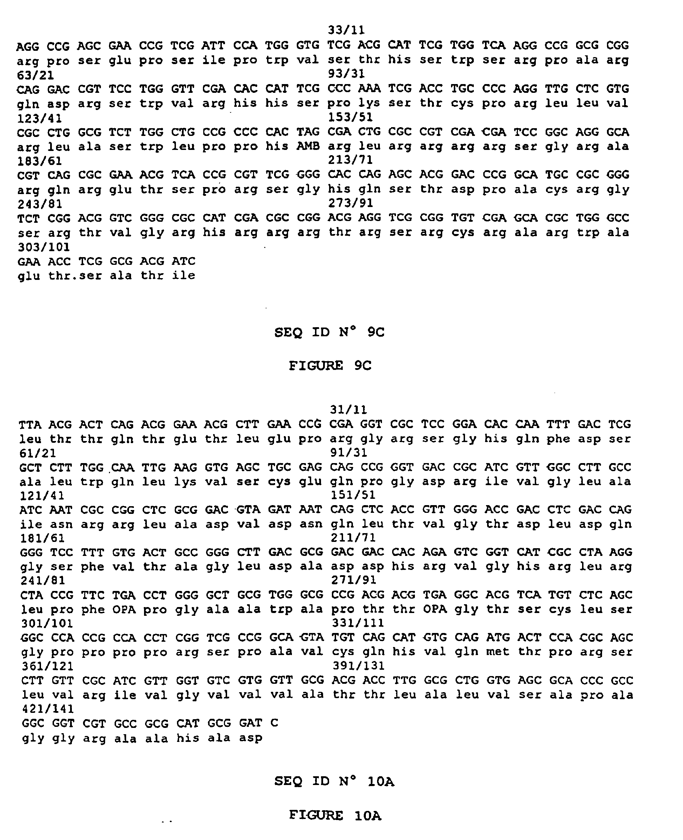

### La série de Figures 9 :

La série de Figures 9 représente la série de séquences nucléotidiques SEQ ID N°9 correspondant à l'insert du vecteur p2D7 (déposé à la CNCM sous le N° I-1821).

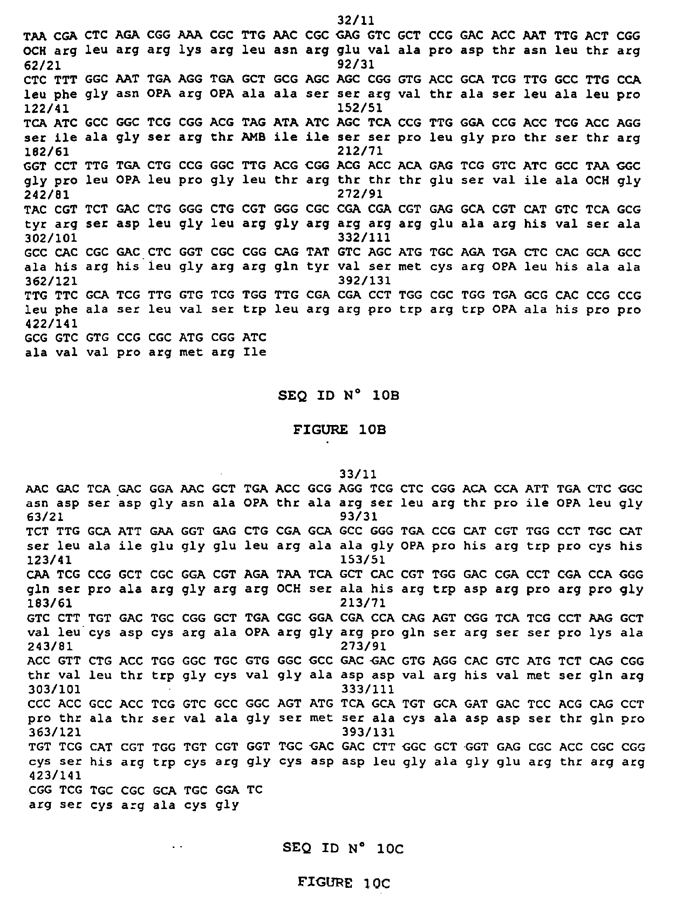

### La série de Figures 10 :

La série de Figures 10 représente la série de séquences nucléotidiques SEQ ID N°10 correspondant à l'insert du vecteur p1B7 (déposé à la CNCM sous le N° I-1843).

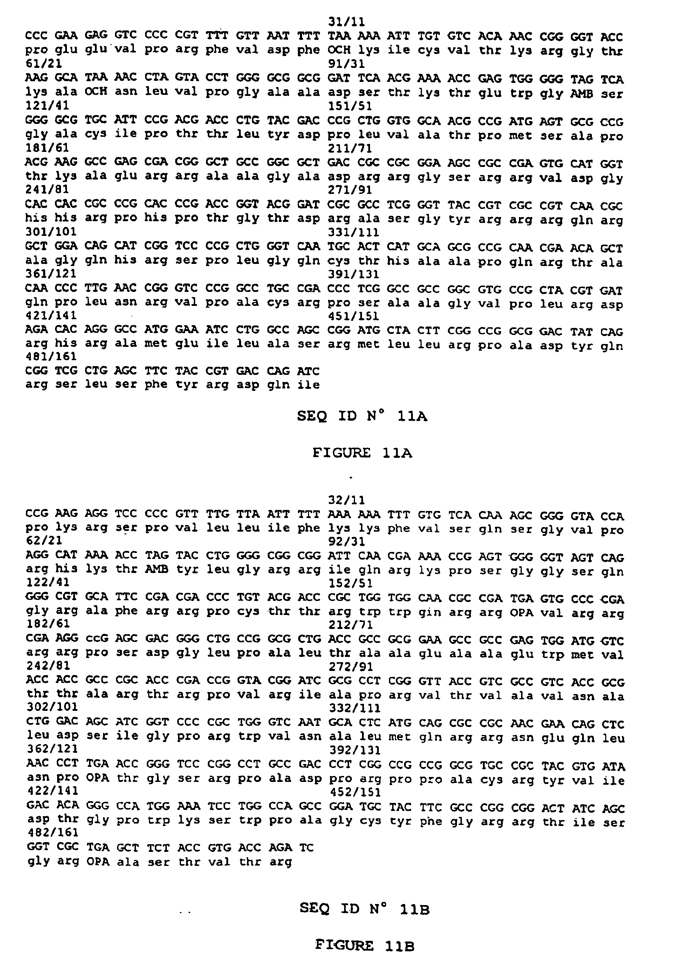

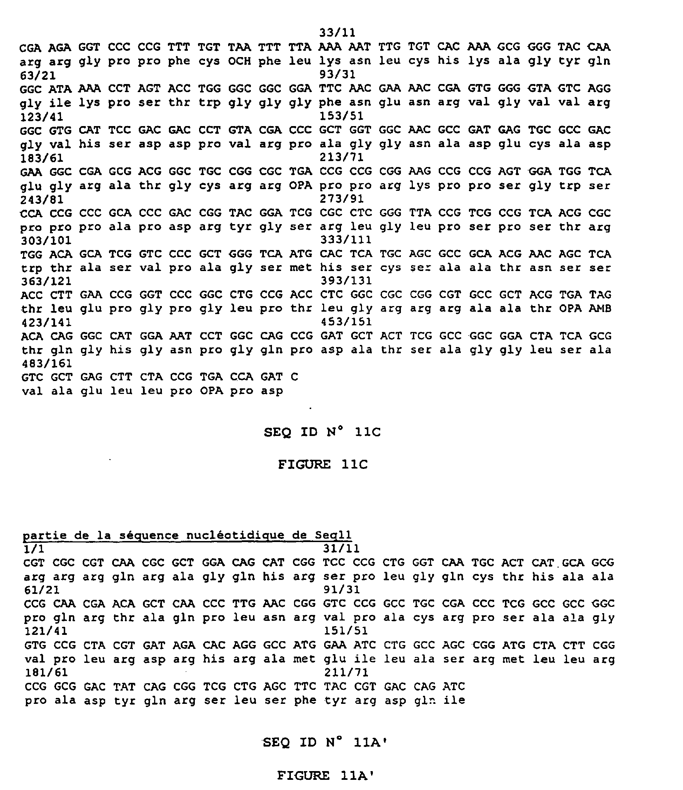

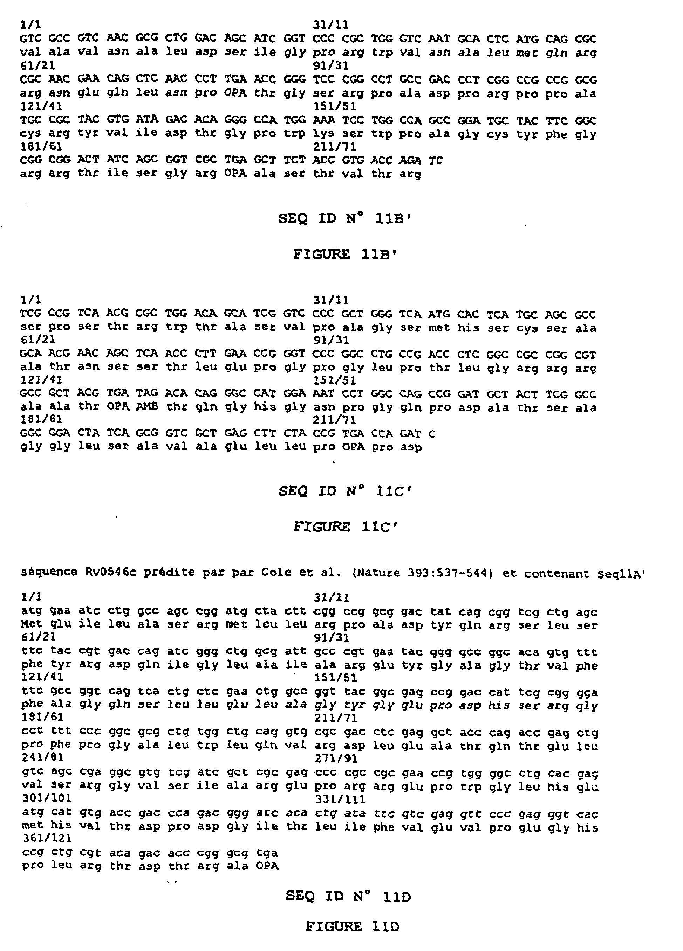

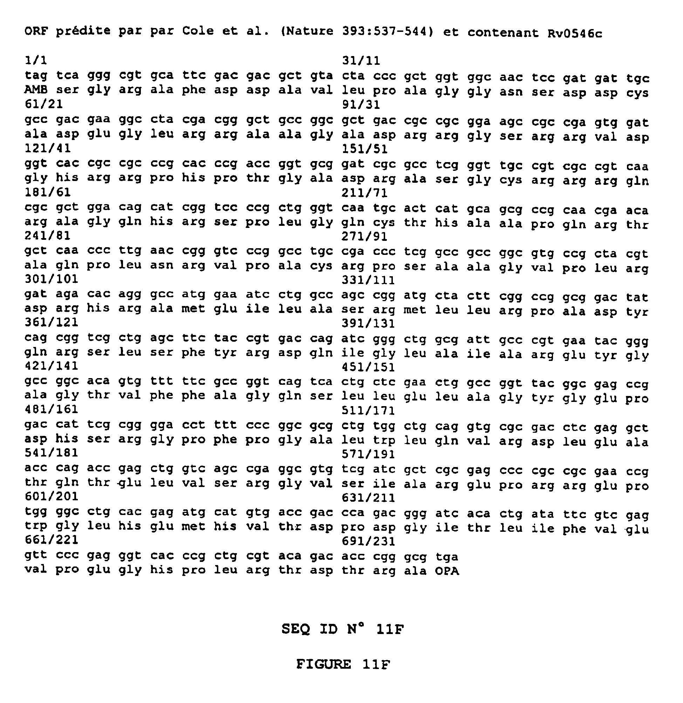

### La série de Figures 11 :

La série de Figures 11 représente la série de séquences nucléotidiques SEQ ID N°11.

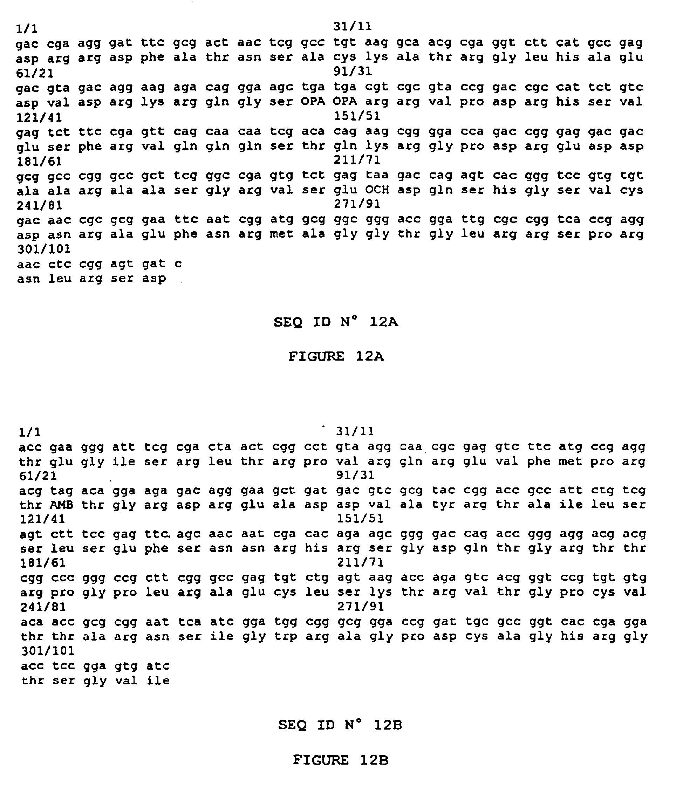

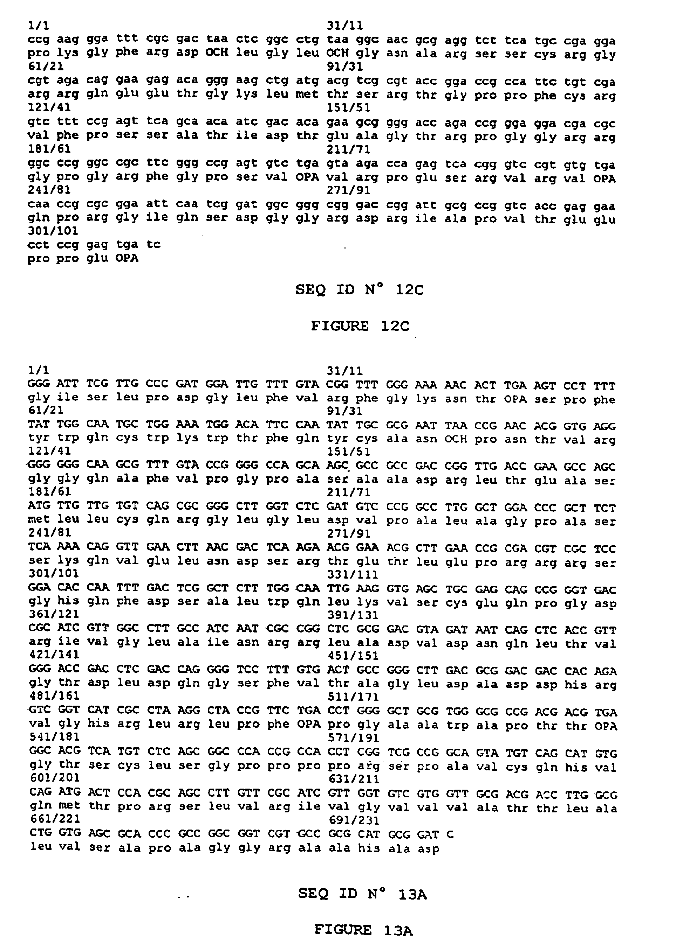

### La série de Figures 12 :

La série de Figures 12 représente la série de séquences nucléotidiques SEQ ID N°12.

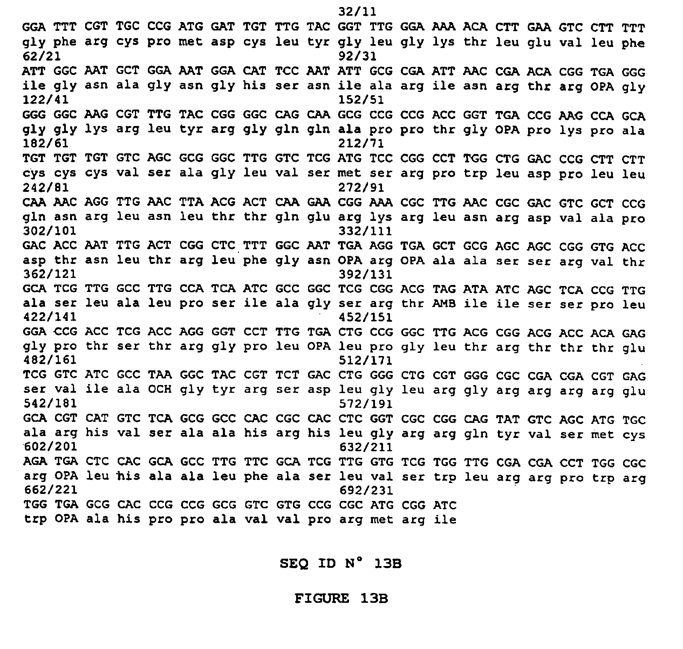

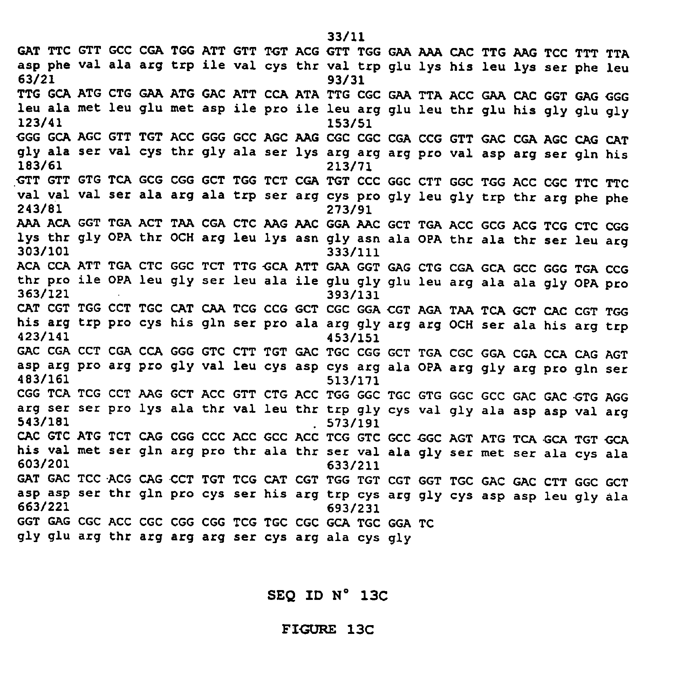

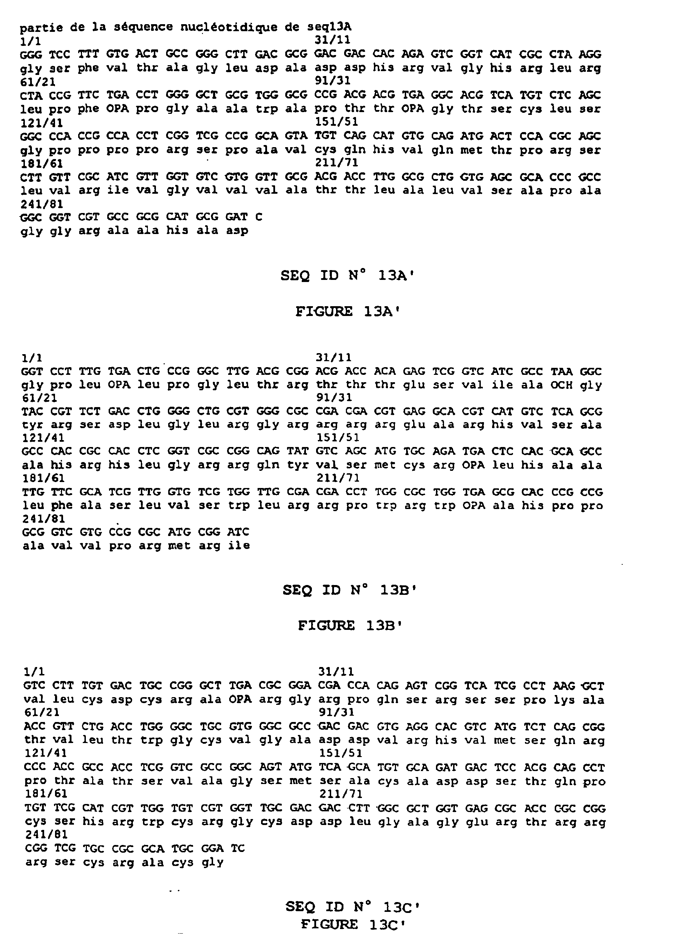

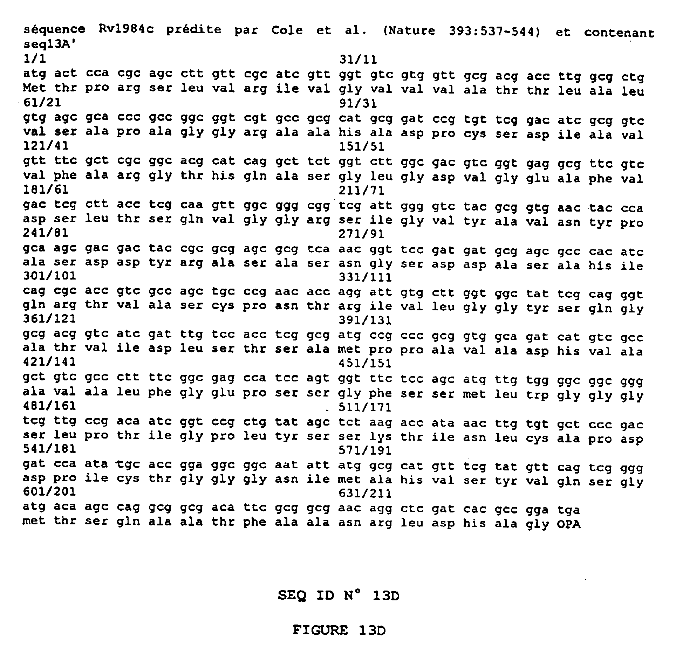

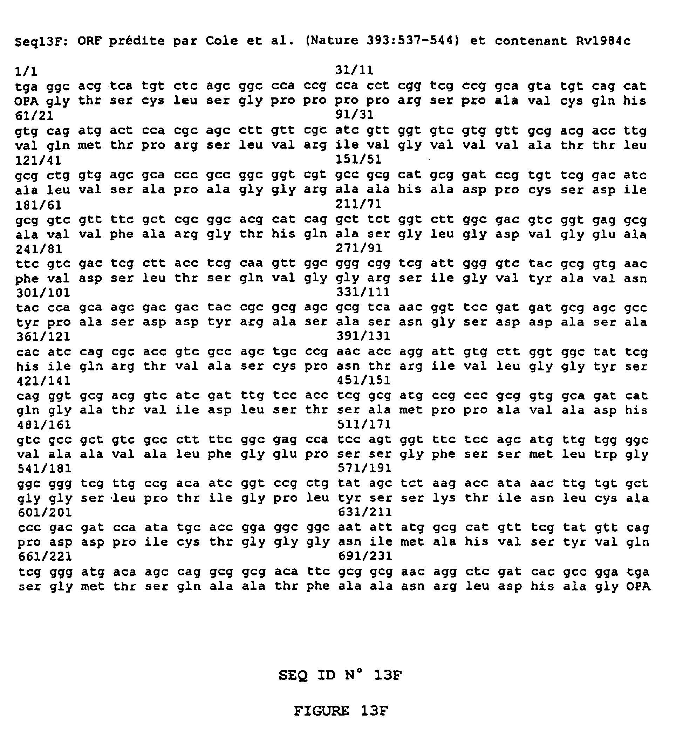

### La série de Figures 13 :

La série de Figures 13 représente la série de séquences nucléotidiques SEQ ID N°13.

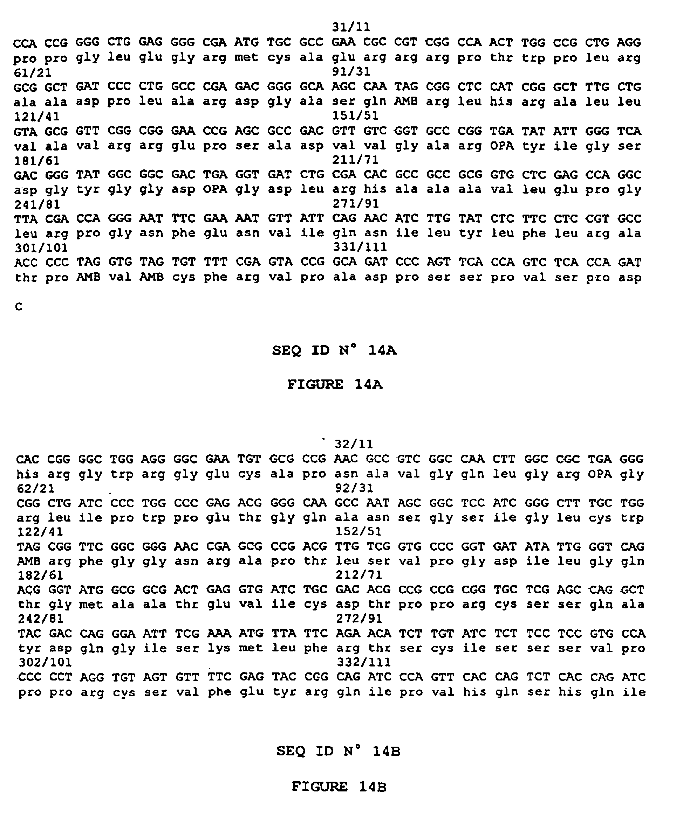

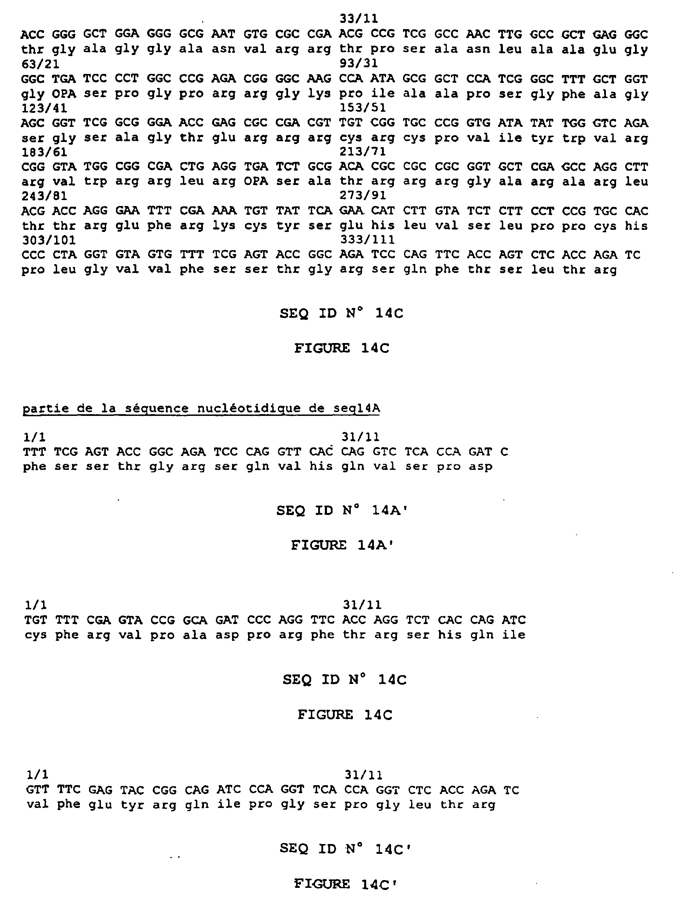

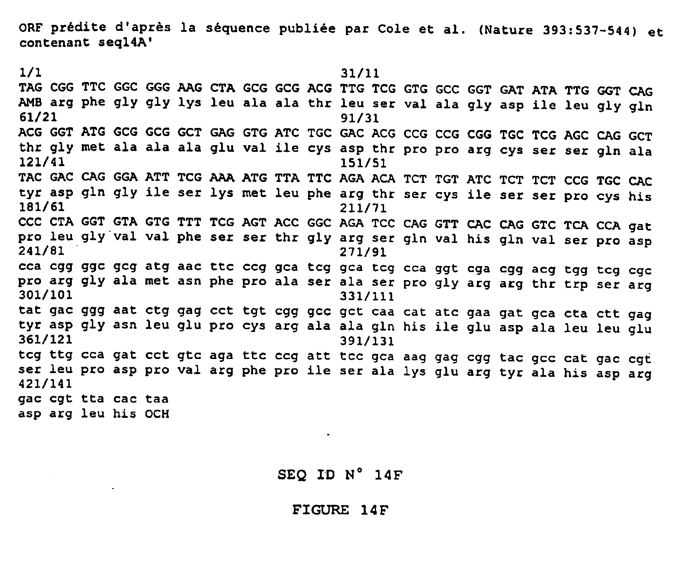

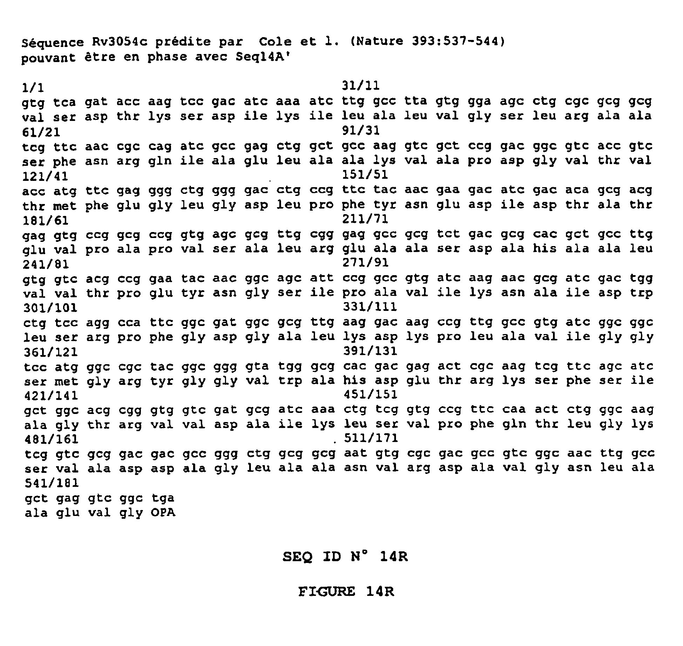

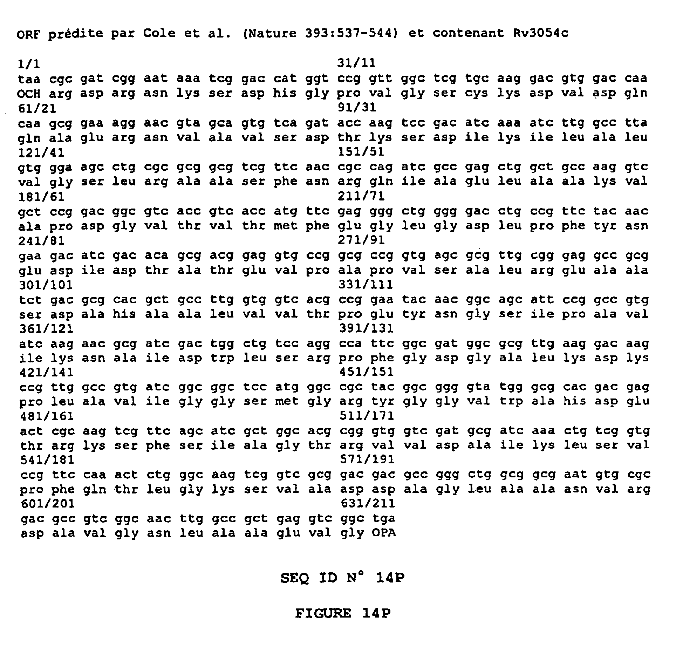

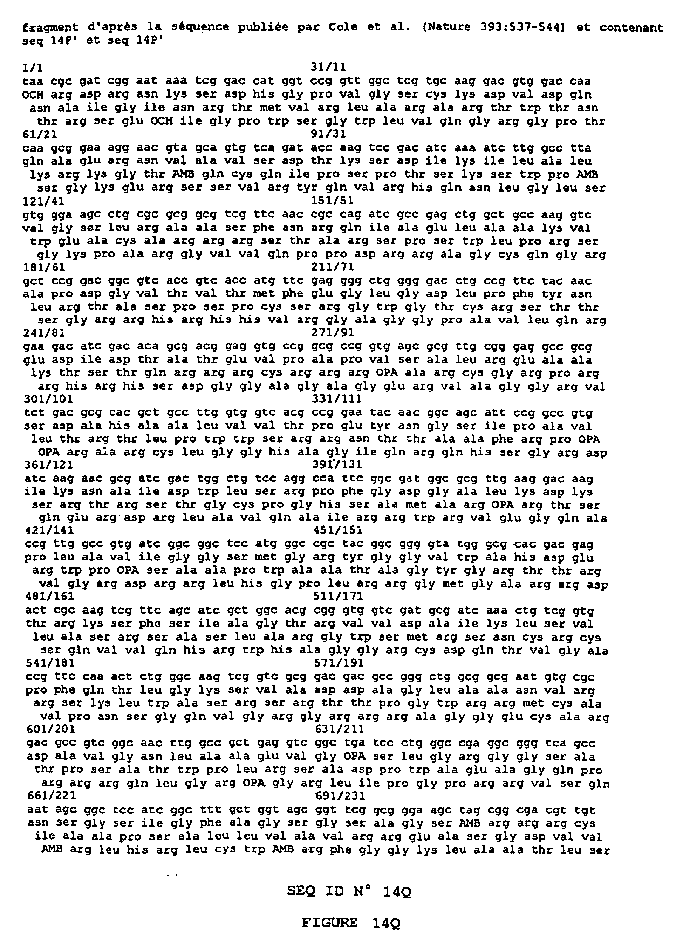

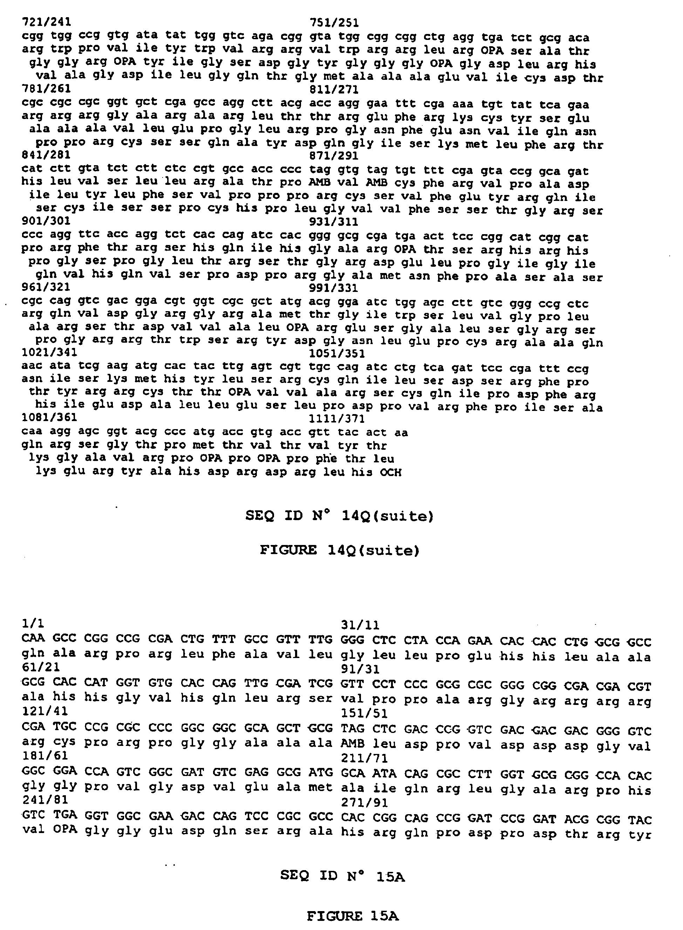

### La série de Figures 14 :

La série de Figures 14 représente la série de séquences nucléotidiques SEQ ID N°14 correspondant à l'insert du vecteur p5B5 (déposé à la CNCM sous le N° I-1819).

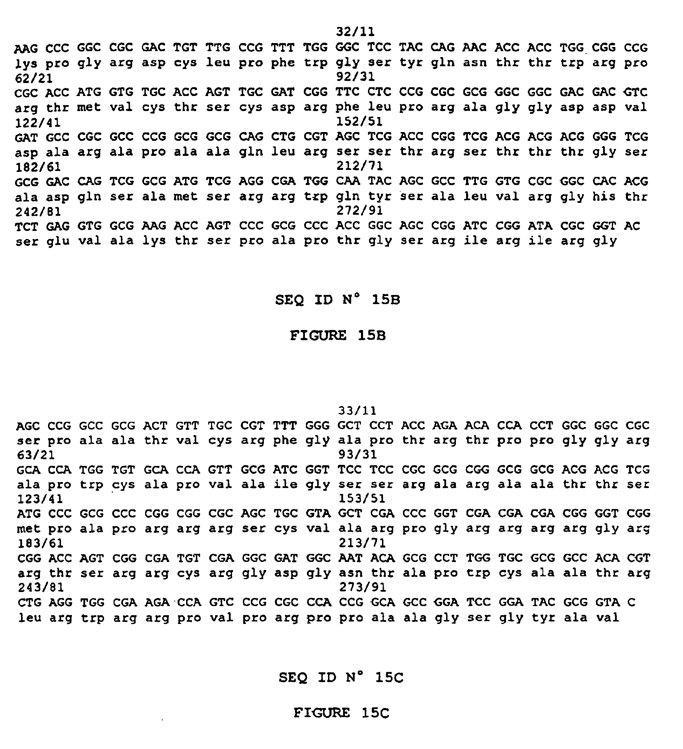

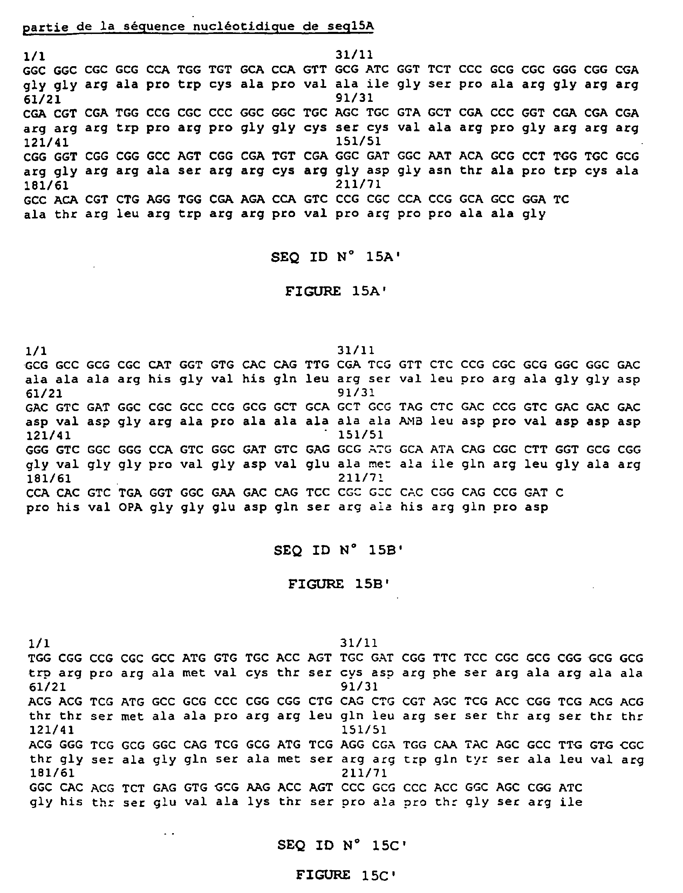

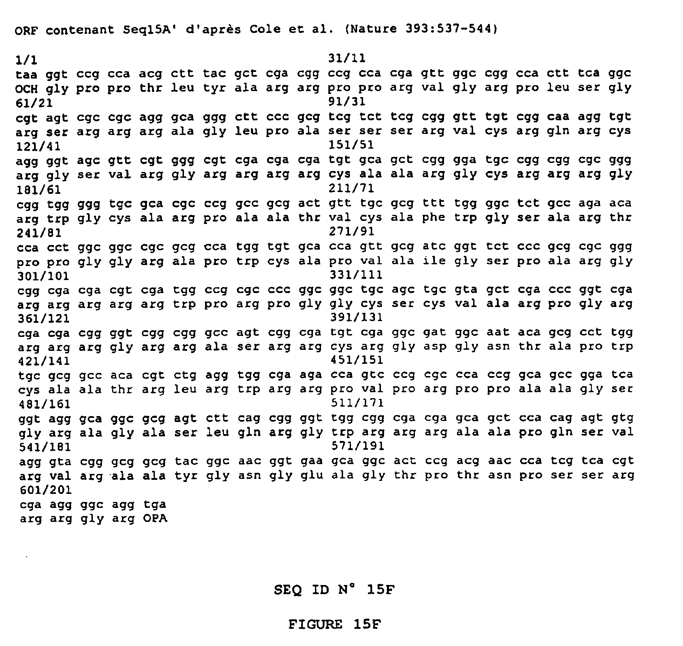

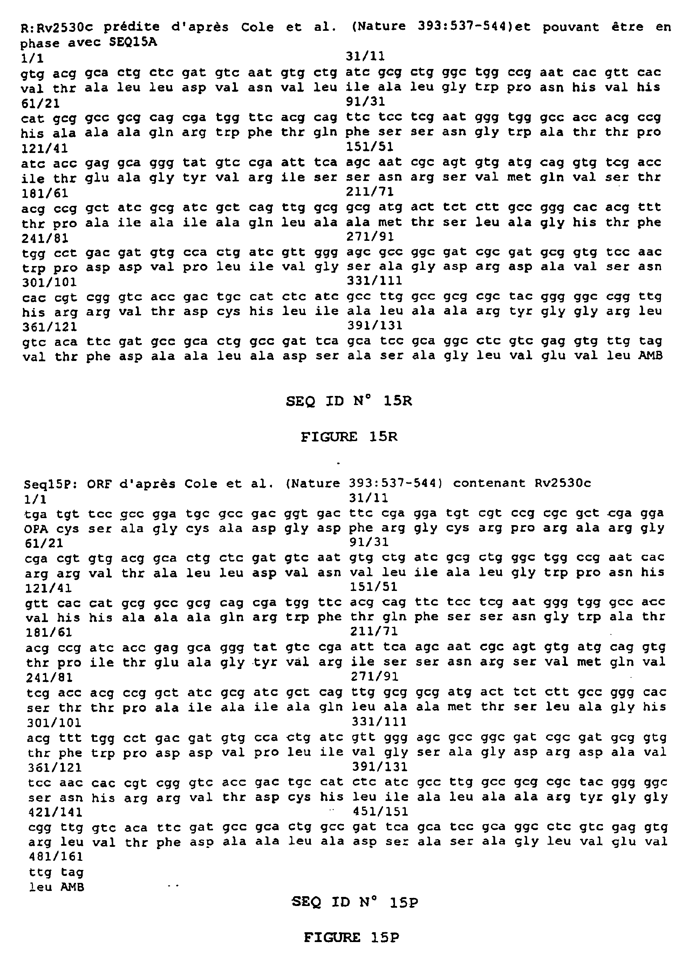

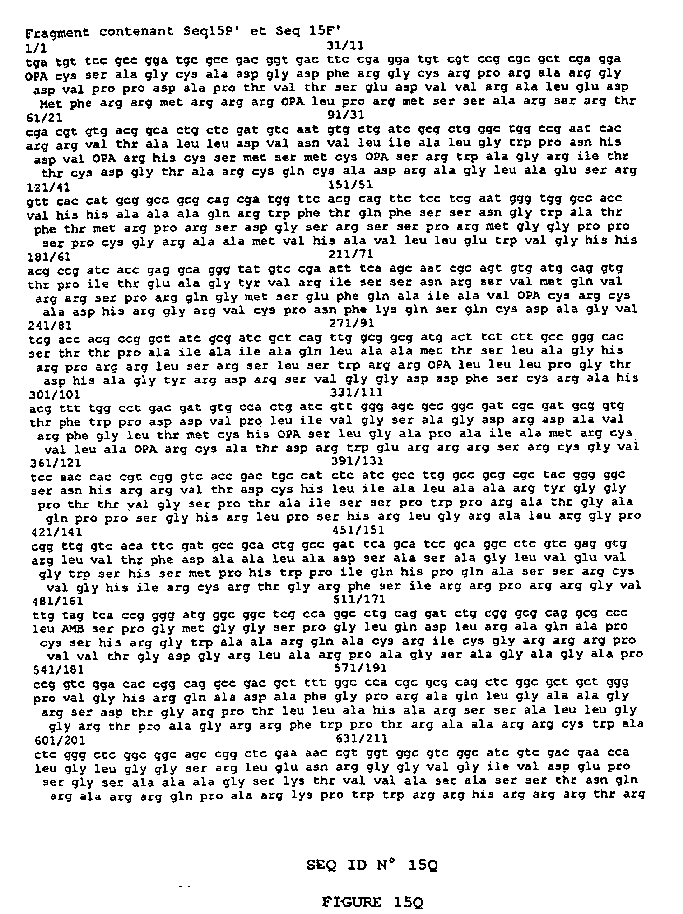

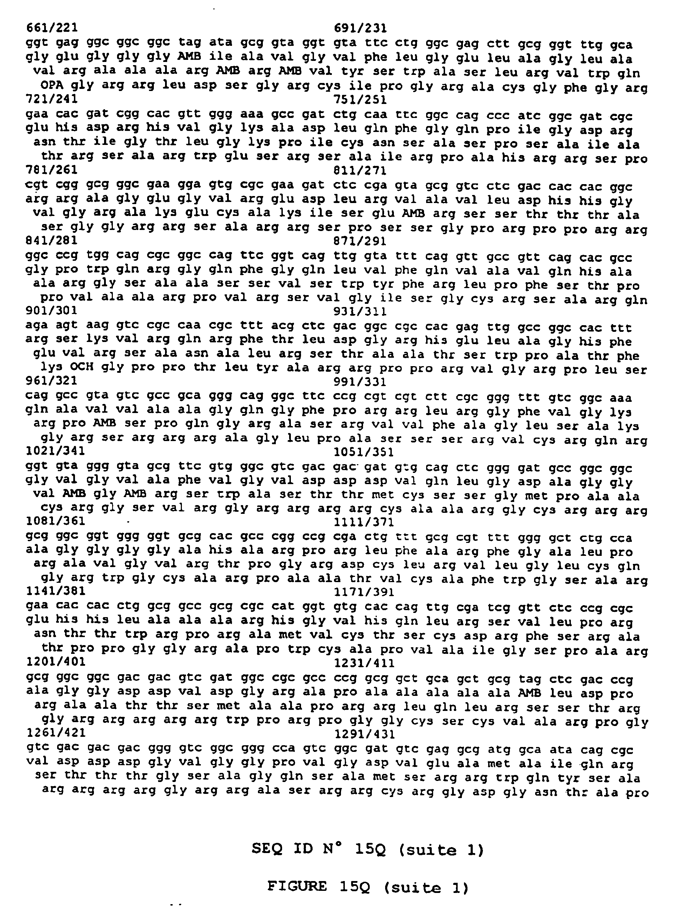

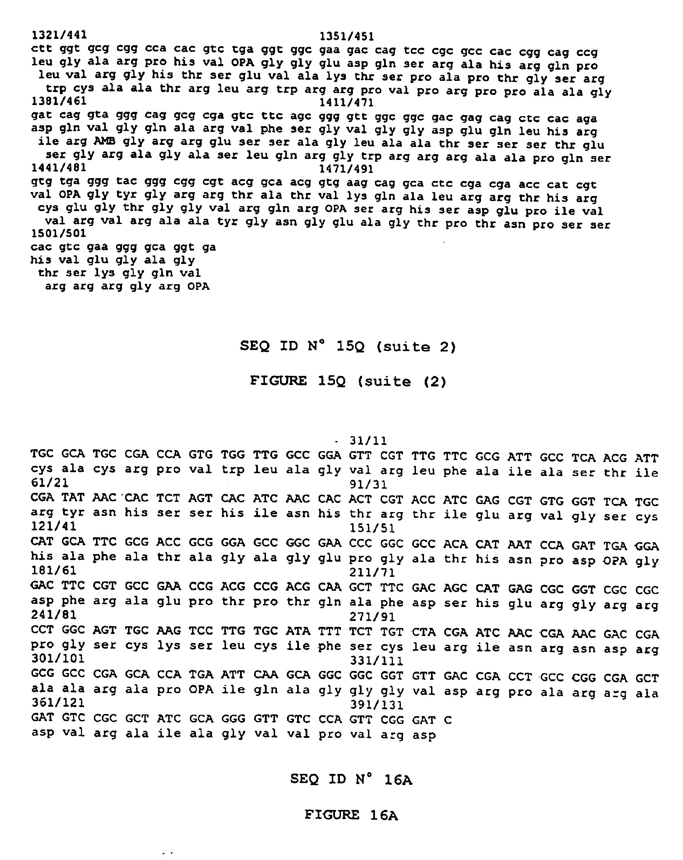

### La série de Figures 15 :

La série de Figures 15 représente la série de séquences nucléotidiques SEQ ID N°15.

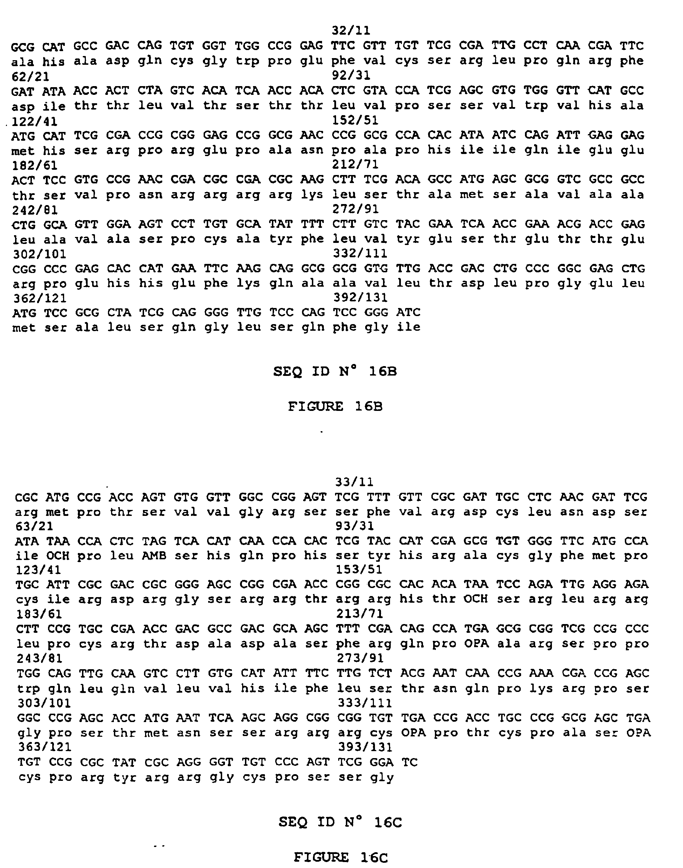

### La série de Figures 16 :

La série de Figures 16 représente la série de séquences nucléotidiques SEQ ID N°16.

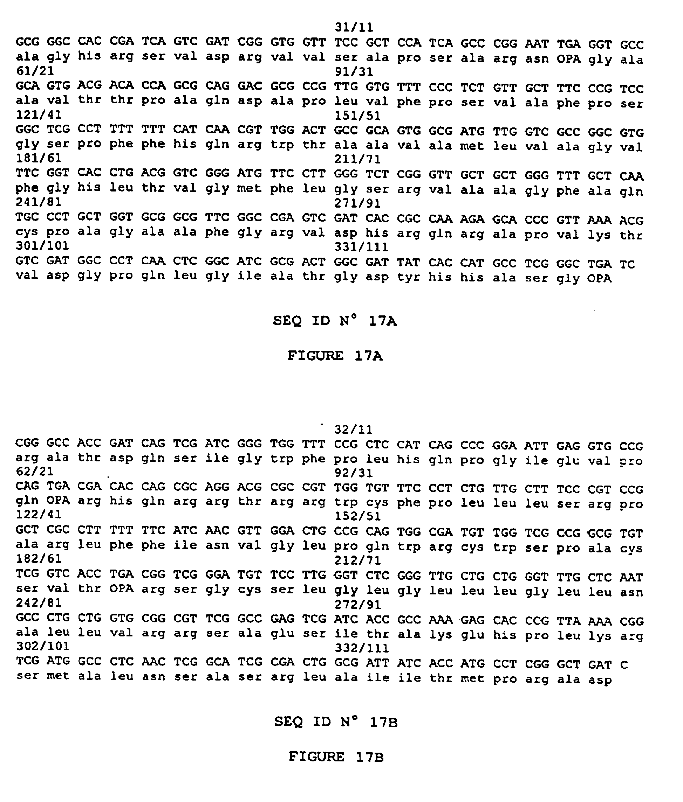

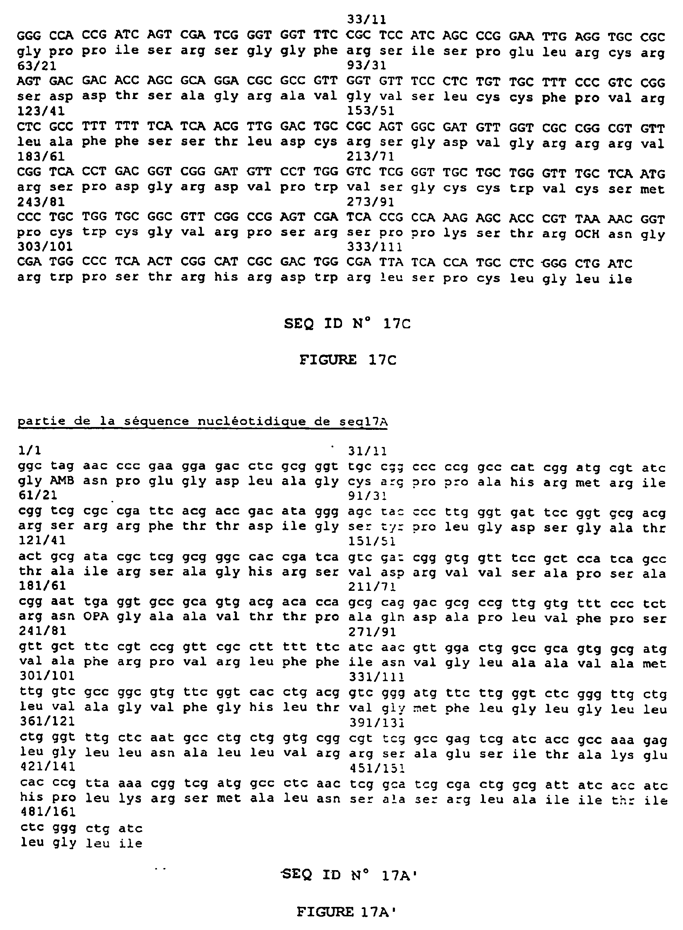

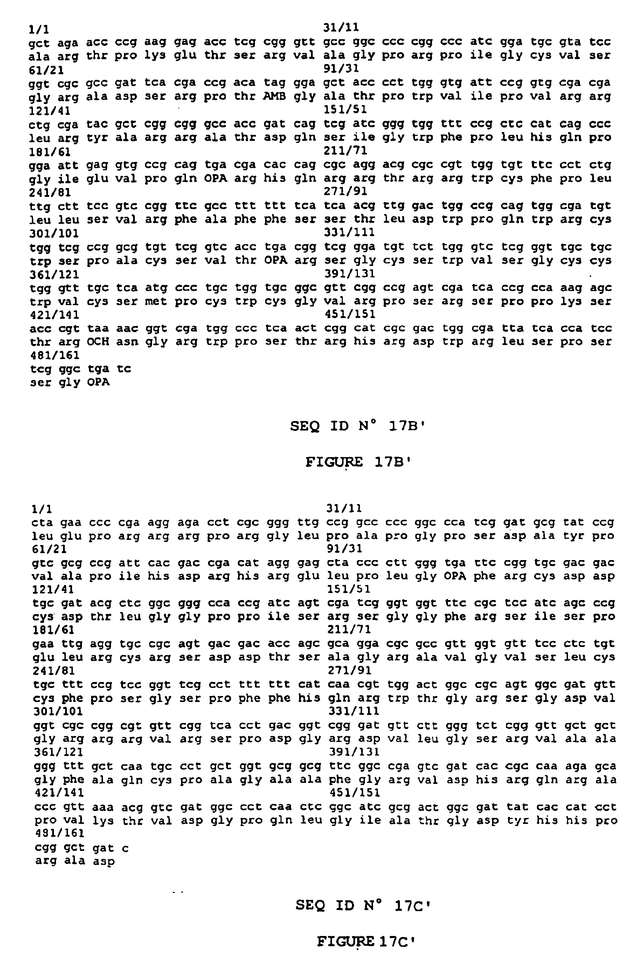

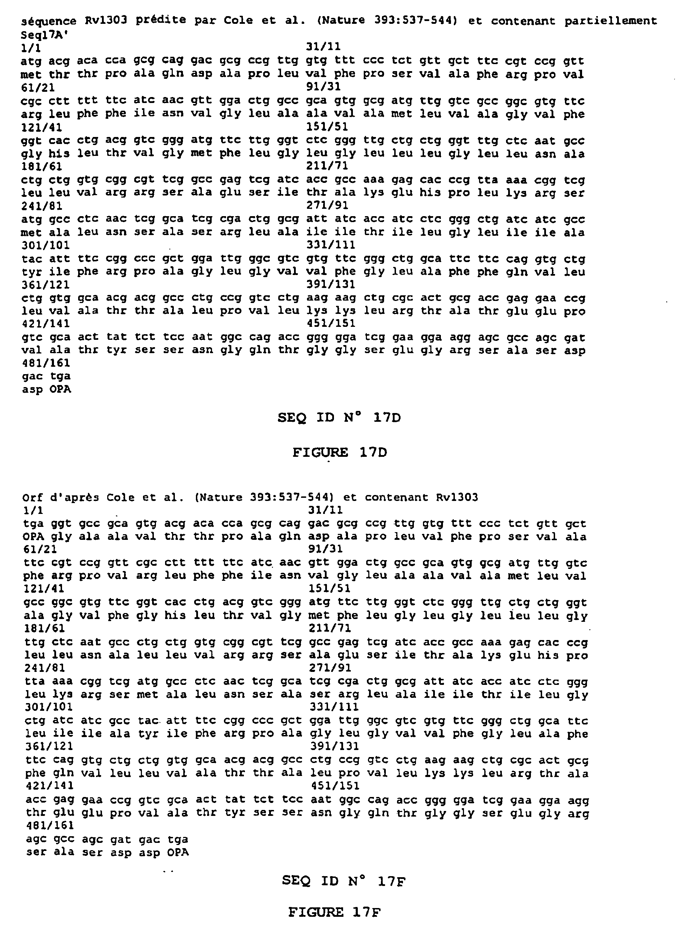

### La série de Figures 17 :

La série de Figures 17 représente la série de séquences nucléotidiques SEQ ID N°17.

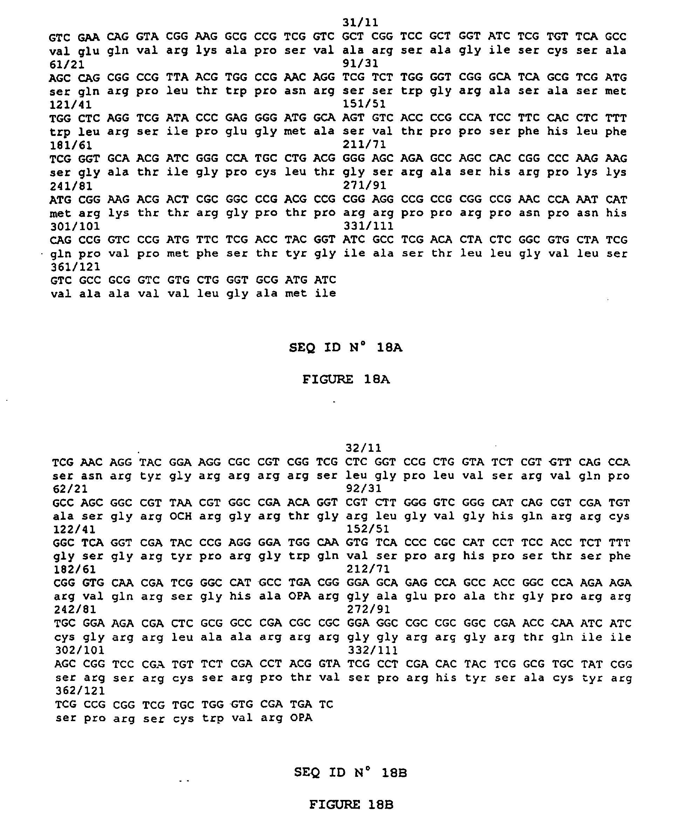

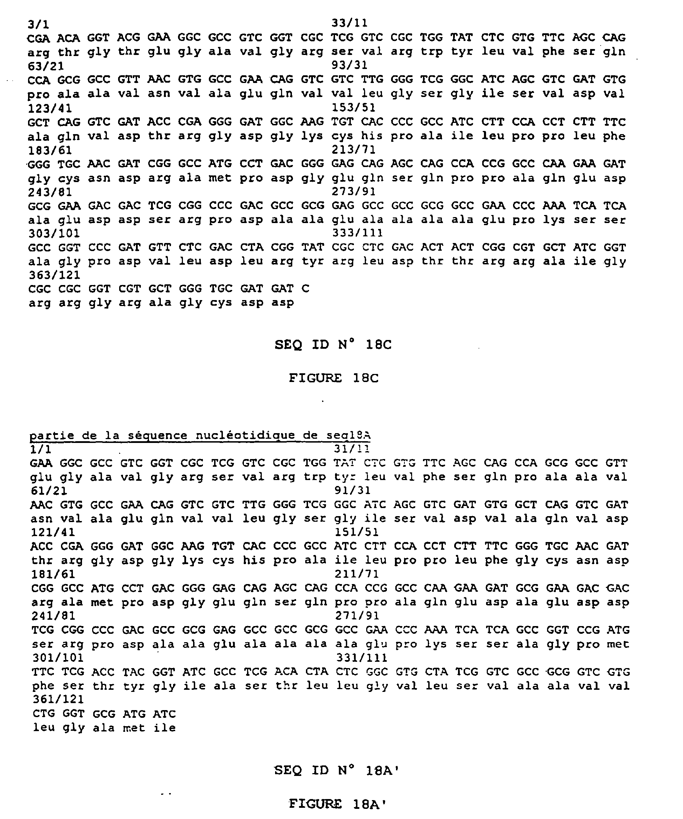

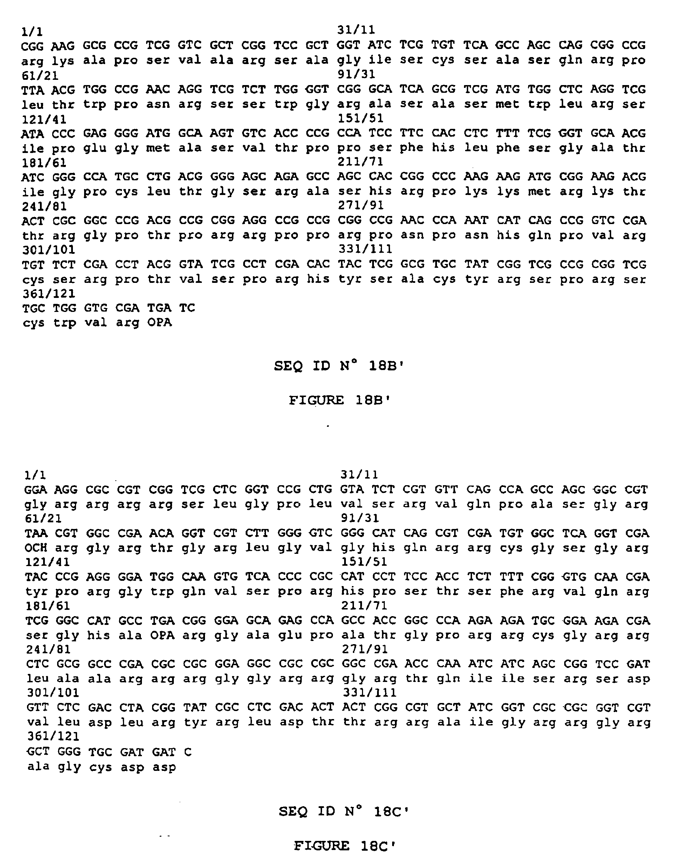

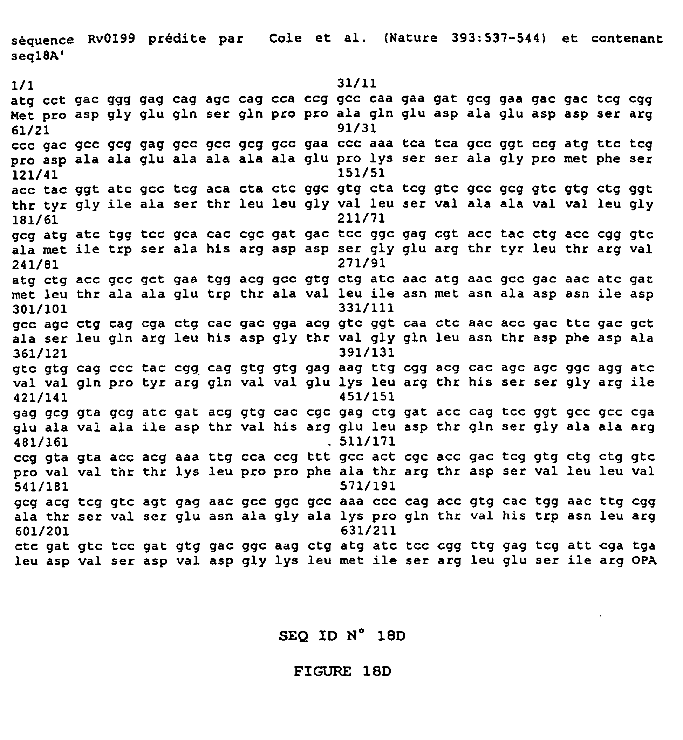

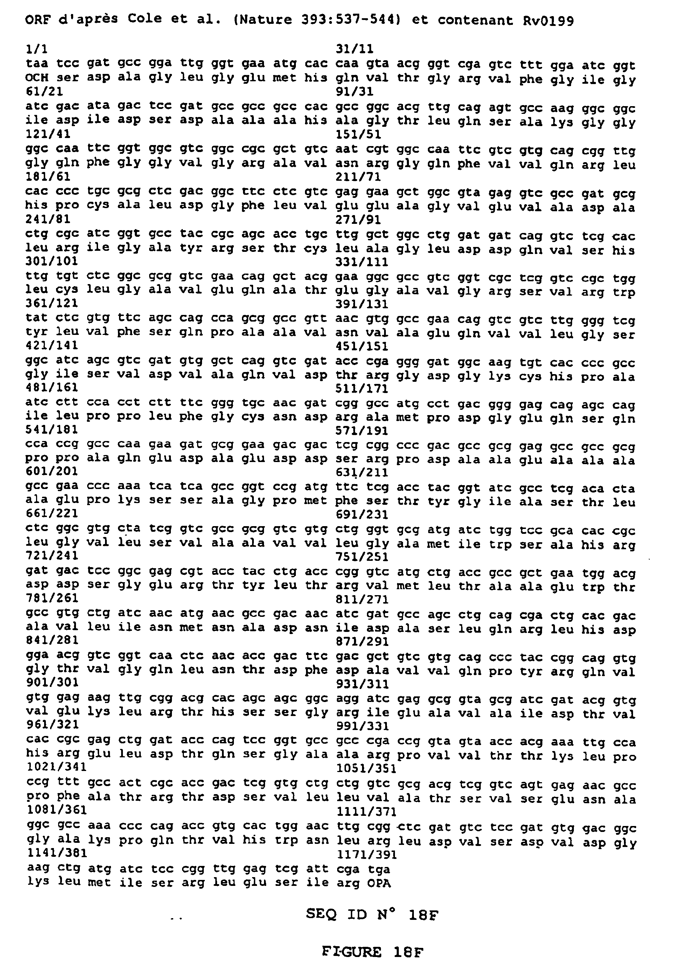

### La série de Figures 18 :

La série de Figures 18 représente la série de séquences nucléotidiques SEQ ID N°18.

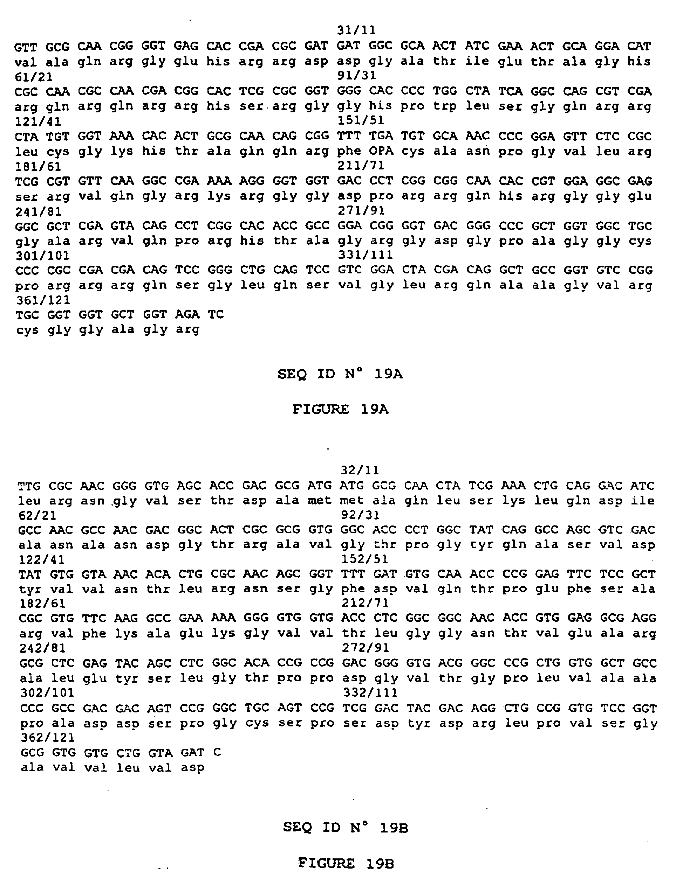

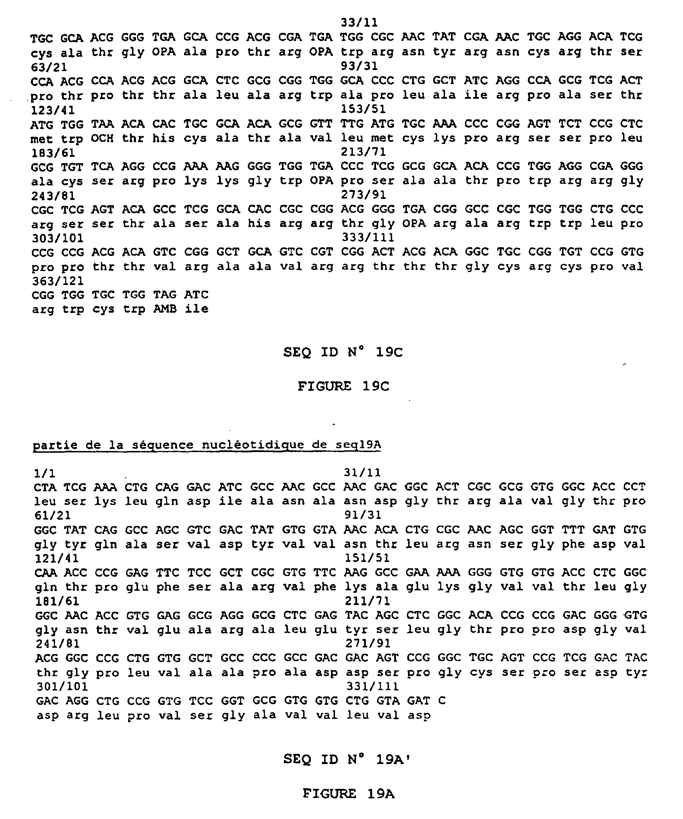

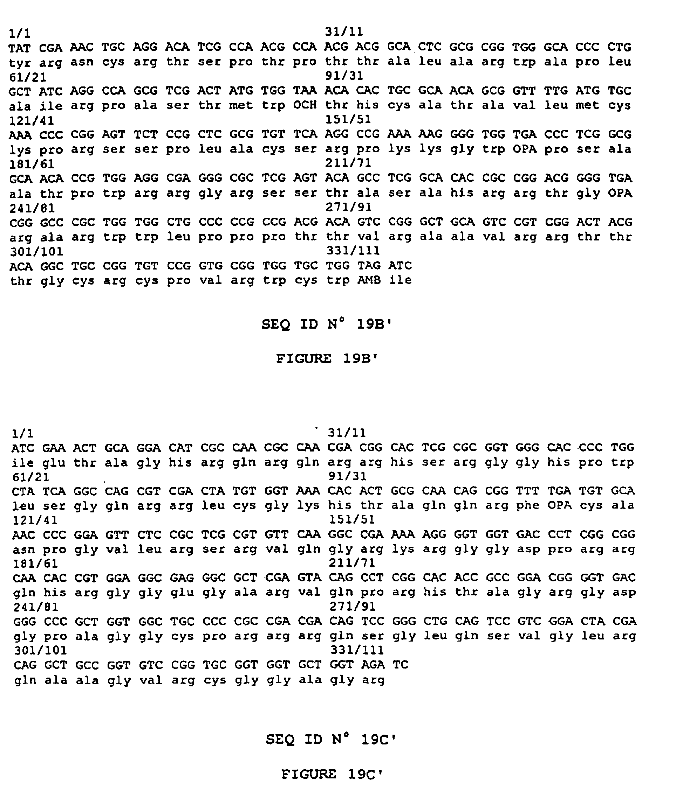

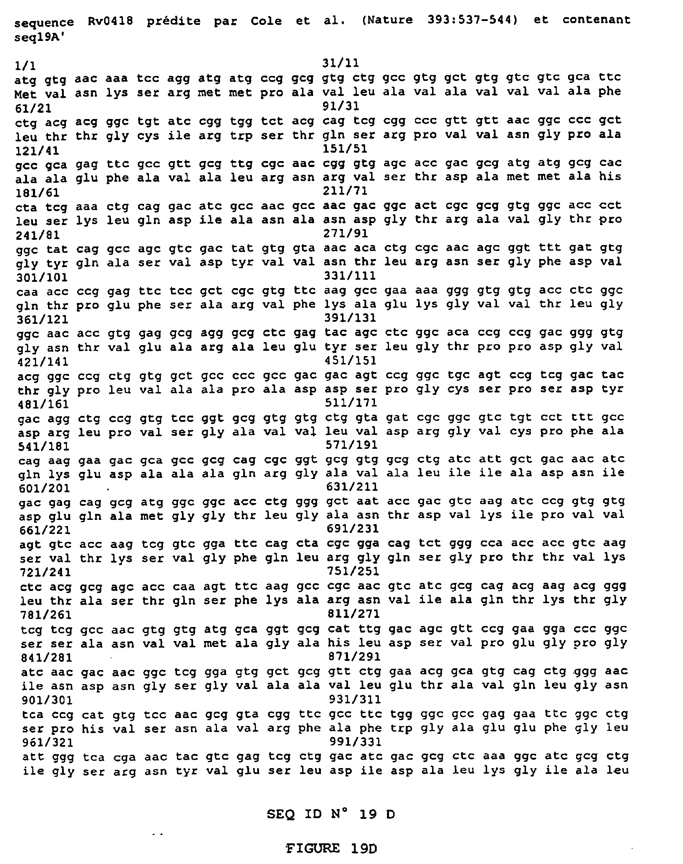

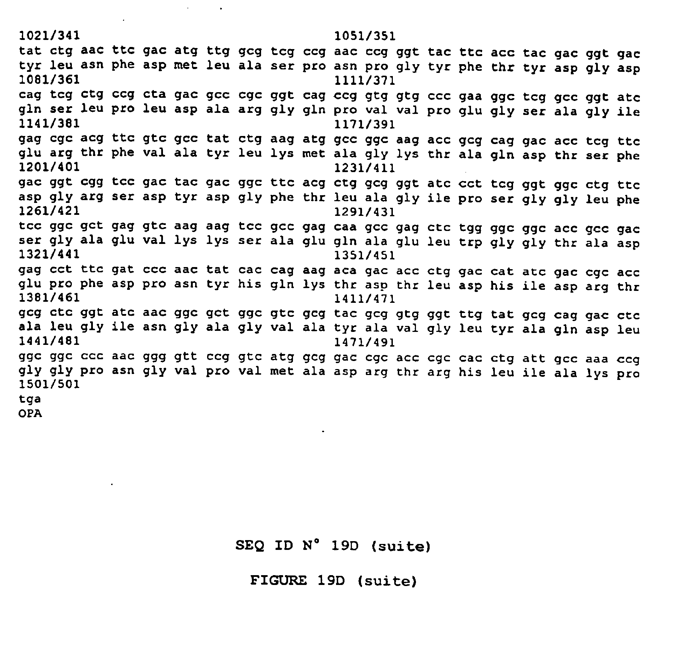

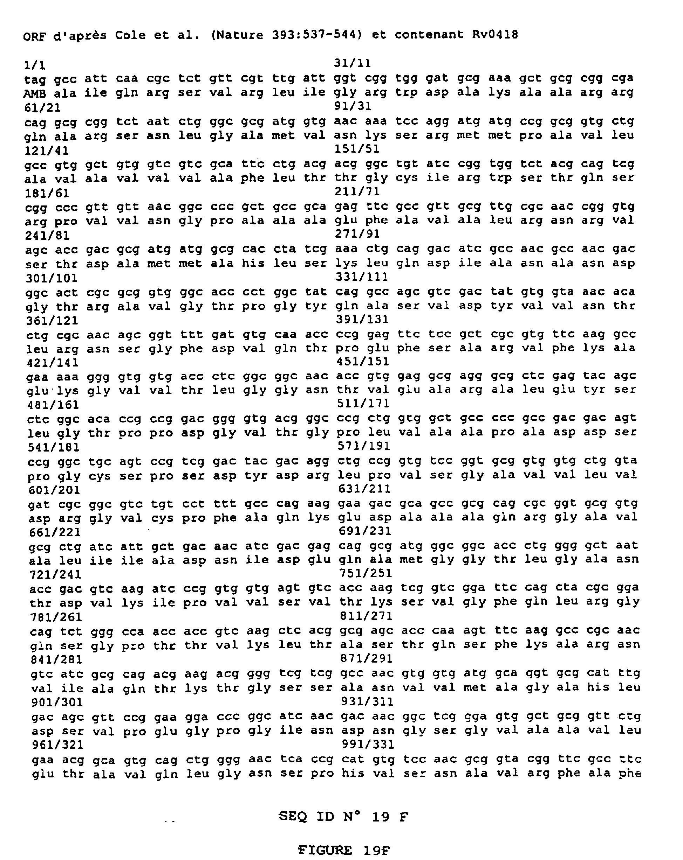

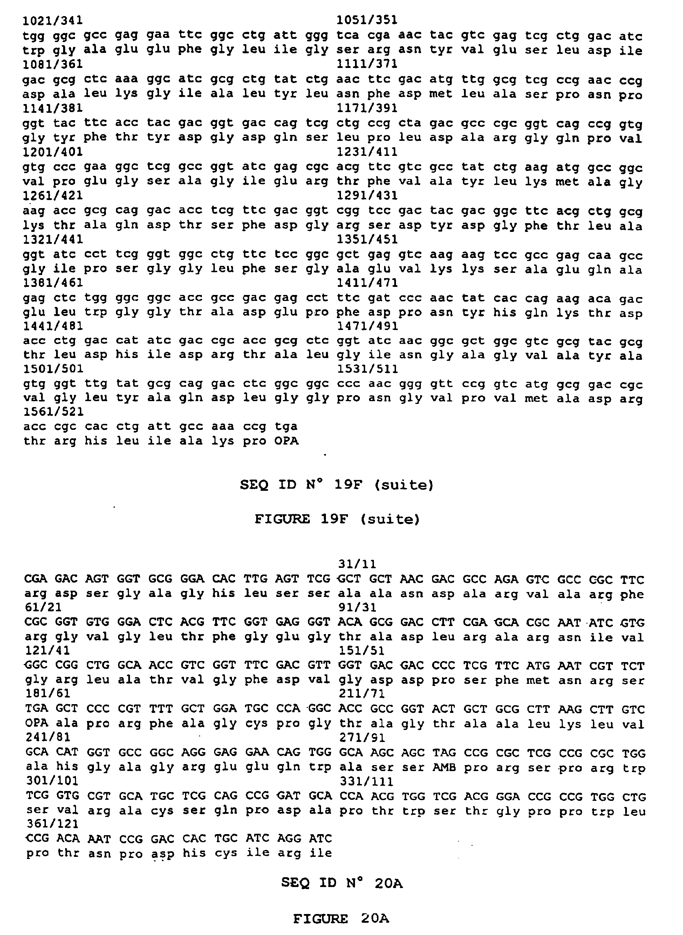

### La série de Figures 19 :

La série de Figures 19 représente la série de séquences nucléotidiques SEQ ID N°19.

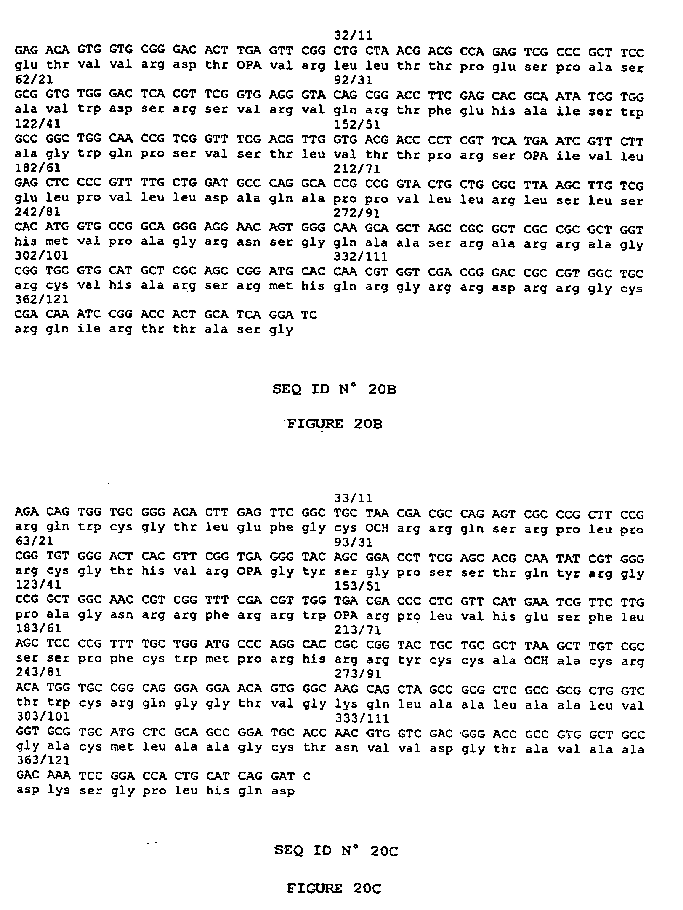

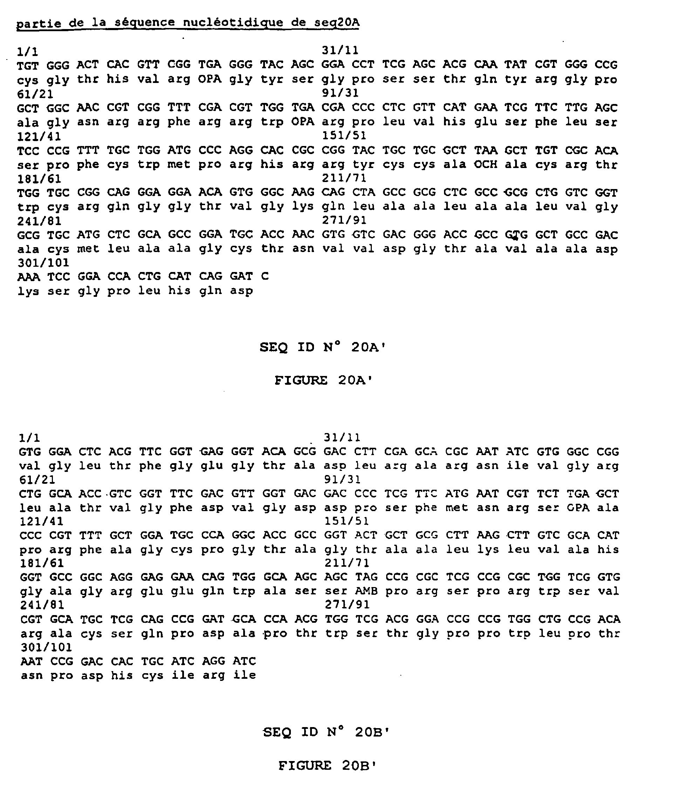

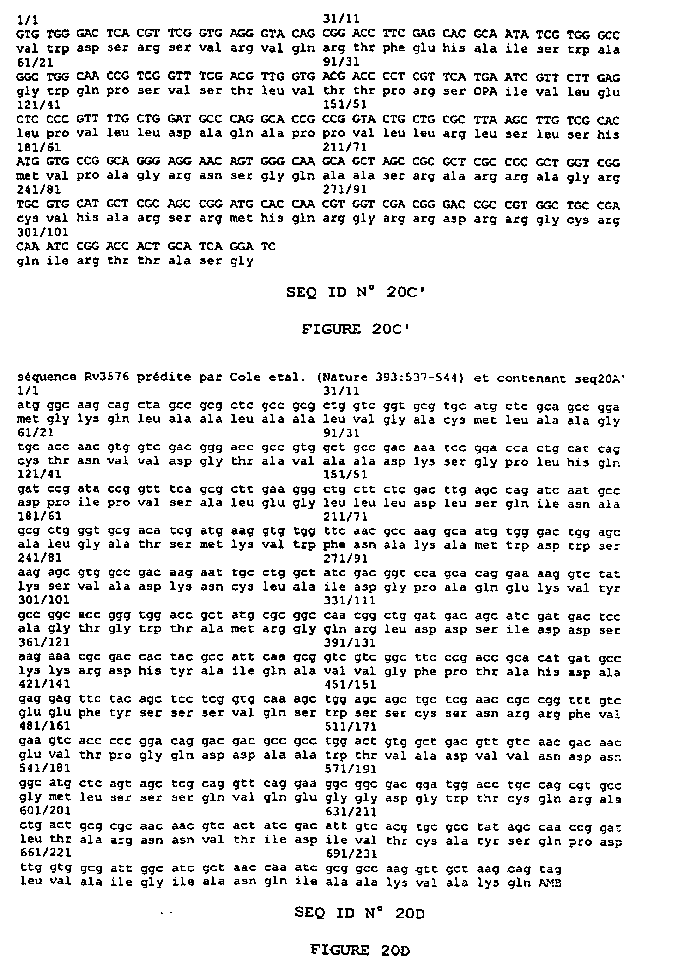

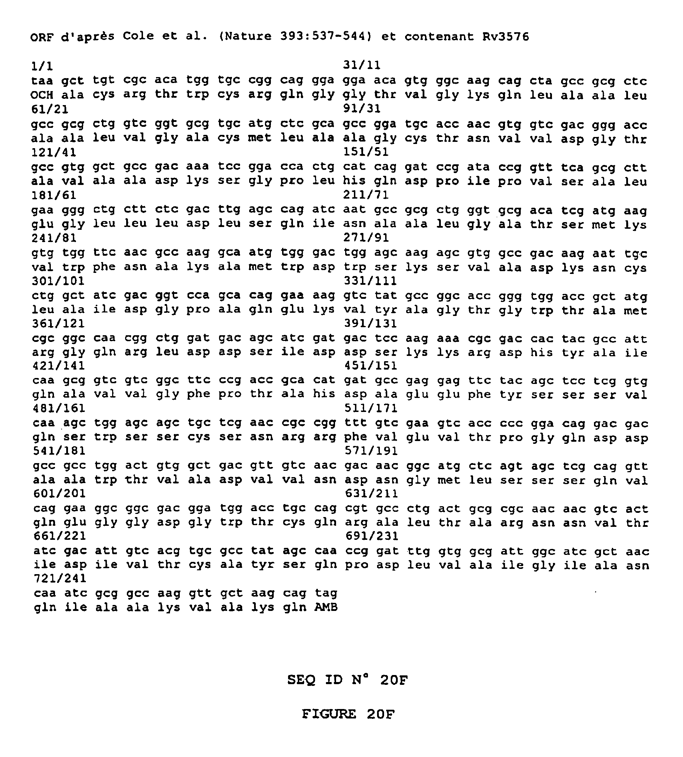

### La série de Figures 20 :

La série de Figures 20 représente la série de séquences nucléotidiques SEQ ID N°20 correspondant à l'insert du vecteur p2A29 (déposé à la CNCM sous le N° I-1817).

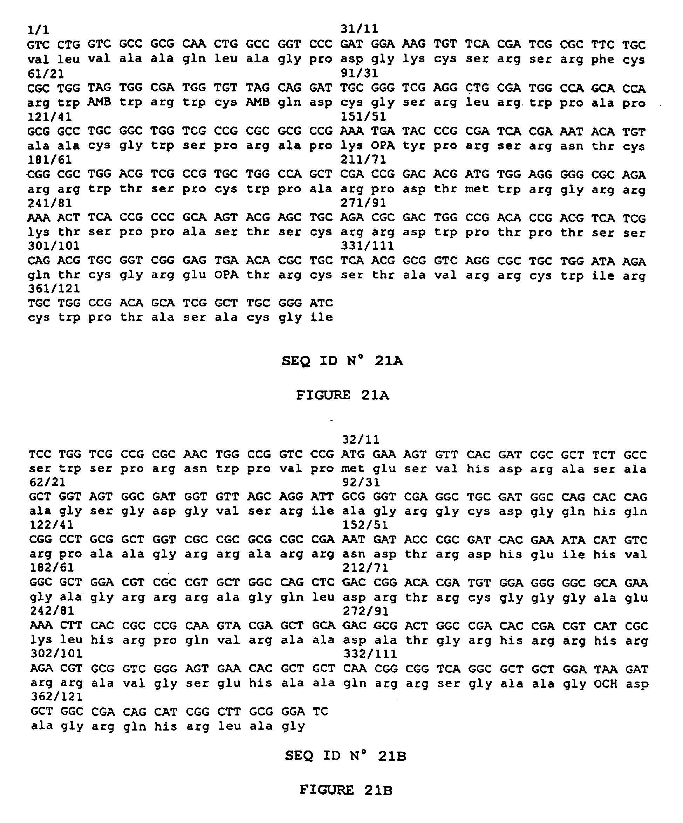

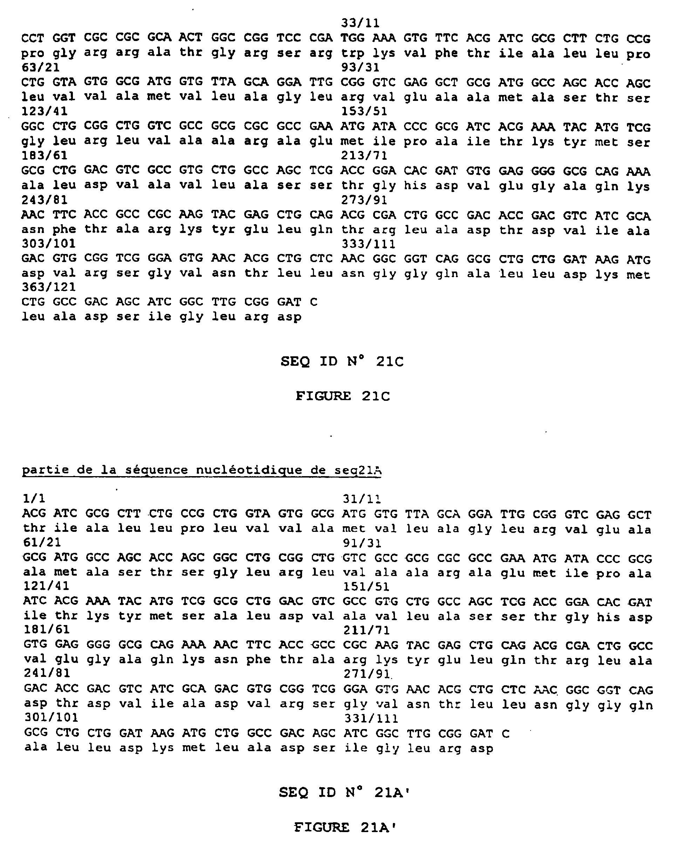

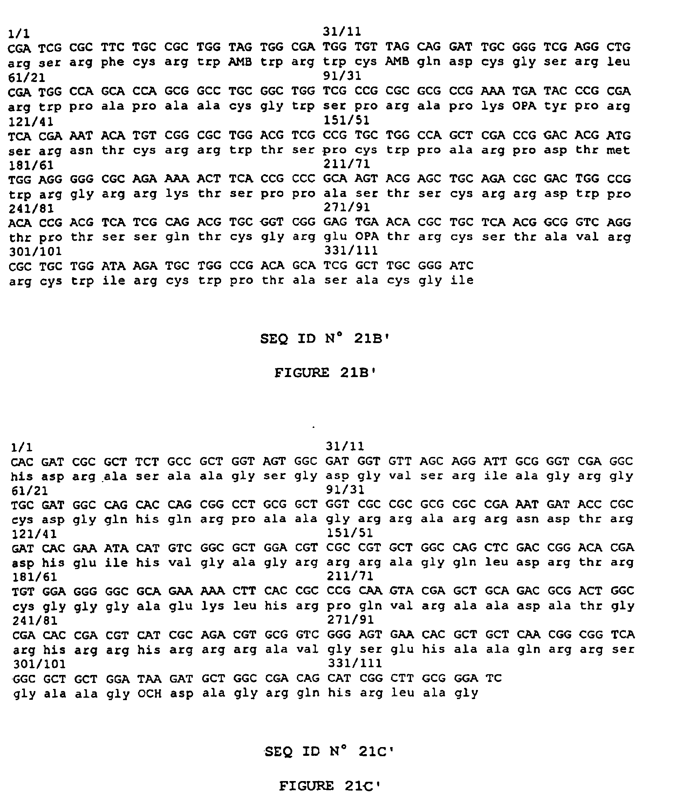

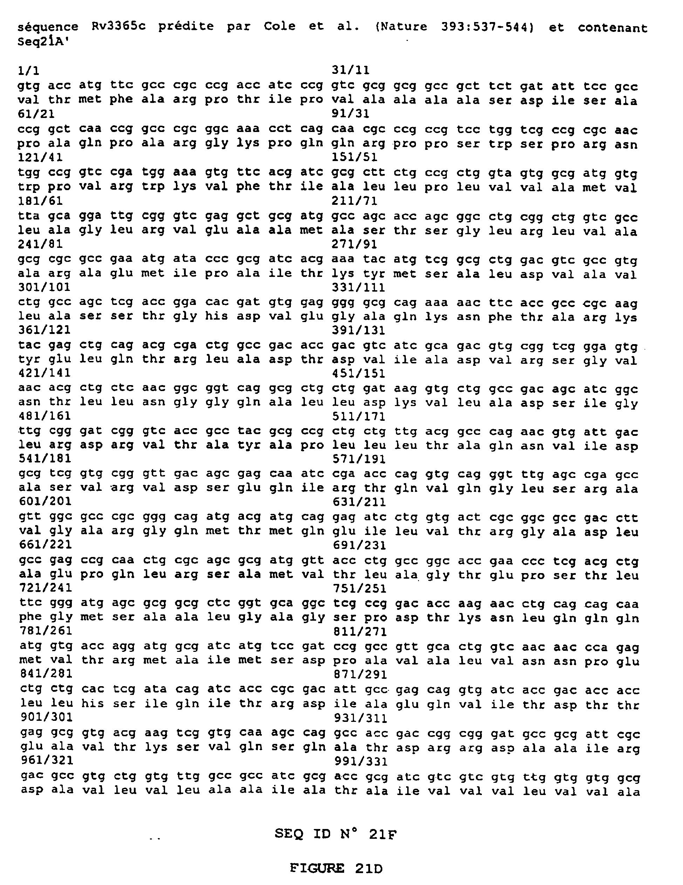

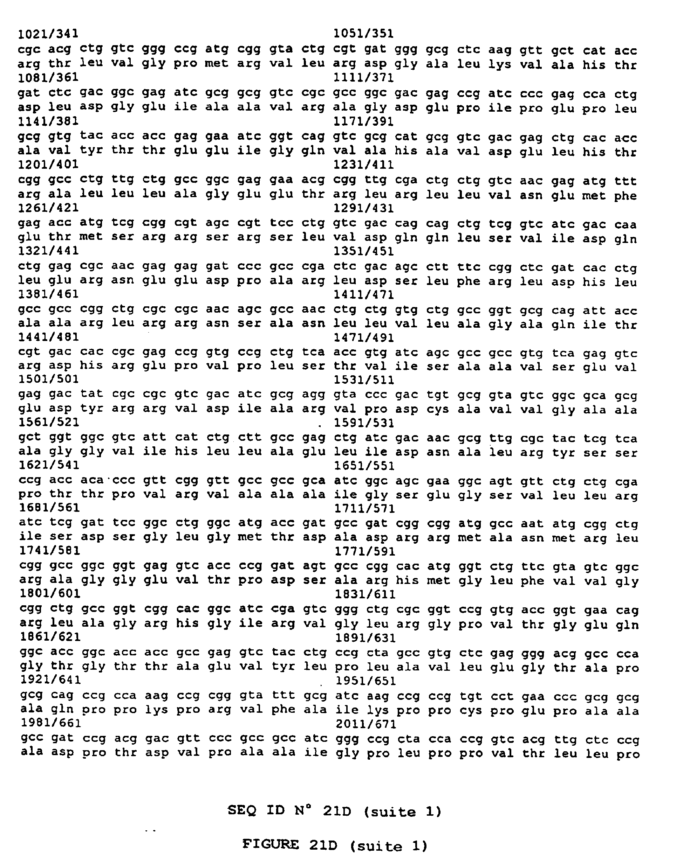

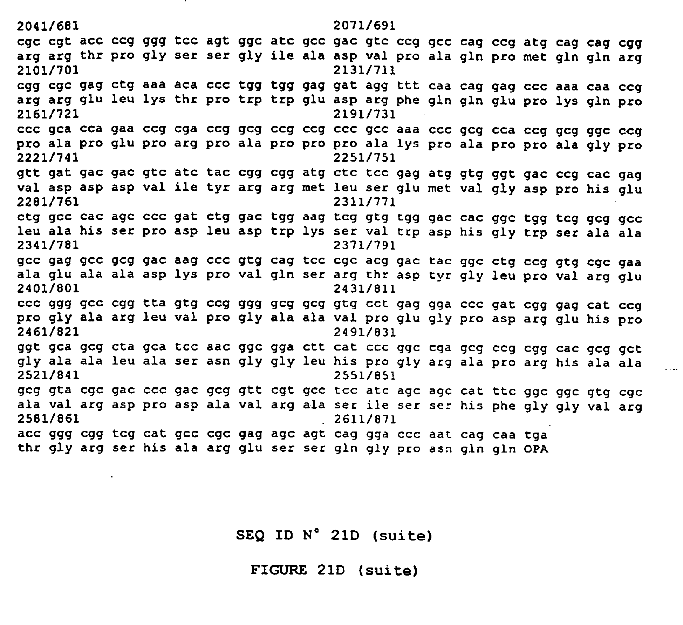

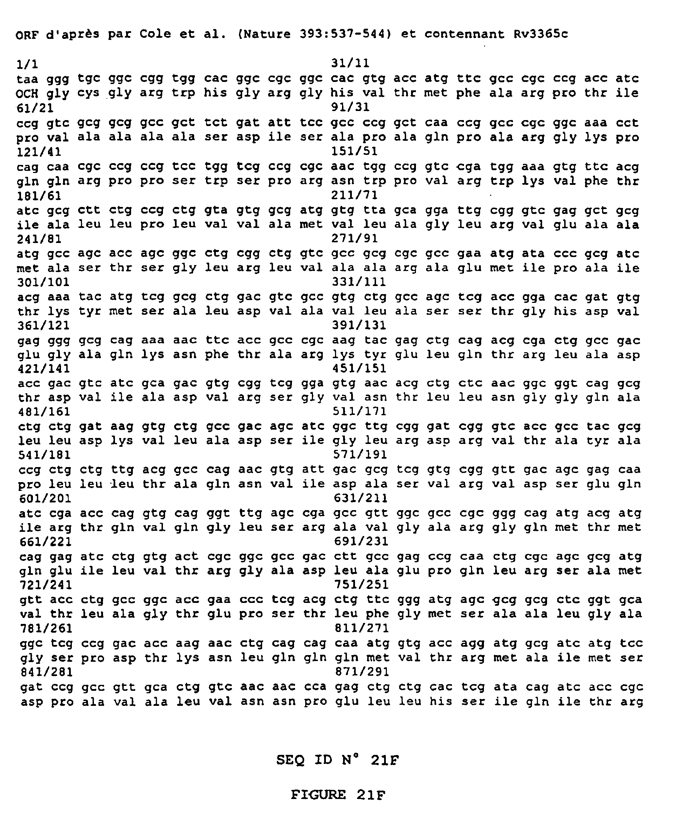

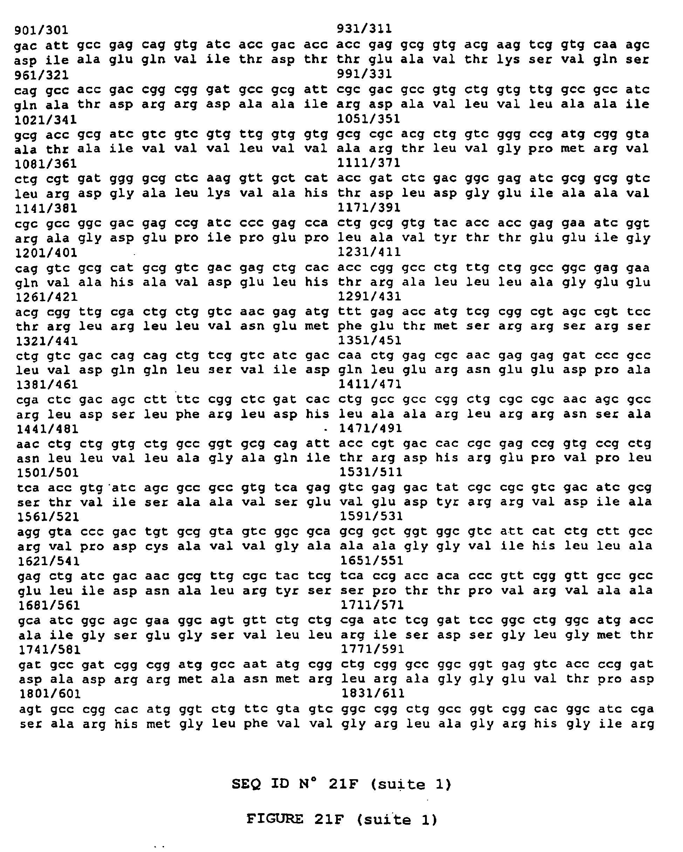

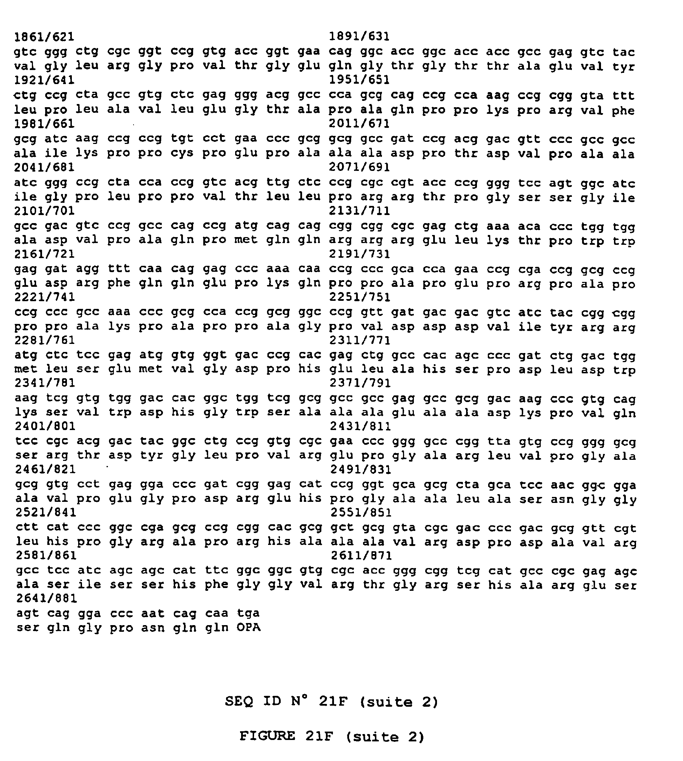

### La série de Figures 21 :

La série de Figures 21 représente la série de séquences nucléotidiques SEQ ID N°21.

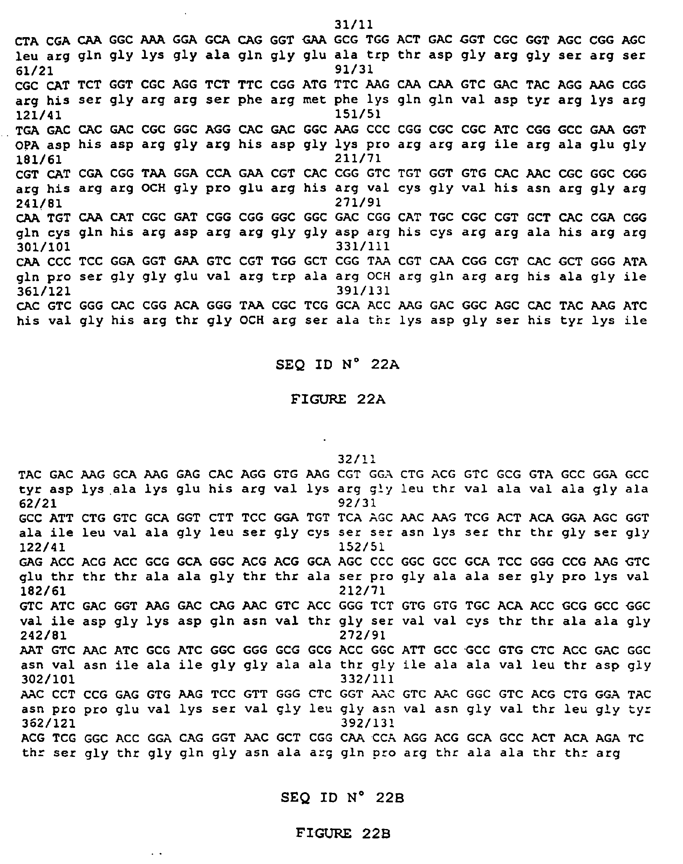

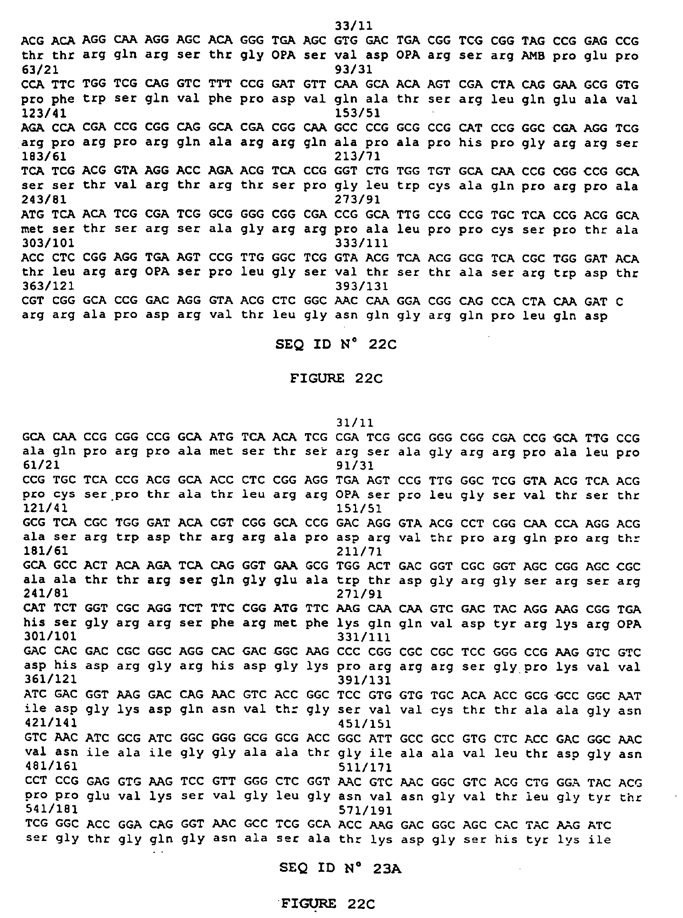

### La série de Figures 22 :

La série de Figures 22 représente la série de séquences nucléotidiques SEQ ID N°22.

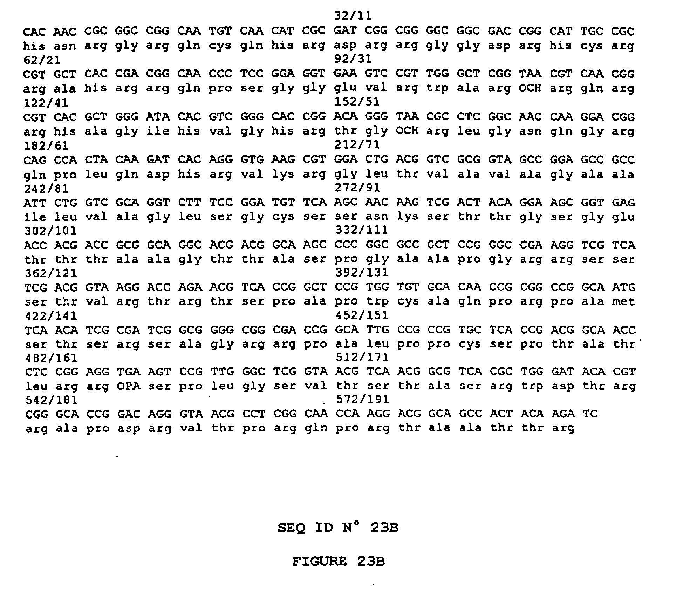

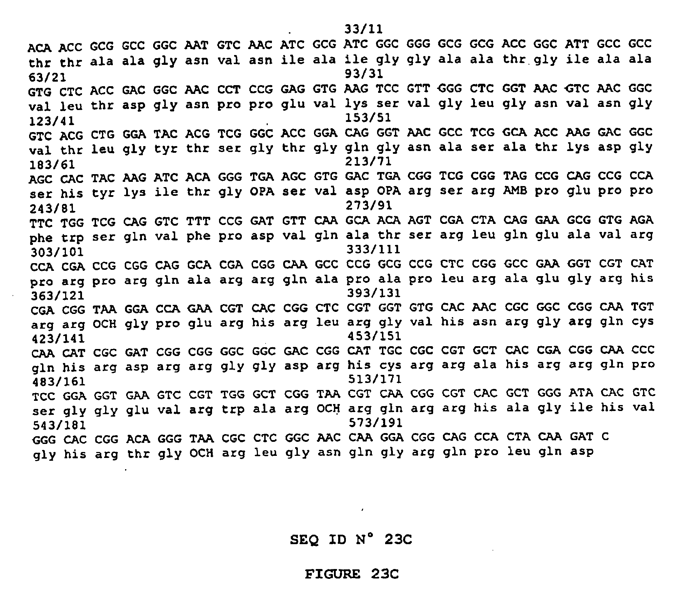

### La série de Figures 23 :

La série de Figures 23 représente la série de séquences nucléotidiques SEQ ID N°23.

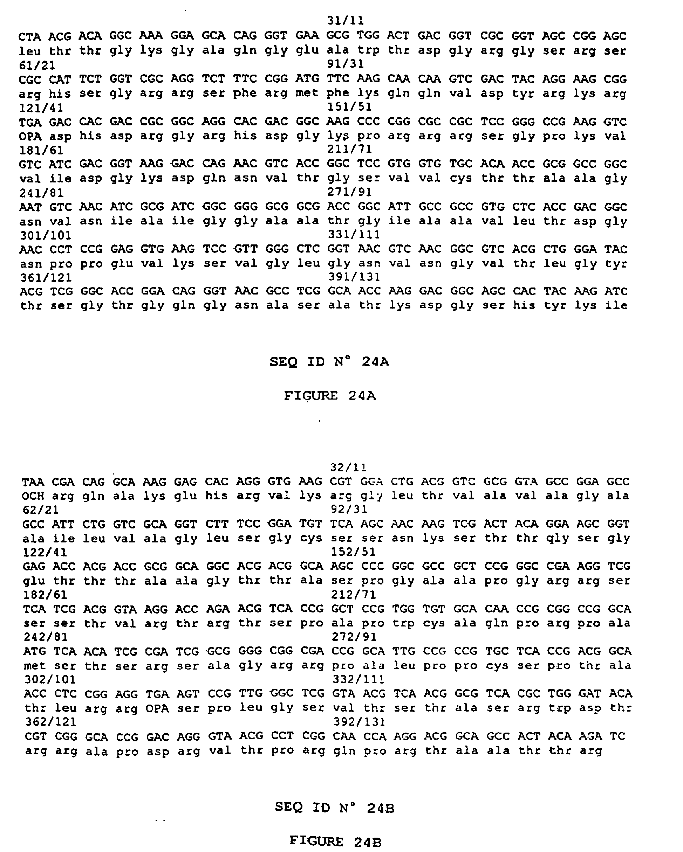

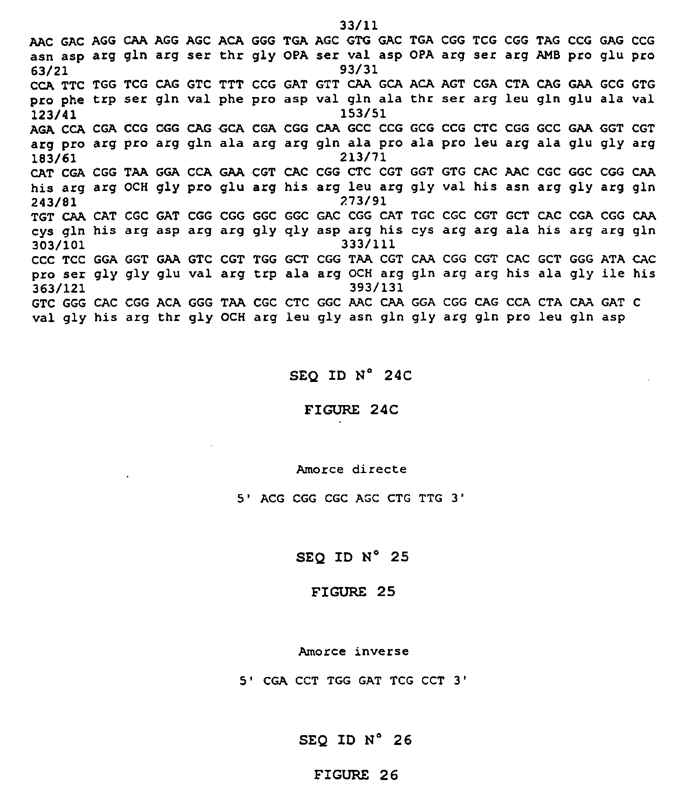

### La série de Figures 24 :

La série de Figures 24 représente la série de séquences nucléotidiques SEQ ID N°24.

### Figures 25 et 26 :

Les figures 25 et 26 illustrent respectivement les séquences SEQ ID N°25 et SEQ ID N°26 représentant un couple d'amorces utilisées pour amplifier spécifiquement par PCR la région correspondant aux nucléotides 964 à 1234 inclus dans la séquence SEQ ID N°1.

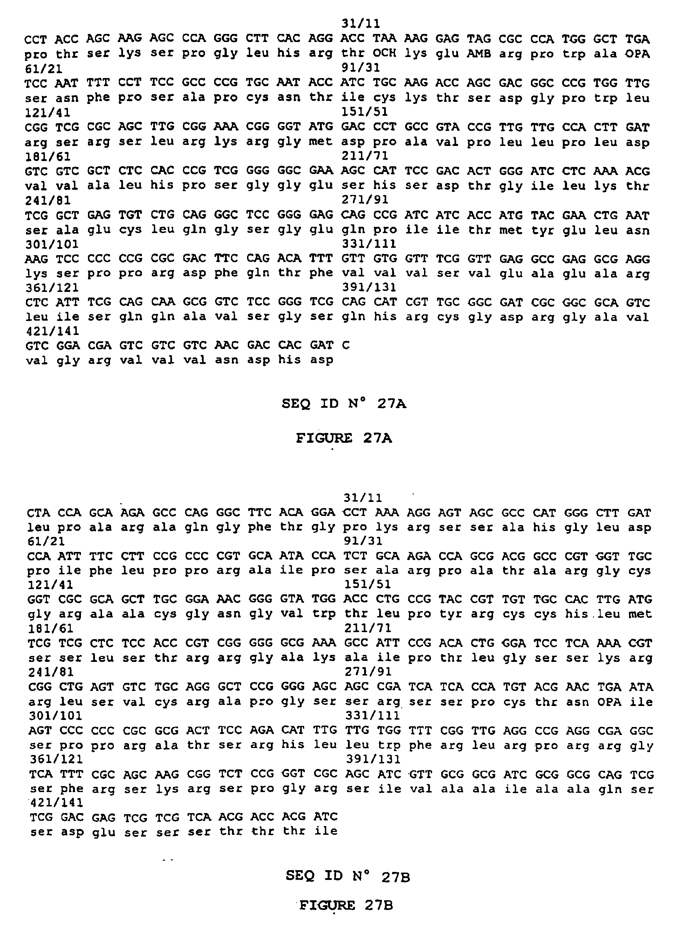

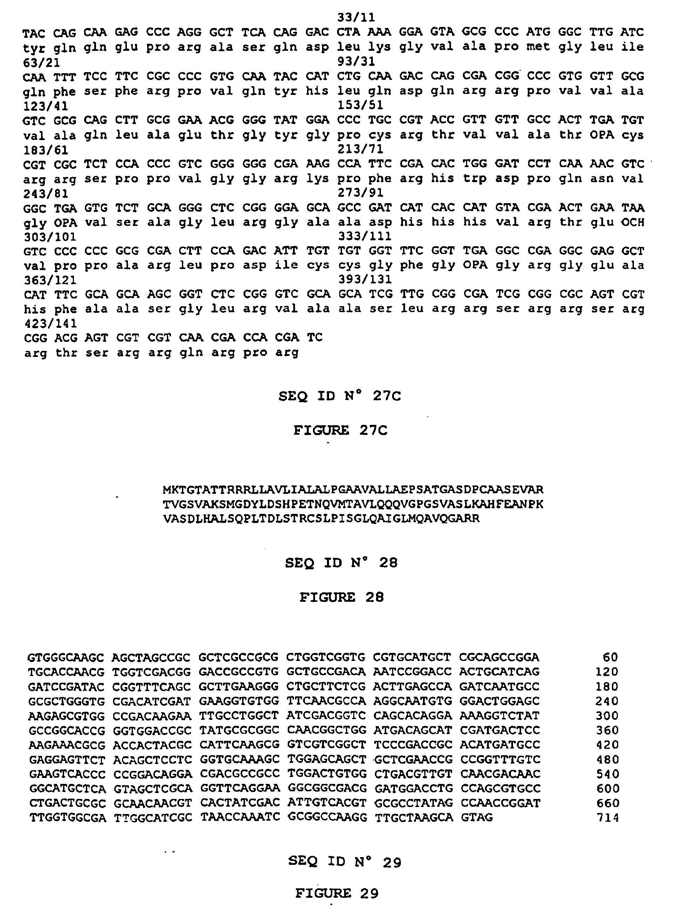

### La série de Figures 27 :

La série de Figures 27 représente la série de séquences nucléotidiques SEQ ID N°27 correspondant à l'insert du vecteur p5A3.

### Figure 28 :

La séquence d'acides aminés telle que définie dans la figure 28 représente la séquence d'acides aminés SEQ ID N°28 correspondant au polypeptide DP428.

### Figure 29 :

La figure 29 représente la séquence nucléotidique SEQ ID N° 29 du gène complet codant pour la protéine MlC25.

### Figure 30 :

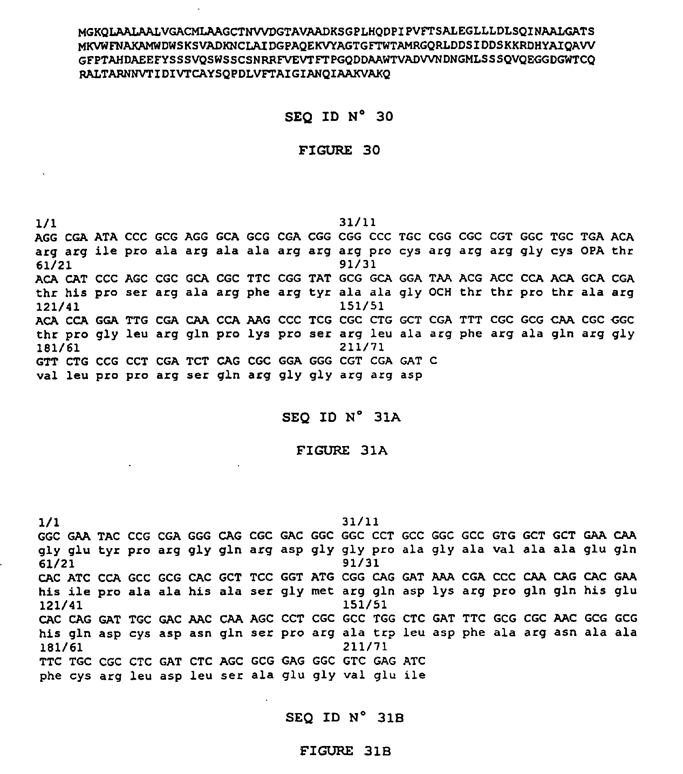

La figure 30 représente la séquence d'acides aminés SEQ ID N° 30 de la protéine MlC25.

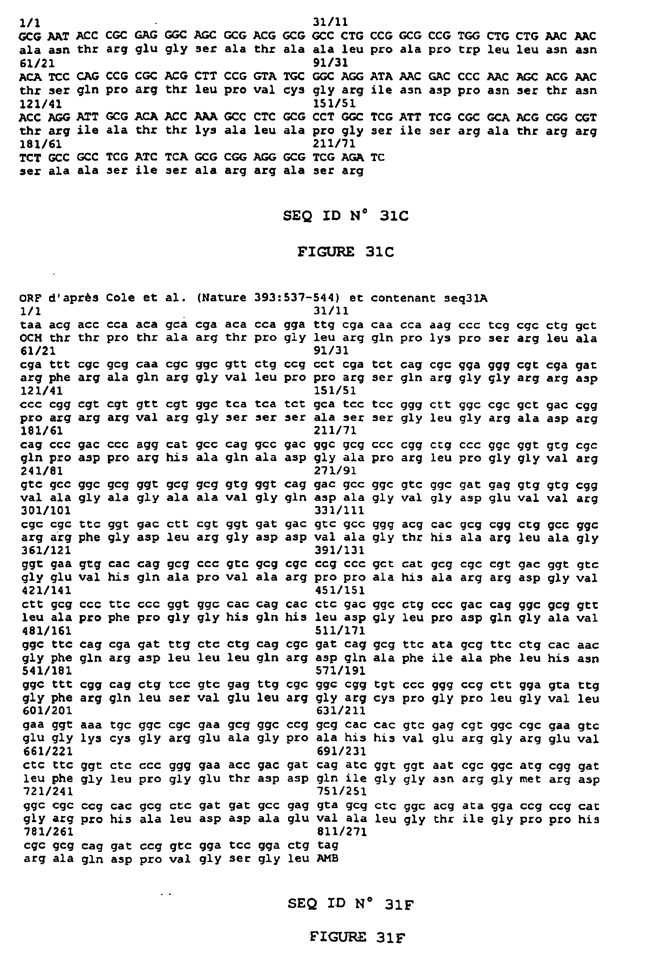

### La série de Figures 31 :

La série de Figures 31 représente la série de séquences nucléotidiques SEQ ID N°31.

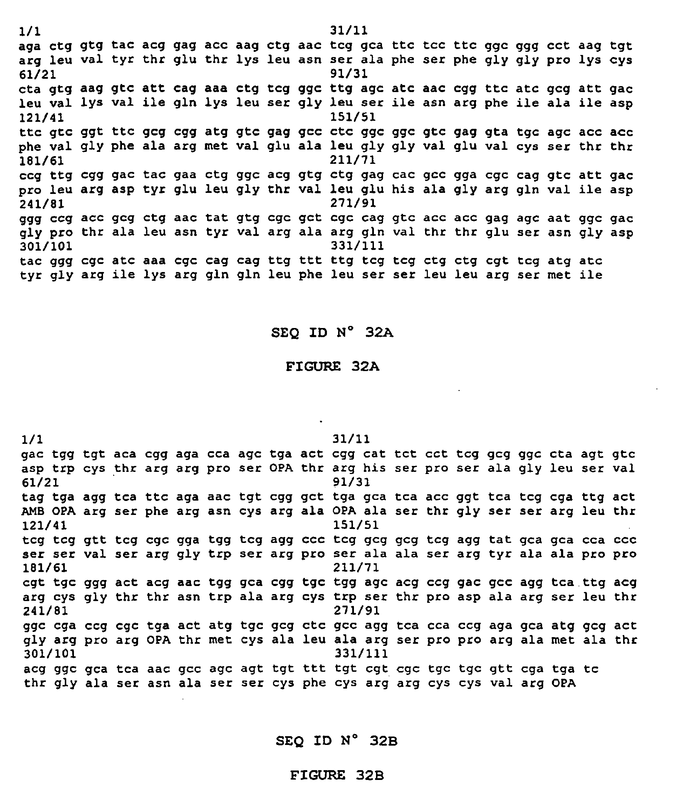

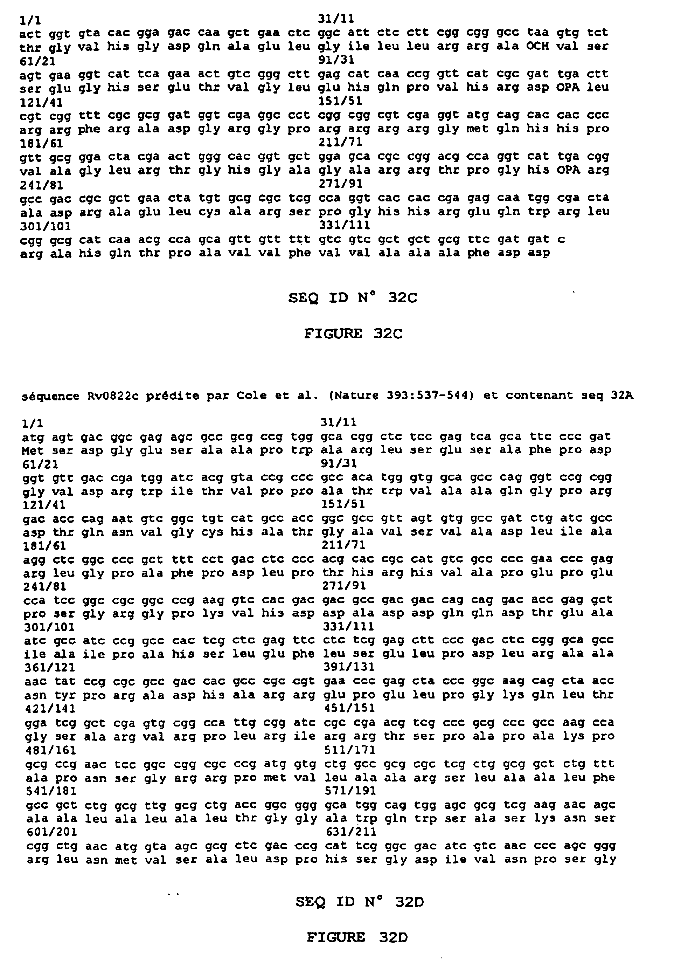

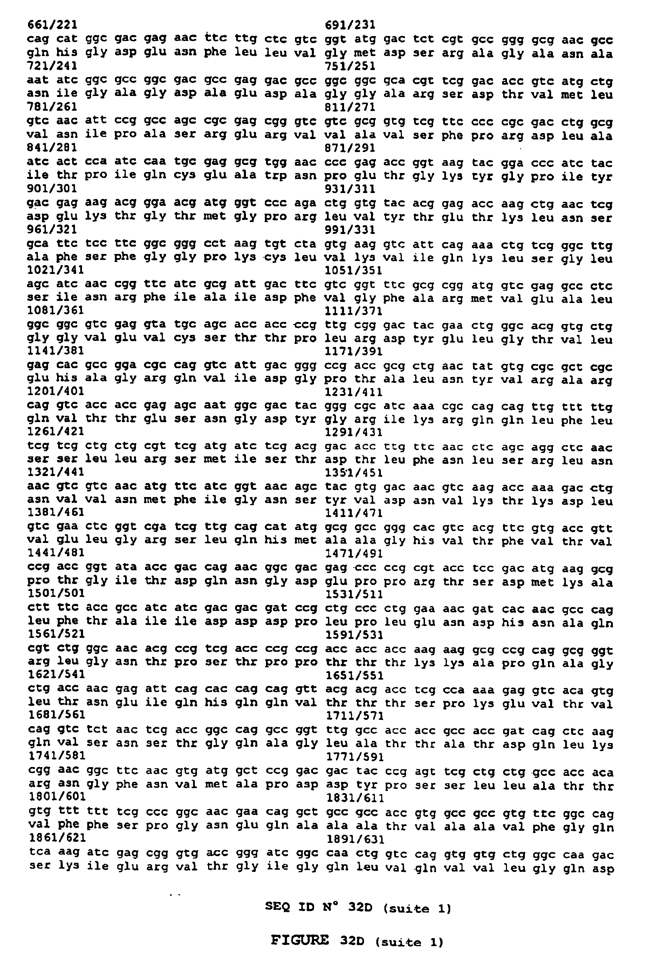

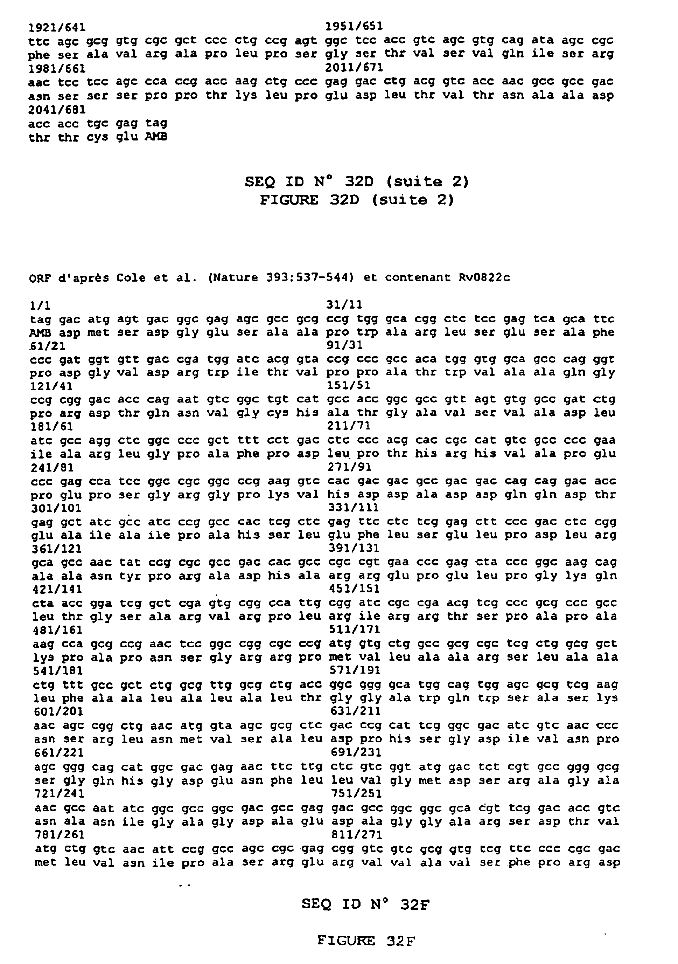

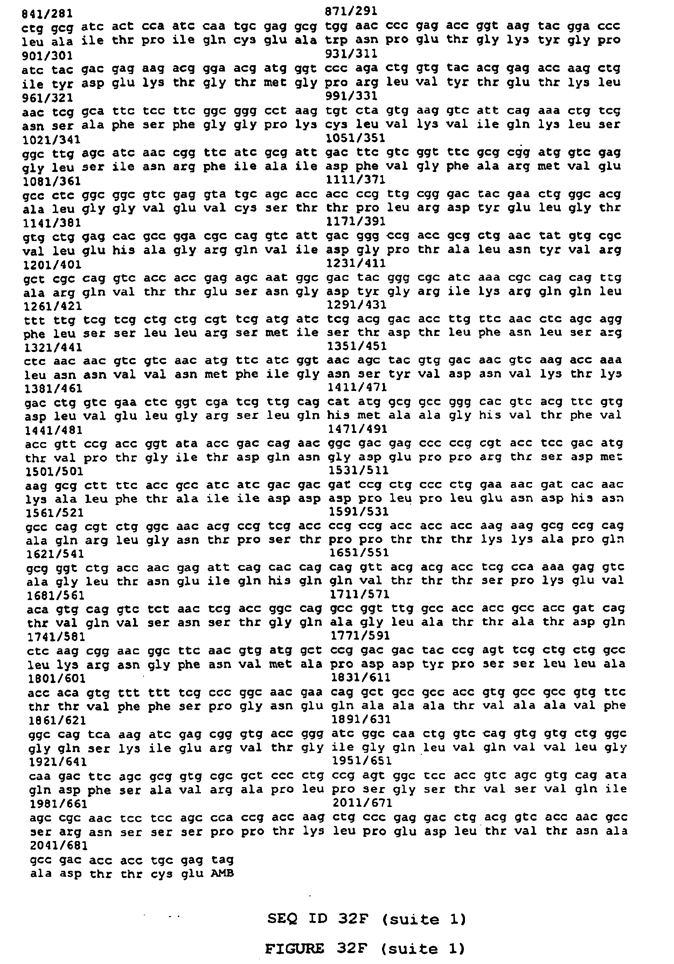

### La série de Figures 32 :

La série de Figures 32 représente la série de séquences nucléotidiques SEQ ID N°32.

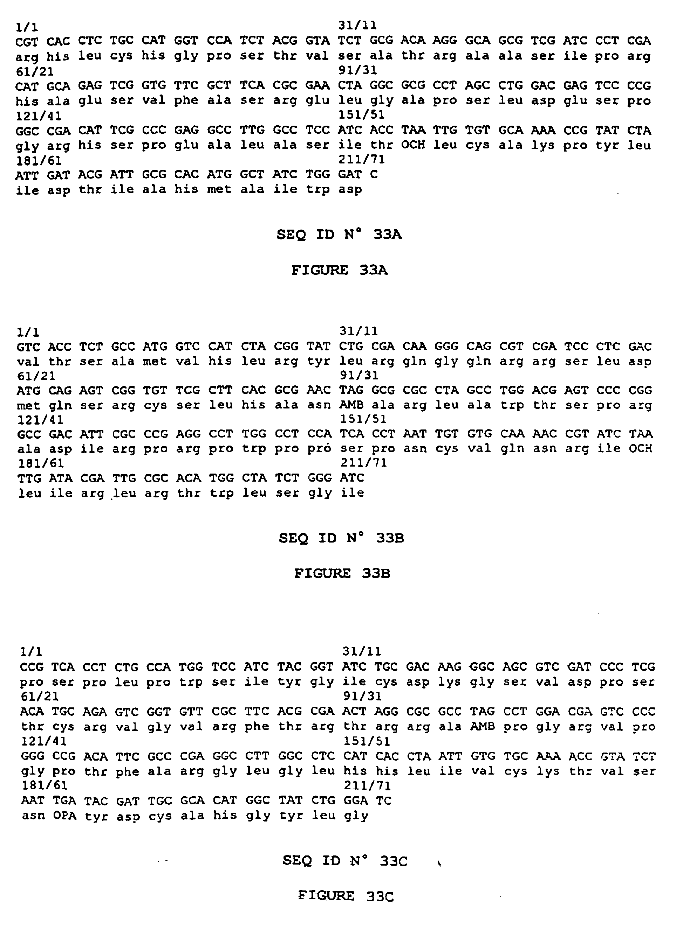

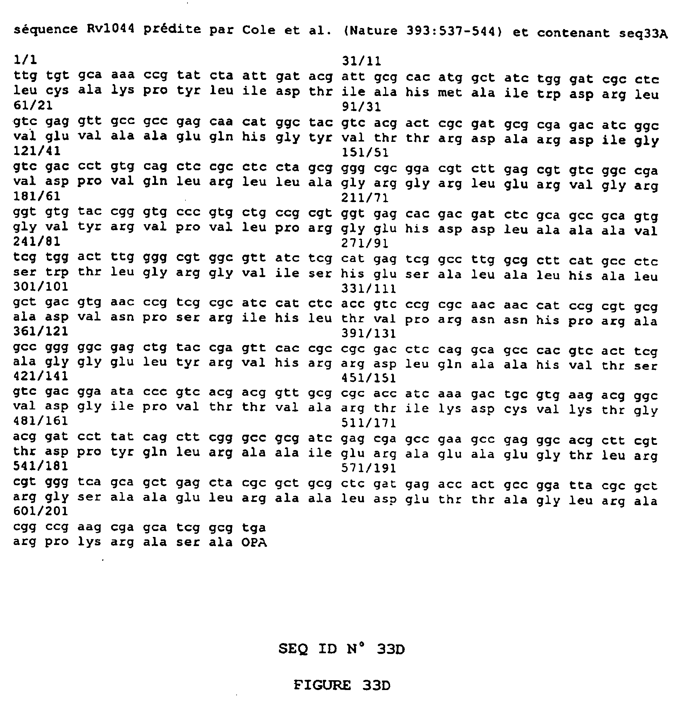

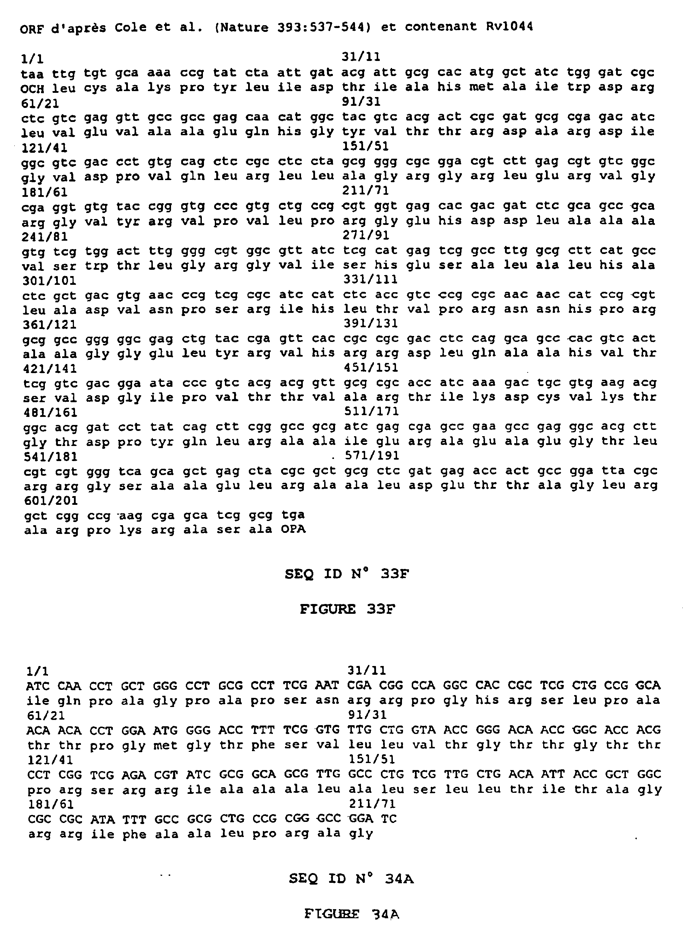

### La série de Figures 33 :

La série de Figures 33 représente la série de séquences nucléotidiques SEQ ID N°33.

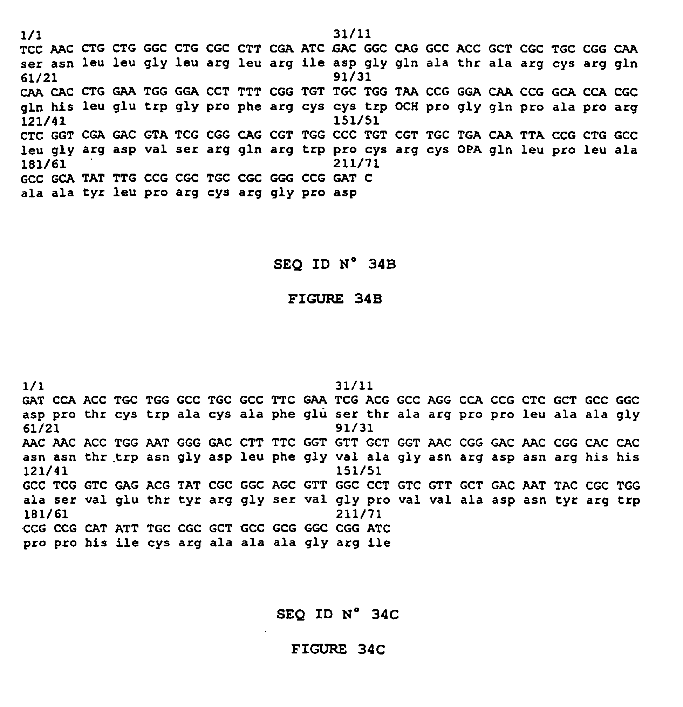

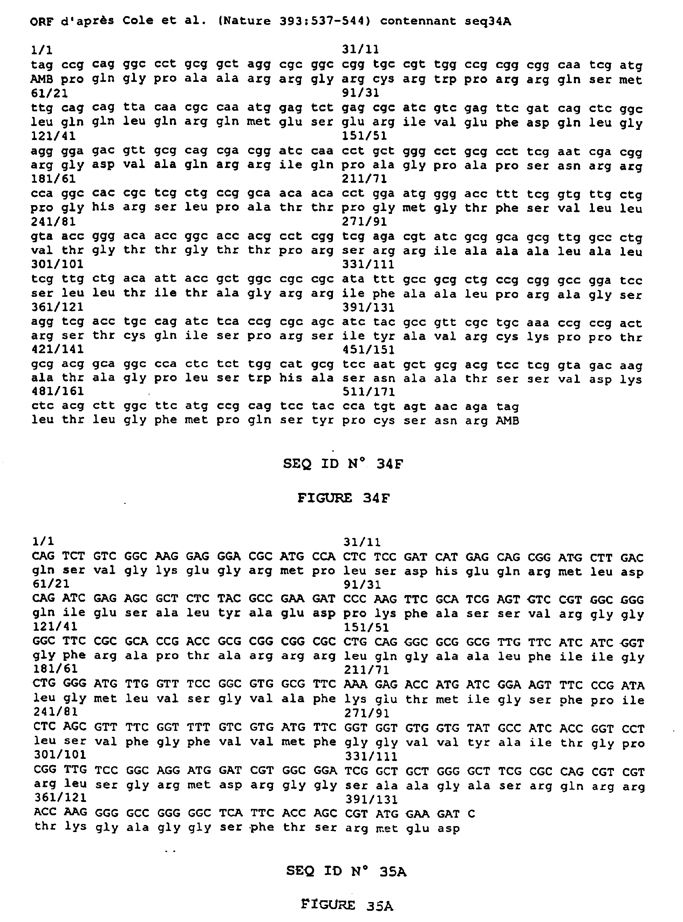

### La série de Figures 34 :

La série de Figures 32 représente la série de séquences nucléotidiques SEQ ID N°34.

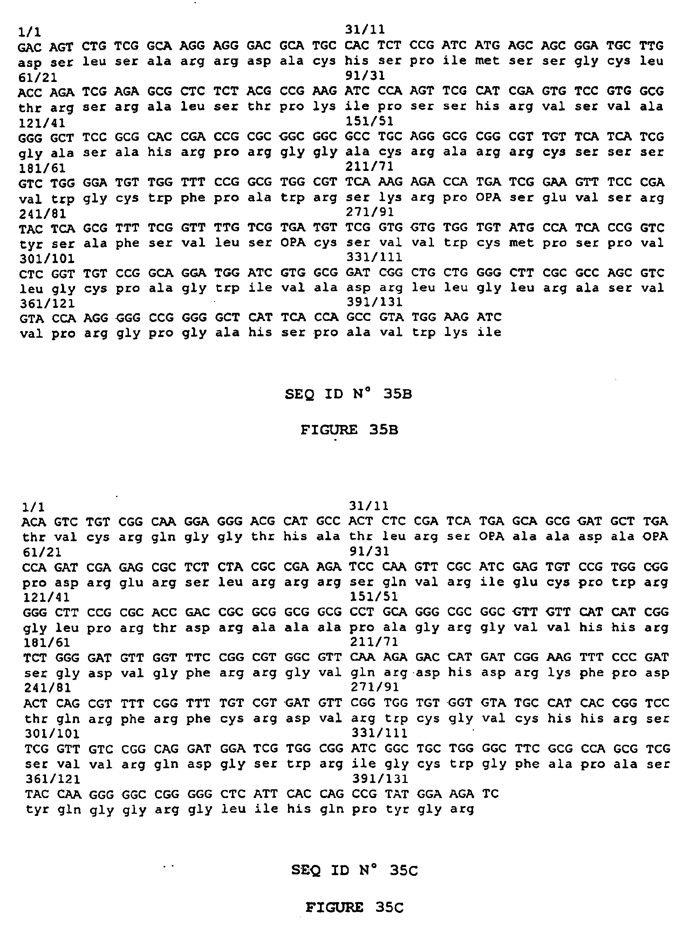

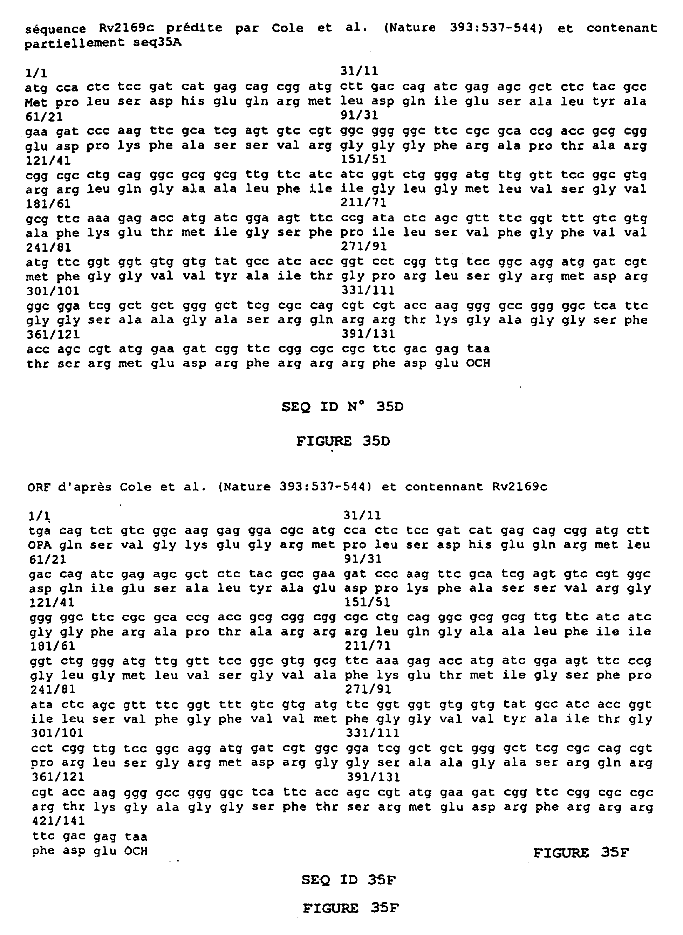

### La série de Figures 35 :

La série de Figures 35 représente la série de séquences nucléotidiques SEQ ID N°35.

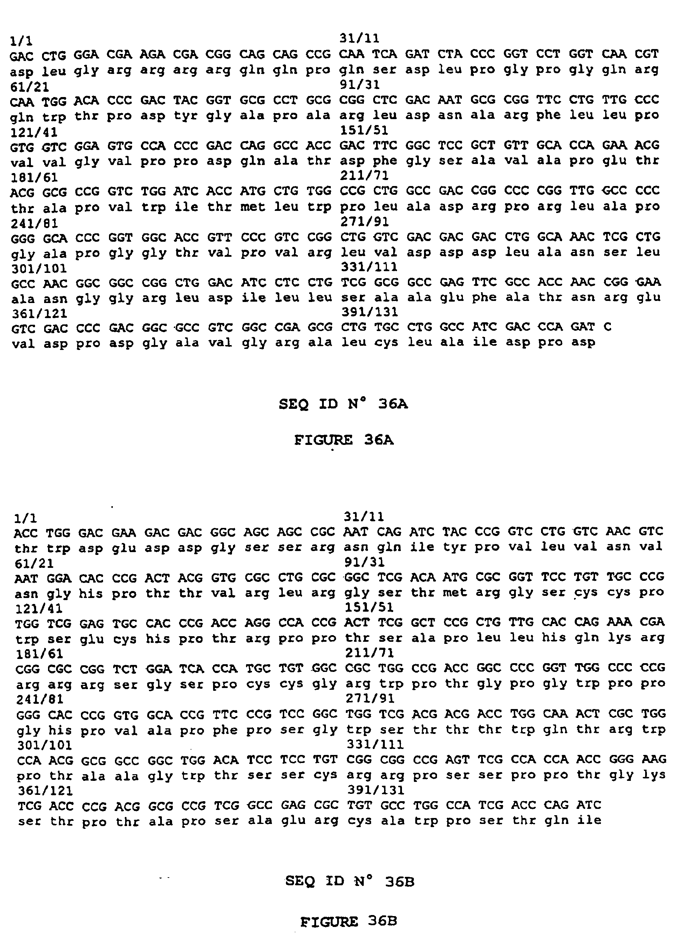

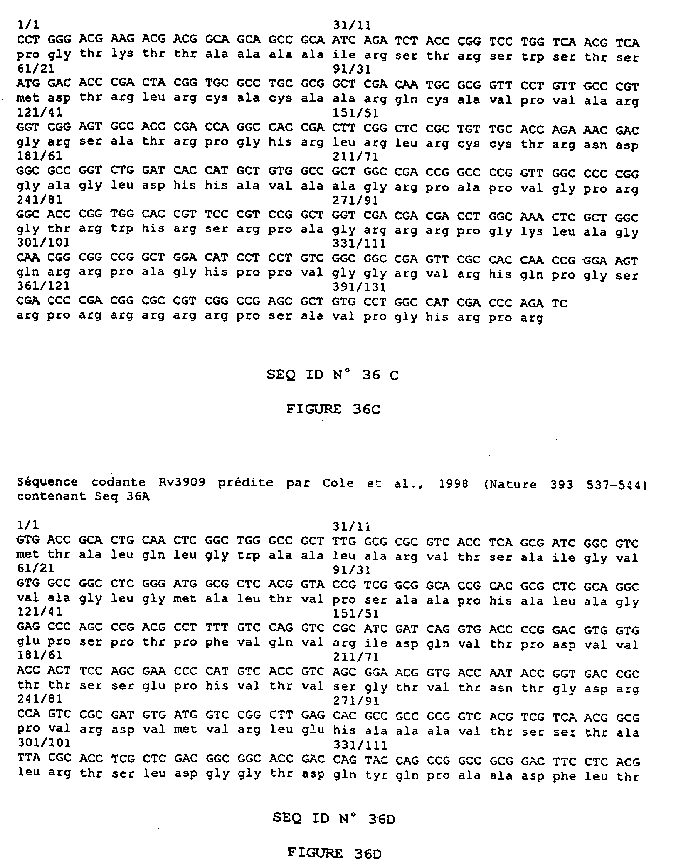

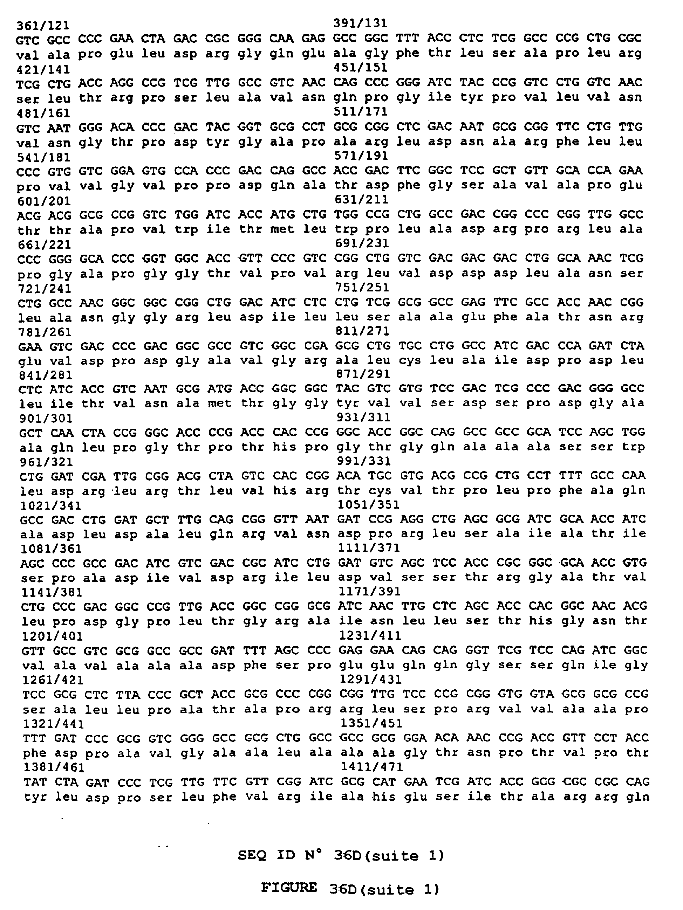

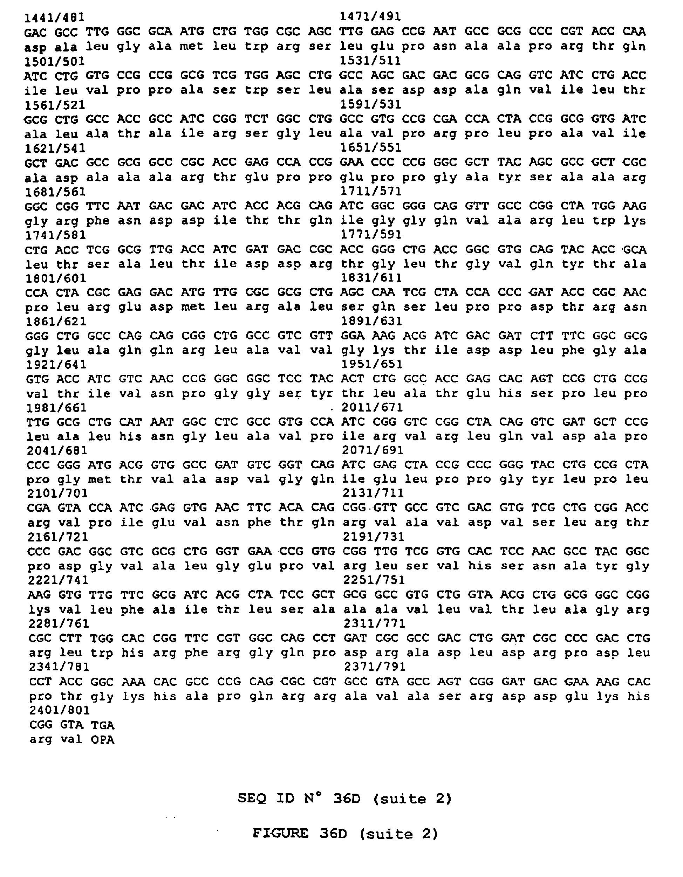

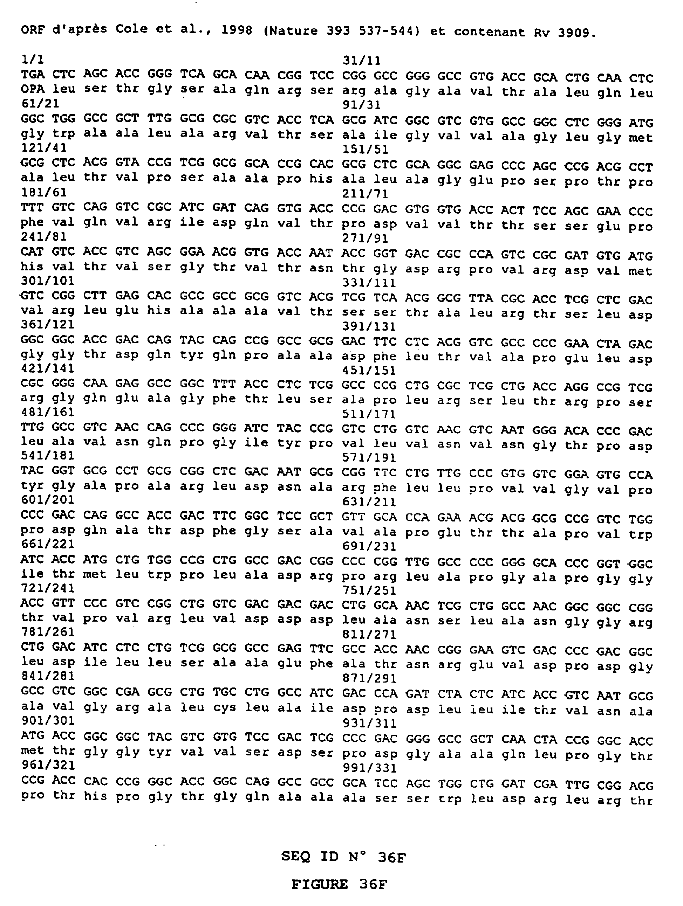

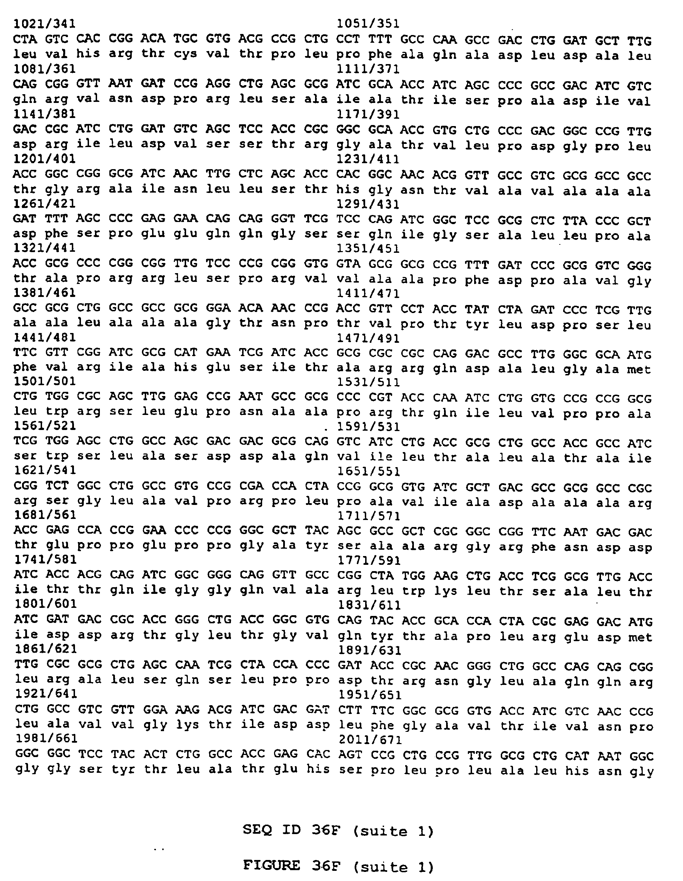

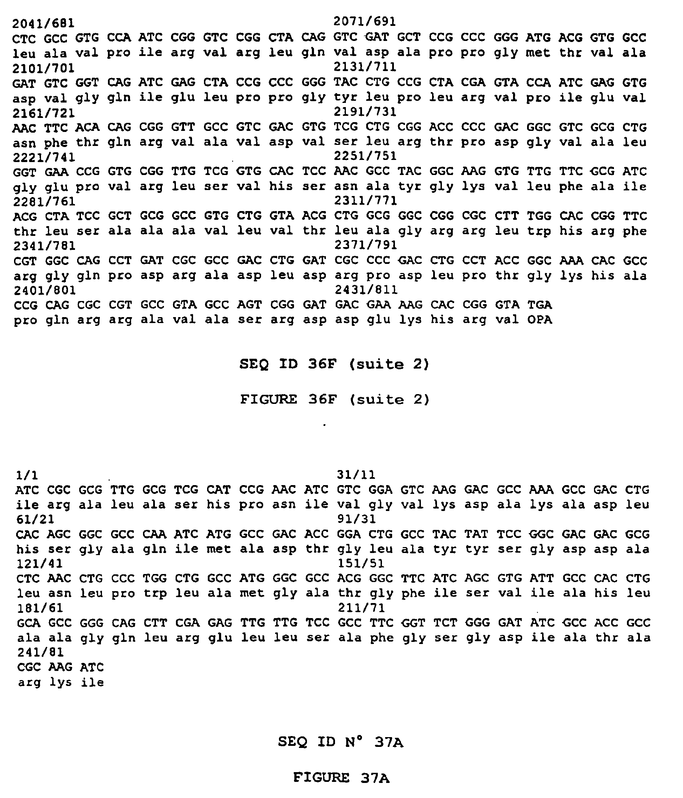

### La série de Figures 36 :

La série de Figures 36 représente la série de séquences nucléotidiques SEQ ID N°36.

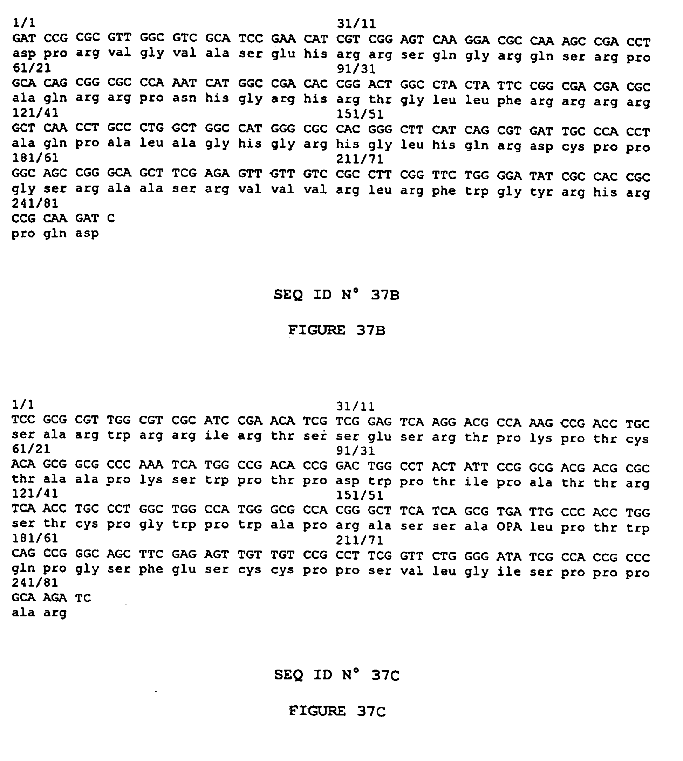

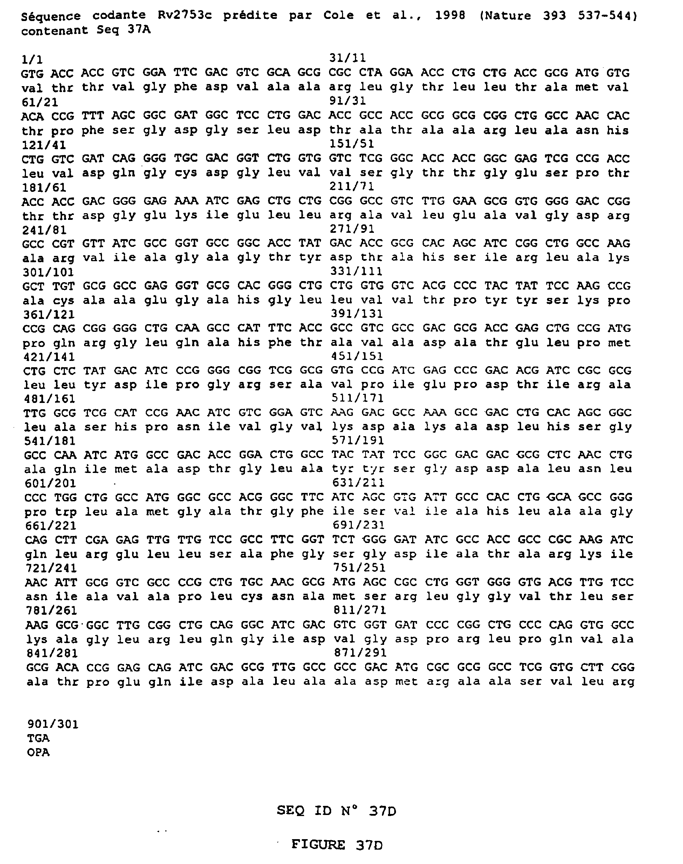

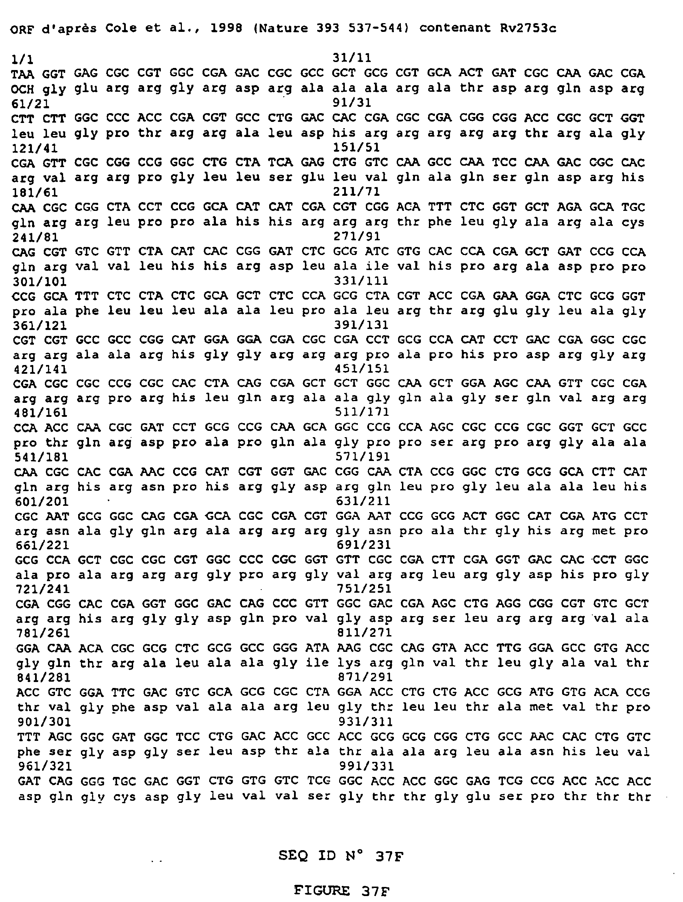

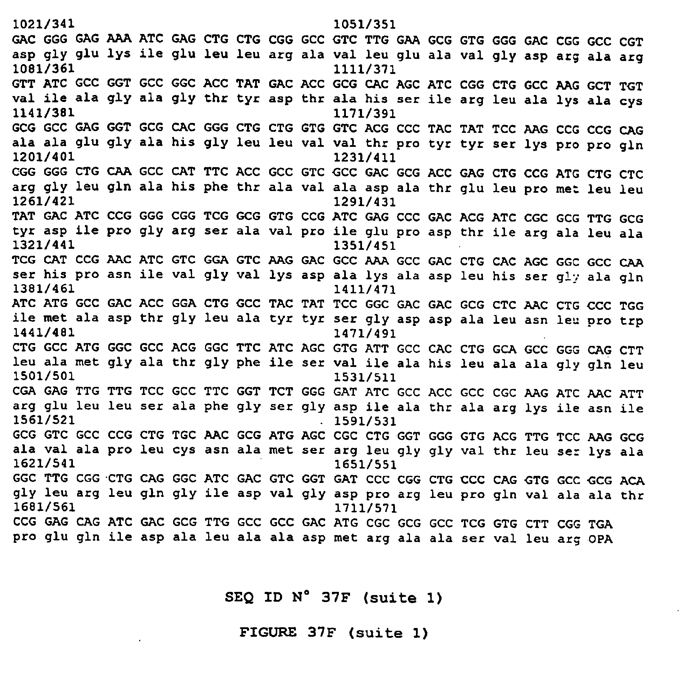

### La série de Figures 37 :

La série de Figures 37 représente la série de séquences nucléotidiques SEQ ID N°37.

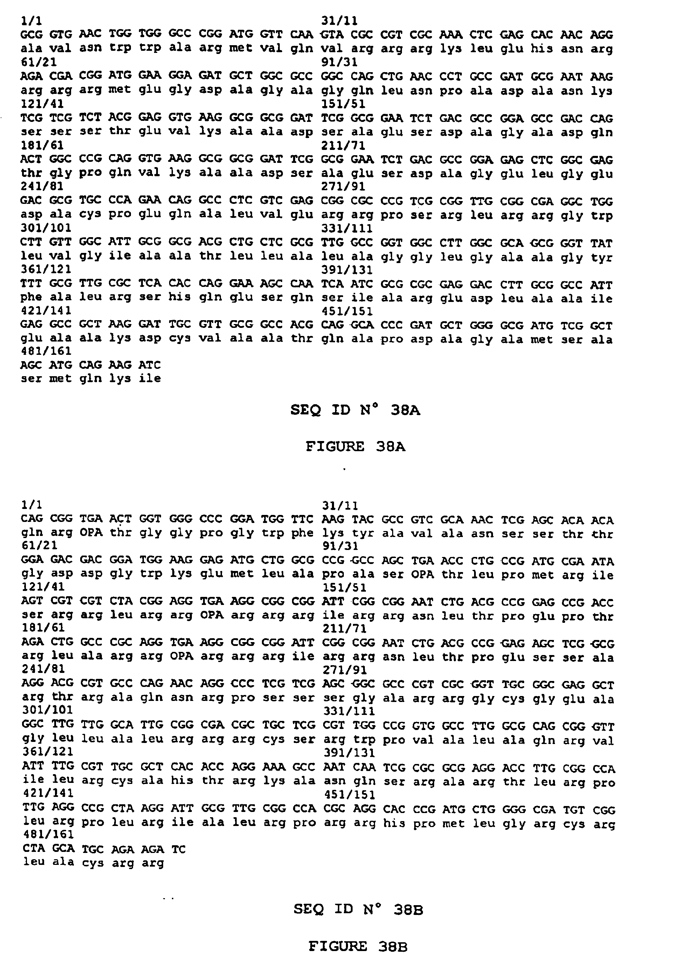

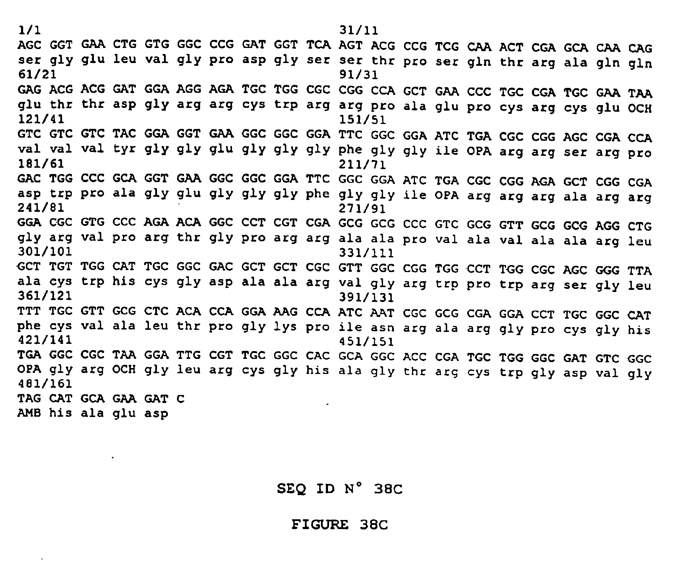

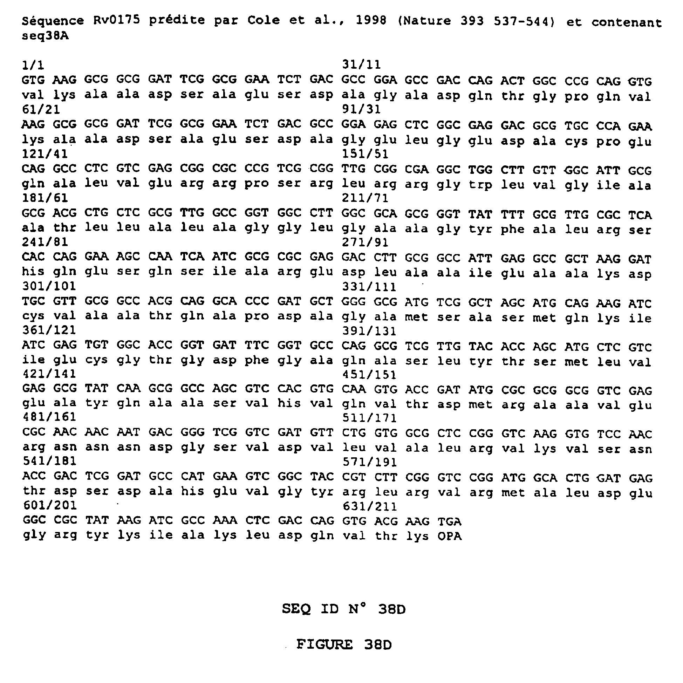

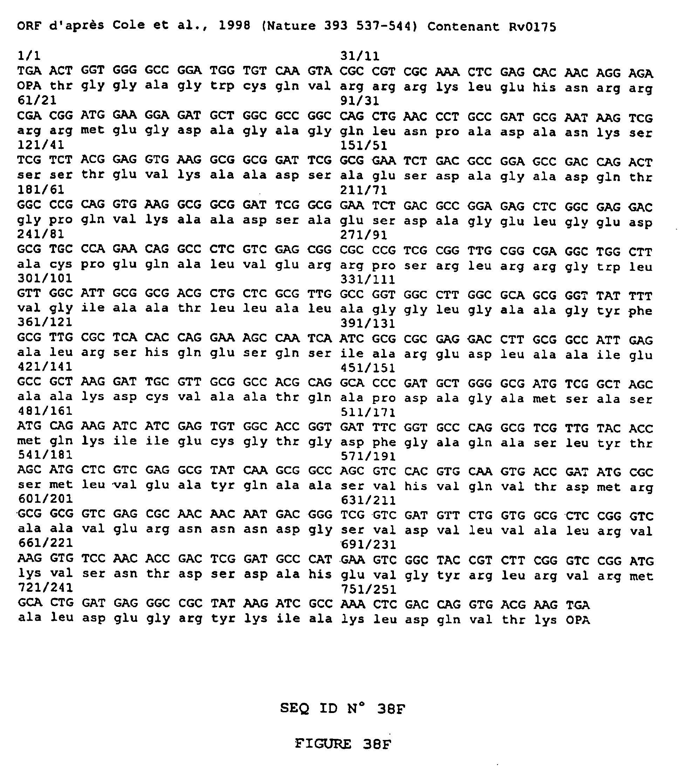

### La série de Figures 38 :

La série de Figures 38 représente la série de séquences nucléotidiques SEQ ID N°38.

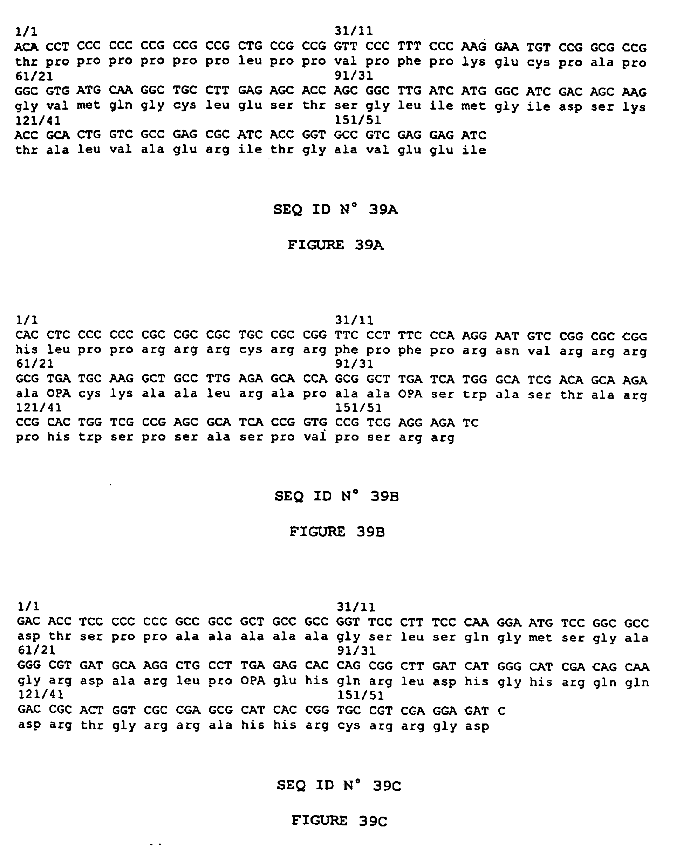

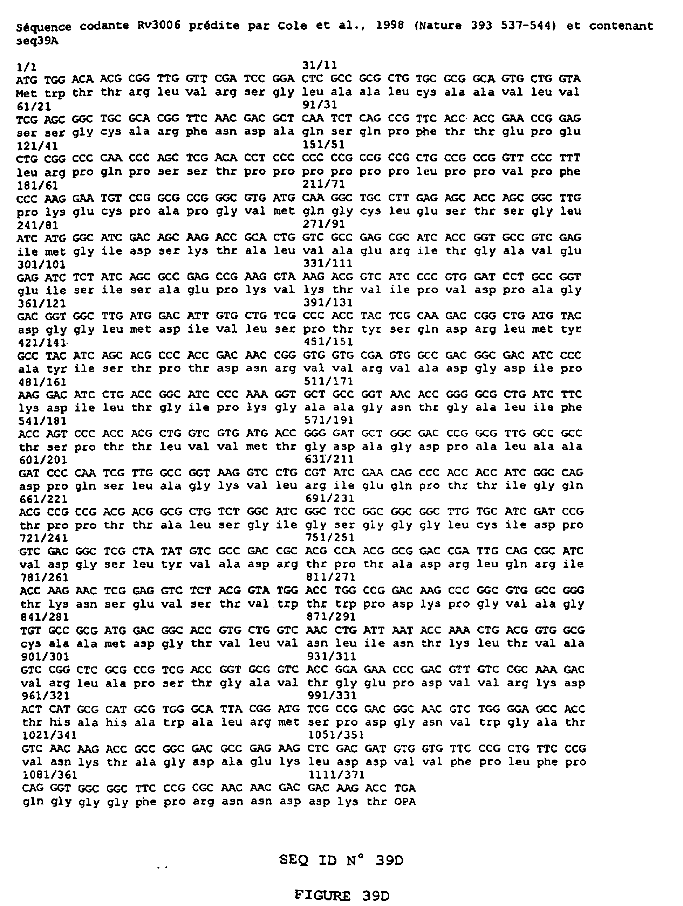

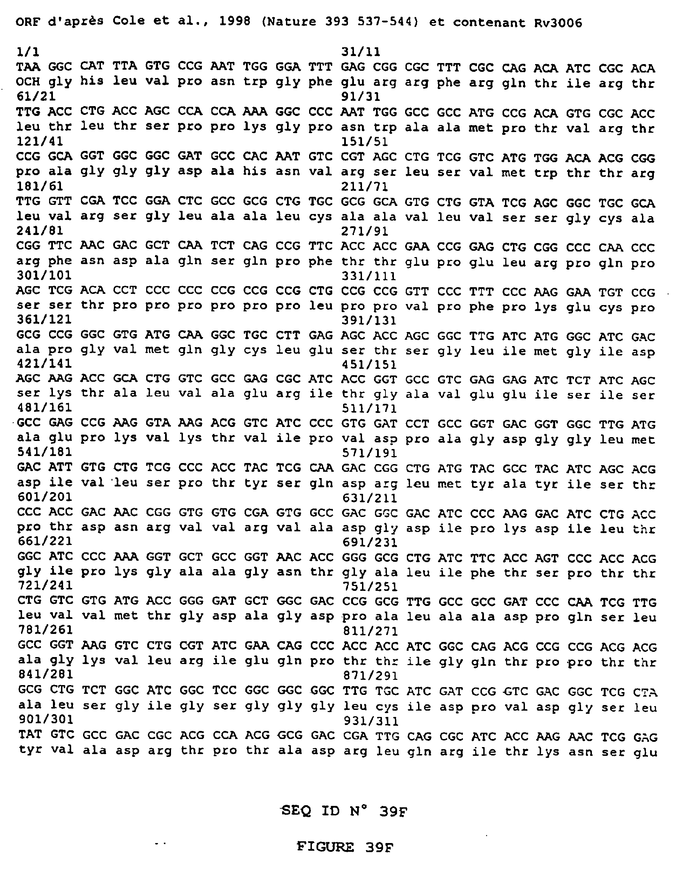

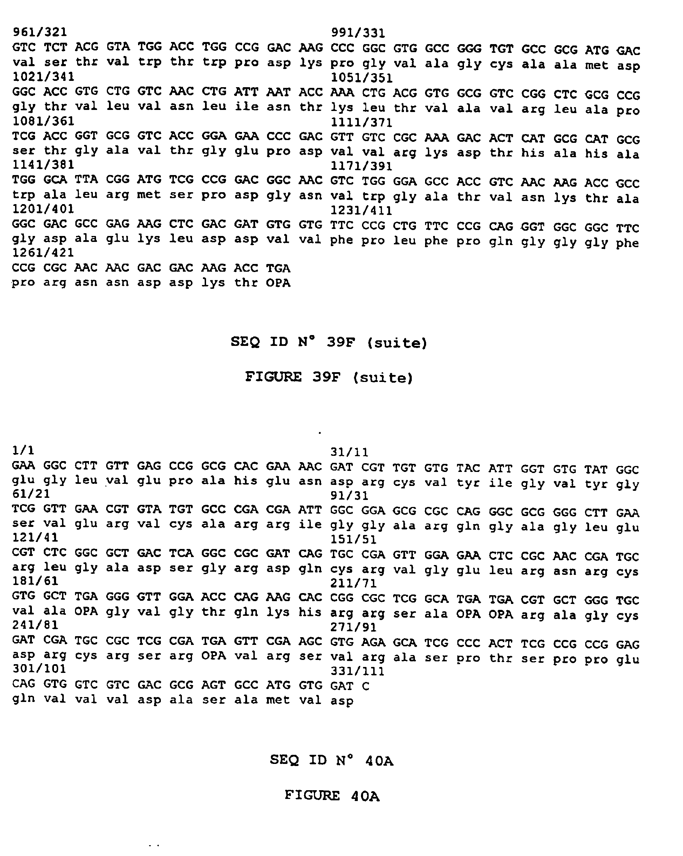

### La série de Figures 39 :

La série de Figures 39 représente la série de séquences nucléotidiques SEQ ID N°39.

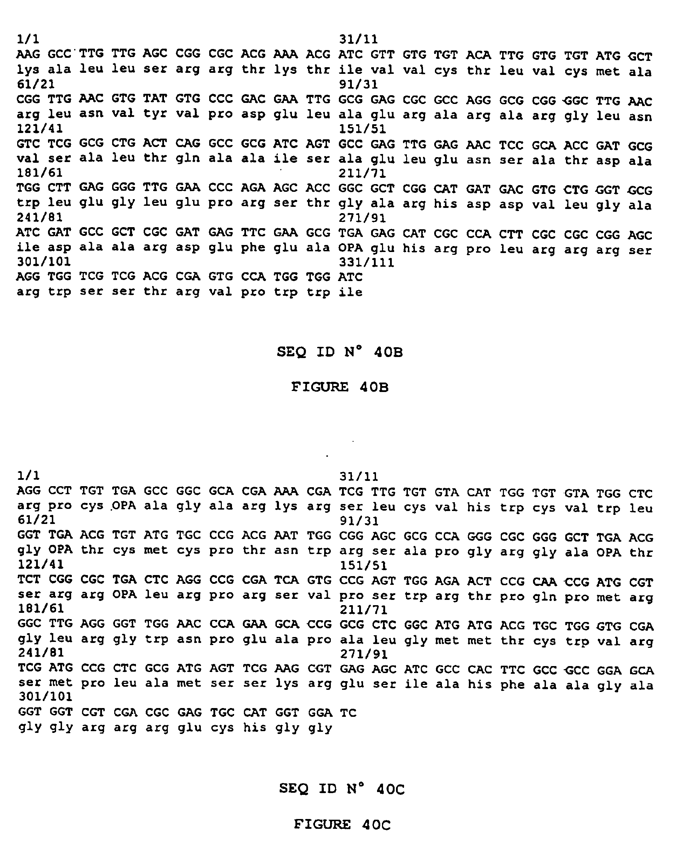

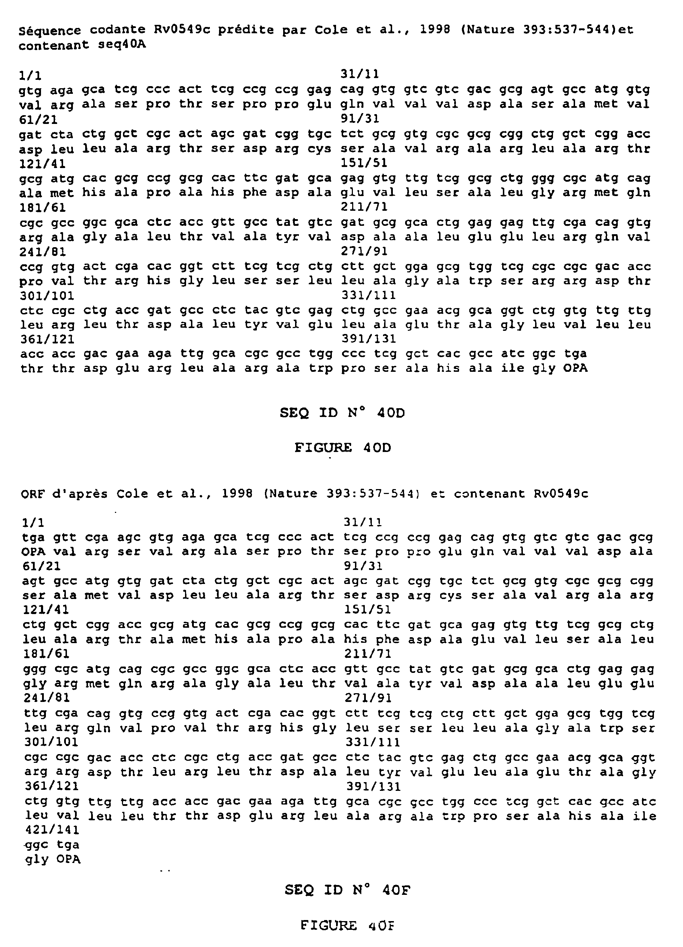

### La série de Figures 40 :

La série de Figures 40 représente la série de séquences nucléotidiques SEQ ID N°40.

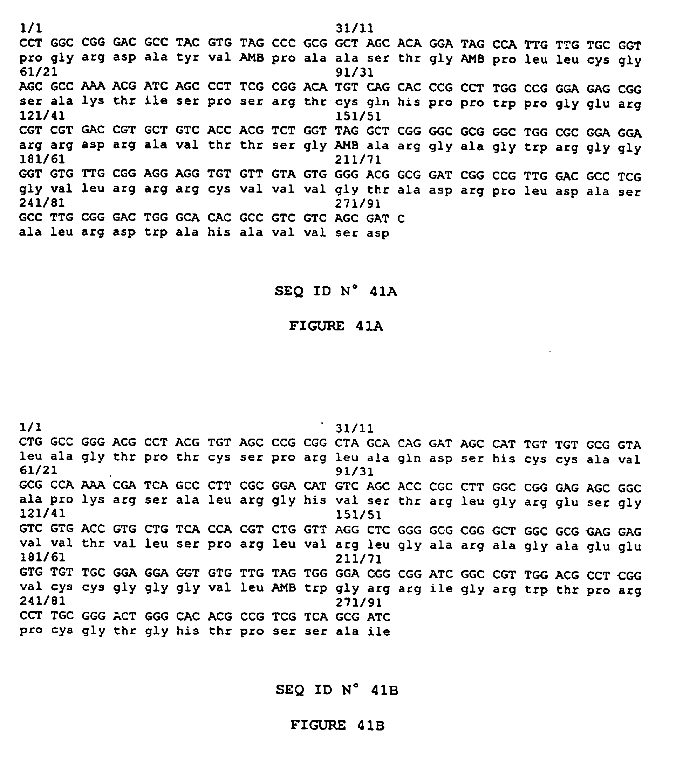

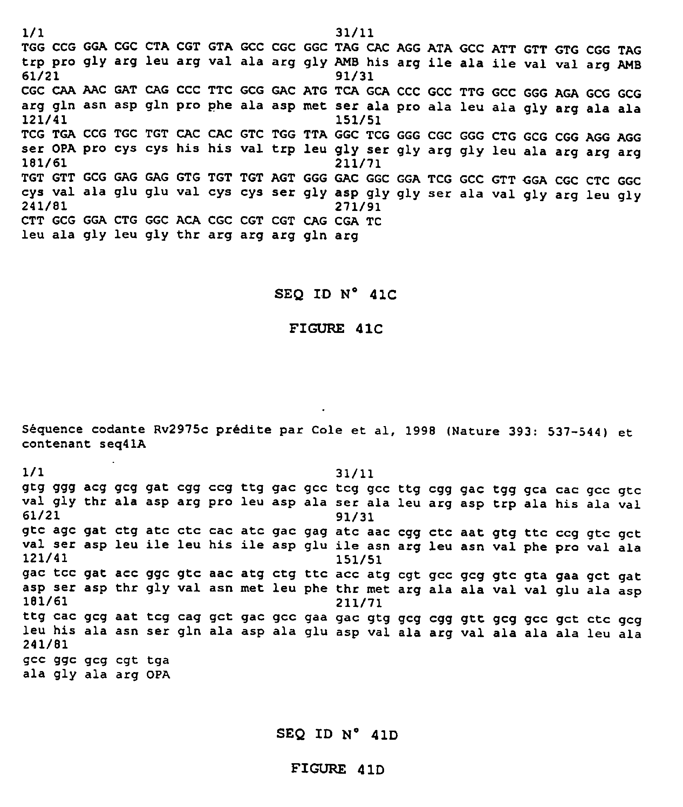

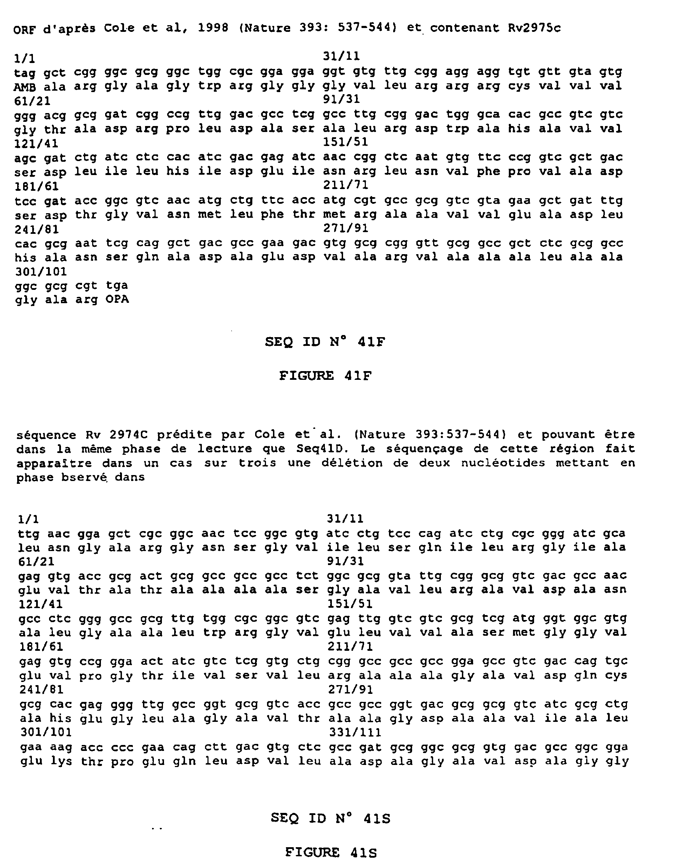

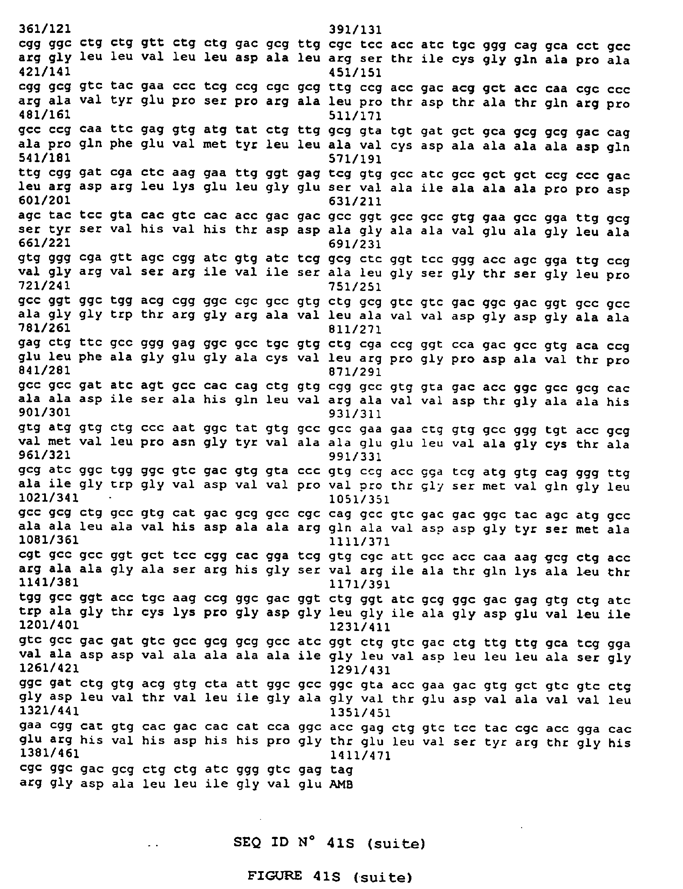

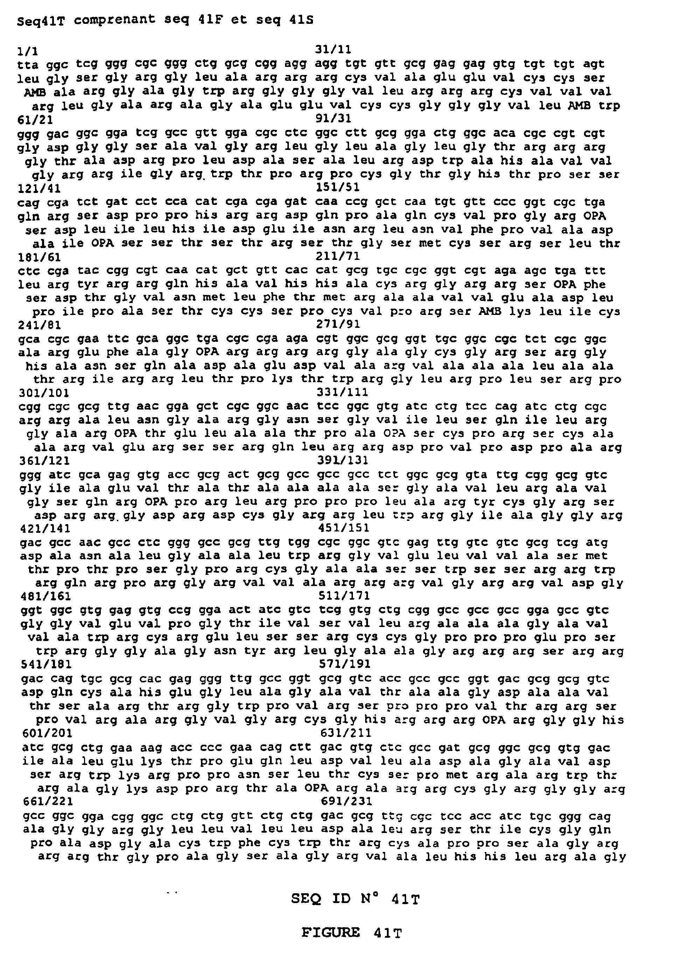

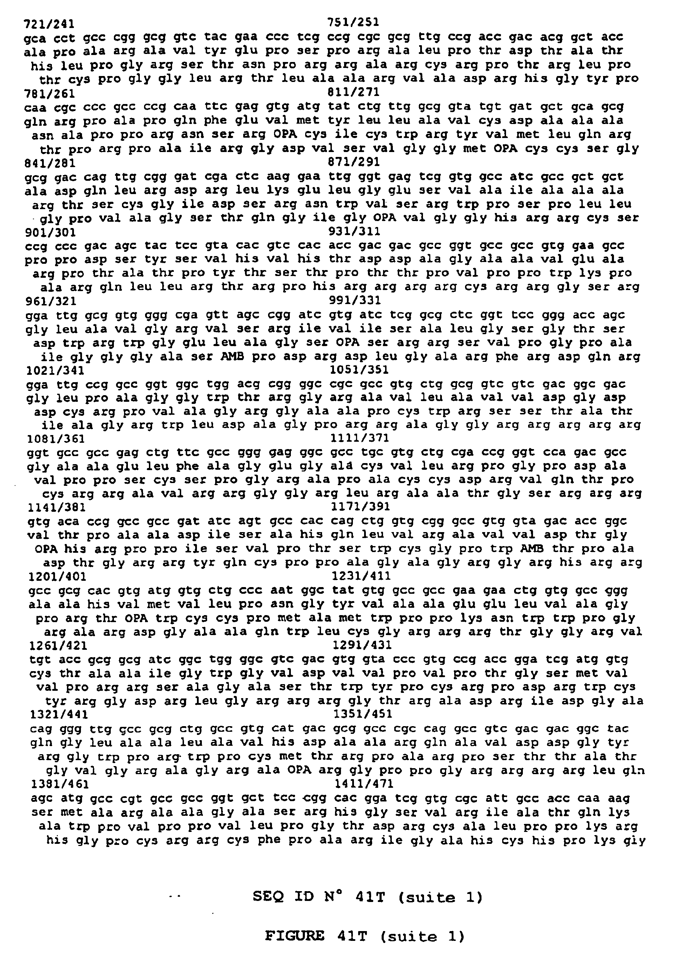

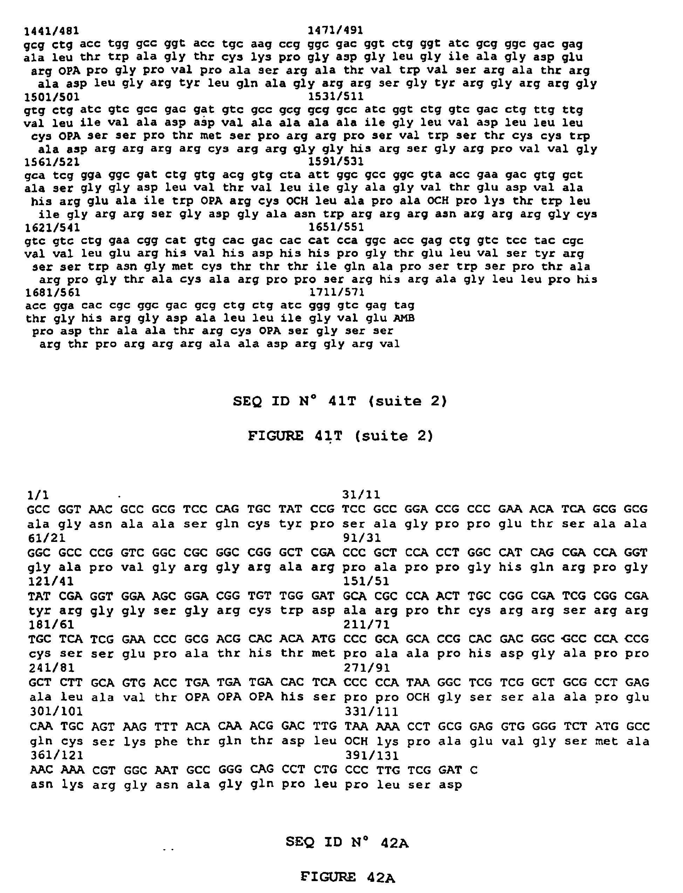

### La série de Figures 41 :

La série de Figures 41 représente la série de séquences nucléotidiques SEQ ID N°41 correspondant à l'insert du vecteur p2D7 (déposé à la CNCM sous le N°I-1821).

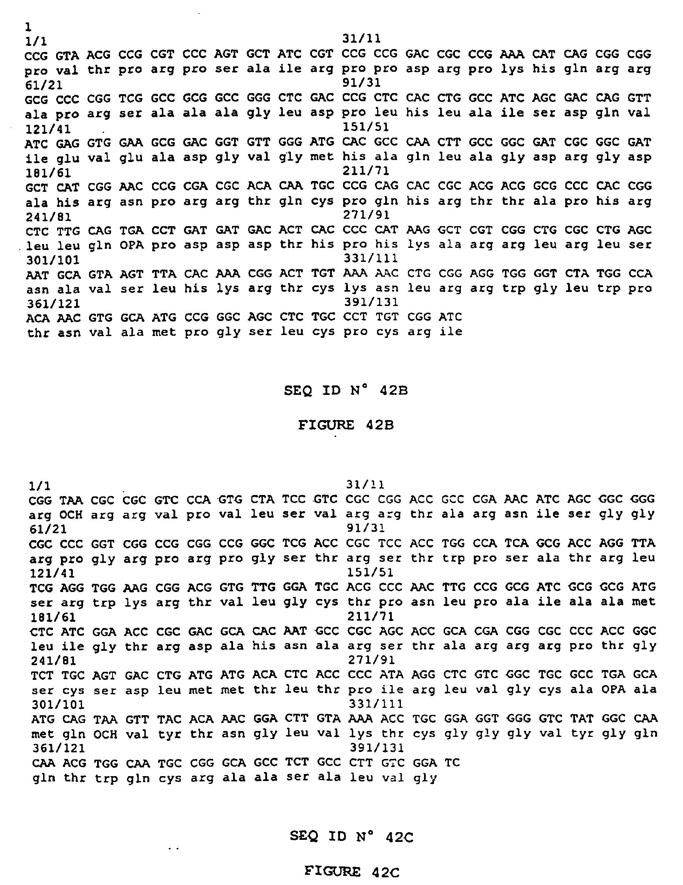

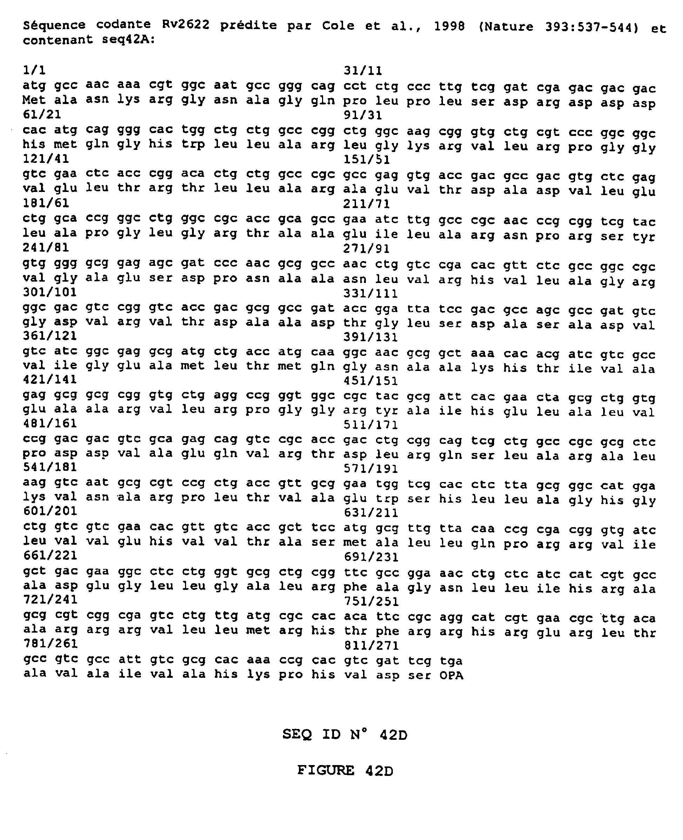

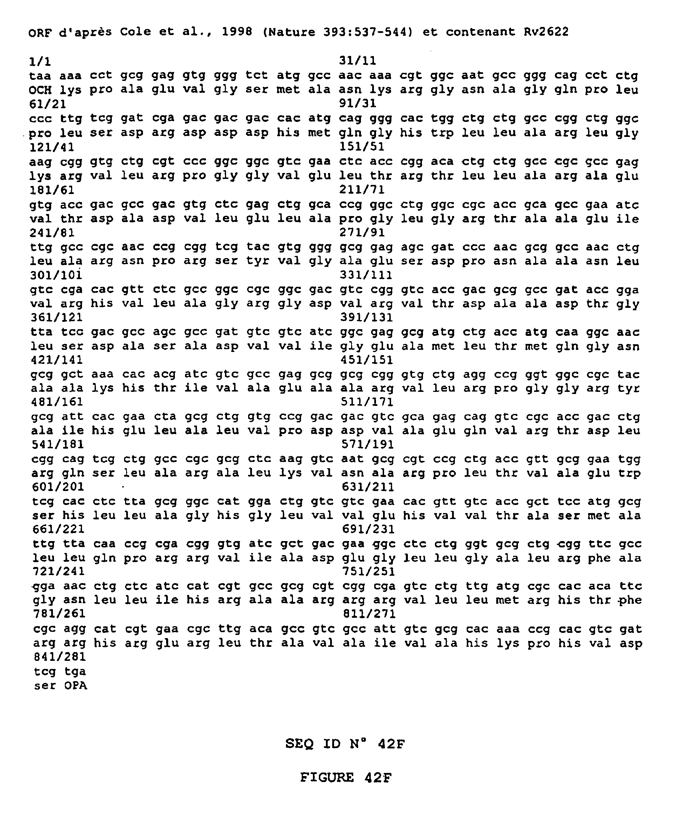

### La série de Figures 42 :

La série de Figures 42 représente la série de séquences nucléotidiques SEQ ID N°42.

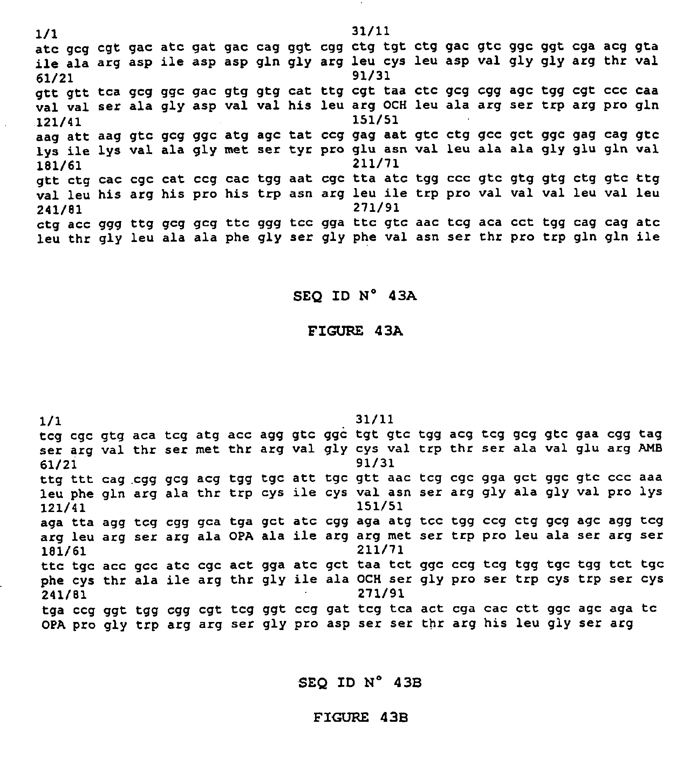

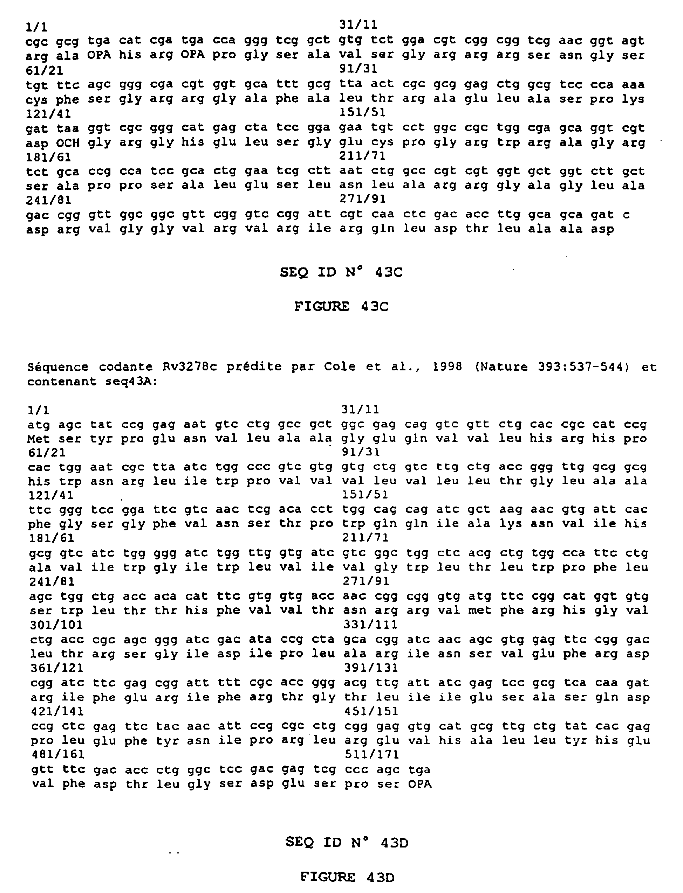

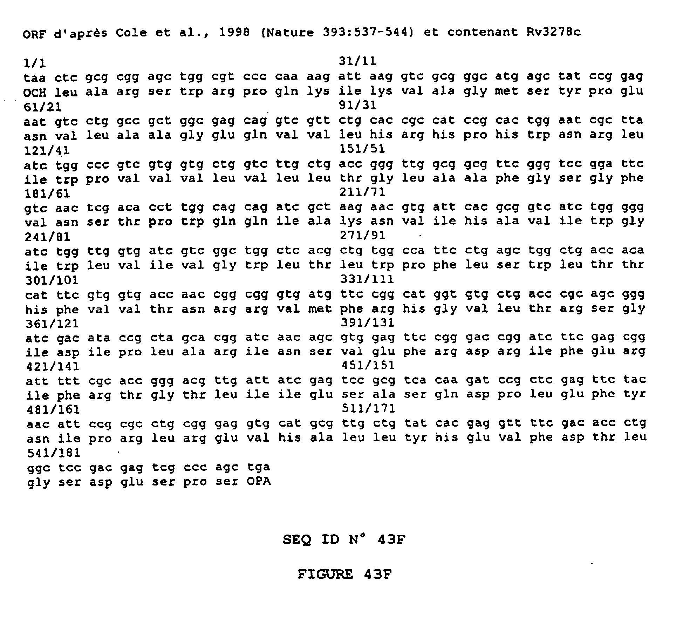

### La série de Figures 43 :

La série de Figures 43 représente la série de séquences nucléotidiques SEQ ID N°43.

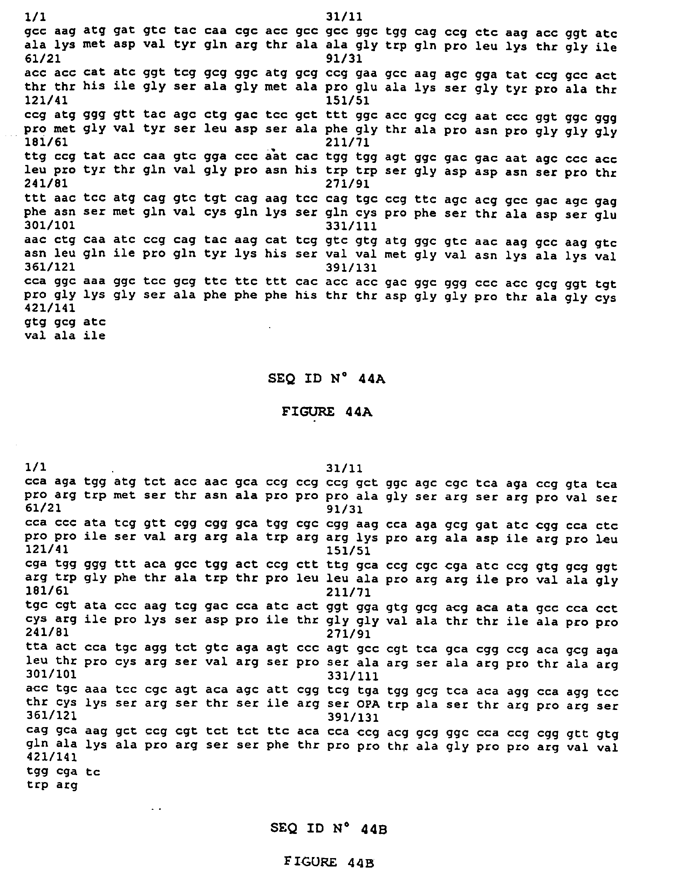

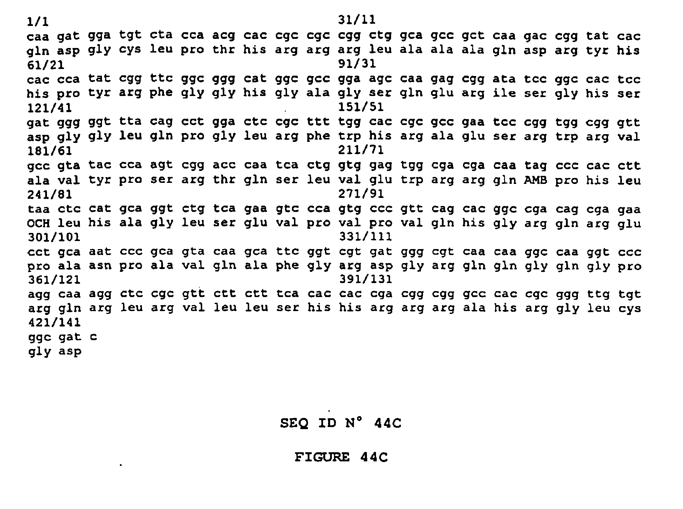

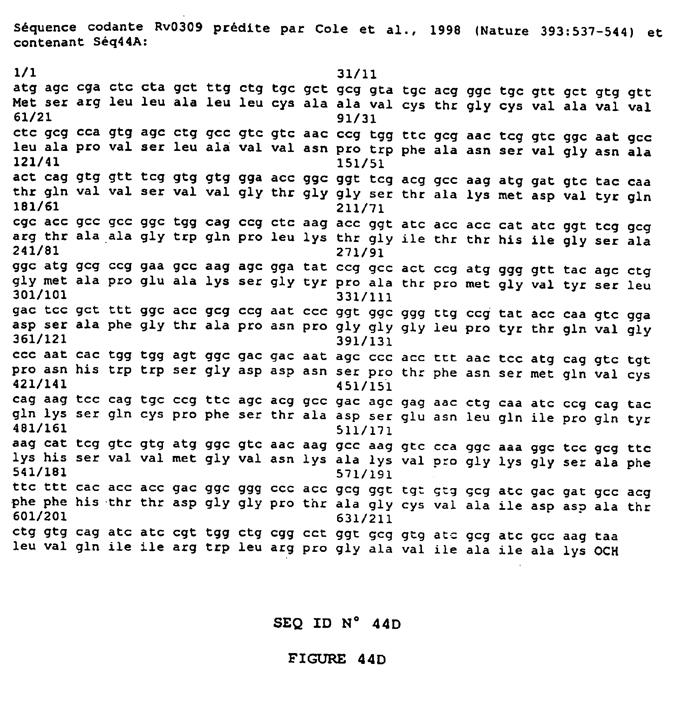

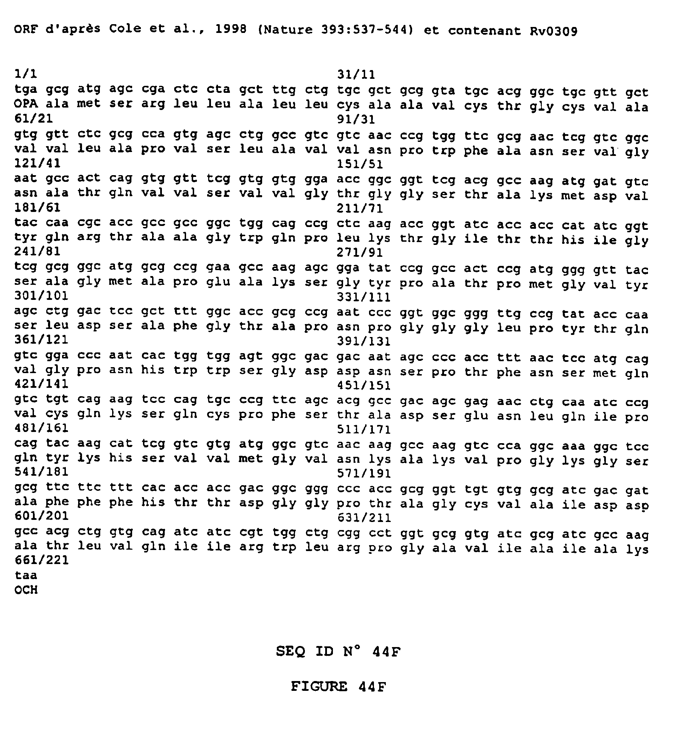

### La série de Figures 44 :

La série de Figures 44 représente la série de séquences nucléotidiques SEQ ID N°44.

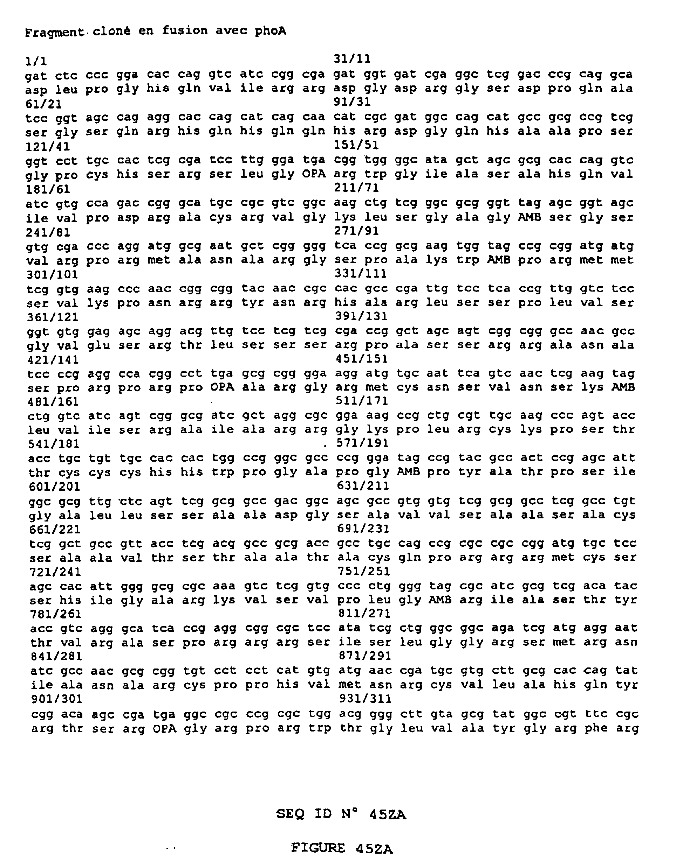

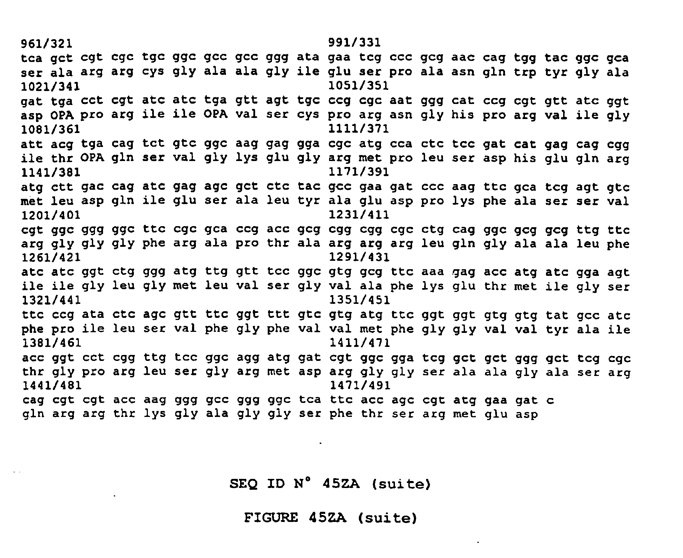

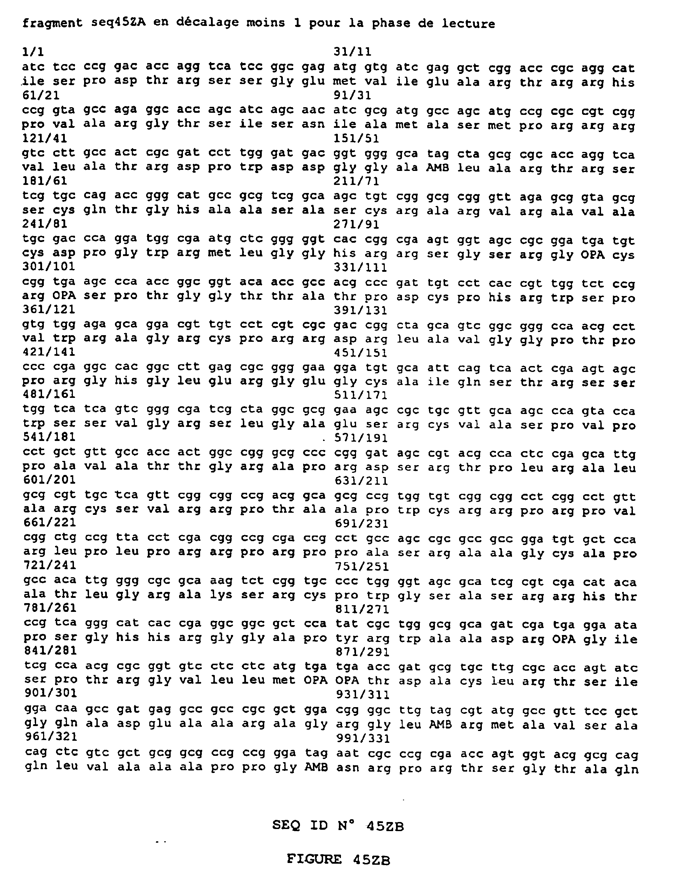

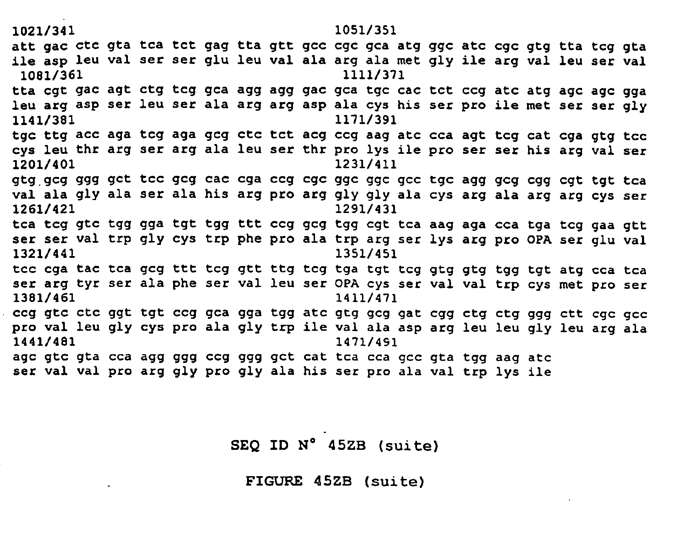

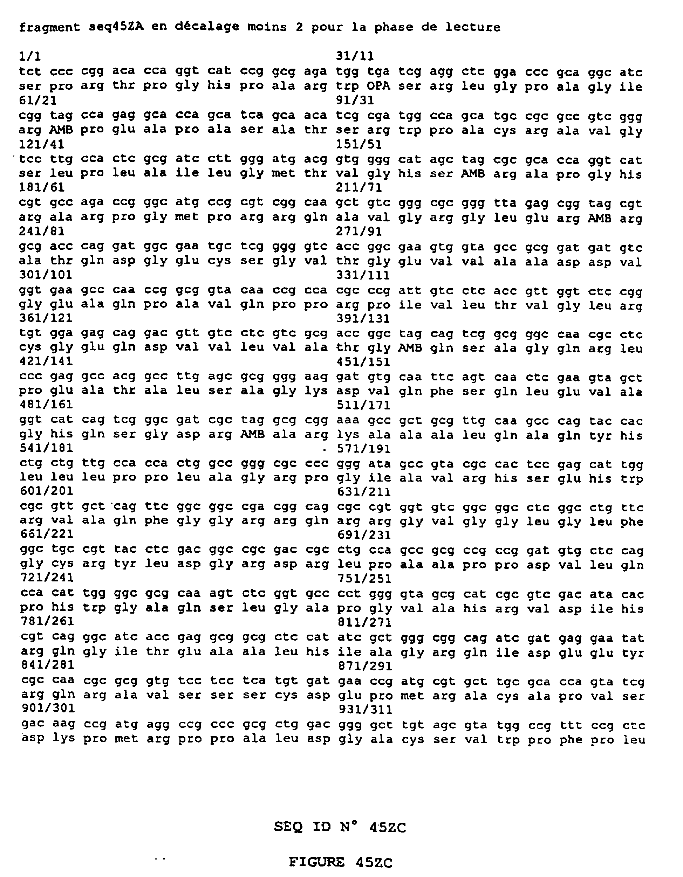

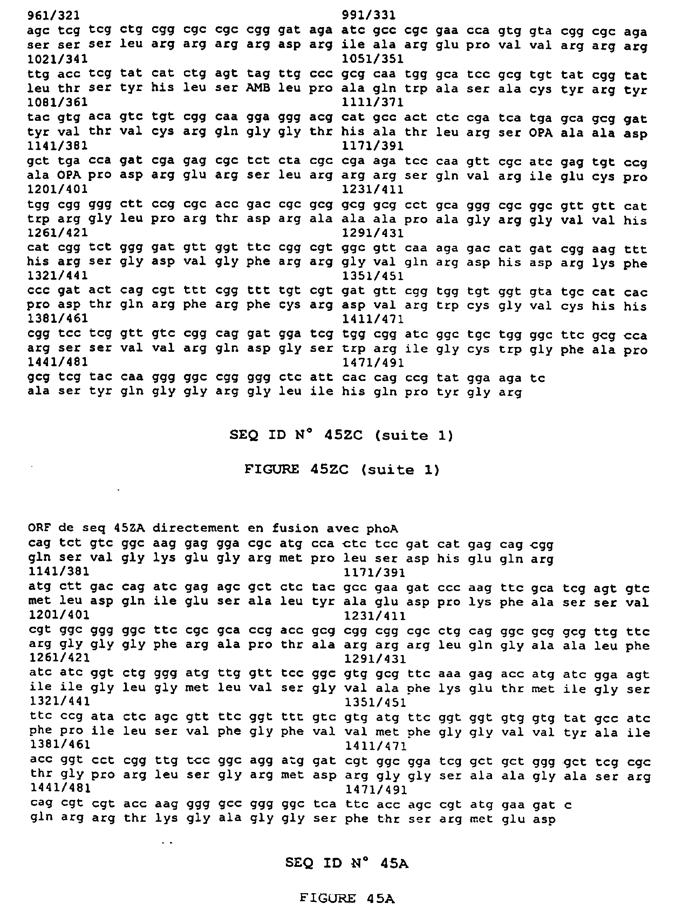

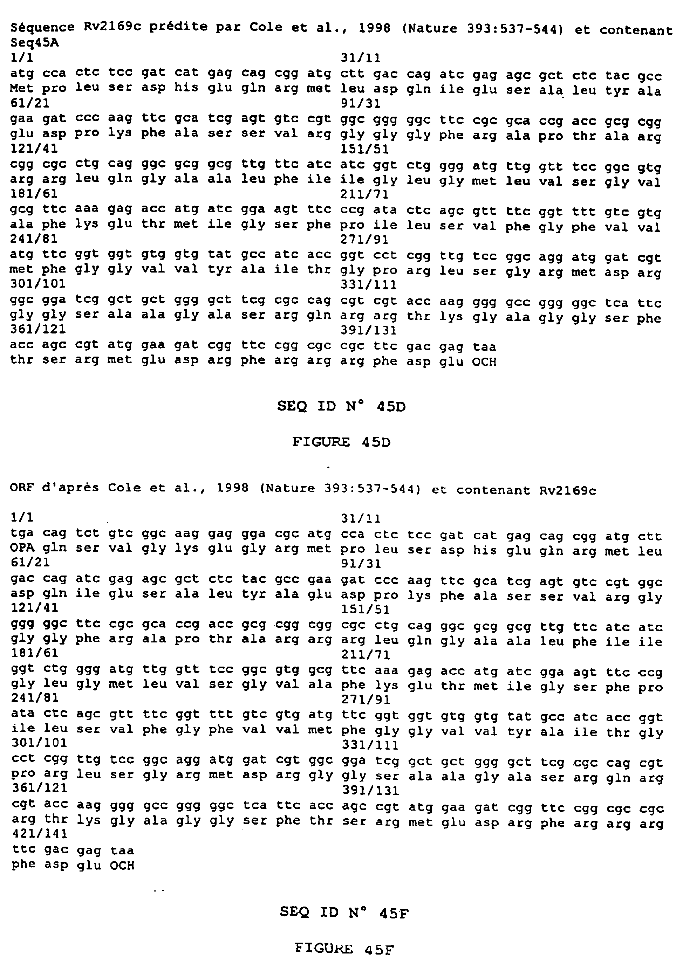

### La série de Figures 45 :

La série de Figures 45 représente la série de séquences nucléotidiques SEQ ID N°45.

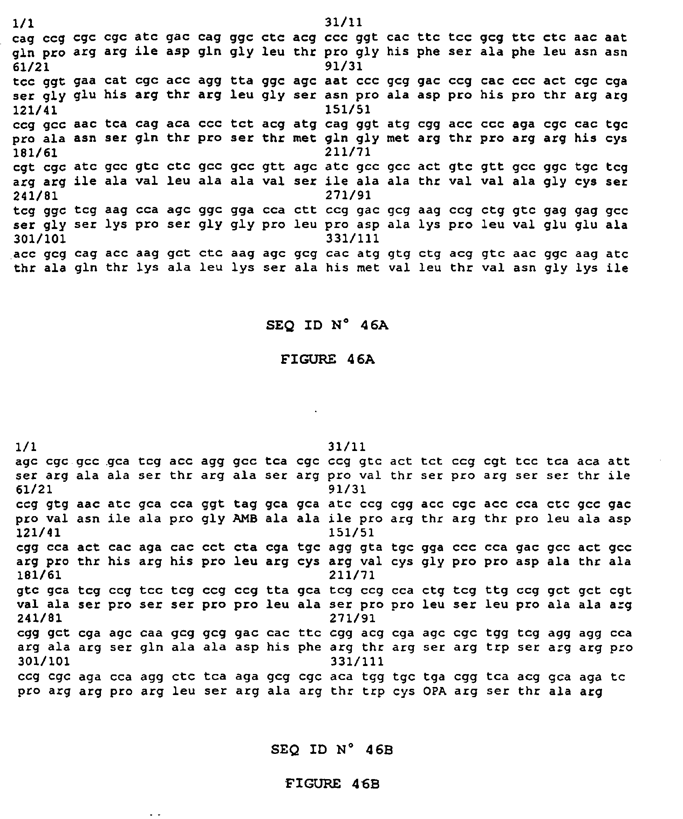

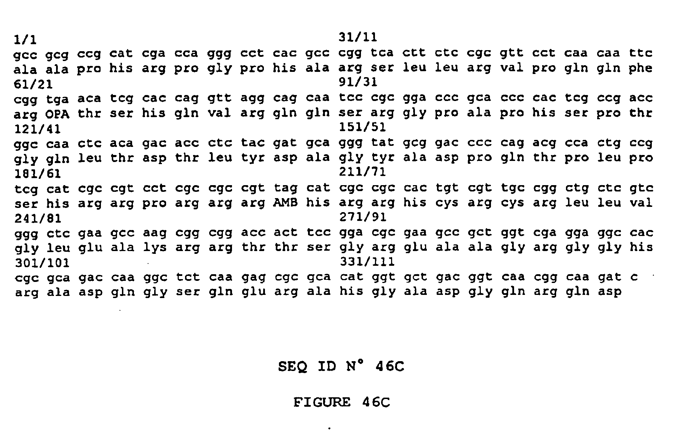

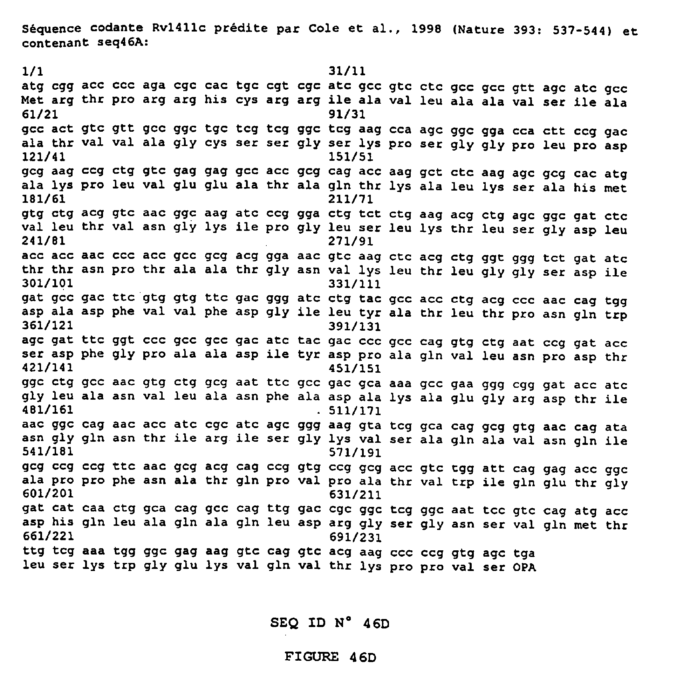

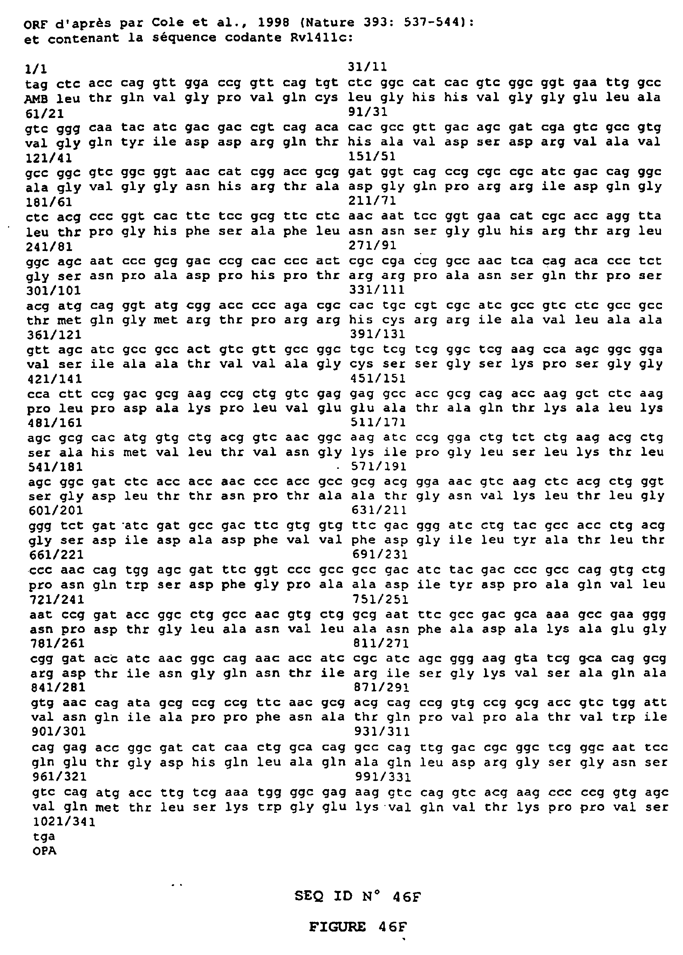

### La série de Figures 46 :

La série de Figures 46 représente la série de séquences nucléotidiques SEQ ID N°46.

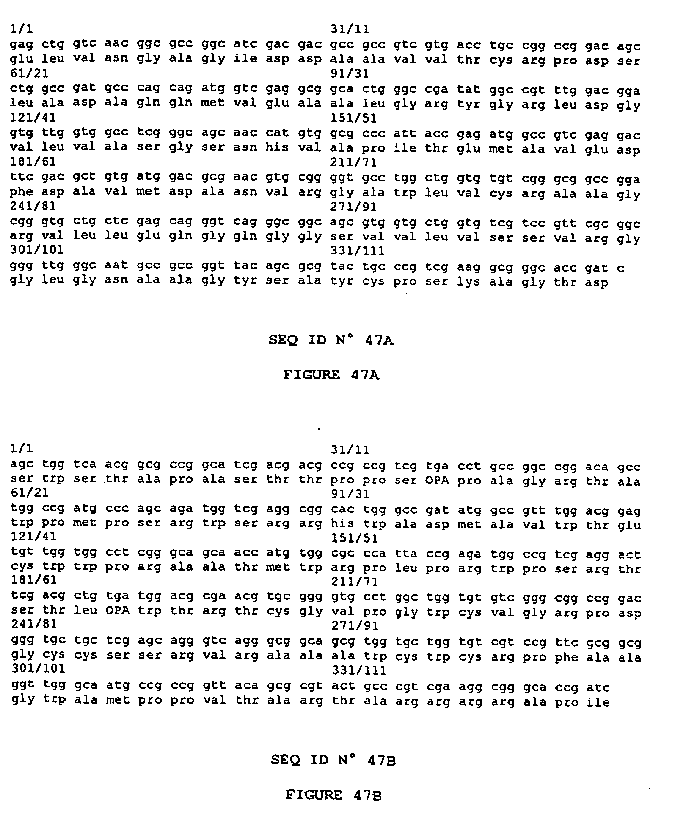

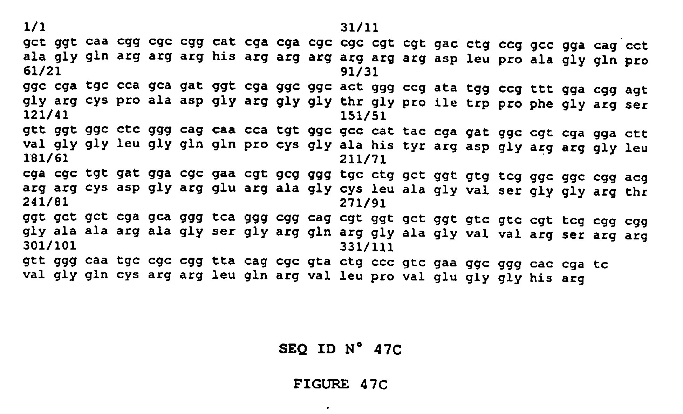

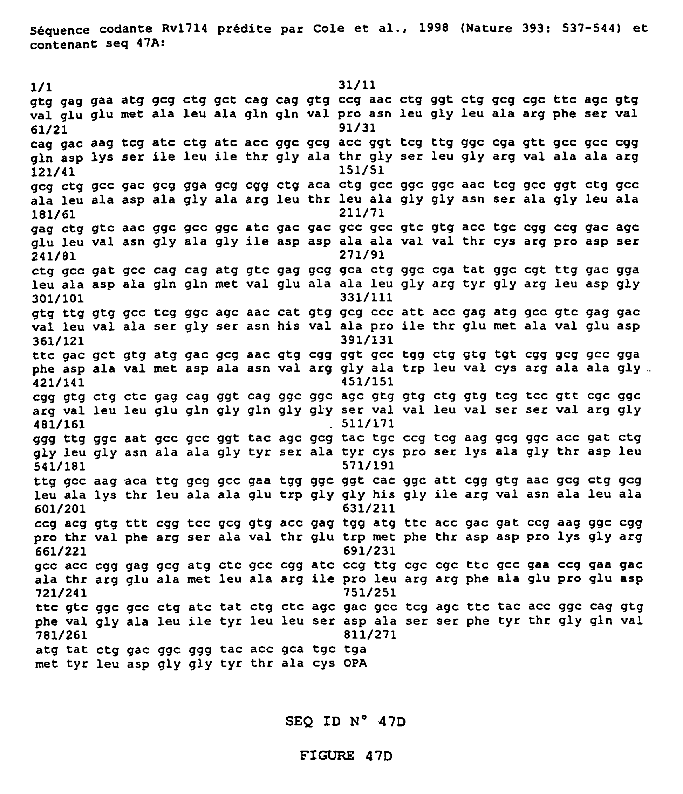

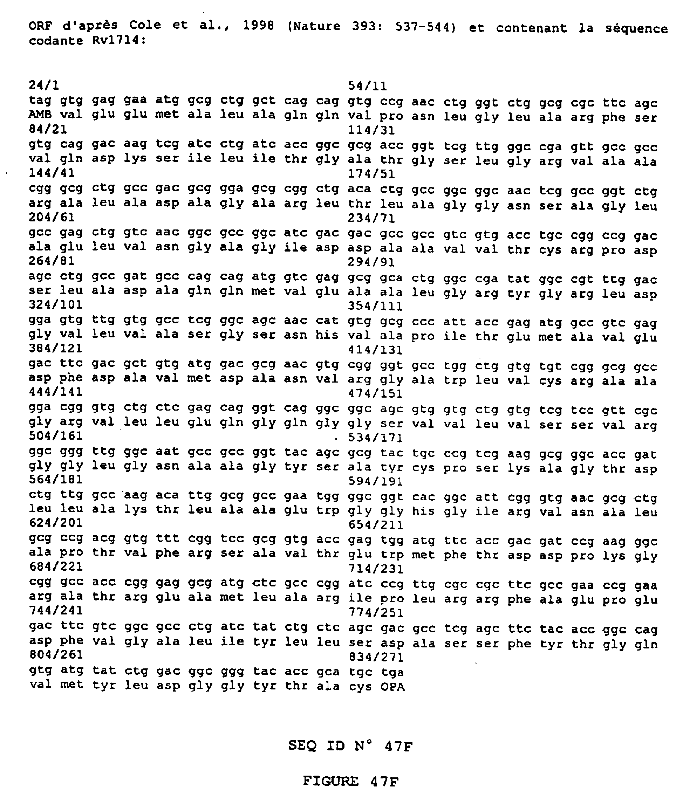

### La série de Figures 47 :

La série de Figures 47 représente la série de séquences nucléotidiques SEQ ID N°47.

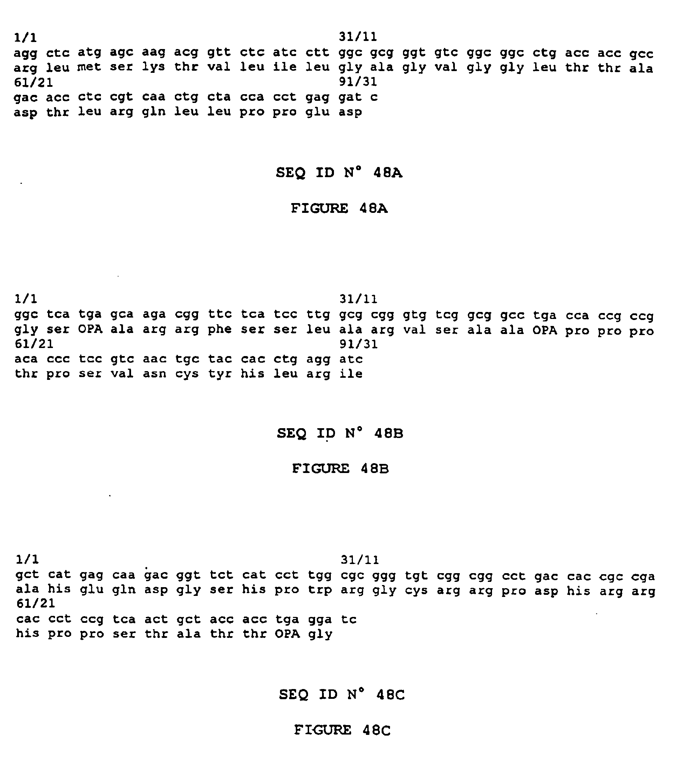

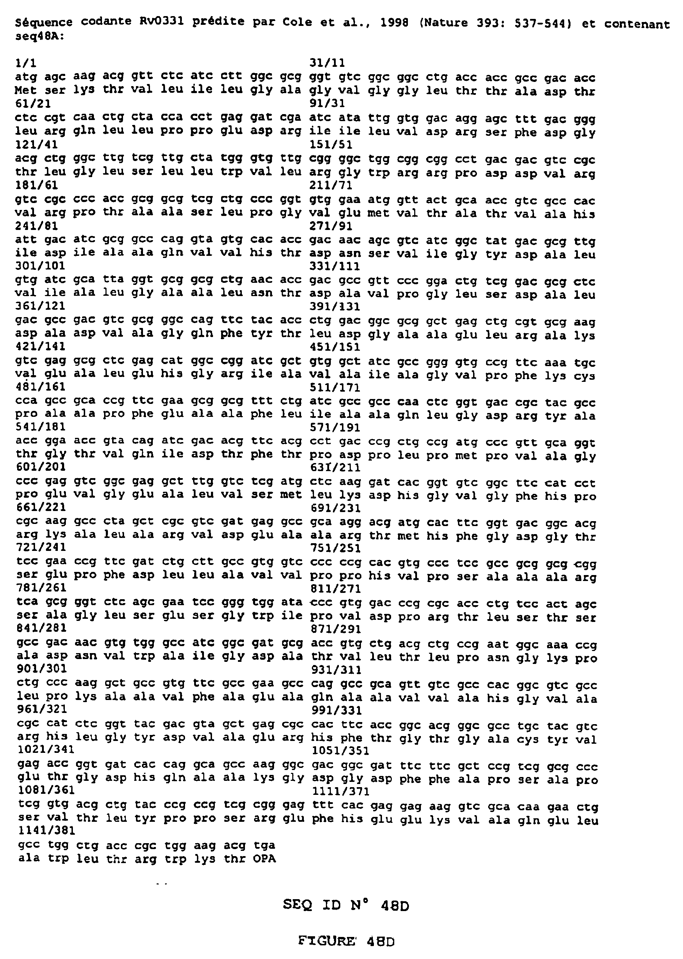

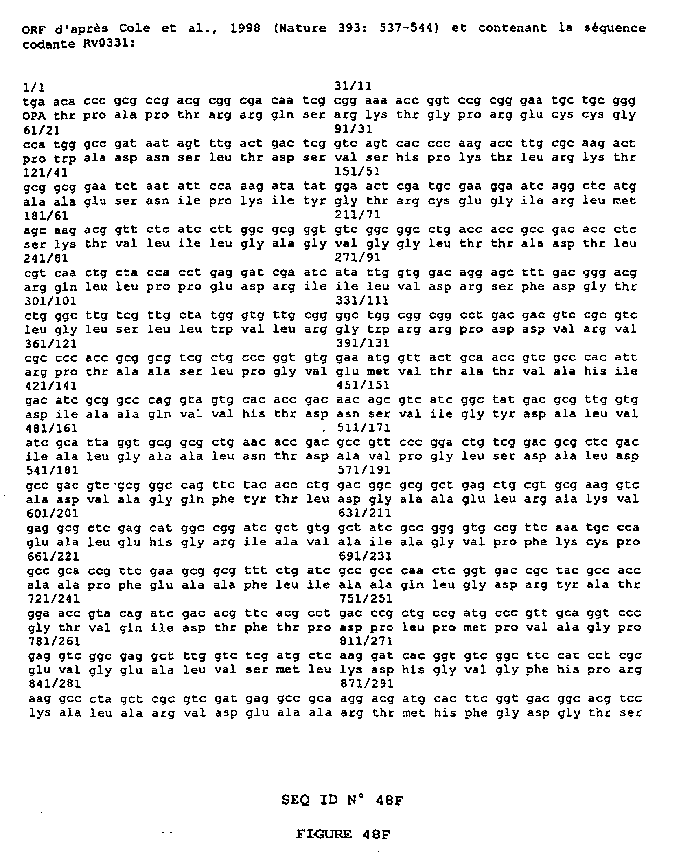

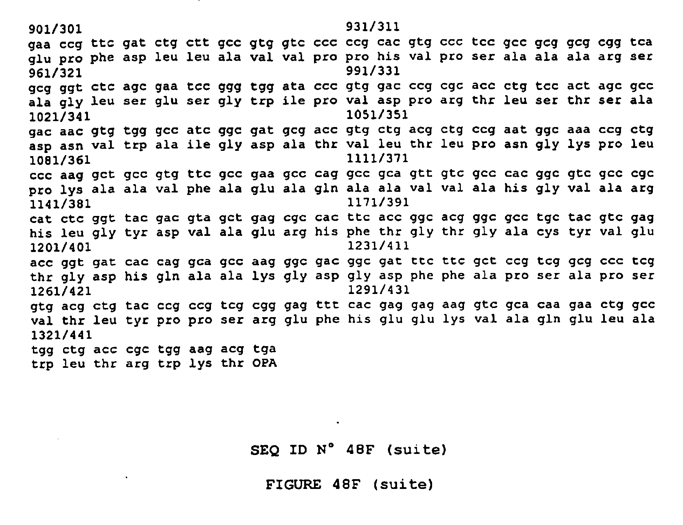

### La série de Figures 48 :

La série de Figures 48 représente la série de séquences nucléotidiques SEQ ID N°48.

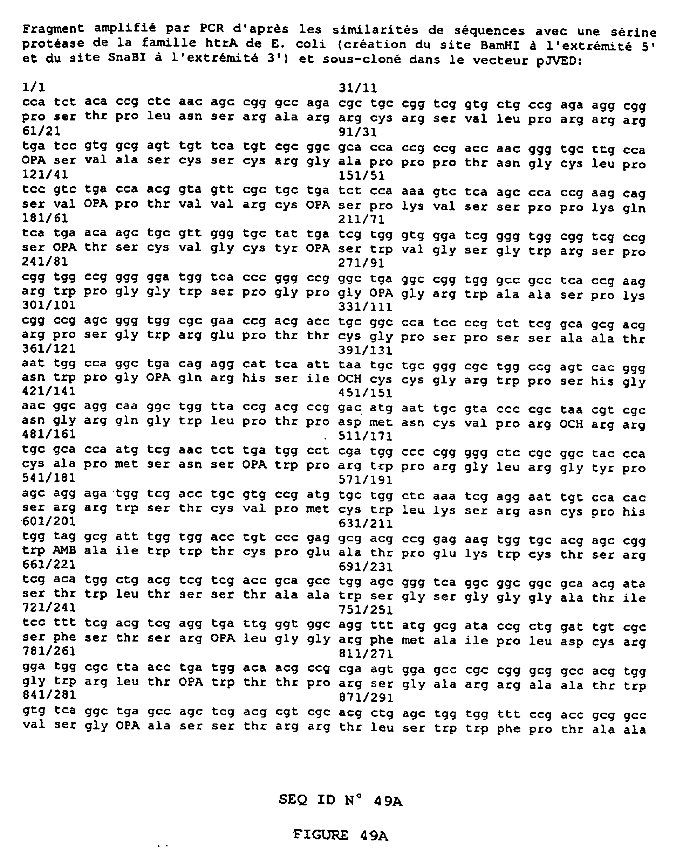

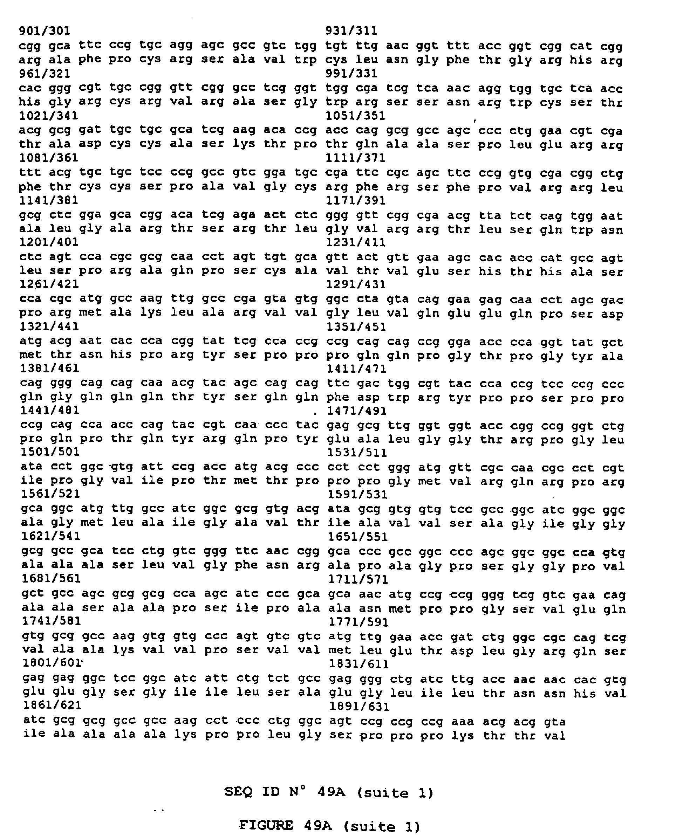

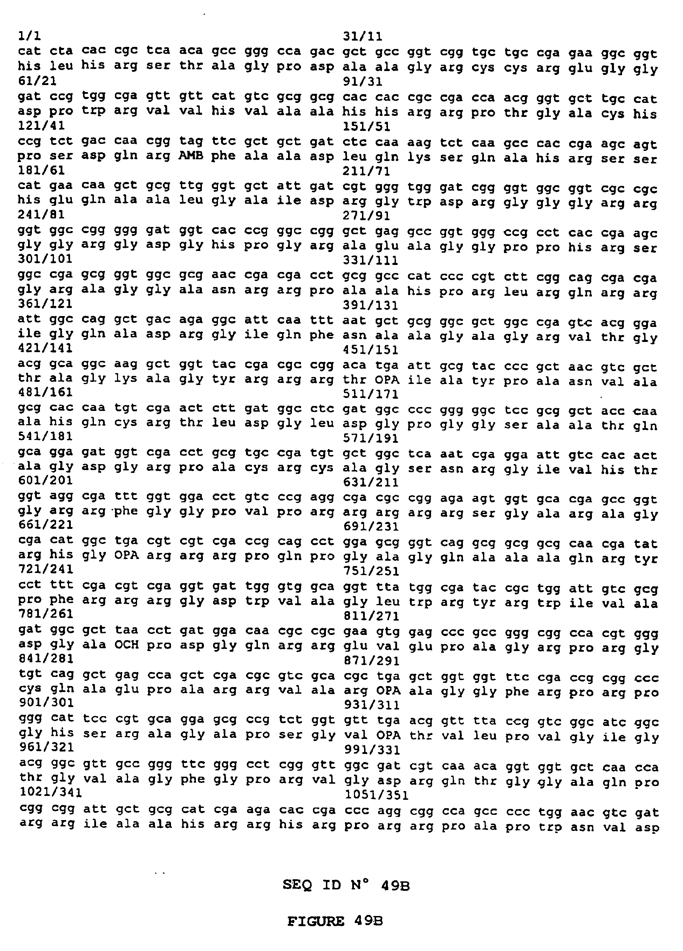

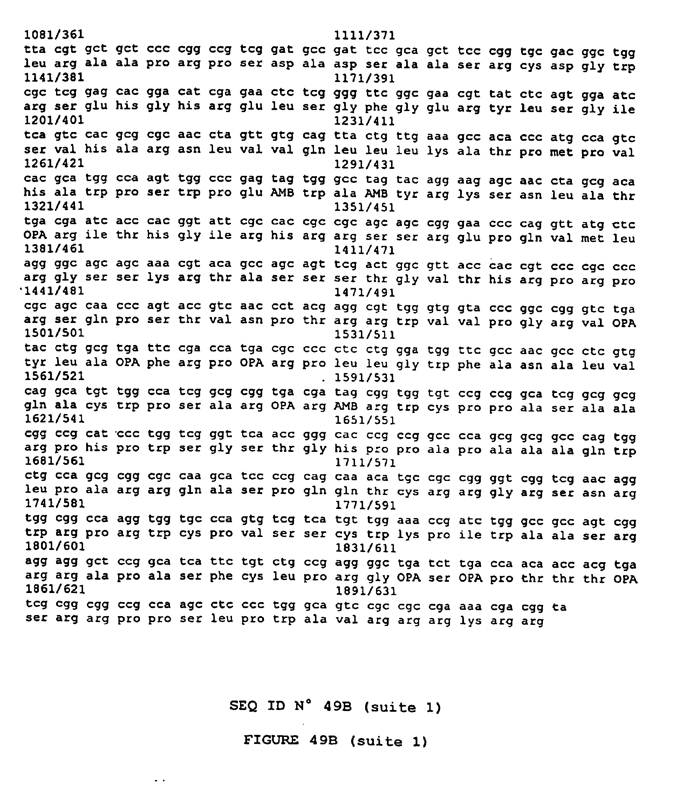

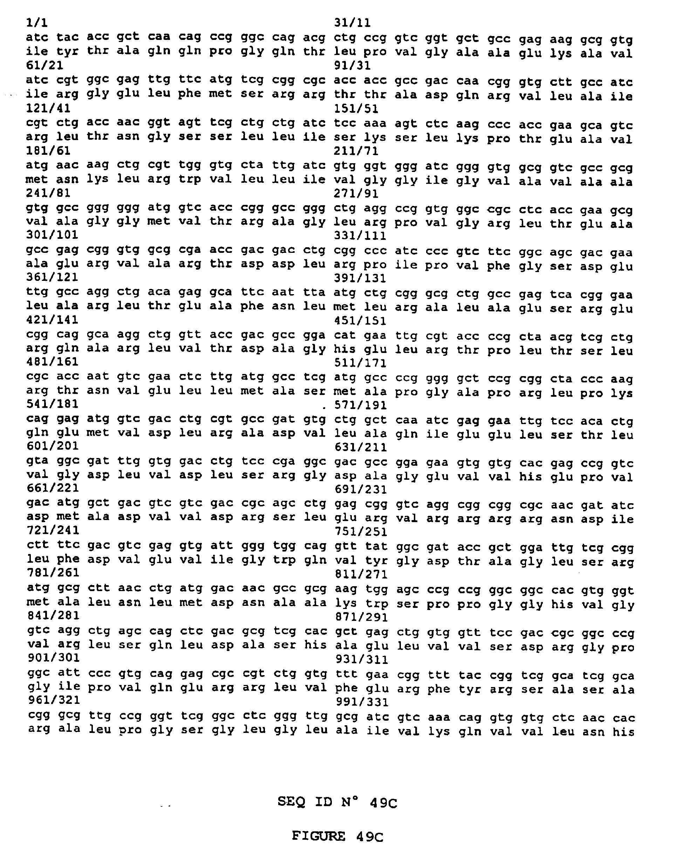

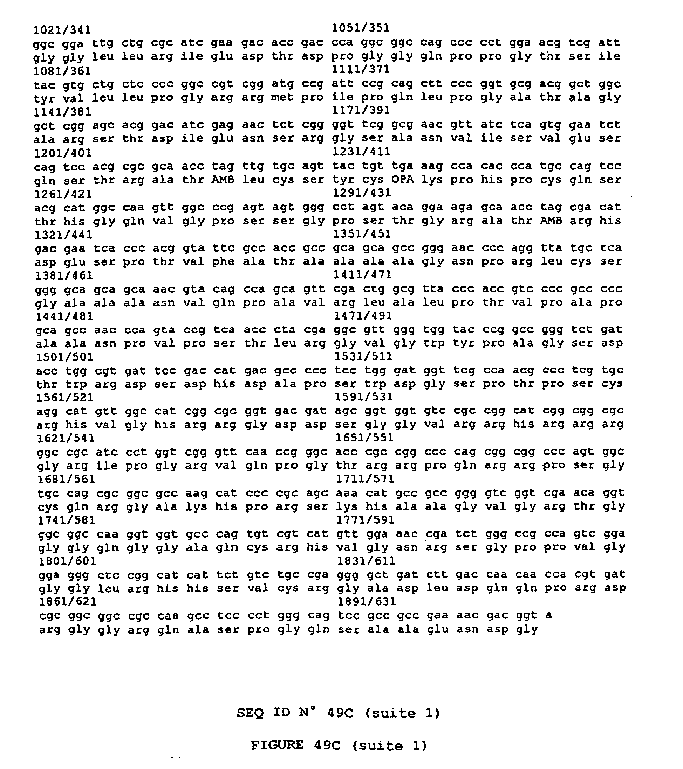

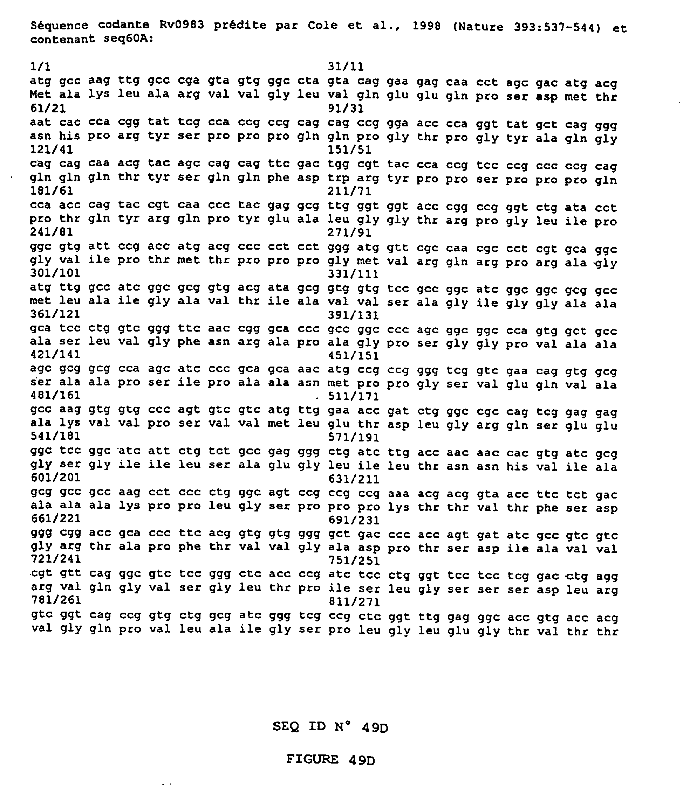

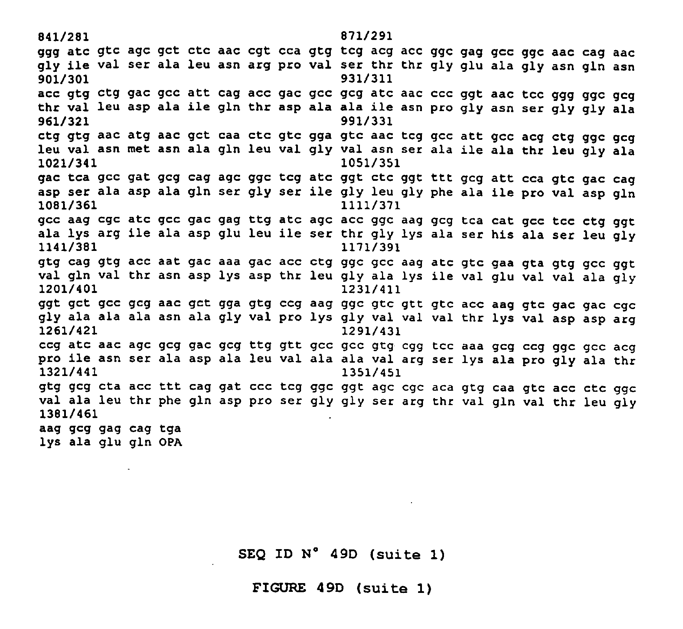

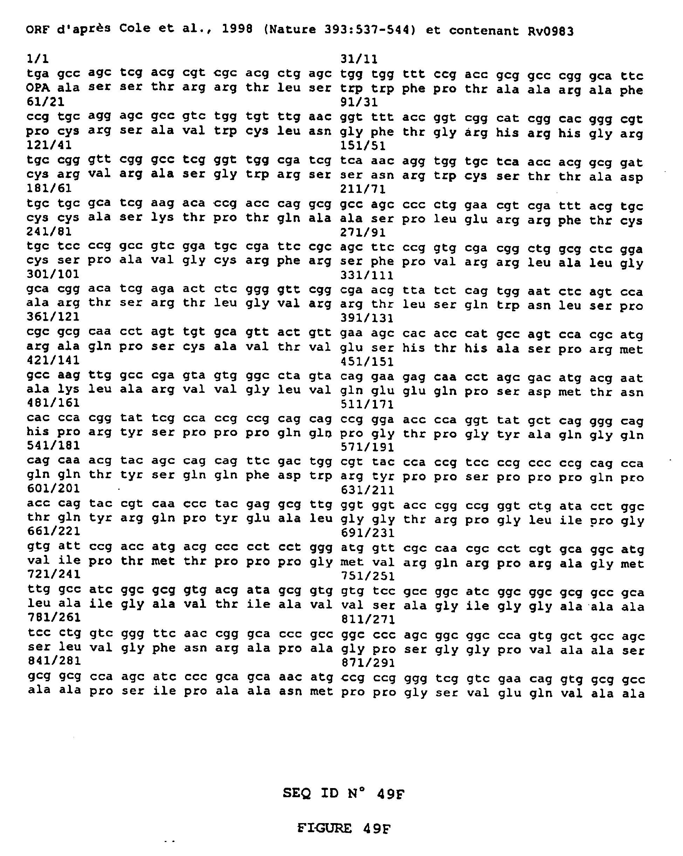

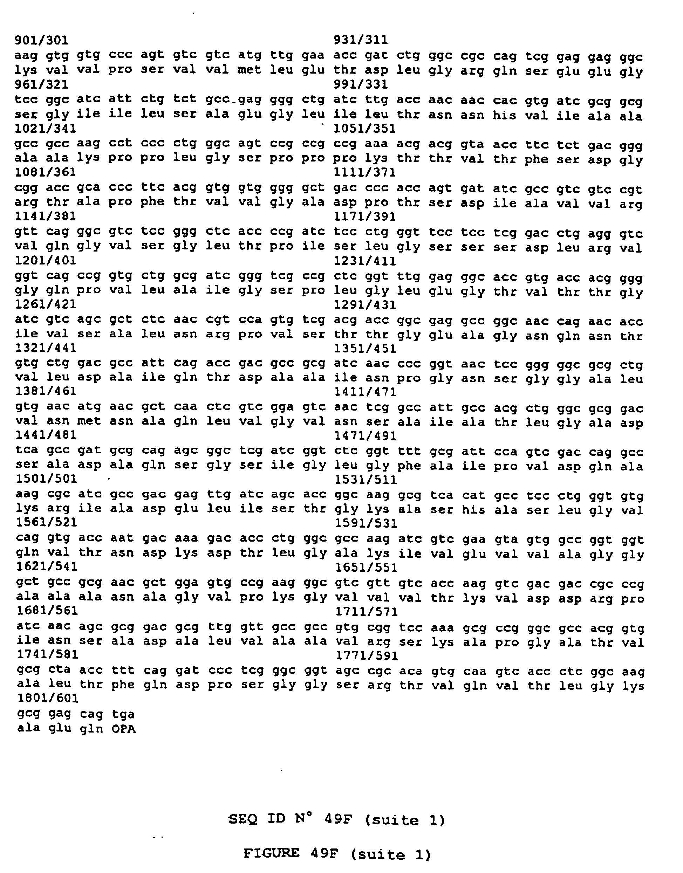

### La série de Figures 49 :

La série de Figures 49 représente la série de séquences nucléotidiques SEQ ID N°49.

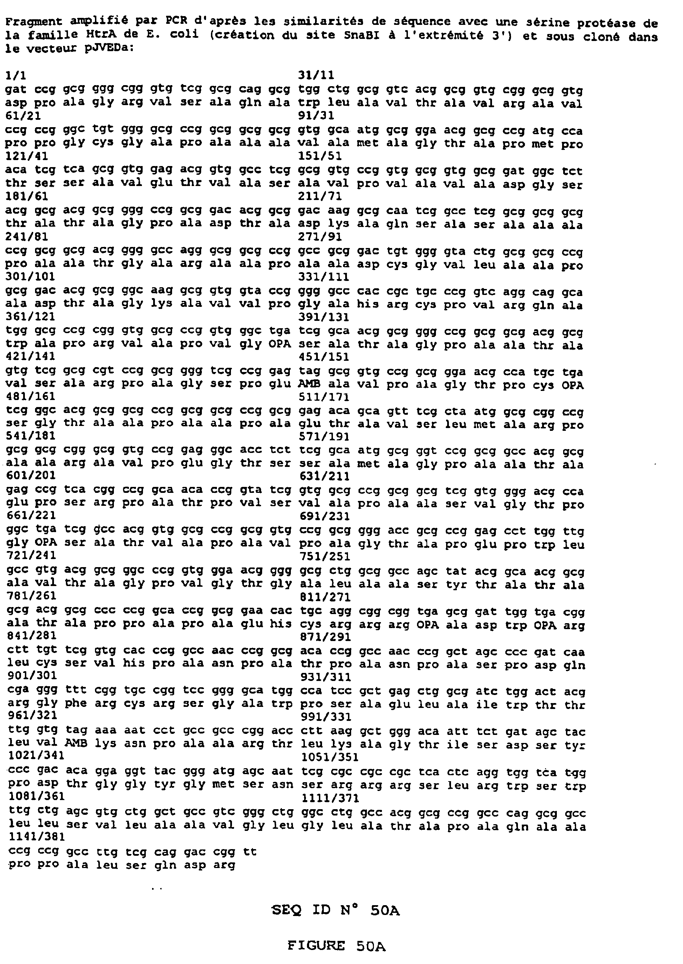

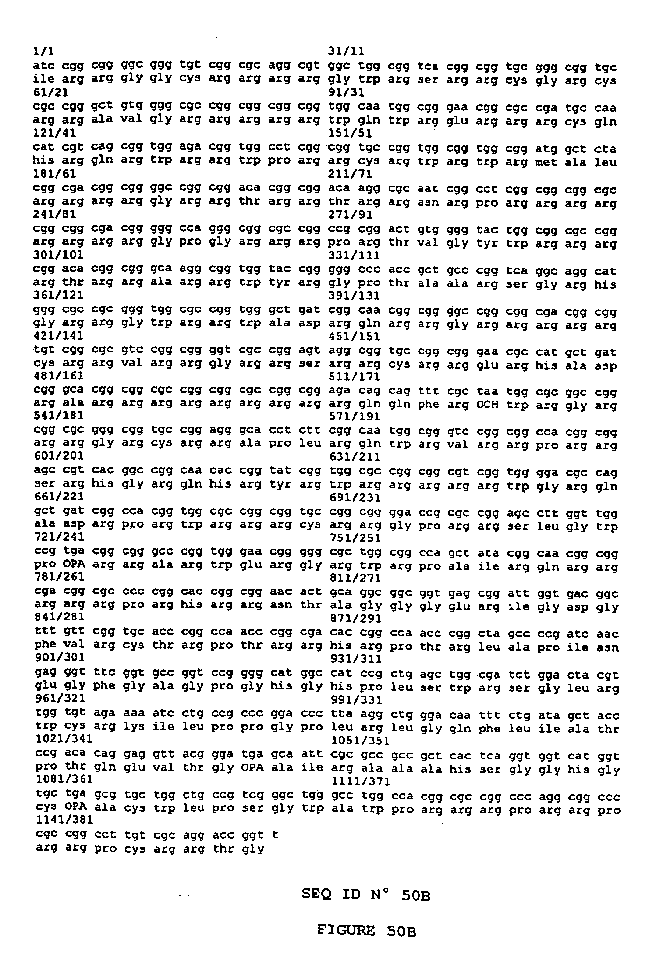

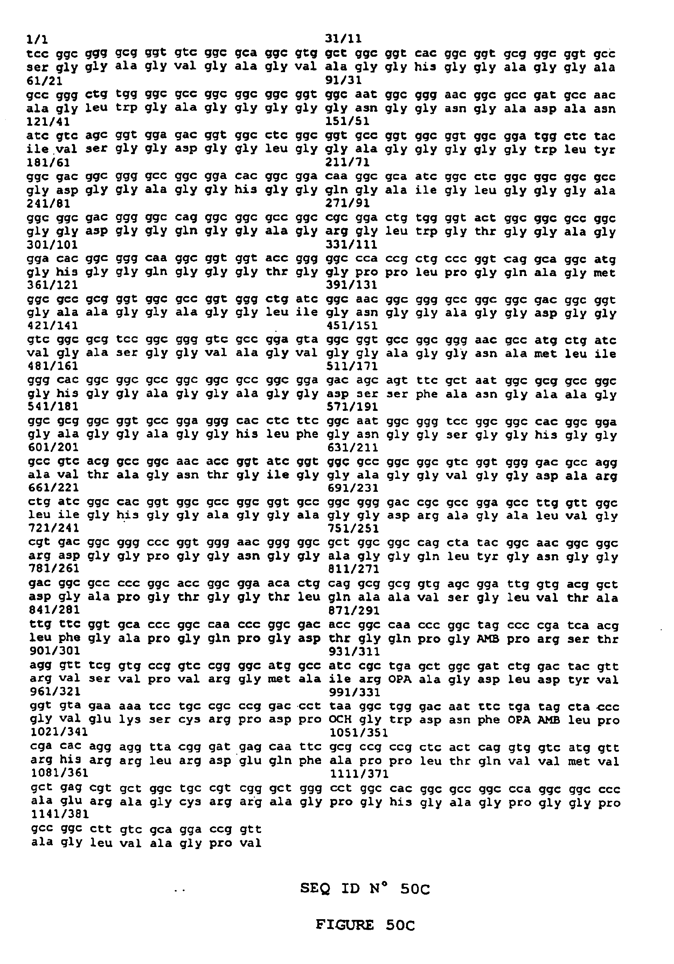

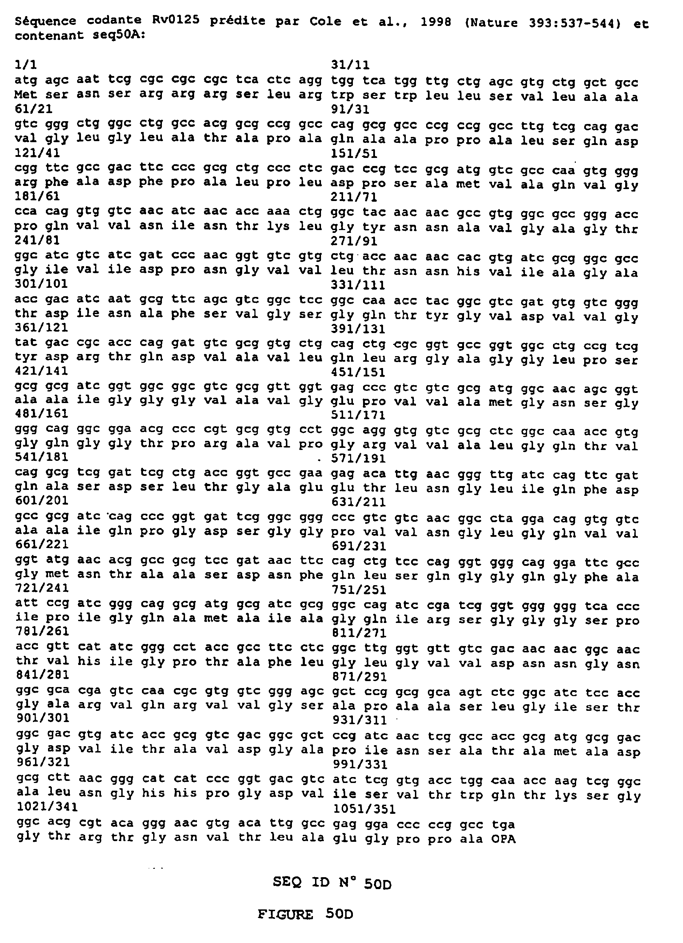

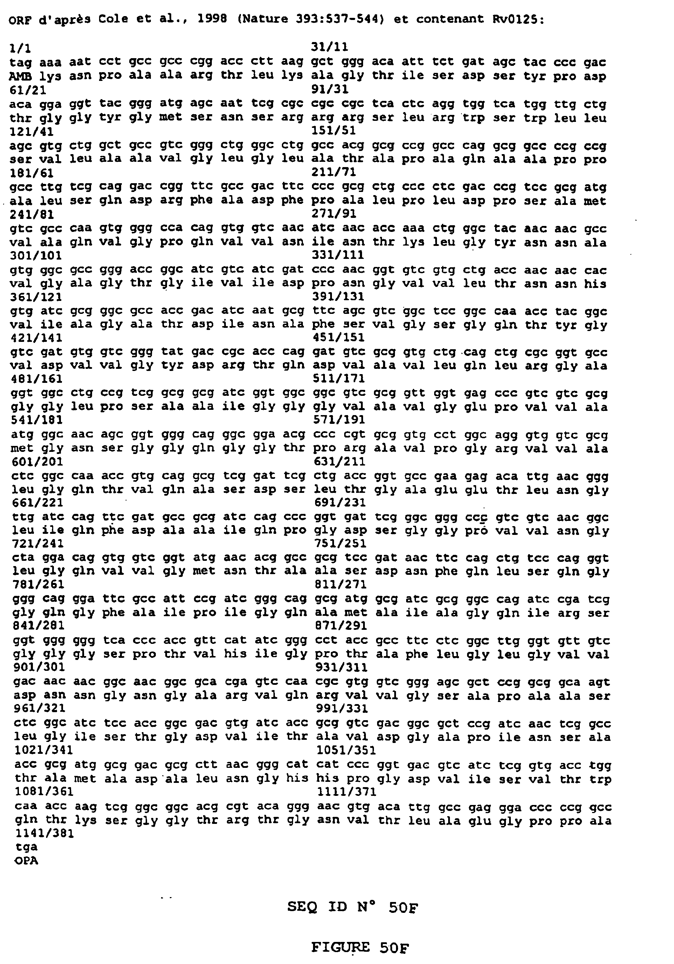

### La série de Figures 50 :

La série de Figures 50 représente la série de séquences nucléotidiques SEQ ID N°50.

### Figure 51 :

A. la construction pJVED: Plasmid navette( pouvant se multiplier chez les mycobactéries ainsi que chez *E.coli*). avec un gène de résistance à la kanamycine (issu de Tn*903*) comme marqueur de sélection. Le gène *phoA* tronqué (Δ *phoA)* et le gène *luc* forment un opéron synthetique.
B. Séquence de la jonction entre *phoA* et *luc.*

### Figure 51 :

Hybridation génomique (Southern blot) de l'ADN génomique de différentes espèces mycobactériennes à l'aide d'une sonde oligonucléotidique dont la séquence est la séquence comprise entre le nucléotide en position nt 964 (extrémité 5' de la sonde) et le nucléotide en position nt 1234 (extrémité 3' de la sonde), extrémités inclues, de la séquence SEQ ID N°1.

### Figures 53 et 54 :

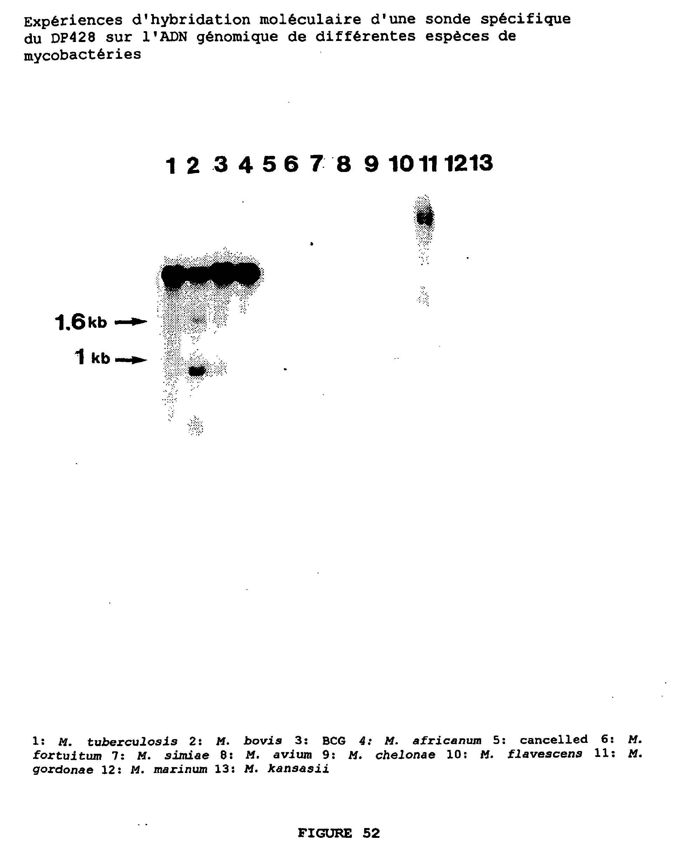

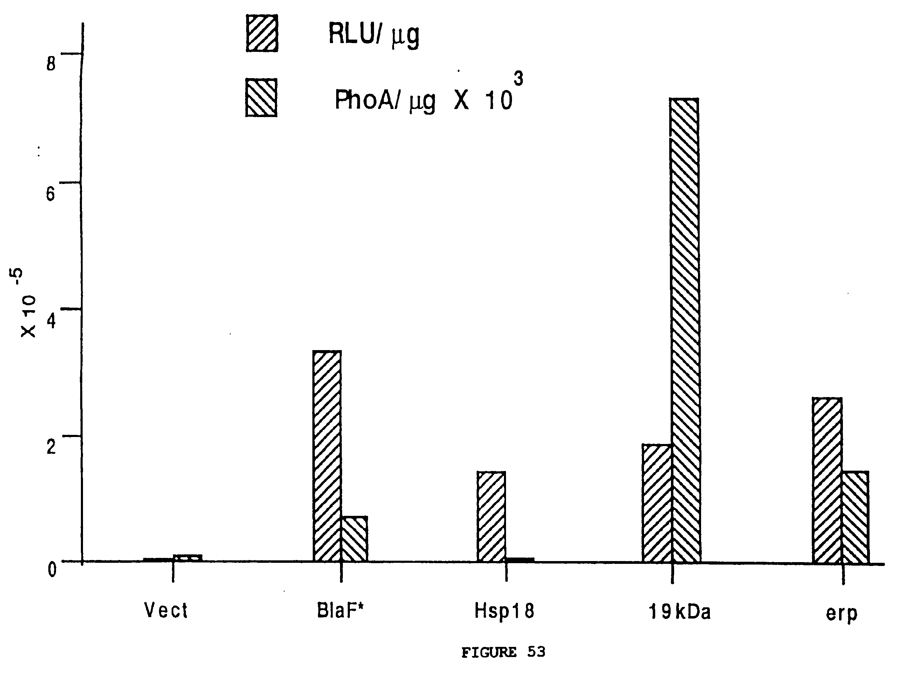

Activités Luc et PhoA de *M. smegmatis* recombinant contenant le pJVED avec différents fragments nucléotidiques comme décrits en exemple. Les figures 52 et 53 représentent les résultats obtenus pour deux expériences distinctes réalisées dans les mêmes conditions.

### Figure 55 :

Représentation de l'hydrophobicité (Kyte et Doolitle) de la séquence codante du polypeptide DP428 avec sa représentation schématique. Le motif LPISG précède immédiatement la région C-terminale hydrophobe. La séquence se termine par deux arginines.

### Figure 56 :

Représentation de l'hydrophoicité (Kyte et Doolitle) de la séquence du polypeptide M1C25 de séquence d'acides aminés SEQ ID N° 30.

### Figure 57 :

A- Gel d'acrylamide (12%) en condition dénaturante d'un extrait bactérien obtenu par sonication de bactéries *E. coli* M15 contenant le plasmide pM1C25 sans et après 4 heures d'induction par l'IPTG, coloré au bleu de Comassie.
   ligne 1: Marqueur de masse molaire (Prestained SDS-PAGE Standards High Range BIO-RAD@.
   ligne 2: Extrait bactérien obtenu par sonication de bactéries *E. coli* M15 contenant le plasmide pM1C25 sans induction par l'IPTG.
   ligne 3: Extrait bactérien obtenu par sonication de bactéries *E. coli* M15 contenant le plasmide pM1C25 après 4 heures d'induction par l'IPTG.
   ligne 4: Marqueur de masse molaire (Prestained SDS-PAGE Standards Low Range BIO-RAD@).
B- Western blot d'un gel semblable gel (acrylamide 12%) révélé grâce à l'anticorps penta-His commercialisé par la société Quiagen.
   ligne 1: représentation du marqueur de masse molaire (Prestained SDS-PAGE Standards High Range BIO-RAD@).
   ligne 2:extrait bactérien obtenu par sonication de bactéries *E. coli* M15 contenant le plasmide pM1C25 sans induction par l'IPTG.
   ligne 3:extrait bactérien obtenu par sonication de bactéries *E. coli* M15 contenant le plasmide pMlC25 après 4 heures d'induction par l'IPTG.
   ligne 4: représentation du marqueur de masse molaire (Prestained SDS-PAGE Standards Low Range BIO-RAD@)
La bande présente très majoritairement dans les lignes correspondant aux bactéries induites par l'IPTG par rapport à celles non induites par l'IPTG, comprise entre 34200 et 28400 daltons, correspond à l'expression de l'insert M1C25 cloné dans le vecteur pQE-60 (Qiagen@).

En ce qui concerne les légendes des autres figures qui sont numérotées par un caractère alphanumérique, chacune de ces autres figures représente la séquence nucléotidique et la séquence d'acides aminés de séquence SEQ ID dont la numérotation est identique au caractère alphanumérique de chacune desdites figures.
Les numérotations alphanumériques des figures représentant les SEQ ID comportant un nombre suivi d'une lettre ont les significations suivantes :
- les numérotations alphanumériques présentant le même nombre concernent une même famille de séquence rattachées à la séquence de référence SEQ ID dont la numérotation présente ce même nombre et la lettre A ;
- les lettres A, B et C pour une même famille de séquences distinguent les trois phases de lecture possibles de la séquence nucléotidique SEQ ID de référence (A) ;
- les lettres indexées par un prime (') signifient que la séquence correspond à un fragment de la séquence SEQ ID de référence (A) ;
- la lettre D signifie que la séquence correspond à la séquence du gène prédit par Cole et al., 1998 ;
- la lettre F signifie que la séquence correspond à la phase ouverte de lecture (ORF pour "Open Reading Frame") contenant la séquence "D" correspondante d'après Cole et al., 1998 ;
- la lettre G signifie que la séquence est une séquence prédite par Cole et al., 1998, et présentant une homologie de plus de 70% avec la séquence SEQ ID de référence (A) ;
- la lettre H signifie que la séquence correspond à la phase ouverte de lecture contenant la séquence "G" correspondante d'après Cole et al., 1998 ;
- la lettre R signifie que la séquence correspond à une séquence prédite par Cole et al., 1998, en amont de la séquence "D" correspondante et pouvant être en phase avec la séquence "D" en raison d'erreurs de séquençage possibles ;
- la lettre P signifie que la séquence correspond à la phase ouverte de lecture contenant la séquence "R" correspondante ;
- la lettre Q signifie que la séquence correspond à une séquence contenant les séquences "F" et "P" correspondantes.
   En ce qui concerne la famille de séquences SEQ ID N° 4, l'insert précédent *phoA* contient deux fragments non contigus sur le génome, SEQ ID 4J et SEQ ID 4A, et donc issus d'un clonage multiple permettant l'expression et l'exportation de *phoA.* Ces deux fragments non contigus, les gènes et les phases ouvertes de lecture qui les contiennent d'après Cole et al., 1998, sont importants pour l'exportation d'un polypeptide antigène :

- les lettres J, K et L distinguent les trois phases de lecture possibles de la séquence nucléotidique "J" correspondante ;
- la lettre M signifie que la séquence correspond à la séquence prédite par Cole et al., 1998, et contenant la séquence SEQ ID N° 4J ;
- la lettre N signifie que la séquence correspond à la phase ouverte de lecture contenant la séquence SEQ ID N° 4M.
En ce qui concerne la famille de séquences SEQ ID N° 45, la lettre Z signifie que la séquence correspond à la séquence d'un fragment cloné fusionné avec *phoA.*
Enfin, en ce qui concerne la famille de séquence SEQ ID N° 41, la lettre S signifie que la séquence correspond à une séquence prédite par Cole et al., 1998 et pouvant être dans la même phase de lecture que la séquence "D" correspondante, la lettre T signifiant que la séquence correspondante contient les séquences "F" et "S" correspondantes.

### EXEMPLES

### Matériel et méthodes

### Cultures bactériennes, plasmides et milieux de cultures

*E. coli* a été cultivé sur milieu liquide ou solide Luria-Bertani (LB). *M. smegmatis* a été cultivé sur milieu liquide Middlebrook 7H9 (Difco) additionné de dextrose albumine (ADC), 0,2 % de glycérol et 0,05 % de Tween, ou sur milieu solide L. Si nécessaire, l'antibiotique kanamycine a été rajouté à une concentration de 20 µg/ml- 1. Les clones bactériens présentant une activité PhoA ont été détectés sur de l'agar LB contenant du 5-bromo-4-chloro-3-indolyle phosphate (X-P, à 40 µg/ml- 1).

### Manipulation d'ADN et séquençage

Les manipulations d'ADN et les analyses par Southern blot ont été effectuées en utilisant les techniques standard (Sambrook et al., 1989). Les séquences d'ADN double brun ont été déterminées avec un kit de séquençage Taq Dye Deoxy Terminator Cycle (Applied Biosystems), dans un Système 9600 GeneAmp PCR (Perkin-Elmer), et après migration sur un système d'analyse ADN modèle 373 (Applied Biosystems) .

### Constructions des plasmides

Le plasmide pJVEDₐ a été construit à partir de pLA71, plasmide de transfert comportant le gène *phoA* tronqué et placé en phase avec *BlaF.* pLA71 a été coupé avec les enzymes de restriction *Kpn*I et *Not*I, retirant ainsi *phoA* sans toucher le promoteur de *BlaF*. Le gène *luc* codant pour la luciférase de luciole a été amplifié à partir de pGEM-*luc* et un site de liaison du ribosome a été rajouté. *phoA* a été amplifié à partir de pJEM11. Les fragments amplifiés ont été coupés avec PstI et ligaturés ensemble. Les oligodéoxynucléotides utilisés sont les suivants :
pPV.luc.Fw : 5'GACTGCTGCAGAAGGAGAAGATCCAAATGG3'
luc.Bw : 5'GACTAGCGGCCGCGAATTCGTCGACCTCCGAGG3'
pJEM.phoA.Fw : 5'CCGCGGATCCGGATACGTAC3'
phoA.Bw: 5'GACTGCTGCAGTTTATTTCAGCCCCAGAGCG3'
Le fragment ainsi obtenu a été réamplifié en utilisant les oligonucléotides complémentaires de ses extrémités, coupé avec *KpnI* et *Not*I, et intégré dans pLA71 coupé avec les mêmes enzymes. La construction résultante a été électroporée dans *E. coli* DHSα et *M. smegmatis* mc2 155. Un clone *M. smegmatis* émettant de la lumière et présentant une activité *phoA* a été sélectionné et appelé pJVED/*blaF*. L'insert a été retiré en utilisant *Bam*HI et la construction refermée sur elle-même, reconstruisant ainsi le pJVEDₐ. Afin d'obtenir le pJVED_{b,c}, le multisite de clonage a été coupé avec ScaI et *KpnI* et refermé en enlevant un (pJVED_{b}) ou deux (pJVED_{c}) nucléotides du site *Sna*BI. Après fusion six cadres de lecture ont pu ainsi être obtenus. L'insert du pJVED/*hsp18* a été obtenu par amplification en chaîne par polymérase (ACP) de pPM1745 (Servant et al., 1995) en utilisant des oligonucléotides de la séquence :
18.Fw : 5'GTACCAGTACTGATCACCCGTCTCCCGCAC3'
18.Back : AGTCAGGTACCTCGCGGAAGGGGTCAGTGCG3'
Le produit a été coupé avec *Kpn*I et *ScaI, et* ligaturé à pJVEDₐ, coupé avec les mêmes enzymes, quittant ainsi le pJVED/*hsp18*.

Le pJVED/*P19kDa* et le pJVED/*erp* furent construits en coupant avec *Bam*HI l'insert de pExp410 et pExp53 respectivement, et en les insérant dans le site *Bam*HI du multisite de clonage de pJVEDₐ.

### Mesure de l'activité phosphatase alkaline

La présence d'activité est détectée par la couleur bleue des colonies croissant sur un milieu de culture contenant le substrat 5-bromo 4-chloro 3-indolyl phosphate (XP), puis l'activité peut être mesurée quantitativement de manière plus préçise de la façon suivante :
*M. smegmatis* ont été cultivés dans un milieu LB additionnés de 0,05 % de Tween 80 (Aldrich) et de kanamycine (20 µg/ml- 1) à 37°C pendant 24 heures. L'activité de la phosphatase alkaline a été mesurée par la méthode de Brockman et Heppel (Brockman et al., 1968) dans un extrait soniqué, avec *p*-nitrophénylphosphate comme substrat de la réaction. La quantité de protéines a été mesurée par essai Bio-Rad. L'activité phosphatase alkaline est exprimée en unité arbitraire (densité optique à 420 nm x µg de protéines- 1 x minutes- 1).

### Mesure de l'activité luciférase

*M. smegmatis* a été cultivé dans un milieu LB additionné de 0,05 % de Tween 80 (Aldrich) et de kanamycine (20 µg/ml- 1) à 37°C pendant 24 heures et utilisé en pleine croissance exponentielle (DO à 600 nm comprise entre 0,3 et 0,8). Les aliquots de suspensions bactériennes ont été brièvement soniqués et l'extrait cellulaire a été utilisé pour mesurer l'activité de la luciférase. 25 µl de l'extrait soniqué ont été mélangés avec 100 µl de substrat (système d'essai luciférase Promega) automatiquement dans un luminomètre et la lumière émise exprimée en ULR ou RLU (Unités Lumineuses Relatives). Les bactéries ont été comptées par dilutions sérielles de la suspension d'origine sur milieu agar LB kanamycine et l'activité de la luciférase exprimée en ULR/µg de protéines bactériennes ou en ULR/10⁻³ bactéries.

Construction de banques génomiques de *M. tuberculosis* et de *M. bovis-BCG*

Les banques ont été obtenues en utilisant essentiellement pJVED_{a,b,c} précédemment décrits.

### Préparation de macrophages issus de la moelle osseuse et infection par M. smegmatis recombinants

Les macrophages issus de la moelle osseuse ont été préparés comme décrits par Lang et al., 1991. En résumé, les cellules de la moelle osseuse ont été prélevés du fémur de souris C57BL/6 agée de 6 à 12 semaines (Iffa-Credo, France). Les cellules en suspensions ont été lavées et resuspendues dans du DMEM enrichi avec 10 % de sérum foetal de veau, 10 % de milieu L-cell conditionné et 2 mM de glutamine, sans antibiotiques. 106 cellules ont été ensemencées sur des plaques 24 puits Costar à fond plat dans 1 ml. Après quatre jours à 37°C dans une atmosphère humide à 10 % de teneur en CO2, les macrophages ont été rincés et réincubés pendant deux à quatre jours supplémentaires. Les cellules d'un puits contrôle ont été lysées avec du triton x 100 à 0,1 % dans l'eau et les noyaux énumérés. Environ 5 x 105 cellules adhérentes ont été comptées. Pour l'infection, *M. smegmatis* portant les différents plasmides a été cultivé en pleine phase exponentielle (DO₆₀₀ₙₘ entre 0,4 et 0,8) et dilué jusqu'à une DO de 0,1 puis 10 fois dans un milieu pour macrophage. 1 ml a été ajouté à chaque puits et les plaques ont été centrifugées et incubées quatre heures à 37°C. Après trois lavages, les cellules ont été incubées dans un milieu contenant de l'amykacine pendant deux heures. Après trois nouveaux lavages, les cellules infectées adhérentes ont été incubées dans un milieu macrophage pendant une nuit. Les cellules ont ensuite été lysées dans 0,5 ml de tampon de lyse (Promega). 100 µl ont été soniqués et la lumière émise a été mesurée sur 25 µm. Simultanément, les bactéries ont été énumérées par étalement sur L-agar-kanamycine (20 µg/ml- 1). La lumière émise est exprimée en ULR/103 bactéries.

### Analyses des banques de données

Les séquences nucléotidiques ont été comparées à EMBL et GenBank en utilisant l'algorithme FASTA et les séquences protéiques ont été analysées par similitude grâce aux banques de données PIR et Swiss Prot en utilisant l'algorithme BLAST .

### Exemple 1 : Les vecteurs pJVED

Les vecteurs pJVED (Figure 51) sont des plasmides portant un gène *phoA* tronqué de *E. coli* dépourvu de codon d'initiation, de séquence signal et de séquence régulatrice. Le site multiple de clonage (SMC) permet l'insertion de fragments des gènes codants pour d'éventuelles protéines exportées ainsi que leurs séquences de régulation. Dès lors, la protéine de fusion peut être produite et présenter une activité phosphatase alcaline si elle est exportée. Seules les fusions en phase pourront être productives. Ainsi, le SMC a été modifié de sorte que les fusions peuvent être obtenues dans six phases de lecture. En aval de *phoA,* le gène *luc* de la luciférase de luciole a été inséré. Le gène complet avec le codon d'initiation mais sans qu'aucun promoteur n'ait été utilisé devrait ainsi s'exprimer avec *phoA* comme dans un opéron synthétique. Un nouveau site de liaison des ribosomes a été inséré huit nucléotides en amont du codon d'initiation de *luc*. Deux terminateurs transcriptionnels sont présents dans les vecteurs pJVED, un en amont du SMC et un second en aval de *luc.* Ces vecteurs sont des plasmides de transfert *E. coli*-mycobacterium avec un gène de résistance à la kanamycine comme marqueur de sélection.

### phoA et luc fonctionnent comme dans un opéron, mais l'exportation est nécessaire pour l'activité phoA.

Quatre plasmides ont été construits par insertion dans le SMC de fragments d'ADN d'origine diverse :
Dans la première construction nommée pJVED/*blaF*, le fragment de 1,4 kb provient du plasmide déjà décrit pLA71 (Lim et al., 1995). Ce fragment issu du gène β-lactamase (*blaF*) de *M. fortuitum* D216 (Timm et al., 1994) inclut le promoteur muté hyperactif, le segment codant pour 32 acides aminés de la séquence signal et les 5 premiers acides aminés de la protéine mature. Ainsi cette construction inclut le promoteur le plus fort connu chez mycobacterium et les éléments nécessaires à l'exportation de la protéine de la fusion *phoA*. Par conséquent, on peut attendre de cette construction une forte émission de lumière et une bonne activité *phoA* (cf figures 53 et 54).
Dans une deuxième construction nommée pJVED/*hsp18,* un fragment de 1,5 kb a été cloné à partir du plasmide déjà décrit pPM1745 (Servant et al., 1995). Ce fragment inclut les nucléotides codants pour les dix premiers acides aminés de la protéine de choc thermique de 18 kb issue de *Streptomyces albus* (heat shock protein 18, HSP 18), le site de liaison du ribosome, le promoteur et, en amont, des sites régulateurs contrôlant son expression. Cette protéine appartient à la famille de alpha-crystalline de HSP à faible poids moléculaire (Verbon et al., 1992). Son homologue issu de *M. leprae,* l'antigène de 18 kDa, est déjà connu pour être induit durant la phagocytose par un macrophage murin de la lignée cellulaire J-774 (Dellagostinet al., 1995). Dans des conditions de culture standard, le pJVED/*hsp18*, montre une faible activité *luc* et aucune activité *phoA* (cf figures 53 et 54).
Dans une troisième construction, nommée pJVED/*P19kDa*, l'insert issu de pExp410 (Lim et al., 1995) a été coupé et cloné dans le SMC de pJVEDₐ. Ce fragment inclut les nucléotides codants pour les 134 premiers acides aminés de la protéine connue de *M. tuberculosis* 19 kDa et de ses séquences régulatrices. Comme cela a pu être mis en évidence, cette protéine est une lipoprotéine glycosylée (Garbe et al., 1993 ; Herrmann et al., 1996). Sur les figures 53 et 54, on observe, pour cette construction, une bonne activité *luc* correspondant à un promoteur fort, mais l'activité *phoA* est la plus forte des quatre constructions. L'activité *phoA* élevée de cette protéine de fusion avec une lipoprotéine s'explique par le fait qu'elle reste attachée à la paroi cellulaire par son extrémité N-terminal.
Dans la quatrième et dernière construction nommée pJVED/*erp* l'insert provient de pExp53 (Lim et al., 1995) et a été cloné dans le SMC de pJVEDₐ. pExp53 est le plasmide initial sélectionné pour son activité *phoA* et contenant une partie du gène *erp* de *M. tuberculosis* qui code pour un antigène de 28 kDa. Ce dernier inclut la séquence signal, une partie de la protéine mature et, en amont du codon d'initiation, le site de liaison de ribosome. Le promoteur a été cartographie. Une boîte fer (iron box) putative du type fur est présente dans cette région et encadre la région -35 du promoteur (Berthet et al ., 1995). Comme prévu (figures 53 et 54) cette construction présente une bonne émission lumineuse et une bonne activité *phoA*. Le fait que cette protéine de fusion, contrairement à la fusion avec la lipoprotéine de 19 kDa, ne semble pas attachée à la paroi cellulaire n'exclut pas que la protéine native y soit associée. De plus, l'extrémité C-terminal de erp est absente de la protéine de fusion.

### Exemple 2 : Construction d'une banque d'ADN génomique de M. tuberculosis dans les vecteurs pJVEDs et identification d'un des membres de ces banques, (DP428), induit au cours de la phagocytose par les macrophages murins dérivés de la moelle osseuse.

Les différentes constructions sont testées pour leur capacité à évaluer l'expression intracellulaire des gènes identifiés par l'expression de *phoA.* Dans cet objectif, l'activité *luc* est exprimée en URL pour 10³ bactéries en culture axénique et/ou dans des conditions intracellulaires. L'induction ou la répression suivant la phagocytose par les macrophages murins dérivés de la moelle osseuse peut être évaluée convenablement par la mesure des activités spécifiques. Les résultats de deux expériences distinctes sont présentés dans le tableau 2.
Le plasmide pJVED/*hsp18* a été utilisé comme contrôle positif pour l'induction durant la phase de croissance intracellulaire. Bien que l'induction du promoteur par le chauffage de la bactérie à 42°C n'ait pas été concluant la phagocytose de la bactérie conduit clairement à une augmentation de l'activité du promoteur. Dans toutes les expériences, l'activité *luc* intracellulaire a été fortement induite, augmentant de 20 à 100 fois l'activité basale initialement faible (Servant, 1995).
Le plasmide pJVED/*blaF* a été utilisé comme contrôle de la modulation non spécifique au cours de la phagocytose. De faibles variations ont pu être mises en évidence, probablement dues à des changements de conditions de cultures. Quoi qu'il en soit, ces faibles variations ne sont pas comparables à l'induction observée avec le plasmide pJVED/*hsp18*.
Tous les membres de la banque d'ADN ont été testés par mesure de l'activité du promoteur durant la croissance intracellulaire. Parmi eux, le DP428 est fortement induit au cours de la phagocytose (tableaux 1 et 2).

**TABLEAU 1**

| Construction | % Récupération | | URL/10³ bactéries extracellulaire | URL/10³ bactéries intracellulaire | | Induction | |
|---|---|---|---|---|---|---|---|
| pJVED/*blaF** | 0,5 | | 1460 | 1727 | | 1,2 | |
| pJVED/*hsp18* | 0,6 | | 8 | 57 | | 7,1 | |
| pJVED/*DP428* | 0,7 | | 0,06 | 18 | | 300 | |
| | | | | | | | |
| Construction | % Récupération | | URL/10³ | URL/10⁻³ | | Induction | |
| | | | bactéries | bactéries | | | |
| | C57BL/6 | Balb/C | extracellulaire | intracellulaire | | C57BL/6 | Balb/C |
| | | | | C57BL/6 | Balb/C | | |
| PJVED/*blaF** | 7 | 1.1 | 662 | 250 | 911 | 0,4 | 1,4 |
| pJVED/*hsp18* | 6,7 | 1,7 | 164 | 261 | 325 | 1,6 | 2 |
| pJVED/*DP428* | 1,6 | 2.1 | 0,08 | 1,25 | 3,3 | 15.6 | 41 |

**TABLEAU 2**

| Construction | % Récupération | URL/10³ bactéries extracellulaire | URL/10³ bactéries intracellulaire | Induction |
|---|---|---|---|---|
| pJVED/*blaF** | 22 | 1477 | 367 | 0,25 |
| pJVED/*hsp18* | 7 | 0,26 | 6,8 | 26 |
| pJVED/*DP428* | 21 | 0,14 | 4 | 28 |

Le fragment nucléotidique codant pour la région N-terminale du polypeptide DP428 de séquence SEQ ID N° 28 est contenu dans le plasmide déposé à la CNCM sous le N° I-1818.
La totalité de la séquence codant pour le polypeptide DP428 a été obtenue comme détaillée ci-après.
Une sonde a été obtenue par PCR à l'aide des oligonucléotides de séquence SEQ ID N° 25 et SEQ ID N° 26. Cette sonde a été marquée par extension aléatoire en présence de ³²P dCTP. Une hybridation de l'ADN génomique de *M. tuberculosis* souche Mt103 préalablement digéré par l'endonucléase Sca1 a été réalisée à l'aide de ladite sonde. Les résultats de l'hybridation ont fait apparaître qu'un fragment d'ADN d'environ 1,7 kb était marqué. Du fait qu'il existe un site Sca1 s'étendant du nucléotide nt 984 au nucléotide nt 989 de la séquence SEQ ID N° 1, c'est-à-dire du côté 5' de la séquence utilisée comme sonde, la fin de la séquence codante est nécessairement présente dans le fragment détecté par hybridation.
L'ADN génomique de la souche Mt 103 de *M. tuberculosis*, après digestion par Sca1, a subi une migration sur un gel d'agarose. Les fragments de tailles comprises entre 1,6 et 1,8 kb ont été clonés dans le vecteur pSL1180 (Pharmacia) préalablement clivé par Sca1 et déphosphorylé. Après transformation de *E. coli* avec les vecteurs recombinants résultants, les colonies obtenues ont été criblées à l'aide de la sonde. Le criblage a permis d'isoler six colonies hybridant avec cette sonde.
Les inserts contenus dans les plasmides des clones recombinants précédemment sélectionnés ont été séquencés, puis les séquences alignées de manière à déterminer la totalité de la séquence codant pour DP428, plus spécifiquement la SEQ ID N° 2.
Un couple d'amorces a été synthétisé afin d'amplifier, à partir de l'ADN génomique de *M. tuberculosis*, souche Mt 103, la totalité de la séquence codant pour le polypeptide DP428. L'amplicon obtenu a été cloné dans un vecteur d'expression.
Des couples d'amorces appropriés pour l'amplification et le clonage de la séquence codant pour le polypeptide DP428 peuvent être aisément réalisés par l'homme du métier, sur la base des séquences nucléotidiques SEQ ID N°1 et SEQ ID N°2.
Un couple d'amorces particulier selon l'invention est le couple d'amorces suivants, capable d'amplifier l'ADN codant pour le polypeptide DP428 dépourvu de sa séquence signal :
- Amorce aller (SEQ ID N° 29), comprenant la séquence allant du nucléotide en position nt 1021 au nucléotide nt 1044 de la séquence SEQ ID N° 2 :
   5' -AGTGCATGCTGCTGGCCGAACCATCAGCGAC- 3'
- Amorce retour (SEQ ID N° 30), comprenant la séquence complémentaire de la séquence allant du nucléotide en position nt 1345 au nucléotide en position nt 1325 de la séquence SEQ ID N° 2 :
   5' -CAGCCAGATCTGCGGGCGCCCTGCACCGCCTG- 3',
dans lesquelles la partie soulignée représente les séquences hybridant spécifiquement avec la séquence SEQ ID N° 2 et les extrémités 5' correspondent à des sites de restriction en vue du clonage de l'amplicon résultant dans un vecteur de clonage et/ou d'expression.
Un vecteur particulier utilisé pour l'expression du polypeptide DP428 est le vecteur pQE70 commercialisé par la société Qiagen.

### Exemple 3 : La séquence complète du gène DP428 et de ses régions flanquantes.

Une sonde de la région codante de DP428 a été obtenue par ACP, et utilisée pour hybrider l'ADN génomique de différentes espèces de mycobactéries. D'après les résultats de la figure 3, le gène est présent uniquement dans les mycobactéries du complexe de *M. tuberculosis.*
L'analyse de la séquence suggère que DP428 pourrait faire partie d'un opéron. La séquence codante et Les régions flanquantes ne présentent aucune homologie avec des séquences connues déposées dans les banques de données.
D'après la séquence codante, ce gène code pour une protéine de 10 kDa avec un peptide signal, une extrémité C-terminal hydrophobe terminée par deux arginines et précédée par un motif LPISG semblable au motif connu LPXTG. Ces deux arginines pourraient correspondre à un signal de rétention et la protéine DP428 pourrait être accrochée par ce motif à des peptidoglycanes comme cela a déjà été décrit chez d'autres bactéries Gram' (Navarre et al., 1994 et 1996).
Le mécanisme de survie et de croissance intracellulaire des mycobactéries est complexe et les relations intimes entre la bactérie et la cellule hôte restent inexpliquées. Quel que soit le mécanisme, la croissance et la survie intracellulaire des mycrobactéries dépend de facteurs produits par la bactérie et capables de moduler la réponse de l'hôte. Ces facteurs peuvent être des molécules exposées à la surface cellulaire telle que LAM ou des protéines associées à la surface cellulaire, ou des molécules activement secrétées.
D'un autre côté, intracellulairement, les bactéries elles-mêmes doivent faire face à un environnement hostile. Elles semblent y répondre par des moyens proches de ceux mis en oeuvre dans les conditions de stress, par l'induction de protéines de choc thermique (Dellagostin et al., 1995), mais aussi par induction ou la répression de différentes protéines (Lee et al., 1995). En utilisant une méthodologie dérivée de la PCR, Plum et Clark-curtiss (Plum et al., 1994) ont montré qu'un gène de *M. avium* inclu dans un fragment d'ADN de 3 kb, est induit après la phagocytose par des macrophages humains. Ce gène code pour une protéine exportée comprenant une séquence leader mais ne présentant pas d'homologie significative avec les séquences proposées par les banques de données. L'induction, pendant la phase de croissance intracellulaire, d'une protéine de choc thermique de faible poids moléculaire issue de *M. leprae* a également été mise en évidence (Dellagostin et al., 1995). Dans une autre étude, les protéines bactériennes de *M. tuberculosis* ont été métaboliquement marquées pendant la phase de croissance intracellulaire ou bien dans des conditions de stress et séparées par électrophorèse sur gel à deux dimensions : 16 protéines de *M. tuberculosis* ont été induites et 28 reprimées. Les mêmes protéines sont mises en jeu au cours de stress provoqué par un faible pH, un choc thermique, H₂O₂, ou au cours de la phagocytose par des monocytes humains de la lignée THP1. Quoi qu'il en soit, le comportement des protéines induites et réprimées était unique dans chaque condition (Lee et al., 1995). Pris ensemble, ces résultats indiquent qu'un dialogue moléculaire subtile est mis en place entre les bactéries et leurs hôtes cellulaires. De ce dialogue dépend probablement le sort de l'organisme intracellulaire.
Dans ce contexte, l'induction de l'expression de DP428 pourrait être d'une importance majeure, indiquant un rôle important de cette protéine dans la survie et la croissance intracellulaire.
La méthode utilisée dans ces expériences pour évaluer l'expression intracellulaire des gènes(cf. Jacobs et al., 1993, pour la méthode de détermination de l'expression de la luciférase de luciole, et Lim et al., 1995, pour la méthode de détermination de l'expression du gène PhoA) présente l'avantage d'être simple comparée aux autres techniques comme la technique décrite par Mahan et al. (Mahan et al., 1993) adaptée aux mycobactéries et proposée par Bange et al. (Bange et al., 1996), ou la méthode substractive basée sur l'ACP décrite par Plum et Clark-curtiss (Plum et al., 1994). Il existe indiscutablement une variabilité comme le montre la comparaison des différentes expériences. Bien que provoquer l'induction ou la répression soit suffisant, il est désormais possible de l'évaluer fournissant ainsi un outil supplémentaire d'études physiologiques des protéines exportées identifiées par fusion avec *phoA.*

### Exemple 4 :

### Recherche d'une modulation de l'activité des promoteurs lors des phases intramacrophagiques.

Des macrophages de moelle osseuse de souris sont préparés comme décrit par Lang et Antoine (Lang et al., 1991). Les bactéries de *M. segmentis* recombinantes, dont on a déterminé l'activité luciférase par 10³ bactéries comme précédemment, sont incubées à 37°C sous atmosphère humidifiée et enrichie en CO₂ à 5%, pendant 4 heures en présence de ces macrophages de telle manière qu'elles soient phagocytées. Après rinçage pour éliminer les bactéries extracellulaires restantes, on ajoute au milieu de culture de l'amikacine (100 µg/ml) pendant deux heures. Après un nouveau rinçage, le milieu est remplacé par un milieu de culture (DMEM enrichi de 10 % de sérum de veau et 2 mM de glutamine) sans antibiotiques. Après une nuit d'incubation comme précédemment, les macrophages sont lysés à froid (4°C) à l'aide d'un tampon de lyse (cee lysis buffer, Promega), et l'activité luciférase par 10³ bactéries déterminée. Le rapport des activités à la mise en culture et après une nuit donne le coefficient d'induction.

### Exemple 5 :

### Isolement d'une série de séquences par séquençage directement à partir des colonies.

Une série de séquences permettant l'expression et l'exportation de *phoA* ont été isolées à partir de l'ADN de *M. Tuberculosis* ou de *M. Bovis* BCG. Parmi ce groupe de séquences, deux d'entre elles ont été d'avantage étudiées, les gènes entiers correspondant aux inserts ont été clonés, séquencés, et des anticorps contre le produit de ces gènes ont servi à montrer en microscopie électronique que ces gènes codaient pour des antigènes retrouvés à la surface des bacilles de la tuberculose. L'un de ces gènes erp codant pour une séquence signal d'exportation consensus, l'autre des ne possédait aucune caractéristique de gène codant pour une protéine exportée, d'après la séquence. Un autre gène DP428 a été séquence avant que la séquence du génome de *M. Tuberculosis* ne soit disponible. Il contient une séquence ressemblant à la séquence consensus d'attachement au peptidoglycane, ce qui suggère qu'il s'agit aussi d'un antigène vraisemblablement retrouvé à la suface des bacilles de la tuberculose. L'étude des trois gènes *erp*, *des,* et celui codant pour DP428 montre que le système *phoA* que nous avons développé chez les mycobactéries permet de repérer des gènes codant pour des protéine exportées sans déterminant repérable par des études *in silico.* Ceci est particulièrement vrai pour les polypeptides qui ne possèdent pas de séquence signal consensus (des) ou non pas de similarité avec des protéines de fonction connue (*erp* et *DP428).*

Un certain nombre d'inserts ont été identifiés et séquencés avant la connaissance du génome de *M. Tuberculosis,* d'autres après. Ces séquences peuvent être considérées comme des amorces permettant de rechercher des gènes codant pour des protéines exportées. A ce jour, une série d'amorces ont été séquencées et les gènes entiers correspondants ont été soit séquencés, soit identifiés d'après la séquence publiée du génome. Pour tenir compte des erreurs de séquençage toujours possibles, les régions en amont ou en aval de certaines amorces ont été considérées comme pouvant faire partie de séquences codant pour des protéines exportées. Dans certains cas des similarités avec des gènes codant pour des protéines exportées ou des séquences caractéristiques de signaux d'exportation ou des caractéristiques topologiques de protéines membranaires ont été détectées.

Des séquences amorces s'avèrent correspondre à des gènes appartenant à des familles de gènes possédant plus de 50 % de similarité. On peut ainsi indiquer que les autres gènes détectés par similarité avec une amorce codent pour des protéines exportées. C'est le cas de la séquence SEQ ID N° 8G et SEQ ID N° 8H possédant plus de 77 % de similarité avec SEQ ID N° 8A'.
Les séquences pouvant coder pour des protéines exportées sont les suivantes : SEQ ID N° 1, 8, 9, 8G, 8H, 13, 3, 10, 19, 20, 6, 16, 22, 23, 24, 39, 44, 46, et 50.

Des gènes identifiés d'après les amorces à partir de la séquence du génome n'ont aucune caractéristique (d'après la séquence) de protéines exportées. Il s'agit des séquences suivantes : SEQ ID N° 4, 27, 11, 12, 14, 7, 15, 17, 18, 21, 31, 32, 33, 34, 35, 36, 37, 38, 40, 41, 42, 43, 45, 47, 48, et 49.

D'après la séquence d'autres organismes comme *E. coli,* on peut rechercher dans la séquence du génome de *M. tuberculosis,* des gènes possédant des similarités avec des protéines connues pour être exportées chez d'autres organismes bien que ne possédant pas de séquence signal d'exportation. Dans ce cas une fusion avec *phoA* est un protocole avantageux pour déterminer si ces séquences de *M. tuberculosis* codent pour des protéines exportées bien que ne présentant pas de séquence signal consensus. Il a été en effet possible de cloner SEQ ID N° 49, une séquence similaire à un gène de *E. coli* de la famille *htrA.* Une fusion de SEQ ID N° 49 avec *phoA* conduit à l'expression et à l'exportation de *phoA.* Des colonies *M. smegmatis* hébergeant une fusion SEQ ID N° 49 *phoA* sur un plasmide pJVED sont bleues.
SEQ ID N° 49 est donc considérée comme une protéine exportée.
La méthode *phoA* est donc utile pour détecter d'après la séquence de *M. Tuberculosis* des gènes codant pour des protéines exportées sans qu'ils ne codent pour des séquences caractéristiques des protéines exportées.

Même si une séquence possède des déterminants de protéines exportées, cela ne démontre pas une exportation fonctionnelle. Le système *phoA* permet de montrer que le gène suspecté code réellement pour une protéine exportée. Ainsi, il a été vérifié que la séquence SEQ ID N° 50 possédait bien des signaux d'exportation.

**TABLEAU 3**

| SEQ ID N° | Référence de la séquence correspondante prédite par Cole et al. | | Annotation |
|---|---|---|---|
| SEQ ID N°1 | Rv 0203 | * | Séquence hydrophobe en N-terminal |
| SEQ ID N°4 | Rv 2050 | | Pas de prédiction |
| SEQ ID N°27 | | | |
| SEQ ID N°8 | | | |
| SEQ ID N°9 | Rv 2563 | * | Protéine membranaire |
| SEQ ID N° 8G',H' | Rv 0072 | * | Possible protéine de transport transmembranaire de type ABC |
| SEQ ID N°11 | Rv 0546c | ML | Protein S-D Lactoyl Glutathione-méthyl glyoxal lyase |
| SEQ ID N°12 | pas de prédiction | | non retrouvé dans *M. tuberculosis* H37rv |
| SEQ ID N°13 | Rv 1984c | * | probable précurseur cutinase avec une séquence signal N-terminale |
| SEQ ID N°3 | | | |
| SEQ ID N°10 | | | |
| SEQ ID N°14 | | | |
| SEQ ID N°7 | pas de prédiction | | pas de prédiction |
| SEQ ID N°15 | avec décalage de lecture, pourrait être en phase avec Rv 2530c | | pas de prédiction |
| SEQ ID N°17 | Rv 1303 | ML | pas de prédiction |
| SEQ ID N°18 | Rv 0199 | ML | pas de prédiction |
| SEQ ID N°19 | Rv 0418 | * | site de fixation de lipoprotéine membranaire procaryote, similarité avec la N-acétyl puromycyne acétyl hydrolase |
| SEQ ID N°20 | Rv 3576 | * | contient un site de fixation de lipoprotéine membranaire procaryote, similarité avec une |
| SEQ ID N°6 | | | |
| | | | sérine/thréonine protéine kinase |
| SEQ ID N° 21 | Rv 3365c | ML | similarité avec une métallo peptidase à zinc |
| SEQ ID N°31 | non prédite | | pas de prédiction |
| | | | |
| SEQ ID N°32 | Rv 0822c | ML | Existence d'une région consensus avec la famille drac |
| SEQ ID N°33 | Rv 1044 | | pas de prédiction |
| SEQ ID N°34 | non prédite | | pas de prédiction |
| SEQ ID N°35 | Rv 2169c | | pas de prédiction |
| SEQ ID N°36 | Rv 3909 | ML | pas de prédiction |
| SEQ ID N°37 | Rv 2753c | | similarité avec des dihydropricolinate synthases |
| SEQ ID N°38 | Rv 0175 | | pas de prédiction |
| SEQ ID N°39 | Rv 3006 | * ML | prédiction de séquence signal de lipoprotéine |
| SEQ ID N°40 | Rv 0549c | | pas de prédiction |
| SEQ ID N°41 | Rv 2975c pouvant être en phase avec Rv 2974c | | similarité avec protéine de substilis |
| SEQ ID N°42 | Rv 2622 | | similarité avec une méthyl transférase |
| SEQ ID N°43 | Rv 3278c | ML | pas de prédiction |
| SEQ ID N°44 | Rv 0309 | * | pas de prédiction |
| SEQ ID N°45 | Rv 2169c | ML | pas de prédiction |
| SEQ ID N°96 | Rv 1411c | * | probable lipoprotéine avec une séquence signal N-terminale |
| SEQ ID N°47 | Rv 1714 | | similarité avec une gluconate 3-déhydrogénase |
| SEQ ID N°48 | Rv 0331 | | similarité avec une sulfide déhydrogénase et une sulfide quinone réductase |
| SEQ ID N°49 | Rv 0983 | ML | Similarité avec une sérine protéase HtrA |
| | | | |
| SEQ ID N°5 | | | |
| SEQ ID N°16 | Rv 3810 | * ML | Protéine de surface Berthelet et al. 1995 |
| SEQ ID N°22 | Rv 3763 | * | Contient un site de fixation de lipoprotéine membranaire eucaryote |
| SEQ ID N°23 | | | |
| SEQ ID N°24 | | | |
| SEQ ID N°50 | Rv 0125 | * | Site actif des sérines protéases Séquence signal N-terminale possible |

| | | | |
|---|---|---|---|
| Légende du tableau 3 : Correspondance des séquences selon l'invention avec les séquences prédites par Cole et al. 1998, Nature, 393, 537-544. * : Prédiction que la protéine codée par la séquence soit exportée ML : Prédiction de similarité avec *M. leprae.* | | | |

### Exemple 6 :

### Caractéristiques et obtention de la protéine MlC25

L'extrémité N terminale de la protéine MlC25 a été détectée par le système PhoA comme permettant l'exportation de la protéine de fusion, nécessaire à l'obtention de son activité phosphatase.
La séquence d'ADN codant pour l'extrémité N terminale de la protéine MlC25 est contenue dans la séquence SEQ ID N° 20 de la présente demande de brevet.
A partir de cette séquence amorce, le gène complet codant pour la protéine M1C25 a été recherché dans le génome de *M. tuberculosis* (Fondation Welcome Trust, site Sanger) .
Le centre Sanger a attribué à MlC25 les noms:
Rv3576,
MTCY06G11.23,
pknM

### Séquence SEQ ID N° 29 du gène complet M1C25 (714 bases): cf. Figure 29

Ce gène code pour une protéine de 237 AA, de 25 kDa de masse molaire. Cette protéine est référencée dans les banques sous les appellations:
PID:e306716,
SPTREMBL:P96858

### Séquence SEO ID N° 30 de la protéine MlC25 (237 acides aminés): cf. Figure 30

MlC25 contient un site de fixation à la partie lipidique des lipoprotéines de membrane des procaryotes (PS00013 Prokaryotic membrane lipoprotein lipid attachment site:

### CTGGTCGGTG CGTGCATGCT CGCAGCCGGA TGC).

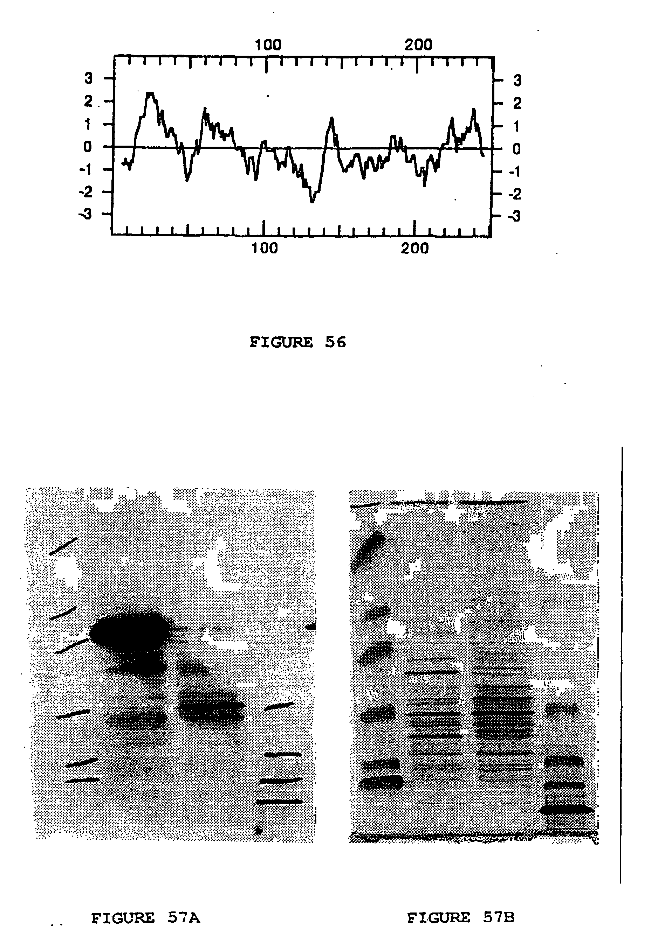

La fonction de M1C2S n'est pas certaine mais elle possède très probablement une activité "sérine/thréonine-protéine kinase". Des ressemblances sont à noter avec la moitié C terminale de K08G_MYCTU Q11053 Rv1266c (MTCY50.16). Des similarités sont aussi retrouvées avec KY28_MYCTU.
En 5' du gène codant pour M1C25 se trouve un gène codant potentiellement pour une protéine régulatrice (PID:e306715, SPTREMBL:P96857, Rv3575c, (MTCY06G11.22c))
Le profil d'hydrophobicité (Kyte et Doolitle) de M1C25 est représenté à la figure 56.
Un site de clivage de la séquence signal est prédit (SignalP Vl.1; World Wide Web Prediction Server, Center for Biological Sequence Analysis) entre les acides aminés 31 et 32: AVA-AD. Ce site de coupure est derrière un motif "AXA" classique. Cette prédiction est compatible avec le profil d'hydrophobicité. Dans cette séquence signal potentielle il est a remarqué la répétition trois fois de la séquence des trois acides aminés LAA.

### Clonage du gène M1C25 en vue de la production de la protéine qu'il code:

Un couple d'amorces a été synthétisé afin d'amplifier, à partir de l'ADN génomique de M. tuberculosis, souche H37Rv, la totalité de la séquence codant pour le polypeptide M1C25. L'amplicon obtenu a été cloné dans un vecteur d'expression.

Des couples d'amorces appropriés pour l'amplification et le clonage de la séquence codant pour M1C25 ont été synthétisés :
- amorce aller :
   5' -ATAATACCATGGGCAAGCAGCTAGCCGCGC- 3'
- amorce retour :
   5' -ATTTATAGATCTCTGCTTAGCAACCTTGGCCGCG- 3'

La partie soulignée représente les séquences hybridant spécifiquement avec la séquence M1C25 et les extrémités 5' correspondent à des sites de restriction en vue du clonage de l'amplicon résultant dans un vecteur de clonage et/ou d'expression.
Un vecteur particulier utilisé pour l'expression du polypeptide M1C25 est le vecteur pQE60 commercialisé par la société Qiagen, en suivant le protocole et les recommandations proposés par cette marque.
Les cellules utilisées pour le clonage sont des bactéries : *E. coli* XL1-Blue (résistante à la tétracycline).
Les cellules utilisées pour l'expression sont des bactéries : *E*. *coli* M15 (résistante à la kanamycine) contenant le plasmide pRep4 (M15 pRep4).
La production de la protéine MC25 est illustrée par les figures 57 A et B. (Extraits bactériens de la souche E. coli M15 contenant le plasmide pMlC25. Les cultures bactériennes et les extraits sont préparés selon Sambrook et al. (1989). L'analyse des extraits bacrériens est effectuée selon les instructions de Quiagen (1997).

### Références bibliographiques

AIDS therapies, 1993, in Mycobacterial infections, ISBN 0-9631698-1-5, pp. 1-11.
Altschul, S.F. et al., 1990, J. Mol. Biol., 215 : 403-410.
Andersen, P. et al., 1991, Infect. Immun., 59 :1905-1910.
Andersen, P. et al., 1995, J. Immunol., 154, 3359-3372.
Bange, F.C., A.M. Brown, and W.R. Jacobs JR., 1996, Leucine auxotrophy restricts growth of Mycobacterium bovis BCG in macrophages. Infect. Immun., 64,: 1794-1799.
Barany, F., 1911, Proc. Natl. Acad. Sci. USA, 88 :189-193.
Bates, J. et al., 1986, Am. Rev. Respir. Dis., 134 :415-417.
Bates, J. 1979. Chest. 76(Suppl.):757-763.
Bates, J. et al.. 1986. Am. Rev. Respir. Dis. 134 :415-417.
Berthet, F.X., J. Rauzier, E.M. Lim, W. Philipp, B. Gicquel, and D. Portnoï, 1995, Characterization of the M. tuberculosis erp gene encoding a potential cell surface protein with repetitive structures. Microbiology. In press Borremans, M. et al., 1989, Biochemistry, 7 : 3123-3130.
Bouvet, E. 1994. Rev. Fr. Lab. 273 :53-56.
Brockman, R.W. and Heppel L.A., 1968, On the localization of alkaline phosphatase and cyclic phosphodiesterase in Escherichia coli, Biochemistry, 7 : 2554-2561.
Burg, J.L. et al., 1996, Mol. and Cell. Probes, 10 :257-271.
Chevrier, D. et al., 1993, Mol. and Cell. Probes, 7 :187-197.
Clemens, D.L., 1996, Characterization of the Mycobacterium tuberculosis phagosome, Trends Microbiol., 4 : 113-118.
Chu ,B.C.F. et al., 1986, Nucleic Acids Res., 14 :5591-5603.
Clemens, D.L. and Horwitz M.A., 1995, Characterization of the Mycobacterium tuberculosis phagosome and evidence that phagosomal maturation is inhibited, J. Exp. Med., 181 : 257-270.
Colignon J.E., 1996. Immumologic studies in humans. Measurement of proliferative responses of culturered lymphocytes. Current Protocols in Immunology, NIH, 2, Section II.
Daniel, T.M. et al. 1987. Am. Rev. Respir. Dis., 135 :1137-1151).
Dellagostin, O.A., Esposito G., Eales L.-J., Dale J.W. and. McFadden J.J., 1995, Activity of mycobacterial promoters during intracellular and extracellular growth. Microbiol., 141 : 2123-2130.
Drake, T.A. et al. 1987. J. Clin. Mocrobiol. 25:1442-1445.
Dramsi et al., 1997, Infection and Immunity, 65, 5 : 1615-1625.
Duck, P. et al., 1990, Biotechniques, 9:142-147.
Erlich, H.A. 1989. In PCR Technology. Principles and Applications for DNA Amplification. New York: Stockton Press.
Felgner et al., 1987, Proc. Natl. Acad. Sci., 84:7413.
Fraley et al., 1980, J. Biol. Chem., 255:10431.
Gaillard, J.L., Berche P., Frehel C., Gouin E. and Cossart P., 1991, Entry of L. monocytogenes into cells is mediated by internalin, a repeat protein reminiscent of surface antigens from Gram-positive cocci, Cell., 65 : 1127-1141.
Garbe, T., Harris D., Vordermeir M., Lathigra R., Ivanyi J. and Young D., 1993, Expression of the Mycobacterium tuberculosis 19-kilodalton antigen in Mycobacterium smegmatis: immunological analysis and evidence of glycosylation, Infect. Immun., 61 : 260-267.
Guateli, J.C. et al., 1990, Proc. Natl. Acad. Sci. USA, 87:1874-1878.
Harboe et al., 1996, Infect. Immun., 64, 16-22.
Herrmann, J.L., O'Gaora P., Gallagher A., Thole J.E.R. and Young D.B., 1996, Bacterial glycoproteins: a link between glycosylation and proteolytic cleavage of a 19 kDa antigen from Mycobacterium tuberculosis, EMBO J. 15 : 3547-3554.
Houbenweyl, 1974, in Meuthode der Organischen Chemie, E. Wunsch Ed., Volume 15-I et 15-II, Thieme, Stuttgart.
Huygen, K. et al., 1996, Nature Medicine, 2(8):893-898.
Innis, M.A. et al. 1990. in PCR Protocols. A guide to Methods and Apllications. San Diego: Academic Press.
Isberg, R.R., Voorhis D.L. and Falkow S., 1987, Identification of invasin: a protein that allows enteric bacteria to penetrate cultured mammalian cells, Cell, 50 : 769-778.
Jacobs, W.R. et al., 1991. Construction of mycobacterial genomic libraries in shuttle cosmids. Genetic Systems for Mycobacteria, Methods in Enzymology, 204 : 537-555.
Jacobs, W.R. et al., 1993, Science, 260 :819-822.
Kaneda, et al., 1989, Science, 243:375
Kiehn, T.E., et al. 1987. J. Clin. Microbiol. 25 :1551-1552.
Kievitis ,T. et al., 1991, J. Virol. Methods, 35 :273-286.
Kohler, G. et al., 1975, Nature, 256(5517):495-497.
Kwoh, D.Y. et al., 1989, Proc. Natl. Acad. Sci. USA, 86 :1173-1177.
Landegren ,U. et al., 1988, Science, 241, :1077-1080.
Lang, T. and Antoine J.-C., 1991, Localization of MHC classII molécules in murine bone marrow-derived macrophages. Immunology, 72 : 199-205.
Lee, B.Y. and Horwitz M.A., 1995, Identification of macrophage and stress-induced proteins of Mycobacterium tuberculosis, J. Clin. Invest., 96 : 245-249.
Lim, E.M., Rauzier J., Timm J., Torrea G., Murray A., Gicquel B. and Portnoï D., 1995, Identification of Mycobacterium tuberculosis DNA sequences encoding exported proteins, using phoA gene fusions, J. Bacteriol., 177 : 59-65.
Lizardi, P.M. et al., 1988, Bio/technology, 6 :1197-1202.
Mahan, M.J. et al., 1993. Selection of bacterial virulence genes that are specifically induced in host tissues, Science, 259 : 686-688.
Manoil L., Mekolanos J.J. and Beckwith J., J. Bacteriol., 1990, 172, 515-518.
Matthews, J.A. et al., 1988, Anal. Biochem., 169:1-25.
Merrifield, R.D., 1966, J. Am. Chem. Soc., 88(21):5051-5052.
Midoux, 1993, Nucleic Acids Research, 21:871-878/
Miele, E.A. et al., 1983, J. Mol. Biol., 171:281-295.
Minton, N.P., 1984, Gene, 31, 269-273.
Montgomery et al., 1993, DNA Cell Biol., 12:777-783.
Navarre,W.W.et al.,1994, Molecular Microbiologie, 14(1):115-121.
Navarre,W.W.et al.,1996, J. of Bacteriology, 178, 2 :441-446.
Pagano et al., 1967, J. Virol., 1 :891
Pastore, 1994, Circulation, 90:1-517.
Patel, et al. 1990. J. Clin. Microbiol. :513-518.
Prentki, B. et Krish H.M., 1984, Gene, 29 : 303-313.
Pettersson R., Nordfelth J., Dubinina E., Bergman T., Gustafonon M., Magnusson K.E. and Wolf-Watz H., 1996, Modulation of virulence factor expression by pathogen target cell contact. Science., 273 : 1231-1233.
Plum, G. and Clark-Curtiss J.E., 1994, Induction of Mycobacterium avium gene expression following phagocytosis by human macrophages. Infect. Immun., 62 : 476-483.
Roberts, M.C., et al. 1987. J. Clin. Microbiol. 25,:1239-1243.
Rolfs, A. et al. 1991. In PCR Topics. Usage of Polymerase Chain reaction in Genetic and Infectious Disease. Berlin: Springer-Verlag.
Sambrook, J. et al. 1989. In Molecular cloning : A Laboratory Manual. Cold Spring Harbor, NY: Cold Spring Harbor Laboratory Press.
Sanchez-Pencador, R., 1988, J. Clin. Microbiol., 26(10),:1934-1938.
Schneewind, O. et al., 1995, Science, 268 : 103-106.
Segev D., 1992, in « Non-radioactive Labeling and Detection of Biomolecules ». Kessler C. Springer Verlag, Berlin, New-York, 197-205.
Servant, P. and Mazodier P., 1995, Characterization of Streptomyces albus 18-kilodalton heat shock-responsive protein. J. Bacteriol., 177 : 2998-3003.
Shiver, J.W., 1995, in Vaccines 1995, eds Chanock, R.M. Brown, F. Ginsberg, H.S. & Norrby, E.) , pp.95-98, Cold Spring Harbor Laboratory Press, Cold Spring Harbor, New York.
Sorensen et al., 1995, Infect. Immun., 63, 1710-1717.
Stone, B.B. et al., 1996, Mol. and Cell. Probes, 10 :359-370.
Stover, C.K., Bansal G.P., Hanson M.S., Burlein S.R., Palaszynski S.R., Young J.F., Koenig S., Young D.B., Sadziene A. and Barbour A.G., 1993, Protective immunity elecited by recombinant Bacille Calmette-Guerin (BCG) expressing outer surface protein A (OspA) lipoprotein: a candidate Lyme disease vaccine. J. Exp. Med., 178 : 197-209.
Sturgill-Koozycki, S., Schleainger P.H., Chakroborty P., Haddix P.L., Collins H.L., Fok A.K., Allen R.D., Gluck S.L., Heuser J. and Russell D.G., 1994, Lack of acidification in Mycobacterium phagosomes by exclusion of the vesicular proton-ATPase. Science., 263 : 678-681.
Tascon, R.E et al.., 1996, Nature Medicine, 2(8):888-892.
Technique assemblage oligonucléotides, 1983, Proc. Natl. Acad. Sci. USA, 80 :7461-7465.
Technnique des béta-cyanethylphosphoramidites, 1986, Bioorganic Chem., 4 :274-325.
Thierry, D. et al.,1990, Nucl. Acid Res., 18 :188.
Timm, J., Perilli M.G., Duez C., Trias J., Orefici G., Fattorini L., Amicosante G., Oratore A., Boris B., Frere J.M., Pugsley A.P. and Gicquel B., 1994, Transcription and expression analysis, using lacZ and phoA gene fusions, of Mycobacterium fortuitum B-lactamase genes cloned from a natural isolate and a high-level B-lactamase producer. Mol. Microbiol., 12 : 491-504.
Tuberculosis Prévention Trial, 1980, Mendis, « Trial of BCG vaccines in South India for Tuberculosis Infection », Indian Journal of Medical research, 1972 (Suppl.):1-74.
Urdea, M.S. et al., 1991, Nucleic Acids Symp. Ser., 24 :197-200.
Urdea, M.S., 1988, Nucleic Acids Research, 11: 4937-4957.
Verbon, A., Hartskeerl R.A., Schuitema A., Kolk A.H., Young D.B. and Lathigra R., 1992, The 14,000-molecular-weight antigen of Mycobacterium tuberculosis is related to the alpha-crystallin family of low-molecular-weight heat shock proteins. J Bacteriol., 174 : 1352-1359.
Walker, G.T. et al., 1992, Nucleic Acids Res., 20:1691-1696.
Walker, G.T. et al., 1992, Proc. Natl. Acad. Sci. USA, 89:392-396.
Wiker, H.G. et al., 1992, Microbiol. Rev., 56 :648-661.
Yamaguchi, R. et al., 1989, Infect. Immun., 57 :283-288 ; Xu, S., Cooper A., Sturgill-Koozycki S., van Heyningen T., Chatterjee D., Orme I., Allen P. and Russel D.G., 1994, Intracellular trafficking in Mycobacterium tuberculosis and Mycobacterium avium-infected macrophages, J. Immuno., 153: 2568-2578.
Young, D.B. et al., 1992, Mol. Microbiol., 6 :133-145.
Yuen, L.K.W. et al., 1993, J. Clin. Microbiol., 31 : 1615-1618.

## Revendications

1. Vecteur recombinant de criblage, de clonage et/ou d'expression, **caractérisé en ce qu'**il se réplique chez des mycobactéries et **en ce qu'**il contient :
1) un réplicon fonctionnel chez les mycobactéries ;
2) un marqueur de sélection ;
3) une cassette rapporteur comprenant :
a) un site de clonage multiple (polylinker),
b) éventuellement un terminateur de transcription actif chez les mycobactéries, en amont du polylinker,
c) une première séquence nucléotidique codant pour un marqueur d'exportation et/ou de sécrétion de protéine, ladite séquence nucléotidique étant dépourvue de son codon d'initiation et de ses séquences de régulation, et
d) en aval, une seconde séquence nucléotidique codant pour un marqueur d'activité de promoteurs contenus dans le fragment cloné, ladite séquence nucléotidique étant pourvue de son codon d'initiation.

2. Vecteur recombinant selon la revendication 1, **caractérisé en ce que** la séquence nucléotidique codant pour un marqueur d'exportation et/ou de sécrétion de protéine est une séquence codante issue du gène *phoA* de la phosphatase alcaline.

3. Vecteur recombinant selon l'une des revendications 1 et 2, **caractérisé en ce que** la séquence nucléotidique codant pour un marqueur d'exportation et/ou de sécrétion de protéine est une séquence codante du gène de la β-agarase, de la nucléase d'un staphylocoque ou de la β-lactamase d'une mycobactérie.

4. Vecteur recombinant selon l'une des revendications 1 à 3, **caractérisé en ce que** la séquence nucléotidique codant pour un marqueur d'activité de promoteurs contenus dans le fragment cloné est une séquence codante issue du gène *luc* de la luciférase de luciole.

5. Vecteur recombinant selon l'une des revendications 1 à 4, **caractérisé en ce que** la séquence nucléotidique codant pour un marqueur d'activité de promoteurs contenus dans le fragment cloné est une séquence codante issue du gène GFP de la Green Fluorescent Protein.

6. Vecteur recombinant selon l'une des revendications 1 à 5, **caractérisé en ce que** le terminateur de transcription actif chez les mycobactéries est le terminateur du coliphage T4 (tT4).

7. Vecteur recombinant selon l'une des revendications 1 à 6, **caractérisé en ce qu'**il est un plasmide choisi parmi les plasmides suivants déposés à la CNCM (Collection Nationale de Cultures de Microorganismes, Paris, France) :
a) pJVEDa déposé à la CNCM sous le N° I-1797, le 12/12/1996,
b) pJVEDb déposé à la CNCM sous le N° 1-1906, le 25 juillet 1997,
c) pJVEDc déposé à la CNCM sous le N° I-1799, le 12/12/1996.

8. Vecteur recombinant selon l'une des revendications 1 à 7, **caractérisé en ce qu'**il comprend en l'un des sites de clonage du polylinker une séquence d'acide nucléique de mycobactérie chez laquelle on détecte la présence d'une séquence codant pour un polypeptide susceptible d'être exporté et/ou sécrété, et/ou d'être induit ou réprimé lors de l'infection par ladite mycobactérie ou encore exprimé ou produit de façon constitutive, ainsi que les séquences promotrices et/ou régulatrices associées susceptibles de permettre ou de favoriser l'exportation et/ou la sécrétion dudit polypeptide, ou tout ou partie de gène codant pour ledit polypeptide.

9. Vecteur recombinant selon l'une des revendications 1 à 8, **caractérisé en ce que** la séquence d'acide nucléique de mycobactérie qu'il contient est obtenue par fragmentation physique ou par digestion enzymatique de l'ADN génomique ou de l'ADN complémentaire d'un ARN d'une mycobactérie.

10. Vecteur recombinant selon l'une des revendications 1 à 9, **caractérisé en ce que** ladite mycobactérie est *M. tuberculosis.*

11. Vecteur recombinant selon l'une des revendications 1 à 9, **caractérisé en ce que** ladite mycobactérie est choisie parmi *M. africanum, M. bovis*, *M. avium* ou *M. leprae.*

12. Procédé de criblage de séquences de nucléotides issues de mycobactéries pour déterminer la présence de séquences correspondant à des polypeptides exportés et/ou sécrétés pouvant être induits ou réprimés lors de l'infection, leurs séquences promotrices et/ou régulatrices associées susceptibles notamment de permettre ou de favoriser l'exportation et/ou la sécrétion desdits polypeptides d'intérêt, ou tout ou partie de gènes d'intérêt codant pour lesdits polypeptides, **caractérisé en ce qu'**il met en oeuvre un vecteur selon l'une des revendications 1 à 11.

13. Procédé de criblage selon la revendication 12, **caractérisé en ce qu'**il comprend les étapes suivantes:
a) la fragmentation physique des séquences d'ADN de mycobactéries ou leur digestion par au moins une enzyme déterminée et la récupération des fragments obtenus ;
b) l'insertion des fragments obtenus à l'étape a) dans un site de clonage, compatible le cas échéant avec l'enzyme de l'étape a), du polylinker d'un vecteur selon l'une des revendications 1 à 11 ;
c) si besoin, l'amplification desdits fragments contenus dans le vecteur, par exemple par réplication de ce dernier après insertion du vecteur ainsi modifié dans une cellule déterminée, de préférence *E coli ;*
d) la transformation de cellules hôtes par le vecteur amplifié à l'étape c), ou en l'absence d'amplification, par le vecteur de l'étape b) ;
e) la culture de cellules hôtes transformées dans un milieu permettant la mise en évidence du marqueur d'exportation et/ou de sécrétion, et/ou du marqueur d'activité de promoteurs contenu dans le vecteur ;
f) la détection des cellules hôtes positives (colonies positives) pour l'expression du marqueur d'exportation et/ou de sécrétion, et/ou du marqueur d'activité de promoteurs ;
g) l'isolement de l'ADN des colonies positives et l'insertion de cet ADN dans une cellule identique à celle de l'étape c) ;
h) la sélection des insertions contenues dans le vecteur, permettant l'obtention de clones positifs pour le marqueur d'exportation et/ou de sécrétion, et/ou pour le marqueur d'activité de promoteurs ;
i) l'isolement et la caractérisation des fragments d'ADN de mycobactéries contenues dans ces insérats, et l'étape i) du procédé pouvant comporter en outre une étape de séquençage des insertions sélectionnées.

14. Mycobactérie recombinante **caractérisée en ce qu'**elle est transformée par un vecteur recombinant selon l'une des revendications 1 à 11.

## Claims

1. Recombinant screening, cloning and/or expression vector, **characterized in that** it replicates in mycobacteria and **in that** it contains:
1) a replicon which is functional in mycobacteria;
2) a selectable marker;
3) a reporter cassette comprising:
a) a multiple cloning site (polylinker),
b) optionally a transcription terminator which is active in mycobacteria, upstream of the polylinker,
c) a first nucleotide sequence encoding a protein export and/or secretion marker, said nucleotide sequence lacking its initiation codon and its regulatory sequences, and
d) downstream, a second nucleotide sequence encoding a marker for the activity of promoters which are contained in the cloned fragment, said nucleotide sequence lacking its initiation codon.

2. Recombinant vector according to claim 1, **characterized in that** the nucleotide sequence encoding a protein export and/or secretion marker is a coding sequence derived from the alkaline phosphatase *phoA* gene.

3. Recombinant vector according to either of claims 1 and 2, **characterized in that** the nucleotide sequence encoding a protein export and/or secretion marker is a coding sequence of the gene for β-agarase, for the nuclease of a staphylococcus or for the β-lactamase of a mycobacterium.

4. Recombinant vector according to one of claims 1 to 3, **characterized in that** the nucleotide sequence encoding a marker for the activity of promoters which are contained in the cloned fragment is a coding sequence derived from the firefly luciferase *luc* gene.

5. Recombinant vector according to one of claims 1 to 4, **characterized in that** the nucleotide sequence encoding a marker for the activity of promoters which are contained in the cloned fragment is a coding sequence derived from the Green Fluorescent Protein GFP gene.

6. Recombinant vector according to one of claims 1 to 5, **characterized in that** the transcription terminator which is active in mycobacteria is the T4 coliphage terminator (tT4).

7. Recombinant vector according to one of claims 1 to 6, **characterized in that** it is a plasmid chosen from the following plasmids which have been deposited at the CNCM (Collection Nationale de Cultures de Microorganismes, Paris, France):
a) pJVEDa which was deposited at the CNCM under the No. 1-1797, on 12/12/1996,
b) pJVEDb which was deposited at the CNCM under the No. I-1906, on 25 July 1997,
c) pJVEDc which was deposited at the CNCM under the No. I-1799, on 12/12/1996.

8. Recombinant vector according to one of claims 1 to 7, **characterized in that** it comprises at one of the cloning sites of the polylinker a nucleic acid sequence of a mycobacterium in which the detection is carried out of the presence of a nucleic sequence encoding a polypeptide capable of being exported and/or secreted, and/or of being induced or repressed during the infection with said mycobacterium or expressed or produced constitutively, as well as the associated promoter and/or regulatory sequences which are capable of allowing or promoting the export and/or the secretion of said polypeptide, or all or part of a gene encoding said polypeptide.

9. Recombinant vector according to one of claims 1 to 8, **characterized in that** the mycobacterial nucleic acid sequence which it contains is obtained by physical fragmentation or by enzymatic digestion of the genomic DNA or of the DNA which is complementary to an RNA of a mycobacterium.

10. Recombinant vector according to one of claims 1 to 9, **characterized in that** said mycobacterium is *M. tuberculosis.*

11. Recombinant vector according to one of claims 1 to 9, **characterized in that** said mycobacterium is chosen from *M. africanum, M. bovis, M. avium* or *M. leprae.*

12. Method of screening nucleotide sequences derived from mycobacteria in order to determine the presence of sequences corresponding to exported and/or secreted polypeptides which may be induced or repressed during the infection, their associated promoter and/or regulatory sequences which are capable in particular of allowing or promoting the export and/or secretion of said polypeptides of interest, or all or part of genes of interest encoding said polypeptides, **characterized in that** it uses a vector according to one of claims 1 to 11.

13. Method of screening according to claim 12, **characterized in that** it comprises the following steps:
a) physical fragmentation of the mycobacterial DNA sequences or their digestion with at least one defined enzyme and recovery of the fragments obtained;
b) insertion of the fragments obtained in step a) into a cloning site, which is compatible, where appropriate, with the enzyme of step a), of the polylinker of a vector according to one of claims 1 to 11;
c) if necessary, amplification of said fragments contained in the vector, for example by replication of the latter after insertion of the vector thus modified into a defined cell, preferably *E. coli;*
d) transformation of host cells with the vector amplified in step c), or in the absence of amplification, with the vector of step b);
e) culture of transformed host cells in a medium allowing the detection of the export and/or secretion marker, and/or of the promoter activity marker which is contained in the vector;
f) detection of the host cells which are positive (positive colonies) for the expression of the export and/or secretion marker, and/or of the promoter activity marker;
g) isolation of the DNA from the positive colonies and insertion of this DNA into a cell which is identical to that in step c);
h) selection of the inserts contained in the vector, allowing the production of clones which are positive for the export and/or secretion marker, and/or for the promoter activity marker;
i) isolation and characterization of the mycobacterial DNA fragments contained in these inserts, and it being possible for step i) to comprise, in addition, a step for sequencing the inserts selected.

14. Recombinant mycobacterium, **characterized in that** it is transformed with a recombinant vector according to one of claims 1 to 11.

## Patentansprüche

1. Rekombinanter Screening-, Klonierungs- und/oder Expressionsvektor, **dadurch gekennzeichnet, dass** er sich in Mycobakterien repliziert und dass er enthält:
1) ein in Mycobakterien funktionsfähiges Replikon;
2) einen Selektionsmarker;
3) eine Reporterkassette, umfassend:
a) eine Mehrfachklonierungsstelle (Polylinker),
b) gegebenenfalls einen in Mycobakterien aktiven Transkriptionsterminator strangaufwärts von dem Polylinker,
c) eine erste Nukleotidsequenz, welche einen Proteinexportierungs- und/oder -sekretionsmarker kodiert, wobei der Nukleotidsequenz ihr Startcodon und ihre Regulationssequenzen fehlen, und
d) strangabwärts eine zweite Nukleotidsequenz, welche einen Aktivitätsmarker von Promotoren, die in dem klonierten Fragment enthalten sind, kodiert, wobei der Nukleotidsequenz ihr Startcodon fehlt.

2. Rekombinanter Vektor nach Anspruch 1, **dadurch gekennzeichnet, dass** die Nukleotidsequenz, welche einen Proteinexportierungs- und/oder -sekretionsmarker kodiert, eine kodierende Sequenz ist, welche aus dem phoA-Gen der alkalischen Phosphatase stammt.

3. Rekombinanter Vektor nach einem der Ansprüche 1 und 2, **dadurch gekennzeichnet, dass** die Nukleotidsequenz, welche einen Proteinexportierungs- und/oder -sekretionsmarker kodiert, eine kodierende Sequenz des Gens der β-Agarase, der Nuklease einer Staphylokokke oder der β-Lactamase eines Mycobakteriums ist.

4. Rekombinanter Vektor nach einem der Ansprüche 1 bis 3, **dadurch gekennzeichnet, dass** die Nukleotidsequenz, die einen Aktivitätsmarker von Promotoren, die in dem klonierten Fragment enthalten sind, kodiert, eine kodierende Sequenz ist, die aus dem *luc-*Gen der Glühwürmchen-Luciferase stammt.

5. Rekombinanter Vektor nach einem der Ansprüche 1 bis 4, **dadurch gekennzeichnet, dass** die Nukleotidsequenz, die einen Aktivitätsmarker von Promotoren, die in dem klonierten Fragment enthalten sind, kodiert, eine kodierende Sequenz ist, die aus dem *GFP*-Gen des Green Fluorescent Protein stammt.

6. Rekombinanter Vektor nach einem der Ansprüche 1 bis 5, **dadurch gekennzeichnet, dass** der in Mycobakterien aktive Transkriptionsterminator der Terminator des Coliphagen T4 (tT4) ist.

7. Rekombinanter Vektor nach einem der Ansprüche 1 bis 6, **dadurch gekennzeichnet, dass** er ein Plasmid ist, das unter den folgenden, bei der CNCM (Collection Nationale de Cultures de Microorganismes, Paris, Frankreich) hinterlegten Plasmiden ausgewählt wird:
a) pJVEDa, hinterlegt bei der CNCM am 12/12/1996 unter der Nr. I-1797,
b) pJVEDb, hinterlegt bei der CNCM am 25. Juli 1997 unter der Nr. I-1906,
c) pJVEDc, hinterlegt bei der CNCM am 12/12/1996 unter der Nr. I-1799.

8. Rekombinanter Vektor nach einem der Ansprüche 1 bis 7, **dadurch gekennzeichnet, dass** er in einer der Klonierungsstellen des Polylinkers eine Nukleinsäuresequenz eines Mycobakteriums, bei welcher man das Vorhandensein einer Nukleinsäuresequenz detektiert, welche ein Polypeptid, welches während der Infektion durch das Mycobakterium exportiert und/oder sekretiert und/oder induziert oder reprimiert werden kann oder ferner auf konstitutive Weise exprimiert oder produziert wird, kodiert, wie auch die damit assoziierten Promotor- und/oder regulatorischen Sequenzen, die in der Lage sind, die Exportierung und/oder die Sekretion des Polypeptids zu erlauben oder zu begünstigen, oder die Gesamtheit oder einen Teil eines Gens, welches das Polypeptid kodiert, umfasst.

9. Rekombinanter Vektor nach einem der Ansprüche 1 bis 8, **dadurch gekennzeichnet, dass** die Nukleinsäuresequenz eines Mycobakteriums, die er enthält, erhalten wird durch physische Fragmentierung oder durch enzymatischen Verdau der genomischen DNA oder der komplementären DNA einer RNA eines Mycobakteriums.

10. Rekombinanter Vektor nach einem der Ansprüche 1 bis 9, **dadurch gekennzeichnet, dass** das Mycobakterium *M. tuberculosis* ist.

11. Rekombinanter Vektor nach einem der Ansprüche 1 bis 9, **dadurch gekennzeichnet, dass** das Mycobakterium unter *M. africanum, M. bovis, M. avium* oder *M. leprae* ausgewählt wird.

12. Verfahren zum Screenen von Nukleotidsequenzen, welche von Mycobakterien stammen, zum Bestimmen des Vorhandenseins von Sequenzen, welche exportierten und/oder sekretierten Polypeptiden, die während der Infektion induziert oder reprimiert werden können, den mit diesen assoziierten Promotor- und/oder regulatorischen Sequenzen, die insbesondere die Exportierung und/oder die Sekretion der Polypeptide von Interesse erlauben oder begünstigen können, oder der Gesamtheit oder von einem Teil von Genen von Interesse, welche diese Polypeptide kodieren, entsprechen, **dadurch gekennzeichnet, dass** es einen Vektor nach einem der Ansprüche 1 bis 11 zum Einsatz bringt.

13. Verfahren zum Screenen nach Anspruch 12, **dadurch gekennzeichnet, dass** es die folgenden Schritte umfasst:
a) die physische Fragmentierung der DNA-Sequenzen von Mycobakterien oder deren Verdau durch wenigstens ein bestimmtes Enzym und die Gewinnung der erhaltenen Fragmente;
b) die Insertion der in Schritt a) erhaltenen Fragmente in eine gegebenenfalls mit dem Enzym des Schritts a) kompatible Klonierungsstelle des Polylinkers eines Vektors nach einem der Ansprüche 1 bis 11;
c) sofern erforderlich, die Amplifizierung der in dem Vektor enthaltenen Fragmente, beispielsweise durch Replikation dieses Letzteren nach Einführen des so modifizierten Vektors in eine bestimmte Zelle, vorzugsweise *E. coli;*
d) die Transformation von Wirtszellen durch den in Schritt c) amplifizierten Vektor oder bei Fehlen einer Amplifizierung durch den Vektor des Schritts b);
e) die Kultivierung von transformierten Wirtszellen in einem Medium, welches den Nachweis des Exportierungs- und/oder Sekretionsmarkers und/oder des Aktivitätsmarkers von Promotoren, die in dem Vektor enthalten sind, erlaubt;
f) den Nachweis der hinsichtlich der Expression des Exportierungs- und/oder Sekretionsmarkers und/oder des Aktivitätsmarkers von Promotoren positiven Wirtszellen (positive Kolonien);
g) Isolierung der DNA der positiven Kolonien und die Einführung dieser DNA in eine Zelle, die mit jener des Schritts c) identisch ist;
h) die Selektion der Insertionen, die in dem Vektor enthalten sind, welche die Gewinnung von hinsichtlich des Exportierungs- und/oder Sekretionsmarkers und/oder hinsichtlich des Aktivitätsmarkers von Promotoren positiven Klonen erlauben;
i) die Isolierung und die Charakterisierung der DNA-Fragmente von Mycobakterien, die in diesen Insertionen enthalten sind, und wobei der Schritt i) des Verfahrens außerdem einen Schritt einer Sequenzierung der ausgewählten Insertionen umfassen kann.

14. Rekombinantes Mycobakterium, **dadurch gekennzeichnet, dass** es durch einen rekombinanten Vektor nach einem der Ansprüche 1 bis 11 transformiert ist.
